# EUROPEAN PATENT APPLICATION

(11) **EP 4 442 686 A1**
(43) Date of publication of application: **09.10.2024**
(21) Application number: 22900230.8
(22) Date of filing: 09.11.2022
(51) Int. Cl.: C07D 471/04, C07D 471/02, A61K 31/4375, A61K 31/517, A61P 35/00

(54) **KRAS INHIBITORS, PREPARATION METHOD THEREFOR, AND PHARMACEUTICAL USE THEREOF**

(30) Priority: 02.12.2021 CN 202111459008; 02.12.2021 CN 202111458180; 29.03.2022 CN 202210320229
(71) Applicant: Abbisko Therapeutics Co., Ltd, Shanghai 201203 (CN)
(72) Inventor: MA, Zhixiong, Shanghai 201203 (CN); XUN, Guoliang, Shanghai 201203 (CN); YU, Hongping, Shanghai 201203 (CN); CHEN, Zhui, Shanghai 201203 (CN); XU, Yaochang, Shanghai 201203 (CN)
(74) Representative: Potter Clarkson
(86) International application number: PCT/CN2022/130784
(87) International publication number: WO 2023/098425

(57) **Abstract**

Disclosed are KRAS inhibitors, a preparation method therefor, and a pharmaceutical use thereof. In particular relating to KRas G12D inhibitors having the structure of formula (I), a preparation method therefor, a pharmaceutical composition containing same, a use of same as an KRas G12D inhibitor, and a use of same in the treatment and/or prevention of cancer or tumors related to KRas G12D. Each substituent of formula (I) has the same definition as in the description.

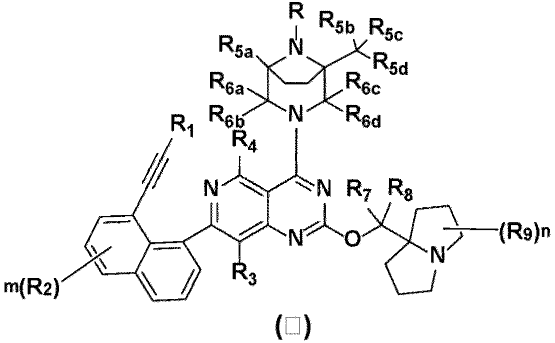

## Description

### TECHNICAL FIELD

The present invention belongs to the field of pharmaceutical synthesis, and particularly relates to a KRAS inhibitor, preparation method therefor, and pharmaceutical use thereof.

### BACKGROUND

The RAS gene family includes HRAS, KRAS and NRAS, which act as oncogenes and are frequently mutated in cancer. Mutated RAS proteins are found in 20-30% of human tumors. Activated RAS proteins contribute to the malignant phenotype of cancer cells, including dysregulation of cell growth and programmed cell death, increased invasiveness and neovascularization. The development of drugs targeting RAS proteins has been slow due to their high affinity for GTP/GDP and lack of a clear binding pocket.

Under normal conditions, the RAS protein acts as a molecular switch, alternating between a GDP-boundinactive state, and a GTP-boundactive state. After stimulation by exogenous growth factors, promoted by guanine nucleotide exchange factors (GEFs), the RAS protein changes from an inactive GDP-bound form, to an activated GTP-bound form, which can bind and activate downstream signaling pathways. Subsequently, the RAS protein reverts to the inactiveGDP-bound form, with the help of its intrinsic GTPase activity and GTPase activating/accelerating protein (GAP).

Missense mutations in codons 12, 13, or 61 lead to abnormal activation of RAS. These mutations prolong the time the RAS protein remains bound to GTP, resulting in sustained activation of downstream signaling pathways. K-RAS is the most common mutation subtype of the RAS family in human cancers, including pancreatic cancer (71%), small intestine cancer (35%), colon cancer (35%), biliary tract cancer (26%), endometrial cancer (17%) and lung cancer (19%). In terms of mutation sites, G12D/G12V/G12C/G13D are the most common mutation types of K-RAS in pancreatic cancer, lung cancer, and colon cancer.

The development of KRAS inhibitors is challenging because the protein lacks a clear pocket. Recent research has uncovered a previously undiscovered pocket in the GDP-bound form of KRAS. Based on these new discoveries, covalently bound inhibitors targeting the mutated cysteine at codon 12 have become a hot spot in the research and development of KRAS inhibitors, which have made certain progress. However, in addition to the G12C mutation, other activating mutations targeting KRAS still need to be solved, especially the KRAS G12D mutation. Therefore, it is necessary to develop safe and effective KRAS G12D inhibitors to treat KRAS-G12D-mediated cancers.

### SUMMARY

The objective of the present invention is to provide a KRas G12D inhibitor, a preparation method therefor and a pharmaceutical use thereof. The series of compounds of the present invention have a strong inhibitory effect on KRas G12D and can be widely used in the preparation of drugs for the treatment and/or prevention of cancers or tumors related to KRas G12D, thereby promising the development of a new generation of KRas G12D inhibitors.

The first aspect of the present invention provides a compound of formula (I), a stereoisomer or pharmaceutically acceptable salt thereof: 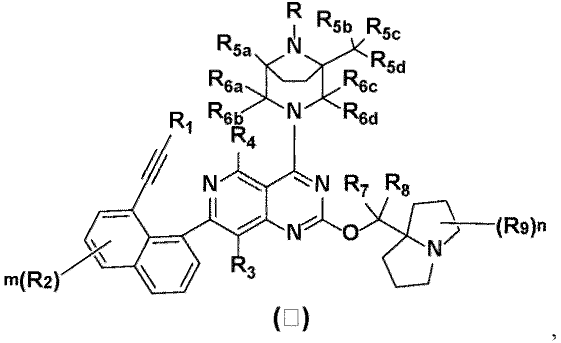
wherein, R is selected from the group consisting of hydrogen, C₁₋₁₀ alkyl, C₂₋₁₀ alkenyl, C₃₋₁₂ cycloalkyl, 3-12 membered heterocyclyl, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-C(O)R₁₅, -C₀₋₈ alkyl-O-C(O)R₁₅, -C₀₋₈ alkyl-NR₁₆R₁₇, -C₀₋₈ alkyl-C(O)NR₁₆R₁₇ and -C₀₋₈ alkyl-N(R₁₆)-C(O)R₁₅, above groups are independently optionally further substituted with one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
R₁ is selected from the group consisting of hydrogen, deuterium, halogen, cyano, C₁₋₄ alkyl, C₃₋₆ cycloalkyl, 3-6 membered heterocyclyl and -C₀₋₈ alkyl-NR₁₆R₁₇, above groups are independently optionally further substituted with one or more substituents selected from the group consisting of deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl and 5-10 membered heteroaryl, above groups are more independently optionally further substituted with one or more substituents selected from the group consisting of deuterium and halogen;
each R₂ is independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, above groups are independently optionally further substituted with one or more substituents selected from the group consisting of deuterium and halogen;
R₃ is selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, above groups are independently optionally further substituted with one or more substituents selected from the group consisting of deuterium and halogen;
R₄ is selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, above groups are independently optionally further substituted with one or more substituents selected from the group consisting of deuterium and halogen;
R₅ₐ is selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, above groups are independently optionally further substituted with one or more substituents selected from the group consisting of deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, above groups are independently optionally further substituted with one or more substituents selected from the group consisting of deuterium and halogen;
R_{5b}, R_{5c} and R_{5d} are each independently selected from the group consisting of hydrogen, deuterium, halogen, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-NH-S(O)₂R₁₃, -C₀₋₈ alkyl-NH-S(O)₂NR₁₆R₁₇, -C₀₋₈ alkyl-S(O)ᵣR₁₃, -C₀₋₈ alkyl-S(O)ᵣNR₁₆R₁₇, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, above groups are independently optionally further substituted with one or more substituents selected from the group consisting of deuterium, halogen, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, above groups are independently optionally further substituted with one or more substituents selected from the group consisting of deuterium and halogen;
or, R_{5b} and R_{5c}, together with the carbon atom directly attached thereto, form a C(O), C₃₋₁₀ cycloalkyl or 3-10 membered heterocyclyl, above groups are independently optionally further substituted with one or more substituents selected from the group consisting of deuterium, halogen, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, above groups are independently optionally further substituted with one or more substituents selected from the group consisting of deuterium and halogen, wherein R_{5d} is defined as above;
or, R_{5b} and R_{5c}, together with the carbon atom directly attached thereto, form 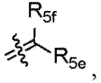 wherein R_{5d} is defined as above;
or, R_{5b}, R_{5c} and R_{5d}, together with the carbon atom directly attached thereto, form 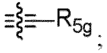
R₅ₑ, R_{5f} and R_{5g} are each independently selected from the group consisting of hydrogen, deuterium, halogen, cyano, C₁₋₄ alkyl, C₃₋₆ cycloalkyl, 3-6 membered heterocyclyl and -C₀₋₈ alkyl-NR₁₆R₁₇, above groups are independently optionally further substituted with one or more substituents selected from the group consisting of deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl and 5-10 membered heteroaryl, above groups are more independently optionally further substituted with one or more substituents selected from the group consisting of deuterium and halogen;
R₆ₐ, R_{6b}, R_{6c} and R_{6d} are each independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, or, R₆ₐ and R_{6b}, R_{6c} and R_{6d}, together with the carbon atom directly attached thereto, form a C₃₋₁₀ cycloalkyl or 3-10 membered heterocyclyl, above groups are independently optionally further substituted with one or more substituents selected from the group consisting of deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, above groups are more independently optionally further substituted with one or more substituents selected from the group consisting of deuterium and halogen;
R₇ and R₈ are each independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, or, R₇ and R₈, together with the carbon atom directly attached thereto, form a C₃₋₁₀ cycloalkyl or 3-10 membered heterocyclyl, above groups are independently optionally further substituted with one or more substituents selected from the group consisting of deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, above groups are more independently optionally further substituted with one or more substituents selected from the group consisting of deuterium and halogen;
each R₉ is independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, or, two adjacent R₉, together with the moiety to which they are directly attached thereto, form a C₃₋₁₀ cycloalkyl or 3-10 membered heterocyclyl, above groups are independently optionally further substituted with one or more substituents selected from the group consisting of deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, above groups are more independently optionally further substituted with one or more substituents selected from the group consisting of deuterium and halogen;
each R₁₀ is independently selected from the group consisting of hydrogen, deuterium, C₁₋₁₀ alkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, -C₀₋₈ alkyl-S(O)ᵣR₁₃, -C₀₋₈ alkyl-C(O)OR₁₄, -C₀₋₈ alkyl-C(O)R₁₅ and -C₀₋₈ alkyl-C(O)NR₁₆R₁₇, above groups are optionally further substituted with one or more substituents selected from the group consisting of deuterium, halogen, cyano, nitro, azido, C₁₋₁₀ alkyl, C₂₋₁₀ alkenyl, C₂₋₁₀ alkynyl, C₁₋₁₀ haloalkyl, C₁₋₁₀ deuterioalkyl, C₃₋₁₀ cycloalkyl, 3-10 membered heterocyclyl, C₆₋₁₀ aryl, 5-10 membered heteroaryl, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)R₁₅, -C₀₋₈ alkyl-O-C(O)R₁₅, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
R₁₁ and R₁₂ are each independently selected from the group consisting of hydrogen, deuterium, hydroxy, C₁₋₁₀ alkyl, C₂₋₁₀ alkenyl, C₂₋₁₀ alkynyl, C₃₋₁₀ cycloalkyl, 3-10 membered heterocyclyl, C₆₋₁₀ aryl and 5-10 membered heteroaryl, or, R₁₁ and R₁₂, together with the sulfur atom directly attached thereto, form a 3-10 membered heterocyclyl, above groups are optionally further substituted with one or more substituents selected from the group consisting of deuterium, halogen, cyano, nitro, azido, C₁₋₁₀ alkyl, C₂₋₁₀ alkenyl, C₂₋₁₀ alkynyl, C₁₋₁₀ haloalkyl, C₁₋₁₀ deuterioalkyl, C₃₋₁₀ cycloalkyl, 3-10 membered heterocyclyl, C₆₋₁₀ aryl, 5-10 membered heteroaryl, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)R₁₅, -C₀₋₈ alkyl-O-C(O)R₁₅, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
each R₁₃ is independently selected from the group consisting of hydrogen, deuterium, hydroxy, C₁₋₁₀ alkyl, C₂₋₁₀ alkenyl, C₃₋₁₂ cycloalkyl, 3-12 membered heterocyclyl, C₆₋₁₀ aryl, 5-10 membered heteroaryl and -NR₁₆R₁₇, above groups are independently optionally further substituted with one or more substituents selected from the group consisting of deuterium, halogen, hydroxy, =O, C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₁₆R₁₇;
each R₁₄ is independently selected from the group consisting of hydrogen, deuterium, C₁₋₁₀ alkyl, C₂₋₁₀ alkenyl, C₃₋₁₂ cycloalkyl, 3-12 membered heterocyclyl, C₆₋₁₀ aryl and 5-10 membered heteroaryl, above groups are independently optionally further substituted with one or more substituents selected from the group consisting of deuterium, halogen, hydroxy, =O, cyano, C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₁₆R₁₇;
each R₁₅ is independently selected from the group consisting of hydrogen, deuterium, hydroxy, C₁₋₁₀ alkyl, C₁₋₁₀ alkoxy, C₂₋₁₀ alkenyl, C₂₋₁₀ alkynyl, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₁₆R₁₇, above groups are independently optionally further substituted with one or more substituents selected from the group consisting of deuterium, halogen, hydroxy, =O, cyano, C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₁₆R₁₇;
R₁₆ and R₁₇ are each independently selected from the group consisting of hydrogen, deuterium, hydroxy, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, sulfinyl, sulfonyl, methanesulfonyl, isopropylsulfonyl, cyclopropylsulfonyl, p-toluenesulfonyl, aminosulfonyl, dimethylaminosulfonyl and C₁₋₁₀ alkanoyl, above groups are independently optionally further substituted with one or more substituents selected from the group consisting of deuterium, halogen, hydroxy, =O, C₁₋₁₀ alkyl, C₂₋₁₀ alkenyl, C₂₋₁₀ alkynyl, C₁₋₁₀ haloalkyl, C₁₋₁₀ deuterioalkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy, amino, monoC₁₋₁₀ alkylamino, diC₁₋₁₀ alkylamino and C₁₋₁₀ alkanoyl;
or, R₁₆ and R₁₇, together with the nitrogen atom directly attached thereto, form a 4-10 membered heterocyclyl or 5-10 membered heteroaryl, the 4-10 membered heterocyclyl or 5-10 membered heteroaryl is optionally further substituted with one or more substituents selected from the group consisting of deuterium, halogen, hydroxy, =O, C₁₋₁₀ alkyl, C₂₋₁₀ alkenyl, C₂₋₁₀ alkynyl, C₁₋₁₀ haloalkyl, C₁₋₁₀ deuterioalkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy, amino, monoC₁₋₁₀ alkylamino, diC₁₋₁₀ alkylamino and C₁₋₁₀ alkanoyl;
m is 0, 1, 2, 3, 4, 5 or 6;
n is 0, 1, 2, 3, 4, 5 or 6; and
each r is independently 0, 1 or 2.

As a preferred embodiment, in the compound of formula (I), the stereoisomer or pharmaceutically acceptable salt thereof, R is selected from the group consisting of hydrogen, C₁₋₄ alkyl, C₂₋₄ alkenyl, C₃₋₆ cycloalkyl, 3-6 membered heterocyclyl, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-C(O)R₁₅, -C₀₋₄ alkyl-O-C(O)R₁₅, -C₀₋₄ alkyl-NR₁₆R₁₇, -C₀₋₄ alkyl-C(O)NR₁₆R₁₇ and -C₀₋₄ alkyl-N(R₁₆)-C(O)R₁₅, above groups are independently optionally further substituted with one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
R₁ is selected from the group consisting of hydrogen, deuterium, halogen, cyano, C₁₋₄ alkyl, C₃₋₆ cycloalkyl, 3-6 membered heterocyclyl and -C₀₋₄ alkyl-NR₁₆R₁₇, above groups are independently optionally further substituted with one or more substituents selected from the group consisting of deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₆₋₈ aryl and 5-8 membered heteroaryl, above groups are more independently optionally further substituted with one or more substituents selected from the group consisting of deuterium and halogen;
each R₂ is independently selected from the group consisting of hydrogen, deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, above groups are independently optionally further substituted with one or more substituents selected from the group consisting of deuterium and halogen;
R₃ is selected from the group consisting of hydrogen, deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, above groups are independently optionally further substituted with one or more substituents selected from the group consisting of deuterium and halogen;
R₄ is selected from the group consisting of hydrogen, deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, above groups are independently optionally further substituted with one or more substituents selected from the group consisting of deuterium and halogen;
R₅ₐ is selected from the group consisting of hydrogen, deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, above groups are independently optionally further substituted with one or more substituents selected from the group consisting of deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, above groups are more independently optionally further substituted with one or more substituents selected from the group consisting of deuterium and halogen;
R_{5b}, R_{5c} and R_{5d} are each independently selected from the group consisting of hydrogen, deuterium, halogen, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-NH-S(O)₂R₁₃, -C₀₋₄ alkyl-NH-S(O)₂NR₁₆R₁₇, -C₀₋₄ alkyl-S(O)ᵣR₁₃, -C₀₋₄ alkyl-S(O)ᵣNR₁₆R₁₇, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, above groups are independently optionally further substituted with one or more substituents selected from the group consisting of deuterium, halogen, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, above groups are independently optionally further substituted with one or more substituents selected from the group consisting of deuterium and halogen;
or, R_{5b} and R_{5c}, together with the carbon atom directly attached thereto, form a C(O), C₃₋₆ cycloalkyl or 3-6 membered heterocyclyl, above groups are independently optionally further substituted with one or more substituents selected from the group consisting of deuterium, halogen, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, above groups are independently optionally further substituted with one or more substituents selected from the group consisting of deuterium and halogen, wherein R_{5d} is defined as above;
or, R_{5b} and R_{5c}, together with the carbon atom directly attached thereto, form 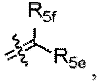 wherein R_{5d} is defined as above;
or, R_{5b}, R_{5c} and R_{5d}, together with the carbon atom directly attached thereto, form 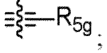
R₅ₑ, R_{5f} and R_{5g} are each independently selected from the group consisting of hydrogen, deuterium, halogen, cyano, C₁₋₄ alkyl, C₃₋₆ cycloalkyl, 3-6 membered heterocyclyl and -NR₁₆R₁₇, above groups are independently optionally further substituted with one or more substituents selected from the group consisting of deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl and 5-8 membered heteroaryl, above groups are more independently optionally further substituted with one or more substituents selected from the group consisting of deuterium and halogen;
R₆ₐ, R_{6b}, R_{6c} and R_{6d} are each independently selected from the group consisting of hydrogen, deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, or, R₆ₐ and R_{6b}, R_{6c} and R_{6d}, together with the carbon atom directly attached thereto, form a C₃₋₁₀ cycloalkyl or 3-10 membered heterocyclyl, above groups are independently optionally further substituted with one or more substituents selected from the group consisting of deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, above groups are more independently optionally further substituted with one or more substituents selected from the group consisting of deuterium and halogen;
R₇ and R₈ are each independently selected from the group consisting of hydrogen, deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, or, R₇ and R₈, together with the carbon atom directly attached thereto, form a C₃₋₁₀ cycloalkyl or 3-10 membered heterocyclyl, above groups are independently optionally further substituted with one or more substituents selected from the group consisting of deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, above groups are more independently optionally further substituted with one or more substituents selected from the group consisting of deuterium and halogen;
each R₉ is independently selected from the group consisting of hydrogen, deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, or, two adjacent R₉, together with the moiety to which they are directly attached thereto, form a C₃₋₁₀ cycloalkyl or 3-10 membered heterocyclyl, above groups are independently optionally further substituted with one or more substituents selected from the group consisting of deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, above groups are more independently optionally further substituted with one or more substituents selected from the group consisting of deuterium and halogen;
wherein, R₁₀, R₁₁, R₁₂, R₁₃, R₁₄, R₁₅, R₁₆, R₁₇, and r are defined as in the compound of formula (I).

As a preferred embodiment, in the compound of formula (I), the stereoisomer or pharmaceutically acceptable salt thereof, each R₁₀ is independently selected from the group consisting of hydrogen, deuterium, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-S(O)ᵣR₁₃, -C₀₋₄ alkyl-C(O)OR₁₄, -C₀₋₄ alkyl-C(O)R₁₅ and -C₀₋₄ alkyl-C(O)NR₁₆R₁₇, above groups are optionally further substituted with one or more substituents selected from the group consisting of deuterium, halogen, cyano, nitro, azido, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, C₆₋₈ aryl, 5-8 membered heteroaryl, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)R₁₅, -C₀₋₄ alkyl-O-C(O)R₁₅, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
each R₁₁ and each R₁₂ are independently selected from the group consisting of hydrogen, deuterium, hydroxy, C₁₋₄ alkyl, C₂₋₄ alkenyl, C₂₋₄ alkynyl, C₃₋₆ cycloalkyl, 3-6 membered heterocyclyl, C₆₋₈ aryl and 5-8 membered heteroaryl, or, R₁₁ and R₁₂, together with the sulfur atom directly attached thereto, form a 3-6 membered heterocyclyl, above groups are optionally further substituted with one or more substituents selected from the group consisting of deuterium, halogen, cyano, nitro, azido, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, C₆₋₈ aryl, 5-8 membered heteroaryl, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)R₁₅, -C₀₋₄ alkyl-O-C(O)R₁₅, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
each R₁₃ is independently selected from the group consisting of hydrogen, deuterium, hydroxy, C₁₋₄ alkyl, C₂₋₄ alkenyl, C₃₋₆ cycloalkyl, 3-6 membered heterocyclyl, C₆₋₈ aryl, 5-8 membered heteroaryl and -NR₁₆R₁₇, above groups are independently optionally further substituted with one or more substituents selected from the group consisting of deuterium, halogen, hydroxy, =O, C₁₋₄ alkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₁₆R₁₇;
each R₁₄ is independently selected from the group consisting of hydrogen, deuterium, C₁₋₄ alkyl, C₂₋₄ alkenyl, C₃₋₆ cycloalkyl, 3-6 membered heterocyclyl, C₆₋₈ aryl and 5-8 membered heteroaryl, above groups are independently optionally further substituted with one or more substituents selected from the group consisting of deuterium, halogen, hydroxy, =O, cyano, C₁₋₄ alkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₁₆R₁₇;
each R₁₅ is independently selected from the group consisting of hydrogen, deuterium, hydroxy, C₁₋₄ alkyl, C₁₋₄ alkoxy, C₂₋₄ alkenyl, C₂₋₄ alkynyl, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₁₆R₁₇, above groups are independently optionally further substituted with one or more substituents selected from the group consisting of deuterium, halogen, hydroxy, =O, cyano, C₁₋₄ alkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₁₆R₁₇;
R₁₆ and R₁₇ are each independently selected from the group consisting of hydrogen, deuterium, hydroxy, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, sulfinyl, sulfonyl, methanesulfonyl, isopropylsulfonyl, cyclopropylsulfonyl, p-toluenesulfonyl, aminosulfonyl, dimethylaminosulfonyl and C₁₋₄ alkanoyl, above groups are independently optionally further substituted with one or more substituents selected from the group consisting of deuterium, halogen, hydroxy, =O, C₁₋₄ alkyl, C₂₋₄ alkenyl, C₂₋₄ alkynyl, C₁₋₄ haloalkyl, C₁₋₄ deuterioalkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy, amino, monoC₁₋₄ alkylamino, diC₁₋₄ alkylamino and C₁₋₄ alkanoyl;
or, R₁₆ and R₁₇, together with the nitrogen atom directly attached thereto, form a 4-8 membered heterocyclyl or 5-8 membered heteroaryl, the 4-8 membered heterocyclyl or 5-8 membered heteroaryl is optionally further substituted with one or more substituents selected from the group consisting of deuterium, halogen, hydroxy, =O, C₁₋₄ alkyl, C₂₋₄ alkenyl, C₂₋₄ alkynyl, C₁₋₄ haloalkyl, C₁₋₄ deuterioalkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy, amino, monoC₁₋₄ alkylamino, diC₁₋₄ alkylamino and C₁₋₄ alkanoyl.

As a preferred embodiment, in the compound of formula (I), the stereoisomer or pharmaceutically acceptable salt thereof, the compound of formula (I) is a compound of formula (II) as shown below: 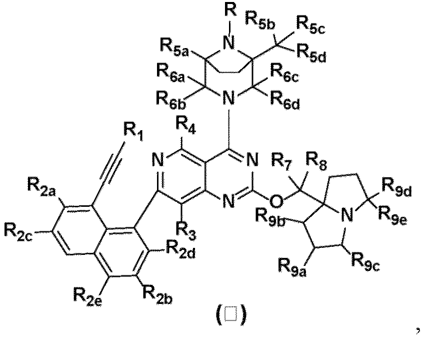
wherein, R is selected from the group consisting of hydrogen, C₁₋₄ alkyl, C₂₋₄ alkenyl, C₃₋₆ cycloalkyl, 3-6 membered heterocyclyl, -S(O)ᵣR₁₃, -O-R₁₄, -C(O)OR₁₄, -C(O)SR₁₄, -C(O)R₁₅, -O-C(O)R₁₅, -NR₁₆R₁₇, -C(O)NR₁₆R₁₇ and -N(R₁₆)-C(O)R₁₅, above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
R₁ is selected from the group consisting of hydrogen, deuterium, halogen, cyano, C₁₋₄ alkyl, C₃₋₆ cycloalkyl, 3-6 membered heterocyclyl and -C₀₋₄ alkyl-NR₁₆R₁₇, above groups are independently optionally further substituted with one or more substituents selected from the group consisting of deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl and 5-8 membered heteroaryl, above groups are more independently optionally further substituted with one or more substituents selected from the group consisting of deuterium and halogen;
R₂ₐ is selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, above groups are independently optionally further substituted with one or more substituents selected from the group consisting of deuterium and halogen;
R_{2b} is selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, above groups are independently optionally further substituted with one or more substituents selected from the group consisting of deuterium and halogen;
R_{2c} is selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, above groups are independently optionally further substituted with one or more substituents selected from the group consisting of deuterium and halogen;
R_{2d} is selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, above groups are independently optionally further substituted with one or more substituents selected from the group consisting of deuterium and halogen;
R₂ₑ is selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, above groups are independently optionally further substituted with one or more substituents selected from the group consisting of deuterium and halogen;
R₃ is selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, above groups are independently optionally further substituted with one or more substituents selected from the group consisting of deuterium and halogen;
R₄ is selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, above groups are independently optionally further substituted with one or more substituents selected from the group consisting of deuterium and halogen;
R₅ₐ is selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, above groups are independently optionally further substituted with one or more substituents selected from the group consisting of deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, above groups are more independently optionally further substituted with one or more substituents selected from the group consisting of deuterium and halogen;
R_{5b}, R_{5c} and R_{5d} are each independently selected from the group consisting of hydrogen, deuterium, halogen, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-NH-S(O)₂R₁₃, -C₀₋₄ alkyl-NH-S(O)₂NR₁₆R₁₇, -C₀₋₄ alkyl-S(O)ᵣR₁₃, -C₀₋₄ alkyl-S(O)ᵣNR₁₆R₁₇, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, above groups are independently optionally further substituted with one or more substituents selected from the group consisting of deuterium, halogen, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, above groups are independently optionally further substituted with one or more substituents selected from the group consisting of deuterium and halogen;
or, R_{5b} and R_{5c}, together with the carbon atom directly attached thereto, form a C(O), C₃₋₆ cycloalkyl or 3-6 membered heterocyclyl, above groups are independently optionally further substituted with one or more substituents selected from the group consisting of deuterium, halogen, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, above groups are independently optionally further substituted with one or more substituents selected from the group consisting of deuterium and halogen, wherein R_{5d} is defined as above;
or, R_{5b} and R_{5c}, together with the carbon atom directly attached thereto, form 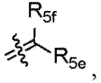 wherein R_{5d} is defined as above; or, R_{5b}, R_{5c} and R_{5d}, together with the carbon atom directly attached thereto, form 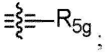
R₅ₑ, R_{5f} and R_{5g} are each independently selected from the group consisting of hydrogen, deuterium, halogen, C₁₋₄ alkyl, C₃₋₆ cycloalkyl, 3-6 membered heterocyclyl and -NR₁₆R₁₇, above groups are independently optionally further substituted with one or more substituents selected from the group consisting of deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl and 5-8 membered heteroaryl, above groups are more independently optionally further substituted with one or more substituents selected from the group consisting of deuterium and halogen;
R₆ₐ, R_{6b}, R_{6c} and R_{6d} are each independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, or, R₆ₐ and R_{6b}, R_{6c} and R_{6d}, together with the carbon atom directly attached thereto, form a C₃₋₆ cycloalkyl and 3-6 membered heterocyclyl, above groups are independently optionally further substituted with one or more substituents selected from the group consisting of deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, above groups are more independently optionally further substituted with one or more substituents selected from the group consisting of deuterium and halogen;
R₇ and R₈ are each independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, or, R₇ and R₈, together with the carbon atom directly attached thereto, form a C₃₋₆ cycloalkyl and 3-6 membered heterocyclyl, above groups are independently optionally further substituted with one or more substituents selected from the group consisting of deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, above groups are more independently optionally further substituted with one or more substituents selected from the group consisting of deuterium and halogen;
R₉ₐ, R_{9b}, R_{9c}, R_{9d} and R₉ₑ are each independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, or, R_{9d} and R₉ₑ, together with the carbon atom directly attached thereto, form a C₃₋₆ cycloalkyl and 3-6 membered heterocyclyl, and the other three are defined as above, the C₃₋₆ cycloalkyl and 3-6 membered heterocyclyl are independently optionally further substituted with one or more substituents selected from the group consisting of deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, above groups are more independently optionally further substituted with one or more substituents selected from the group consisting of deuterium and halogen;
wherein, R₁₀, R₁₁, R₁₂, R₁₃, R₁₄, R₁₅, R₁₆, R₁₇, and r are defined as in the compound of formula (I).

As a preferred embodiment, in the compound of formula (I), the stereoisomer or pharmaceutically acceptable salt thereof, the compound of formula (I) is a compound of formula (III) as shown below: 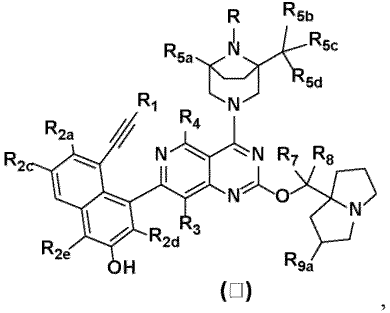
wherein, R is selected from the group consisting of hydrogen, C₁₋₄ alkyl, C₂₋₄ alkenyl, C₃₋₆ cycloalkyl, 3-6 membered heterocyclyl, -S(O)ᵣR₁₃, -O-R₁₄, -C(O)OR₁₄, -C(O)SR₁₄, -C(O)R₁₅, -O-C(O)R₁₅, -NR₁₆R₁₇, -C(O)NR₁₆R₁₇ and -N(R₁₆)-C(O)R₁₅, above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
R₁ is selected from the group consisting of hydrogen, deuterium, halogen, cyano, C₁₋₄ alkyl, C₁₋₄ haloalkyl, C₁₋₄ deuterioalkyl, C₃₋₆ cycloalkyl and 6 membered heterocyclyl;
R₂ₐ is selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, -O-R₁₄ and -S-R₁₃;
R_{2c} is selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, -O-R₁₄ and -S-R₁₃;
R_{2d} is selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, -O-R₁₄ and -S-R₁₃;
R₂ₑ is selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, -O-R₁₄ and -S-R₁₃;
R₃ is selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, -O-R₁₄ and -S-R₁₃;
R₄ is selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, -O-R₁₄, -S-R₁₃ and -NR₁₆R₁₇;
R₅ₐ is selected from the group consisting of hydrogen, deuterium, halogen, cyano, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl and -C₀₋₄ alkyl-O-R₁₄;
R_{5b} and R_{5c} are each independently selected from the group consisting of hydrogen, deuterium, halogen, C₁₋₄ alkyl, C₂₋₄ alkenyl, C₂₋₄ alkynyl, C₃₋₆ cycloalkyl, 3-6 membered heterocyclyl and -O-R₁₄, above groups are independently optionally further substituted with one or more substituents selected from the group consisting of deuterium, halogen, C₁₋₄ alkyl, C₂₋₄ alkenyl, C₂₋₄ alkynyl, C₃₋₆ cycloalkyl, 3-6 membered heterocyclyl, =O, =S, -SF₅, -S(O)ᵣR₁₃ and -O-R₁₄;
R_{5d} is selected from the group consisting of hydrogen, deuterium, halogen, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-NH-S(O)₂R₁₃, -C₀₋₄ alkyl-NH-S(O)₂NR₁₆R₁₇, -C₀₋₄ alkyl-S(O)ᵣR₁₃, -C₀₋₄ alkyl-S(O)ᵣNR₁₆R₁₇, -C₀₋₄ alkyl-O-R₁₄, -C₀₋₄ alkyl-C(O)OR₁₄, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(O)NR₁₆R₁₇ and -C₀₋₄ alkyl-N(R₁₆)-C(O)R₁₅, above groups are independently optionally further substituted with one or more substituents selected from the group consisting of deuterium, halogen, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)ᵣR₁₃, -O-R₁₄, -C(O)OR₁₄, -C(O)R₁₅, -O-C(O)R₁₅, -P(O)(R₁₅)₂, -NR₁₆R₁₇, -C(O)NR₁₆R₁₇ and -N(R₁₆)-C(O)R₁₅, above groups are independently optionally further substituted with one or more substituents selected from the group consisting of deuterium and halogen;
or, R_{5b} and R_{5c}, together with the carbon atom directly attached thereto, form a C(O), C₃₋₆ cycloalkyl or 3-6 membered heterocyclyl, above groups are independently optionally further substituted with one or more substituents selected from the group consisting of deuterium, halogen, C₁₋₄ alkyl, C₂₋₄ alkenyl, C₂₋₄ alkynyl, C₃₋₆ cycloalkyl, 3-6 membered heterocyclyl, =O, =S, -SF₅, -S(O)ᵣR₁₃ and -O-R₁₄, above groups are independently optionally further substituted with one or more substituents selected from the group consisting of deuterium and halogen, and R_{5d} is selected from the group consisting of hydrogen, deuterium, halogen, C₁₋₄ alkyl, C₂₋₄ alkenyl, C₂₋₄ alkynyl, C₃₋₆ cycloalkyl, 3-6 membered heterocyclyl and -O-R₁₄, above groups are independently optionally further substituted with one or more substituents selected from the group consisting of deuterium and halogen;
or, R_{5b} and R_{5c}, together with the carbon atom directly attached thereto, form 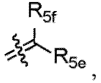 and R_{5d} is selected from the group consisting of hydrogen, deuterium, halogen, C₁₋₄ alkyl, C₂₋₄ alkenyl, C₂₋₄ alkynyl, C₃₋₆ cycloalkyl, 3-6 membered heterocyclyl and -O-R₁₄, above groups are independently optionally further substituted with one or more substituents selected from the group consisting of deuterium and halogen;
or, R_{5b}, R_{5c} and R_{5d}, together with the carbon atom directly attached thereto, form 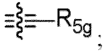
R₅ₑ, R_{5f} and R_{5g} are each independently selected from the group consisting of hydrogen, deuterium, halogen and C₁₋₄ alkyl, above groups are independently optionally further substituted with one or more substituents selected from the group consisting of deuterium, halogen, cyano, C₁₋₄ alkyl, C₃₋₆ cycloalkyl, 3-6 membered heterocyclyl, C₆₋₈ aryl and 5-8 membered heteroaryl, above groups are more independently optionally further substituted with one or more substituents selected from the group consisting of deuterium and halogen;
R₇ and R₈ are each independently selected from the group consisting of hydrogen, deuterium, halogen, cyano, C₁₋₄ alkyl, C₂₋₄ alkenyl, C₂₋₄ alkynyl, C₃₋₆ cycloalkyl and 3-6 membered heterocyclyl, or, R₇ and R₈, together with the carbon atom directly attached thereto, form a C₃₋₆ cycloalkyl and 3-6 membered heterocyclyl, above groups are independently optionally further substituted with one or more substituents selected from the group consisting of deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
R₉ₐ is selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl and 3-6 membered heterocyclyl;
wherein, R₁₀, R₁₁, R₁₂, R₁₃, R₁₄, R₁₅, R₁₆, R₁₇, and r are defined as in the compound of formula (I).

As a further preferred embodiment, in the compound of formula (I), the stereoisomer or pharmaceutically acceptable salt thereof, R_{5b} and R_{5c} are each independently selected from the group consisting of hydrogen, deuterium, fluorine, chlorine, bromine, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl, methoxy, ethoxy, propoxy, isopropoxy, trifluoromethoxy, difluoromethoxy, monofluoromethoxy, trideuteriomethoxy, dideuteriomethoxy, trifluoroethoxy, trideuterioethoxy, cyclopropoxy and cyclobutoxy;
R_{5d} is selected from the group consisting of hydrogen, deuterium, fluorine, chlorine, bromine, C₁₋₄ alkyl, C₂₋₄ alkenyl, C₂₋₄ alkynyl, C₃₋₆ cycloalkyl, 3-6 membered heterocyclyl, 5-8 membered heteroaryl, -NH-S(O)₂R₁₃, -NH-S(O)₂NR₁₆R₁₇, -S(O)ᵣR₁₃, -S(O)ᵣNR₁₆R₁₇, -O-R₁₄, -C(O)OR₁₄, -C(O)R₁₅, -P(O)(R₁₅)₂, -NR₁₆R₁₇, -C(O)NR₁₆R₁₇ and -N(R₁₆)-C(O)R₁₅, above groups are independently optionally further substituted with one or more substituents selected from the group consisting of deuterium, fluorine, chlorine, bromine, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -S(O)ᵣR₁₃, -O-R₁₄, -C(O)OR₁₄, -C(O)R₁₅ and -C(O)NR₁₆R₁₇, above groups are independently optionally further substituted with one or more substituents selected from the group consisting of deuterium and halogen;
or, R_{5b} and R_{5c}, together with the carbon atom directly attached thereto, form a C(O), C₃₋₆ cycloalkyl or 3-6 membered heterocyclyl, above groups are independently optionally further substituted with one or more substituents selected from the group consisting of deuterium, fluorine, chlorine, bromine, C₁₋₄ alkyl, C₂₋₄ alkenyl, C₂₋₄ alkynyl, C₃₋₆ cycloalkyl, =O, =S, methylthio, ethylthio, methoxy, ethoxy and isopropoxy, above groups are independently optionally further substituted with one or more substituents selected from the group consisting of deuterium and halogen, and R_{5d} is selected from the group consisting of hydrogen, deuterium, fluorine, chlorine, bromine, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl, methoxy, ethoxy and isopropoxyl;
or, R_{5b} and R_{5c}, together with the carbon atom directly attached thereto, form 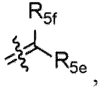 and R_{5d} is selected from the group consisting of hydrogen, deuterium, fluorine, chlorine, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl and azacyclobutyl;
or, R_{5b}, R_{5c} and R_{5d}, together with the carbon atom directly attached thereto, form 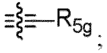
R₅ₑ, R_{5f} and R_{5g} are each independently selected from the group consisting of hydrogen, deuterium, fluorine, chlorine, methyl, ethyl, propyl, isopropyl, trifluoromethyl, difluoromethyl, monofluoromethyl, trideuteriomethyl, dideuteriomethyl and monodeuteriomethyl;
wherein, R₁₃, R₁₄, R₁₅, R₁₆, R₁₇ and r are defined as in the compound of formula (III).

As a further preferred embodiment, in the compound of formula (I), the stereoisomer or pharmaceutically acceptable salt thereof, R₅ₐ is selected from the group consisting of hydrogen, deuterium, fluorine, chlorine, bromine, cyano, hydroxy, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl, methoxy, ethoxy, propoxy, isopropoxy, trifluoromethoxy, difluoromethoxy, trideuteriomethoxy, dideuteriomethoxy, trifluoroethoxy, trideuterioethoxy, cyclopropoxy and cyclobutoxy.

As a further preferred embodiment, in the compound of formula (I), the stereoisomer or pharmaceutically acceptable salt thereof, R is selected from the group consisting of hydrogen, methyl, ethyl, propyl, isopropyl, cyclopropyl, cyclobutyl, oxacyclobutyl, azacyclobutyl and -C(O)OR₁₄, above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
wherein, R₁₀, R₁₁, R₁₂, R₁₃, R₁₄, R₁₅, R₁₆, R₁₇ and r are defined as in the compound of formula (III).

As a further preferred embodiment, in the compound of formula (I), the stereoisomer or pharmaceutically acceptable salt thereof, R₁ is selected from the group consisting of hydrogen, deuterium, fluorine and methyl;
R₂ₐ is selected from the group consisting of hydrogen, deuterium, fluorine, chlorine, bromine, cyano, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl, methoxy, ethoxy, propoxy, isopropoxy, trifluoromethoxy, difluoromethoxy, trideuteriomethoxy, dideuteriomethoxy, trifluoroethoxy, trideuterioethoxy, cyclopropoxy, cyclobutoxy, methylthio and ethylthio;
R_{2c} is selected from the group consisting of hydrogen, deuterium, fluorine, chlorine, bromine, cyano, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl, methoxy, ethoxy, propoxy, isopropoxy, trifluoromethoxy, difluoromethoxy, trideuteriomethoxy, dideuteriomethoxy, trifluoroethoxy, trideuterioethoxy, cyclopropoxy, cyclobutoxy, methylthio and ethylthio;
R_{2d} is selected from the group consisting of hydrogen, deuterium, fluorine, chlorine, bromine, cyano, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl, methoxy, ethoxy, propoxy, isopropoxy, trifluoromethoxy, difluoromethoxy, trideuteriomethoxy, dideuteriomethoxy, trifluoroethoxy, trideuterioethoxy, cyclopropoxy, cyclobutoxy, methylthio and ethylthio;
R₂ₑ is selected from the group consisting of hydrogen, deuterium, fluorine, chlorine, bromine, cyano, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl, methoxy, ethoxy, propoxy, isopropoxy, trifluoromethoxy, difluoromethoxy, trideuteriomethoxy, dideuteriomethoxy, trifluoroethoxy, trideuterioethoxy, cyclopropoxy, cyclobutoxy, methylthio and ethylthio;
R₃ is selected from the group consisting of hydrogen, deuterium, fluorine, chlorine, bromine, cyano, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl, methoxy, ethoxy, propoxy, isopropoxy, trifluoromethoxy, difluoromethoxy, trideuteriomethoxy, dideuteriomethoxy, trifluoroethoxy, trideuterioethoxy, cyclopropoxy, cyclobutoxy, methylthio and ethylthio.

As a further preferred embodiment, in the compound of formula (I), the stereoisomer or pharmaceutically acceptable salt thereof, R₄ is selected from the group consisting of hydrogen, deuterium, fluorine, chlorine, bromine, cyano, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl, hydroxy, methoxy, ethoxy, propoxy, isopropoxy, trifluoromethoxy, difluoromethoxy, monofluoromethoxy, trideuteriomethoxy, dideuteriomethoxy, trifluoroethoxy, trideuterioethoxy, cyclopropoxy, cyclobutoxy, methylthio, ethylthio, amino, monomethylamino, monoethylamino and dimethylamino.

As a further preferred embodiment, in the compound of formula (I), the stereoisomer or pharmaceutically acceptable salt thereof, R₇ and R₈ are each independently selected from the group consisting of hydrogen, deuterium, fluorine, chlorine, bromine, cyano, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl and azacyclobutyl;
R₉ₐ is selected from the group consisting of hydrogen, deuterium, fluorine, chlorine, bromine, cyano, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl and azacyclobutyl.

As a preferred embodiment, in the compound of formula (I), the stereoisomer or pharmaceutically acceptable salt thereof, the compound of formula (I) is a compound of formula (IV-a) as shown below: 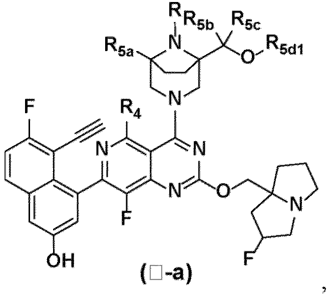
wherein, R is selected from the group consisting of hydrogen, C₁₋₄ alkyl, C₂₋₄ alkenyl, C₃₋₆ cycloalkyl, 3-6 membered heterocyclyl, -S(O)ᵣR₁₃, -O-R₁₄, -C(O)OR₁₄, -C(O)SR₁₄, -C(O)R₁₅, -O-C(O)R₁₅, -NR₁₆R₁₇, -C(O)NR₁₆R₁₇ and -N(R₁₆)-C(O)R₁₅, above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(O)NR₁₆R₁₇ and -N(R₁₆)-C(O)R₁₅;
R₄ is selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, -O-R₁₄, -S-R₁₃ and -NR₁₆R₁₇;
R₅ₐ is selected from the group consisting of hydrogen, deuterium, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl and -C₀₋₄ alkyl-O-R₁₄;
R_{5b} and R_{5c} are each independently selected from the group consisting of hydrogen, deuterium, halogen and C₁₋₄ alkyl, above groups are independently optionally further substituted with one or more substituents selected from the group consisting of deuterium, halogen, C₁₋₄ alkyl, C₃₋₆ cycloalkyl, 3-6 membered heterocyclyl and -O-R₁₄;
R_{5d1} is selected from the group consisting of hydrogen, deuterium, C₁₋₄ alkyl, C₃₋₆ cycloalkyl or 3-6 membered heterocyclyl, above groups are independently optionally further substituted with one or more substituents selected from the group consisting of deuterium, halogen, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)ᵣR₁₃, -O-R₁₄, -C(O)OR₁₄, -C(O)R₁₅, -O-C(O)R₁₅, -P(O)(R₁₅)₂, -NR₁₆R₁₇, -C(O)NR₁₆R₁₇ and -N(R₁₆)-C(O)R₁₅, above groups are independently optionally further substituted with one or more substituents selected from the group consisting of deuterium and halogen;
or, R_{5b} and R_{5c}, together with the carbon atom directly attached thereto, form a C₃₋₆ cycloalkyl or 3-6 membered heterocyclyl, above groups are independently optionally further substituted with one or more substituents selected from the group consisting of deuterium, halogen, C₁₋₄ alkyl, C₂₋₄ alkenyl, C₂₋₄ alkynyl, C₃₋₆ cycloalkyl, 3-6 membered heterocyclyl and -O-R₁₄, above groups are independently optionally further substituted with one or more substituents selected from the group consisting of deuterium and halogen, and R_{5d1} is selected from the group consisting of hydrogen, deuterium, C₁₋₄ alkyl, C₃₋₆ cycloalkyl and 3-6 membered heterocyclyl, above groups are independently optionally further substituted with one or more substituents selected from the group consisting of deuterium and halogen;
wherein, R₁₃, R₁₄, R₁₅, R₁₆, R₁₇ and r are defined as in the compound of formula (I).

As a further preferred embodiment, in the compound of formula (I), the stereoisomer or pharmaceutically acceptable salt thereof, R is selected from the group consisting of hydrogen, methyl, ethyl, propyl, isopropyl, cyclopropyl, cyclobutyl, oxacyclobutyl, azacyclobutyl and -C(O)OR₁₄, above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
wherein, R₁₀, R₁₁, R₁₂, R₁₃, R₁₄, R₁₅, R₁₆, R₁₇ and r are defined as in the compound of formula (IV-a).

As a more further preferred embodiment, in the compound of formula (I), the stereoisomer or pharmaceutically acceptable salt thereof, R is selected from the group consisting of hydrogen, methyl, ethyl, propyl, isopropyl, cyclopropyl, cyclobutyl, oxacyclobutyl, azacyclobutyl and -C(O)OR₁₄, above groups are independently optionally further substituted with one or more substituents selected from the group consisting of deuterium, fluorine, chlorine, cyano, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl and -O-C(O)R₁₅;
wherein, R₁₄ and R₁₅ are defined as in the compound of formula (IV-a).

As a further preferred embodiment, in the compound of formula (I), the stereoisomer or pharmaceutically acceptable salt thereof, R₄ is selected from the group consisting of hydrogen, deuterium, fluorine, chlorine, bromine, cyano, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl, hydroxy, methoxy, ethoxy, propoxy, isopropoxy, trifluoromethoxy, difluoromethoxy, monofluoromethoxy, trideuteriomethoxy, dideuteriomethoxy, trifluoroethoxy, trideuterioethoxy, cyclopropoxy, cyclobutoxy, methylthio, ethylthio, amino, monomethylamino, monoethylamino and dimethylamino.

As a more further preferred embodiment, in the compound of formula (I), the stereoisomer or pharmaceutically acceptable salt thereof, R₄ is selected from the group consisting of hydrogen, fluorine, chlorine, cyano, methyl, ethyl, propyl, isopropyl, trifluoromethyl, difluoromethyl, monofluoromethyl, trideuteriomethyl, dideuteriomethyl, monodeuteriomethyl, hydroxy, methoxy, ethoxy, propoxy, isopropoxy, trifluoromethoxy, difluoromethoxy, monofluoromethoxy, trideuteriomethoxy, dideuteriomethoxy, trifluoroethoxy, trideuterioethoxy, methylthio, ethylthio, amino, monomethylamino, monoethylamino and dimethylamino.

As a further preferred embodiment, in the compound of formula (I), the stereoisomer or pharmaceutically acceptable salt thereof, R₅ₐ is selected from the group consisting of hydrogen, deuterium, fluorine, chlorine, bromine, cyano, hydroxy, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl, methoxy, ethoxy, propoxy, isopropoxy, trifluoromethoxy, difluoromethoxy, trideuteriomethoxy, dideuteriomethoxy, trifluoroethoxy, trideuterioethoxy, cyclopropoxy and cyclobutoxy.

As a more further preferred embodiment, in the compound of formula (I), the stereoisomer or pharmaceutically acceptable salt thereof, R₅ₐ is selected from the group consisting of hydrogen, deuterium, cyano, hydroxy, methyl, ethyl, propyl, isopropyl, trifluoromethyl, difluoromethyl, monofluoromethyl, trideuteriomethyl, dideuteriomethyl, monodeuteriomethyl, methoxy, ethoxy, propoxy, isopropoxy, trifluoromethoxy, difluoromethoxy, trideuteriomethoxy, dideuteriomethoxy, trifluoroethoxy and trideuterioethoxy.

As a further preferred embodiment, in the compound of formula (I), the stereoisomer or pharmaceutically acceptable salt thereof, R_{5b} and R_{5c} are each independently selected from the group consisting of hydrogen, deuterium, fluorine, chlorine, bromine, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl, methoxy, ethoxy, propoxy, isopropoxy, trifluoromethoxy, difluoromethoxy, monofluoromethoxy, trideuteriomethoxy, dideuteriomethoxy, trifluoroethoxy, trideuterioethoxy, cyclopropoxy and cyclobutoxy;
R_{5d1} is selected from the group consisting of hydrogen, deuterium, methyl, ethyl, propyl, isopropyl, cyclopropyl, cyclobutyl, oxacyclobutyl and azacyclobutyl, above groups are independently optionally further substituted with one or more substituents selected from the group consisting of deuterium, fluorine, chlorine, bromine, methyl, ethyl, propyl, isopropyl, cyclopropyl, cyclobutyl, oxacyclobutyl, azacyclobutyl, =O, =S, -S(O)ᵣR₁₃ and -O-R₁₄, above groups are independently optionally further substituted with one or more substituents selected from the group consisting of deuterium and halogen;
or, R_{5b} and R_{5c}, together with the carbon atom directly attached thereto, form a C₃₋₆ cycloalkyl or 3-6 membered heterocyclyl, above groups are independently optionally further substituted with one or more substituents selected from the group consisting of deuterium, fluorine, chlorine, bromine, C₁₋₄ alkyl, C₂₋₄ alkenyl, C₂₋₄ alkynyl, C₃₋₆ cycloalkyl, =O, =S, methylthio, ethylthio, methoxy, ethoxy and isopropoxy, above groups are independently optionally further substituted with one or more substituents selected from the group consisting of deuterium and halogen, and R_{5d1} is selected from the group consisting of hydrogen, deuterium, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl and azacyclobutyl;
wherein, R₁₃, R₁₄ and r are defined as in the compound of formula (IV-a).

As a more further preferred embodiment, in the compound of formula (I), the stereoisomer or pharmaceutically acceptable salt thereof, R_{5b} and R_{5c} are each independently selected from the group consisting of hydrogen, deuterium, fluorine, chlorine, bromine, methyl, ethyl, propyl, isopropyl, trifluoromethyl, difluoromethyl, monofluoromethyl, trideuteriomethyl, dideuteriomethyl, monodeuteriomethyl, methoxy, ethoxy, propoxy, isopropoxy, trifluoromethoxy, difluoromethoxy, monofluoromethoxy, trideuteriomethoxy, dideuteriomethoxy, trifluoroethoxy and trideuterioethoxy;
R_{5d1} is methyl, ethyl, isopropyl or trideuteriomethyl.

As a preferred embodiment, in the compound of formula (I), the stereoisomer or pharmaceutically acceptable salt thereof, the compound of formula (I) is a compound of formula (IV-b) as shown below: 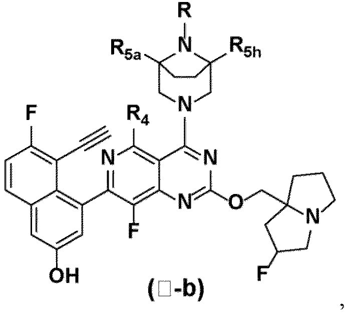
wherein, R is selected from the group consisting of hydrogen, C₁₋₄ alkyl, C₂₋₄ alkenyl, C₃₋₆ cycloalkyl, 3-6 membered heterocyclyl, -S(O)ᵣR₁₃, -O-R₁₄, -C(O)OR₁₄, -C(O)SR₁₄, -C(O)R₁₅, -O-C(O)R₁₅, -NR₁₆R₁₇, -C(O)NR₁₆R₁₇ and -N(R₁₆)-C(O)R₁₅, above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(O)NR₁₆R₁₇ and -N(R₁₆)-C(O)R₁₅;
R₄ is selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, -O-R₁₄, -S-R₁₃ and -NR₁₆R₁₇;
R₅ₐ is selected from the group consisting of hydrogen, deuterium, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl and -C₀₋₄ alkyl-O-R₁₄;
R₅ₕ is selected from the following groups:
   1) 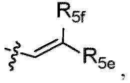 wherein R₅ₑ and R_{5f} are each independently selected from the group consisting of hydrogen, deuterium, halogen and C₁₋₄ alkyl, above groups are independently optionally further substituted with one or more substituents selected from the group consisting of deuterium, halogen, cyano, C₁₋₄ alkyl, C₃₋₆ cycloalkyl, 3-6 membered heterocyclyl, C₆₋₈ aryl and 5-8 membered heteroaryl, above groups are more independently optionally further substituted with one or more substituents selected from the group consisting of deuterium and halogen;
   2) 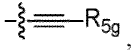 wherein R_{5g} is selected from the group consisting of hydrogen, deuterium, halogen and C₁₋₄ alkyl, above groups are independently optionally further substituted with one or more substituents selected from the group consisting of deuterium, halogen, cyano, C₁₋₄ alkyl, C₃₋₆ cycloalkyl, 3-6 membered heterocyclyl, C₆₋₈ aryl and 5-8 membered heteroaryl, above groups are more independently optionally further substituted with one or more substituents selected from the group consisting of deuterium and halogen;
   3) C₁₋₄ alkyl, C₃₋₆ cycloalkyl or 3-6 membered heterocyclyl, wherein the above C₁₋₄ alkyl is independently optionally further substituted with one or more substituents selected from the group consisting of deuterium, halogen, C₁₋₄ alkyl, C₂₋₄ alkenyl, C₂₋₄ alkynyl, C₃₋₆ cycloalkyl, 3-6 membered heterocyclyl, =O, =S, -SF₅, -S(O)ᵣR₁₃ and -O-R₁₄, above groups are independently optionally further substituted with one or more substituents selected from the group consisting of deuterium and halogen, provided that R₅ₕ is not methyl substituted -O-R₁₄, C₃₋₆ cycloalkyl or 3-6 membered heterocyclyl;
wherein, R₁₃, R₁₄, R₁₅, R₁₆, R₁₇ and r are defined as in the compound of formula (I).

As a further preferred embodiment, in the compound of formula (I), the stereoisomer or pharmaceutically acceptable salt thereof, the compound of formula (I) is a compound of formula (IV-b 1) as shown below: 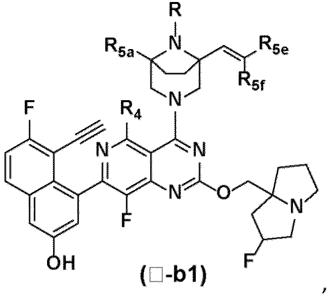
wherein, R is selected from the group consisting of hydrogen, methyl, ethyl, propyl, isopropyl, cyclopropyl, cyclobutyl, oxacyclobutyl, azacyclobutyl and -C(O)OR₁₄, above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl and 3-6 membered heterocyclyl;
R₄ is selected from the group consisting of hydrogen, deuterium, fluorine, chlorine, bromine, cyano, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl, hydroxy, methoxy, ethoxy, propoxy, isopropoxy, trifluoromethoxy, difluoromethoxy, monofluoromethoxy, trideuteriomethoxy, dideuteriomethoxy, trifluoroethoxy, tri deuteri oethoxy, cyclopropoxy, cyclobutoxy, methylthio, ethylthio, amino, monomethylamino, monoethylamino and dimethylamino;
R₅ₐ is selected from the group consisting of hydrogen, deuterium, fluorine, chlorine, bromine, cyano, hydroxy, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl, methoxy, ethoxy, propoxy, isopropoxy, trifluoromethoxy, difluoromethoxy, trideuteriomethoxy, dideuteriomethoxy, trifluoroethoxy, trideuterioethoxy, cyclopropoxy and cyclobutoxy;
R₅ₑ and R_{5f} are each independently selected from the group consisting of hydrogen, fluorine, chlorine, methyl, ethyl and isopropyl, above groups are independently optionally further substituted with one or more substituents selected from the group consisting of deuterium, fluorine, chlorine, cyano, methyl, ethyl, propyl, isopropyl, cyclopropyl, cyclobutyl, oxacyclobutyl and azacyclobutyl, above groups are more independently optionally further substituted with one or more substituents selected from the group consisting of deuterium, fluorine or chlorine;
wherein, R₁₄ is defined as in formula (IV-b).

As a more further preferred embodiment, in the compound of formula (I), the stereoisomer or pharmaceutically acceptable salt thereof, R is hydrogen; R₄ is hydrogen; R₅ₐ is hydrogen; R₅ₑ and R_{5f} are each independently selected from the group consisting of hydrogen, fluorine and methyl.

As a further preferred embodiment, in the compound of formula (I), the stereoisomer or pharmaceutically acceptable salt thereof, the compound of formula (I) is a compound of formula (IV-b2) as shown below: 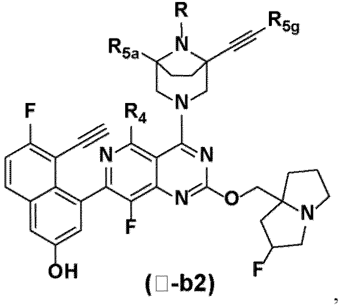
wherein, R is selected from the group consisting of hydrogen, methyl, ethyl, propyl, isopropyl, cyclopropyl, cyclobutyl, oxacyclobutyl, azacyclobutyl and -C(O)OR₁₄, above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl and 3-6 membered heterocyclyl;
R₄ is selected from the group consisting of hydrogen, deuterium, fluorine, chlorine, bromine, cyano, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl, hydroxy, methoxy, ethoxy, propoxy, isopropoxy, trifluoromethoxy, difluoromethoxy, monofluoromethoxy, trideuteriomethoxy, dideuteriomethoxy, trifluoroethoxy, tri deuteri oethoxy, cyclopropoxy, cyclobutoxy, methylthio, ethylthio, amino, monomethylamino, monoethylamino and dimethylamino;
R₅ₐ is selected from the group consisting of hydrogen, deuterium, fluorine, chlorine, bromine, cyano, hydroxy, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl, methoxy, ethoxy, propoxy, isopropoxy, trifluoromethoxy, difluoromethoxy, trideuteriomethoxy, dideuteriomethoxy, trifluoroethoxy, trideuterioethoxy, cyclopropoxy and cyclobutoxy;
R_{5g} is selected from the group consisting of hydrogen, fluorine, chlorine, methyl, ethyl and isopropyl, above groups are independently optionally further substituted with one or more substituents selected from the group consisting of deuterium, fluorine, chlorine, cyano, methyl, ethyl, propyl, isopropyl, cyclopropyl, cyclobutyl, oxacyclobutyl and azacyclobutyl, above groups are more independently optionally further substituted with one or more substituents selected from the group consisting of deuterium, fluorine or chlorine;
wherein, R₁₄ is defined as in the compound of formula (IV-b).

As a more further preferred embodiment, in the compound of formula (I), the stereoisomer or pharmaceutically acceptable salt thereof, R is hydrogen; R₄ is hydrogen; R₅ₐ is hydrogen; R_{5g} is selected from the group consisting of hydrogen, fluorine and methyl.

As a further preferred embodiment, in the compound of formula (I), the stereoisomer or pharmaceutically acceptable salt thereof, the compound of formula (I) is a compound of formula (IV-b3) as shown below: 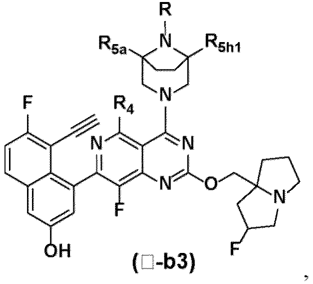
wherein, R is selected from the group consisting of hydrogen, methyl, ethyl, propyl, isopropyl, cyclopropyl, cyclobutyl, oxacyclobutyl, azacyclobutyl and -C(O)OR₁₄, above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl and 3-6 membered heterocyclyl;
R₄ is selected from the group consisting of hydrogen, deuterium, fluorine, chlorine, bromine, cyano, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl, hydroxy, methoxy, ethoxy, propoxy, isopropoxy, trifluoromethoxy, difluoromethoxy, monofluoromethoxy, trideuteriomethoxy, dideuteriomethoxy, trifluoroethoxy, tri deuteri oethoxy, cyclopropoxy, cyclobutoxy, methylthio, ethylthio, amino, monomethylamino, monoethylamino and dimethylamino;
R₅ₐ is selected from the group consisting of hydrogen, deuterium, fluorine, chlorine, bromine, cyano, hydroxy, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl, methoxy, ethoxy, propoxy, isopropoxy, trifluoromethoxy, difluoromethoxy, trideuteriomethoxy, dideuteriomethoxy, trifluoroethoxy, trideuterioethoxy, cyclopropoxy and cyclobutoxy;
R₅ₕ₁ is methyl, ethyl, propyl, isopropyl, cyclopropyl, cyclobutyl, oxacyclobutyl and azacyclobutyl, above groups are independently optionally further substituted with one or more substituents selected from the group consisting of deuterium, halogen, C₁₋₄ alkyl, C₂₋₄ alkenyl, C₂₋₄ alkynyl, C₃₋₆ cycloalkyl, 3-6 membered heterocyclyl, =O, =S, -SF₅, -S(O)ᵣR₁₃ and -O-R₁₄, above groups are independently optionally further substituted with one or more substituents selected from the group consisting of deuterium and halogen, provided that R₅ₕ₁ is not methyl substituted with-O-R₁₄, cyclopropyl, cyclobutyl, oxacyclobutyl and azacyclobutyl;
wherein, R₁₃, R₁₄ and r are defined as in the compound of formula (IV-b).

As a more further preferred embodiment, in the compound of formula (I), the stereoisomer or pharmaceutically acceptable salt thereof, R is hydrogen; R₄ is hydrogen, methyl, ethyl, methoxy or methylthio; R₅ₐ is hydrogen or deuterium;
R₅ₕ₁ is methyl, ethyl, propyl or isopropyl, above groups are independently optionally further substituted with one or more substituents selected from the group consisting of deuterium, fluorine, chlorine, C₃₋₆ cycloalkyl, 3-6 membered heterocyclyl, =O, =S, -SF₅, -S(O)ᵣR₁₃ and -O-R₁₄, above groups are independently optionally further substituted with one or more substituents selected from the group consisting of deuterium and halogen; provided that R₅ₕ₁ is not methyl substituted with -O-R₁₄;
wherein, R₁₃, R₁₄ and r are defined as in the compound of formula (IV-b).

As the most preferred embodiment, the compound of formula (I), the stereoisomer or pharmaceutically acceptable salt thereof includes, but is not limited to, the following compounds:

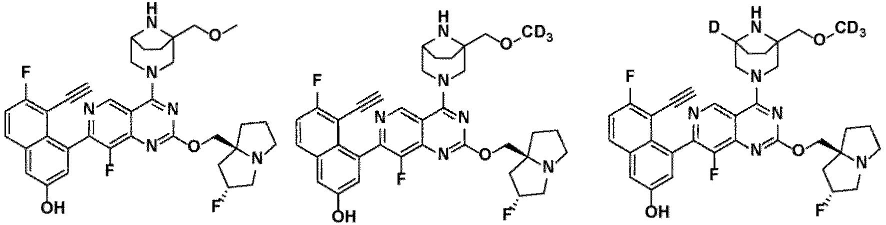

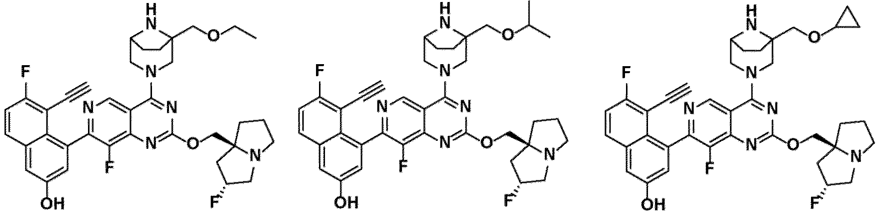

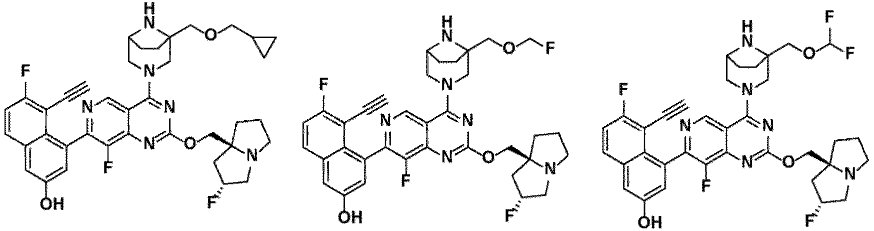

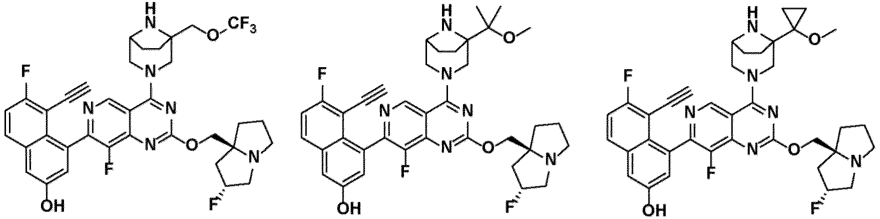

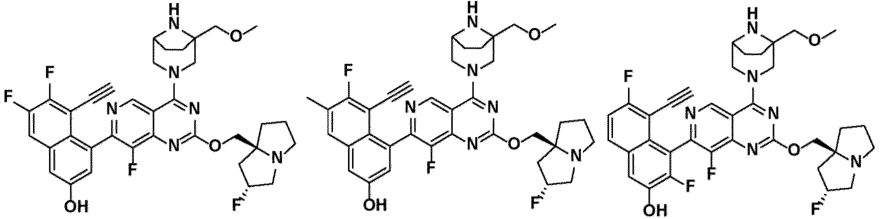

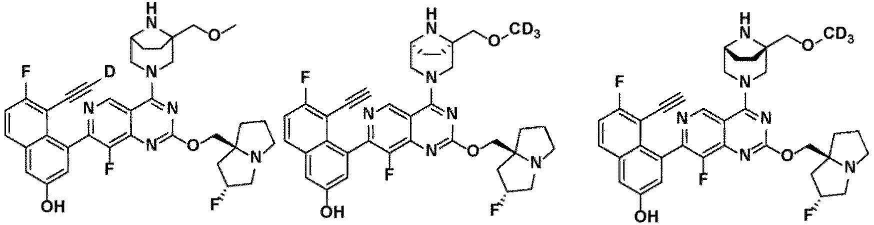

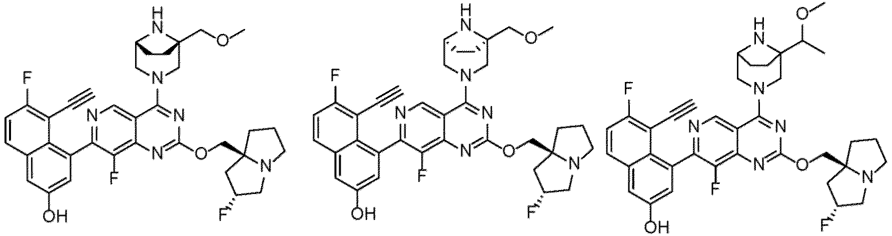

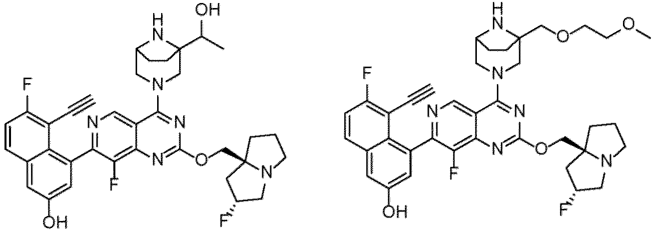

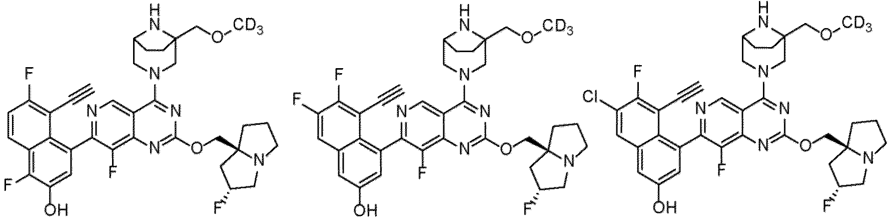

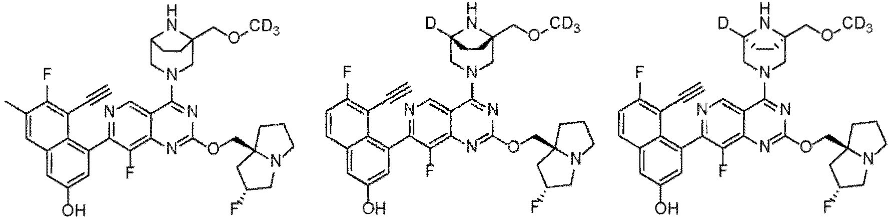

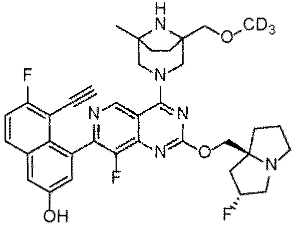

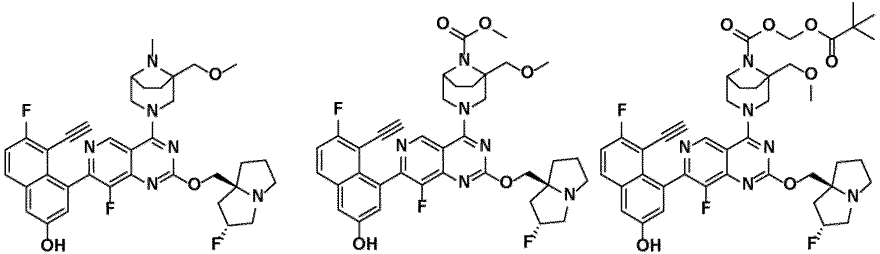

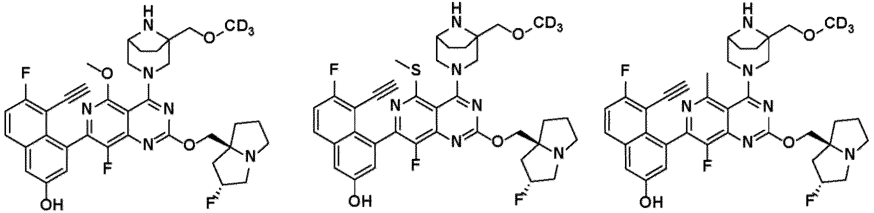

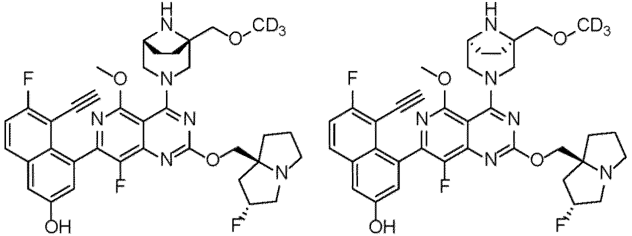

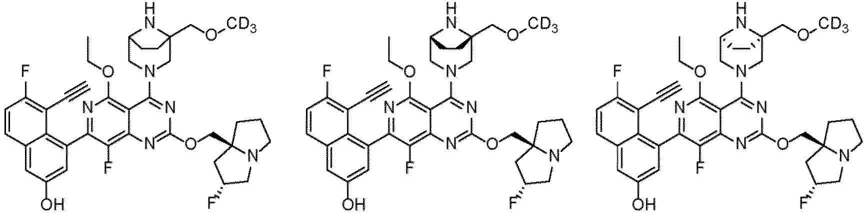

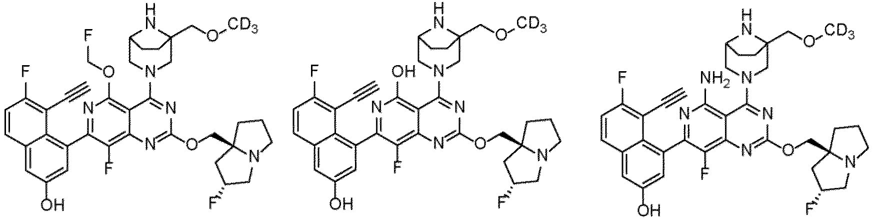

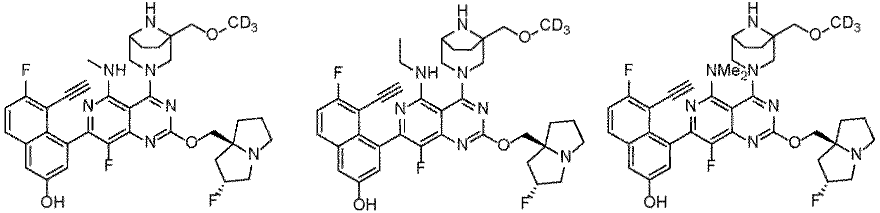

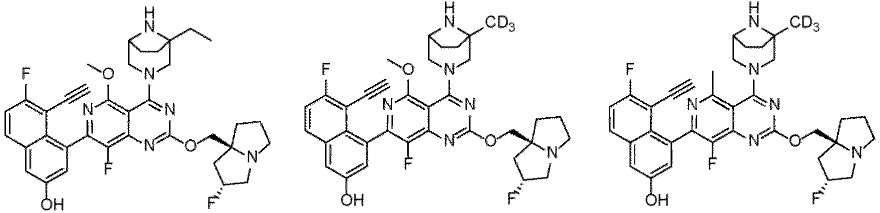

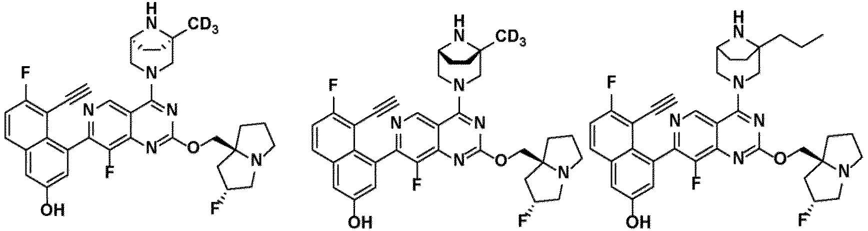

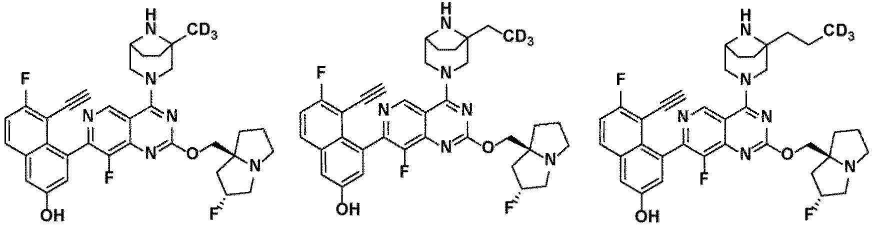

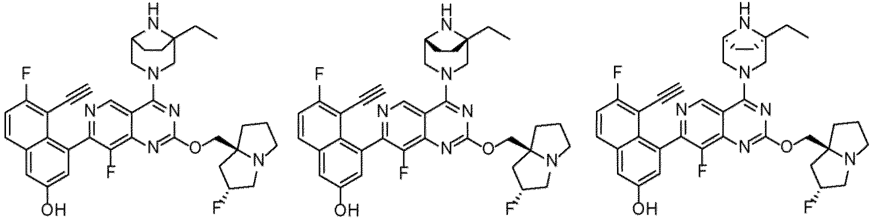

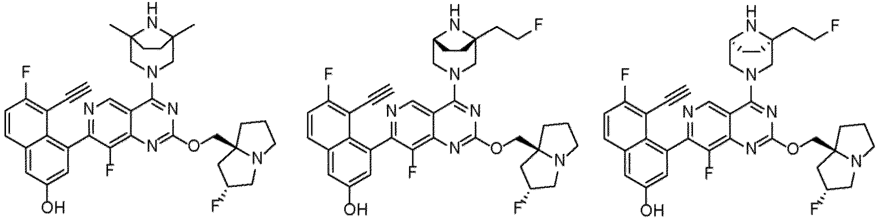

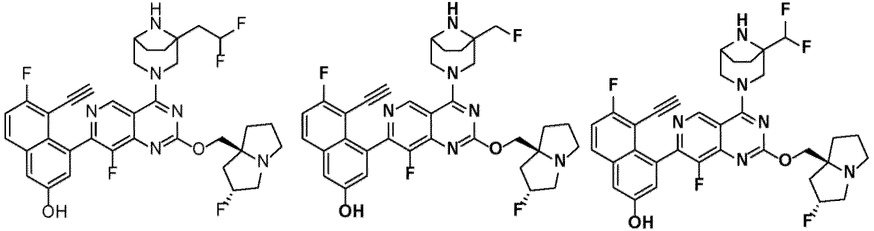

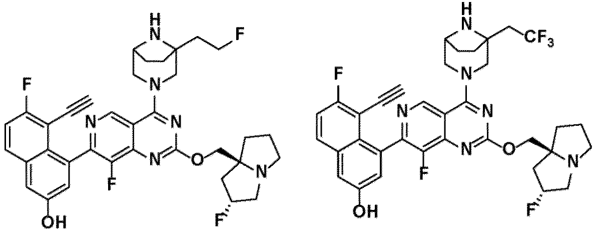

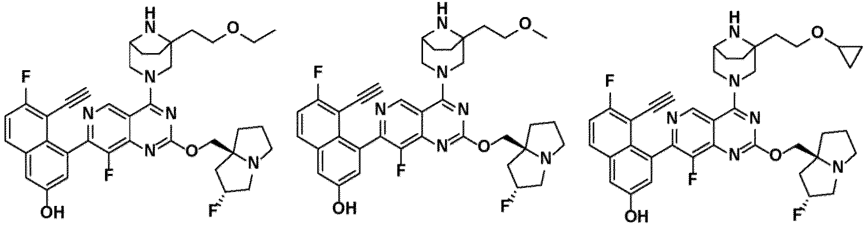

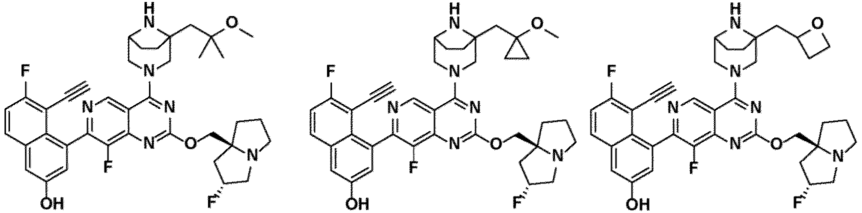

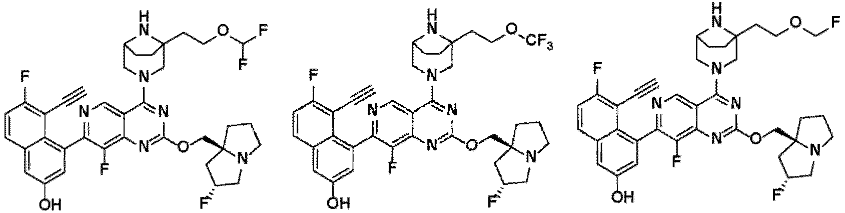

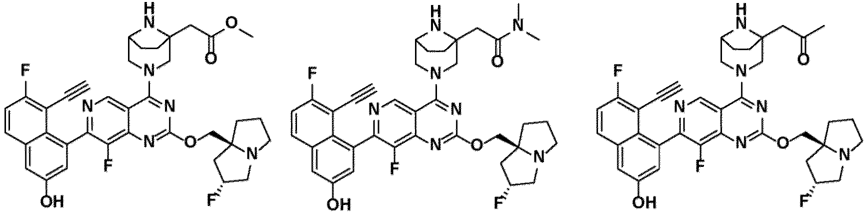

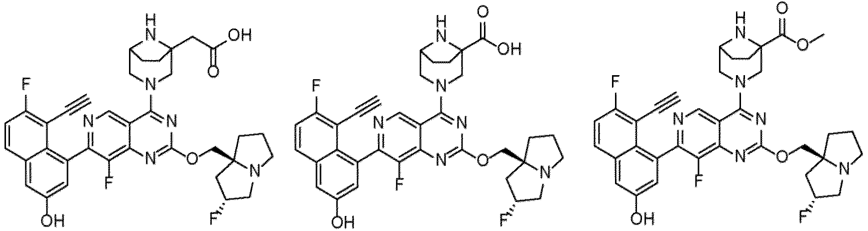

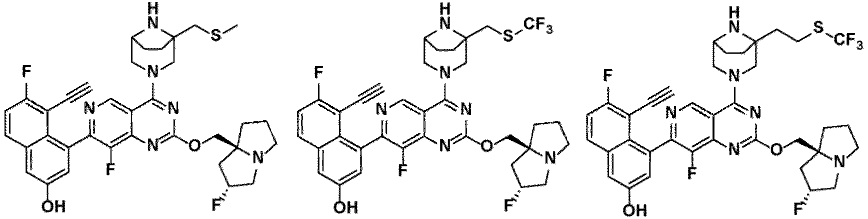

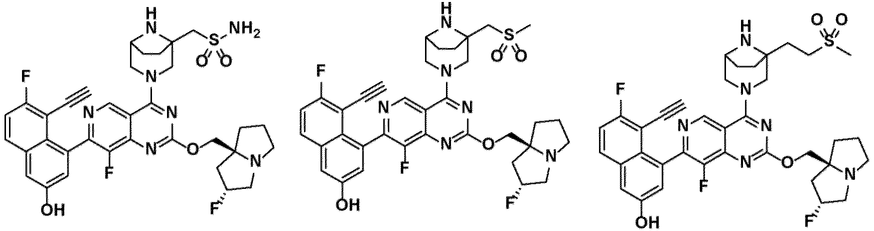

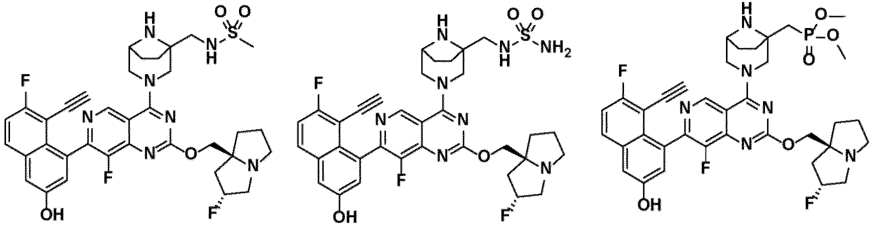

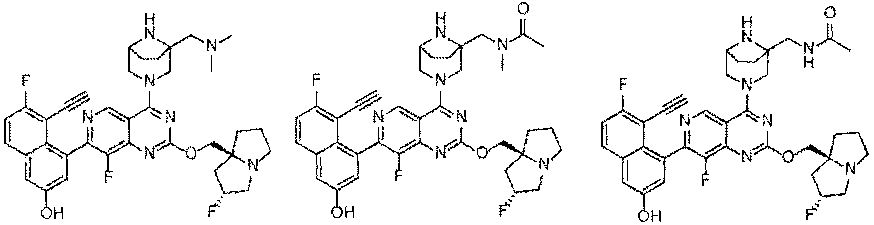

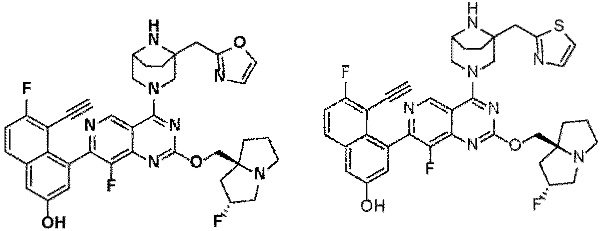

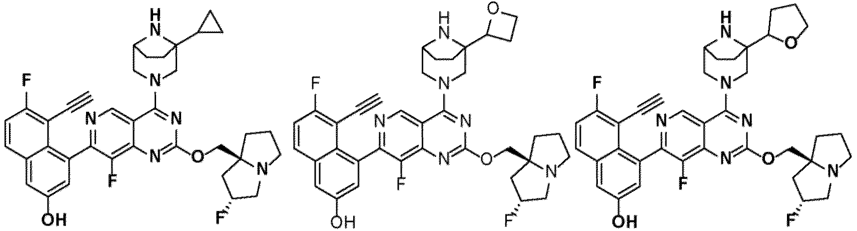

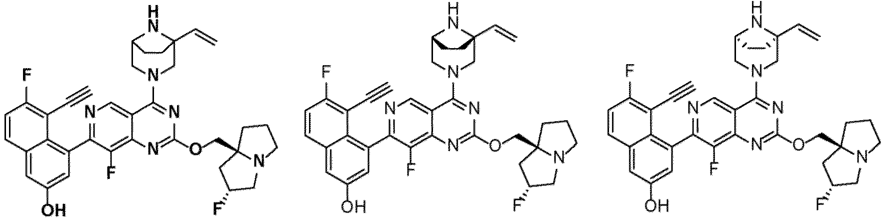

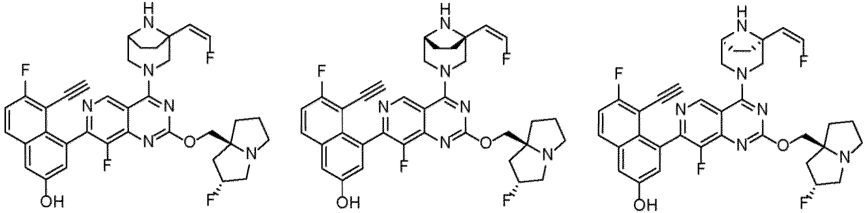

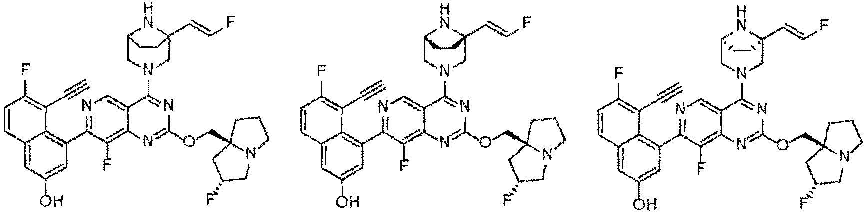

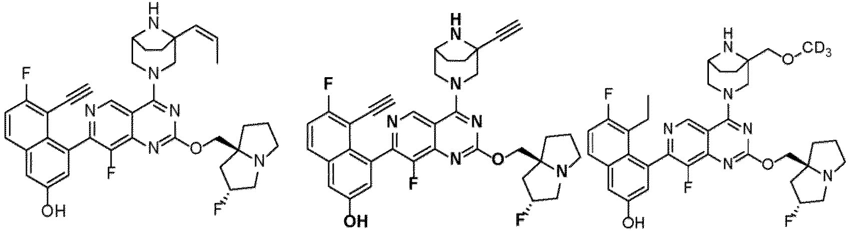

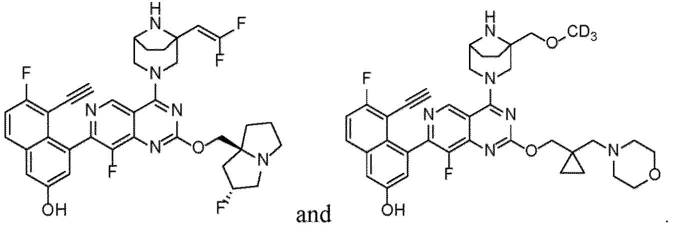

The second aspect of the present invention provides a process for preparing the compound of formula (I), the stereoisomer or pharmaceutically acceptable salt thereof, comprising the following step:

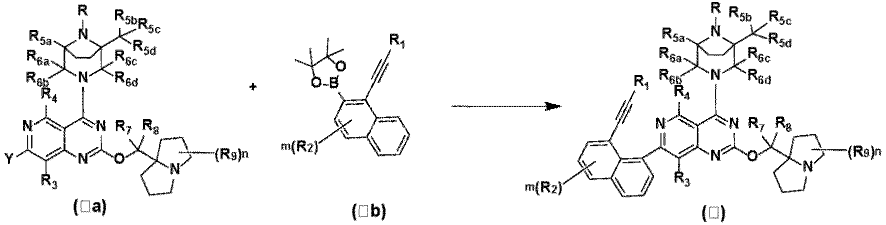

The compound of formula (Ia) or the acid salt thereof reacts with the compound of formula (Ib) or the acid salt thereof to form the compound of formula (I) or the acid salt thereof; or

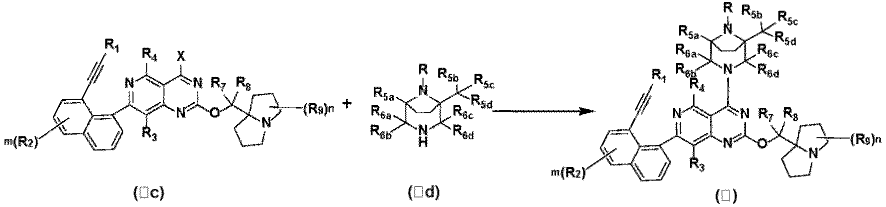

The compound of formula (Ic) or the acid salt thereof reacts with the compound of formula (Id) or the acid salt thereof to form the compound of formula (I) or the acid salt thereof; wherein, X is fluorine, chlorine or bromine, and R, R₁, R₂, R₃, R₄, R_{5b}, R_{5c}, R_{5d}, R₅ₑ, R₆ₐ, R_{6b}, R_{6c}, R_{6d}, R₇, R₈, R₉, m and n are defined as in the compound of formula (I).

The third aspect of the present invention provides a pharmaceutical composition comprising the compound of formula (I), the stereoisomer or pharmaceutically acceptable salt thereof, and a pharmaceutically acceptable carrier.

The present invention also relates to the use of the compound of formula (I), the stereoisomer or pharmaceutically acceptable salt thereof in the preparation of medicaments for the treatment and/or prevention of cancers or tumors related to KRas G12D.

The present invention also relates to the use of the compound of formula (I), the stereoisomer or pharmaceutically acceptable salt thereof in the preparation of medicaments for the treatment and/or prevention of KRas G12D-related sarcoma (angiosarcoma, fibrosarcoma, rhabdomyosarcoma, liposarcoma), myxoma, rhabdomyoma, fibroma, lipoma, teratoma; bronchial carcinoma (squamous cell carcinoma, undifferentiated small cell carcinoma, undifferentiated large cell carcinoma, adenocarcinoma), alveolar (bronchiolar carcinom a) carcinoma, bronchial adenoma, lymphoma, chondromatoid hamartoma, mesothelioma; esophageal carcinoma (squamous cell carcinoma, adenocarcinoma, leiomyosarcoma, lymphoma), gastric carcinoma (lymphoma, leiomyosarcoma), pancreatic carcinoma (ductal adenocarcinoma, insulinoma, glucagonoma, gastrinoma, carcinoid tumor, vasoactive intestinal peptide tumor), small intestine cancer (adenocarcinoma, lymphoma, carcinoid tumor, Kaposi's sarcoma, leiomyoma, hemangioma, lipoma, neurofibroma, fibroma), colorectal carcinoma (adenocarcinoma, tubular adenoma, villous adenoma, hamartoma, leiomyoma); kidney cancer (adenocarcinoma, Wilms' tumor (nephroblastoma), lymphoma, leukemia), bladder cancer and urethral cancer (squamous cell carcinoma, transitional cell carcinoma, adenocarcinoma), prostate cancer (adenocarcinoma, sarcoma), testicular cancer (seminoma, teratoma, embryonal carcinoma, terato-epithelial cancer, choriocarcinoma, sarcoma, mesenchymal cell carcinoma, fibroma, fibroadenoma, adenomatoid tumor, lipoma); liver cancer (hepatocellular carcinoma), cholangiocarcinoma, hepatoblastoma, angiosarcoma, hepatocellular adenoma, hemangioma; gallbladder cancer, ampullary carcinoma, cholangiocarcinoma; osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochondroma (osteochondral exostosis), benign chondroma, chondroblastoma, chondromyxoid fibroma, osteoid osteoma and giant cell tumor; skull cancer (osteoma, hemangioma, granuloma, xanthoma, osteitis deformans), meningeal carcinomatosis (meningioma, meningeal sarcoma, gliomatosis), brain cancer (astrocytoma, medulloblastoma, glioma, ependymoma, blastoma (pineal tumor), glioblastoma multiforme, oligodendroglioma, neurilemmoma, retinoblastoma, congenital tumor, spinal neurofibroma, meningioma, glioma, sarcoma); uterine cancer (endometrial cancer (serous cystadenocarcinoma, mucinous cystadenocarcinoma, unclassified cancer), granulosa-theca cell tumor, Sertoli-Leydig cell tumor, dysgerminoma, malignant teratoma), vulvar cancer (squamous cell carcinoma, intraepithelial carcinoma, adenocarcinoma, fibrosarcoma, melanoma), vaginal cancer (clear cell carcinoma, squamous cell carcinoma, botryoid sarcoma (embryonal rhabdomyosarcoma)), fallopian tube (carcinoma); hematologic cancer (myelogenous leukemia (acute and chronic), acute lymphoblastic leukemia, chronic lymphocytic leukemia, myeloproliferative disease, multiple myeloma, myelodysplastic syndrome), Hodgkin's disease, non-Hodgkin's lymphoma (malignant lymphoma); malignant melanoma, basal cell carcinoma, squamous cell carcinoma, Kaposi's sarcoma, nevus, dysplastic nevus, lipoma, hemangioma, dermatofibroma, keloid, psoriasis, adrenal tumor and neuroblastoma.

The present invention also relates to the compound of formula (I), the stereoisomer or pharmaceutically acceptable salt thereof, for use as a drug.

The present invention also relates to the compound of formula (I), the stereoisomer or pharmaceutically acceptable salt thereof, for use in the treatment and/or prevention of cancers or tumors related to KRas G12D.

The present invention also relates to the compound of formula (I), the stereoisomer or pharmaceutically acceptable salt thereof, for use in the treatment and/or prevention of KRas G12D related-sarcoma (angiosarcoma, fibrosarcoma, rhabdomyosarcoma, liposarcoma), myxoma, rhabdomyoma, fibroma, lipoma, teratoma; bronchial carcinoma (squamous cell carcinoma, undifferentiated small cell carcinoma, undifferentiated large cell carcinoma, adenocarcinoma), alveolar (bronchiolar carcinom a) carcinoma, bronchial adenoma, lymphoma, chondromatoid hamartoma, mesothelioma; esophageal carcinoma (squamous cell carcinoma, adenocarcinoma, leiomyosarcoma, lymphoma), gastric carcinoma (lymphoma, leiomyosarcoma), pancreatic carcinoma (ductal adenocarcinoma, insulinoma, glucagonoma, gastrinoma, carcinoid tumor, vasoactive intestinal peptide tumor), small intestine cancer (adenocarcinoma, lymphoma, carcinoid tumor, Kaposi's sarcoma, leiomyoma, hemangioma, lipoma, neurofibroma, fibroma), colorectal carcinoma (adenocarcinoma, tubular adenoma, villous adenoma, hamartoma, leiomyoma); kidney cancer (adenocarcinoma, Wilms' tumor (nephroblastoma), lymphoma, leukemia), bladder cancer and urethral cancer (squamous cell carcinoma, transitional cell carcinoma, adenocarcinoma), prostate cancer (adenocarcinoma, sarcoma), testicular cancer (seminoma, teratoma, embryonal carcinoma, terato-epithelial cancer, choriocarcinoma, sarcoma, mesenchymal cell carcinoma, fibroma, fibroadenoma, adenomatoid tumor, lipoma); liver cancer (hepatocellular carcinoma), cholangiocarcinoma, hepatoblastoma, angiosarcoma, hepatocellular adenoma, hemangioma; gallbladder cancer, ampullary carcinoma, cholangiocarcinoma; osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochondroma (osteochondral exostosis), benign chondroma, chondroblastoma, chondromyxoid fibroma, osteoid osteoma and giant cell tumor; skull cancer (osteoma, hemangioma, granuloma, xanthoma, osteitis deformans), meningeal carcinomatosis (meningioma, meningeal sarcoma, gliomatosis), brain cancer (astrocytoma, medulloblastoma, glioma, ependymoma, blastoma (pineal tumor), glioblastoma multiforme, oligodendroglioma, neurilemmoma, retinoblastoma, congenital tumor, spinal neurofibroma, meningioma, glioma, sarcoma); uterine cancer (endometrial cancer (serous cystadenocarcinoma, mucinous cystadenocarcinoma, unclassified cancer), granulosa-theca cell tumor, Sertoli-Leydig cell tumor, dysgerminoma, malignant teratoma), vulvar cancer (squamous cell carcinoma, intraepithelial carcinoma, adenocarcinoma, fibrosarcoma, melanoma), vaginal cancer (clear cell carcinoma, squamous cell carcinoma, botryoid sarcoma (embryonal rhabdomyosarcoma)), fallopian tube (carcinoma); hematologic cancer (myelogenous leukemia (acute and chronic), acute lymphoblastic leukemia, chronic lymphocytic leukemia, myeloproliferative disease, multiple myeloma, myelodysplastic syndrome), Hodgkin's disease, non-Hodgkin's lymphoma (malignant lymphoma); malignant melanoma, basal cell carcinoma, squamous cell carcinoma, Kaposi's sarcoma, nevus, dysplastic nevus, lipoma, hemangioma, dermatofibroma, keloid, psoriasis, adrenal tumor and neuroblastoma.

The present invention also relates to a method for treating and/or preventing cancers or tumors related to KRas G12D, comprising administering to a patient in need a therapeutically effective amount of the compound of formula (I), the stereoisomer or pharmaceutically acceptable salt thereof.

The present invention also relates to a method for treating and/or preventing KRas G12D related-sarcoma (angiosarcoma, fibrosarcoma, rhabdomyosarcoma, liposarcoma), myxoma, rhabdomyoma, fibroma, lipoma, teratoma; bronchial carcinoma (squamous cell carcinoma, undifferentiated small cell carcinoma, undifferentiated large cell carcinoma, adenocarcinoma), alveolar (bronchiolar carcinom a) carcinoma, bronchial adenoma, lymphoma, chondromatoid hamartoma, mesothelioma; esophageal carcinoma (squamous cell carcinoma, adenocarcinoma, leiomyosarcoma, lymphoma), gastric carcinoma (lymphoma, leiomyosarcoma), pancreatic carcinoma (ductal adenocarcinoma, insulinoma, glucagonoma, gastrinoma, carcinoid tumor, vasoactive intestinal peptide tumor), small intestine cancer (adenocarcinoma, lymphoma, carcinoid tumor, Kaposi's sarcoma, leiomyoma, hemangioma, lipoma, neurofibroma, fibroma), colorectal carcinoma (adenocarcinoma, tubular adenoma, villous adenoma, hamartoma, leiomyoma); kidney cancer (adenocarcinoma, Wilms' tumor (nephroblastoma), lymphoma, leukemia), bladder cancer and urethral cancer (squamous cell carcinoma, transitional cell carcinoma, adenocarcinoma), prostate cancer (adenocarcinoma, sarcoma), testicular cancer (seminoma, teratoma, embryonal carcinoma, terato-epithelial cancer, choriocarcinoma, sarcoma, mesenchymal cell carcinoma, fibroma, fibroadenoma, adenomatoid tumor, lipoma); liver cancer (hepatocellular carcinoma), cholangiocarcinoma, hepatoblastoma, angiosarcoma, hepatocellular adenoma, hemangioma; gallbladder cancer, ampullary carcinoma, cholangiocarcinoma; osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochondroma (osteochondral exostosis), benign chondroma, chondroblastoma, chondromyxoid fibroma, osteoid osteoma and giant cell tumor; skull cancer (osteoma, hemangioma, granuloma, xanthoma, osteitis deformans), meningeal carcinomatosis (meningioma, meningeal sarcoma, gliomatosis), brain cancer (astrocytoma, medulloblastoma, glioma, ependymoma, blastoma (pineal tumor), glioblastoma multiforme, oligodendroglioma, neurilemmoma, retinoblastoma, congenital tumor, spinal neurofibroma, meningioma, glioma, sarcoma); uterine cancer (endometrial cancer (serous cystadenocarcinoma, mucinous cystadenocarcinoma, unclassified cancer), granulosa-theca cell tumor, Sertoli-Leydig cell tumor, dysgerminoma, malignant teratoma), vulvar cancer (squamous cell carcinoma, intraepithelial carcinoma, adenocarcinoma, fibrosarcoma, melanoma), vaginal cancer (clear cell carcinoma, squamous cell carcinoma, botryoid sarcoma (embryonal rhabdomyosarcoma)), fallopian tube (carcinoma); hematologic cancer (myelogenous leukemia (acute and chronic), acute lymphoblastic leukemia, chronic lymphocytic leukemia, myeloproliferative disease, multiple myeloma, myelodysplastic syndrome), Hodgkin's disease, non-Hodgkin's lymphoma (malignant lymphoma); malignant melanoma, basal cell carcinoma, squamous cell carcinoma, Kaposi's sarcoma, nevus, dysplastic nevus, lipoma, hemangioma, dermatofibroma, keloid, psoriasis, adrenal tumor and neuroblastoma, comprising administering to a patient in need a therapeutically effective amount of the compound of formula (I), the stereoisomer or pharmaceutically acceptable salt thereof.

### DETAILED DESCRIPTION OF EMBODIMENTS

After an extensive and intensive research, the inventors of the present invention develop a KRas G12D inhibitor with the structure of formula (I) for the first time. The series of compounds of the present invention can be widely applied in the preparation of drugs for the treatment and/or prevention of cancers or tumors related to KRas G12D, and are expected to be developed into a new generation of KRas G12D inhibitors. The present invention is achieved on this basis.

Detailed description: unless otherwise stated or specified, the following terms used in the specification and claims have the following meanings.

"Alkyl" refers to linear or branched saturated aliphatic alkyl groups, preferably including a linear or branched alkyl having 1 to 10 or 1 to 6 carbon atoms or 1 to 4 carbon atoms, including but not limited to methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl or various branched isomers thereof, etc. "C₁₋₁₀ alkyl" means a linear or branched alkyl containing 1 to 10 carbon atoms, "C₁₋₄ alkyl" means a linear or branched alkyl containing 1 to 4 carbon atoms, "C₀₋₈ alkyl" means a linear or branched alkyl containing 0 to 8 carbon atoms, "C₀₋₄ alkyl" means a linear or branched alkyl containing 0 to 4 carbon atoms.

Alkyl can be optionally substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅.

"Cycloalkyl" or "carbocyclic ring" refers to monocyclic or polycyclic hydrocarbon substituents that are saturated or partially unsaturated, wherein the partially unsaturated cyclic hydrocarbon refers to a cyclic hydrocarbon that may contain one or more (preferably 1, 2 or 3) double bonds, but none of the rings have a fully conjugated π-electron system. Cycloalkyl may be monocyclic cycloalkyl or polycyclic cycloalkyl, preferably including a cycloalkyl containing 3 to 12 or 3 to 8 or 3 to 6 carbon atoms. For example, "C₃₋₁₂ cycloalkyl" means a cycloalkyl having 3 to 12 carton atoms, "C₃₋₁₀ cycloalkyl" means a cycloalkyl having 3 to 10 carton atoms, "C₃₋₈ cycloalkyl" means a cycloalkyl having 3 to 8 carton atoms, "C₃₋₆ cycloalkyl" means a cycloalkyl having 3 to 6 carton atoms, wherein:
monocyclic cycloalkyl includes but is not limited to cyclopropyl, cyclobutyl, cyclopentyl, cyclopentenyl, cyclohexyl, cyclohexenyl, cyclohexadienyl, cycloheptyl, cycloheptarienyl, cyclooctyl, etc.

Polycyclic cycloalkyl includes spirocycloalkyl, fused cycloalkyl and bridged cycloalkyl. "Spirocycloalkyl" refers to a polycyclic group in which a carton atom (called spiro-atom) is shared among monocyclic rings, wherein these rings may contain one or more (preferably 1, 2 or 3) double bonds, but none of them have a fully conjugated π-electron system. According to the number of the spiro-atoms shared among the rings, the spirocycloalkyl may be monospirocycloalkyl, bispirocycloalkyl or polyspirocycloalkyl, including but not limited to:

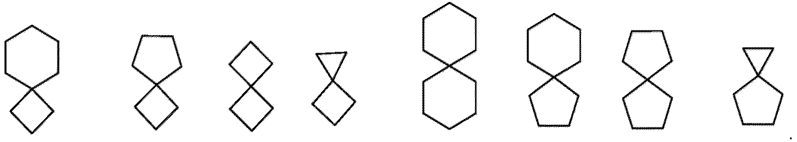

"Fused cycloalkyl" refers to an all-carbon polycyclic group in which each ring shares a pair of adj acent carbon atoms with other rings in the system, wherein one or more of the rings may contain one or more (preferably 1, 2 or 3) double bonds, but none of them have a fully conjugated π-electron system. According to the number of formed rings, the fused cycloalkyl may be bicyclic, tricyclic, tetracyclic or polycyclic, including but not limited to:

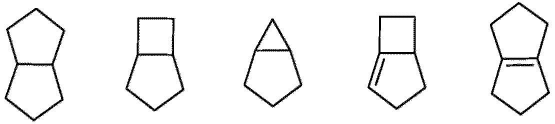

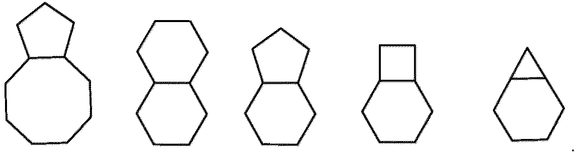

"Bridged cycloalkyl" refers to an all-carbon polycyclic group in which any two rings share two carbon atoms that are not directly connected to each other, wherein these rings may contain one or more (preferably 1, 2 or 3) double bonds, but none of them have a fully conjugated π-electron system. According to the number of formed rings, the bridged cycloalkyl may be bicyclic, tricyclic, tetracyclic or polycyclic, including but not limited to:

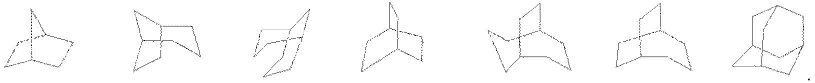

The cycloalkyl ring may be fused to an aryl, heteroaryl or heterocycloalkyl ring, wherein the ring attached to the parent structure is cycloalkyl, which includes, but is not limited to, indenyl, tetrahydronaphthyl, benzocycloheptyl, etc.

Cycloalkyl can be optionally substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅.

"Heterocyclyl" or "heterocycle" refers to a monocyclic or polycyclic hydrocarbon substituent that is saturated or partially unsaturated, wherein the partially unsaturated cyclic hydrocarbon refers to a cyclic hydrocarbon that may contain one or more (preferably 1, 2 or 3) double bonds, but none of the rings have a fully conjugated π-electron system, wherein one or more (preferably 1, 2, 3 or 4) of the rings atoms in heterocyclyl are heteroatoms selected from N, O, N•O or S(O)ᵣ (wherein r is an integer of 0, 1 or 2), excluding ring portions of -O-O-, -O-S- or -S-S-, and the remaining ring atoms are carton atoms. It preferrably includes heterocyclyl containing 3 to 12 or 3 to 8 or 3 to 6 ring atoms. For example, "3-6 membered heterocyclyl" means a heterocyclyl containing 3 to 6 ring atoms, "3-8 membered heterocyclyl" means a heterocyclyl containing 3 to 8 ring atoms, "4-8 membered heterocyclyl" means a heterocyclyl containing 4 to 8 ring atoms, "4-10 membered heterocyclyl" means a heterocyclyl containing 4 to 10 ring atoms, "5-8 membered heterocyclyl" means a heterocyclyl containing 5 to 8 ring atoms, "3-12 membered heterocyclyl" means a heterocyclyl containing 3 to 12 ring atoms.

Monocyclic heterocyclyl includes but is not limited to pyrrolidinyl, piperidinyl, piperazinyl, morpholinyl, thiomorpholinyl, homopiperazinyl, oxacyclobutyl, tetrahydrofuranyl, etc.

Polycyclic heterocyclyl includes spiroheterocyclyl, fused heterocyclyl, and bridged heterocyclyl. "Spiroheterocyclyl" refers to a polycyclic heterocyclyl group in which an atom (called spiro-atom) is shared among monocyclic rings, wherein one or more (preferably 1, 2, 3 or 4) of the ring atoms are heteroatoms selected from N, O, N•O, or S(O)ᵣ (wherein r is an integer of 0, 1 or 2), and the remaining ring atoms are carbon atoms. These rings may contain one or more (preferably 1, 2 or 3) double bonds, but none of them have a fully conjugated π-electronic system. According to the number of sprio-atoms shared among the rings, spiroheterocyclyl may be monospiroheterocyclyl, bispiroheterocyclyl or polyspiroheterocyclyl. Spiroheterocyclyl includes but is not limited to:

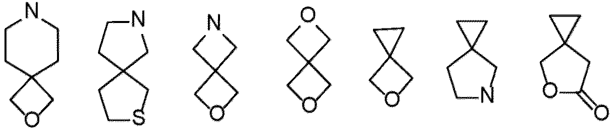

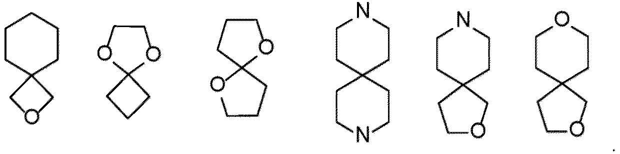

"Fused heterocyclyl" refers to a polycyclic heterocyclyl in which each ring shares a pair of adj acent atoms with the other rings in the system, wherein one or more (preferably 1, 2, 3 or 4) of the rings may contain one or more (preferably 1, 2, or 3) double bonds, but none of them have a fully conjugated π-electron system, wherein one or more (preferably 1, 2, 3, or 4) of the ring atoms are heteroatoms selected from N, O, N•O, or S(O)ᵣ (wherein r is an integer of 0, 1 or 2), and the remaining ring atoms are carbon atoms. According to the number of formed rings, the fused heterocyclyl may be bicyclic, tricyclic, tetracyclic or polycyclic, including but not limited to:

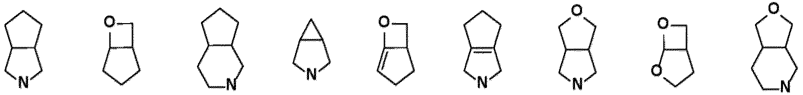

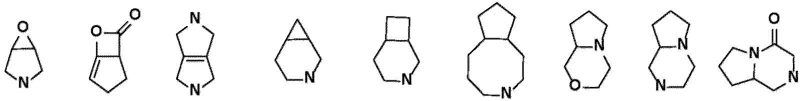

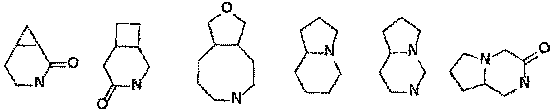

"Bridged heterocyclyl" refers to a polycyclic heterocyclyl in which any two rings share two carton atoms that are not directly attached to each other, wherein these rings may contain one or more (preferably 1, 2, or 3) double bonds, but none of them have a fully conjugated π-electron system, wherein one or more (preferably 1, 2, 3, or 4) of the ring atoms are heteroatoms selected from N, O, N atom, or S(O)ᵣ (wherein r is an integer of 0, 1 or 2), and the remaining ring atoms are carbon atoms. According to the number of formed rings, the bridged heterocyclyl may be bicyclic, tricyclic, tetracyclic or polycyclic, including but not limited to:

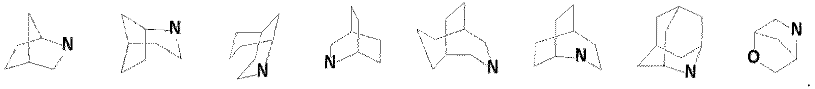

The heterocyclyl ring may be fused to an aryl, heteroaryl or cycloalkyl ring, wherein the ring attached to the parent structure is heterocyclyl, which includes but is not limited to:

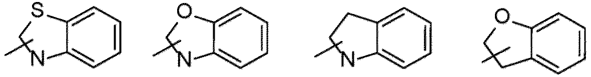

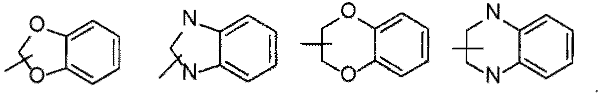

Heterocyclyl can be optionally substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅.

"Aryl" or "aromatic ring" means an all-carbon monocyclic or fused-polycyclic group (i.e., rings that share a pair of adjacent carbon atoms) and a polycyclic group having a conjugated π-electron system (i.e., rings with adjacent pairs of carbon atoms), preferably including all-carbon aryl containing 6-10 or 6-8 carbon atoms. For example, "C₆₋₁₀ aryl" means an all-carbon aryl containing 6-10 carbon atoms, including but not limited to phenyl and naphthyl, "C₆₋₈ aryl" means an all-carbon aryl containing 6-8 carbon atoms. The aryl ring may be fused to an heteroaryl, heterocyclyl or cycloalkyl ring, wherein the ring attached to the parent structure is aryl ring, including but not limited to:

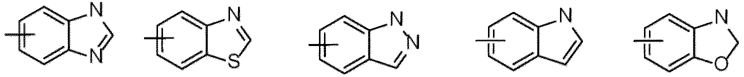

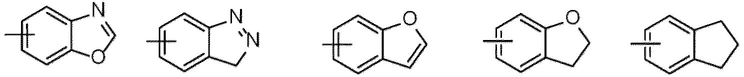

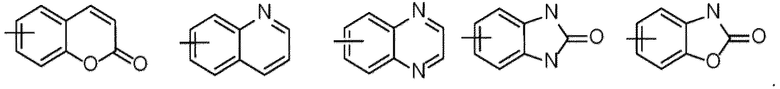

"Aryl" can be substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅.

"Heteroaryl" refers to a heteroaromatic system containing one or more (preferably 1, 2, 3 or 4) of the heteroatoms, wherein the heteroatoms include heteroatoms selected from N, O, N systems, and S(O)r (wherein r is an integer of 0, 1 or 2), preferably including a heteroaromatic system containing 5-10 or 5-8 or 5-6 ring atoms. For example, "5-8 membered heteroaryl" means a heteroaromatic system containing 5-8 ring atoms, "5-10 membered heteroaryl" means a heteroaromatic system containing 5-10 ring atoms, including but not limited to furyl, thiophenyl, pyridyl, pyrrolyl, N-alkylpyrrolyl, pyrimidinyl, pyrazinyl, imidazolyl, tetrazolyl, etc. The heteroaryl ring may be fused to an aryl, heterocyclyl or cycloalkyl ring, wherein the ring attached to the parent structure is heteroaryl ring, including but not limited to:

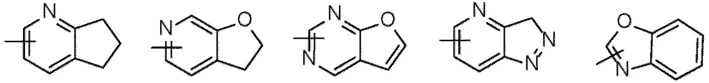

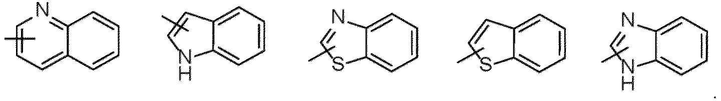

"Heteroaryl" can be optionally substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅.

"Alkenyl" refers to an alkyl defined as above consisting of at least two carbon atoms and at least one carbon-carbon double bond, preferably including a linear or branched alkenyl containing 2-10 or 2-4 carbon atoms. For example, "C₂₋₁₀ alkenyl" means a linear or branched alkenyl containing 2-10 carbon atoms, "C₂₋₄ alkenyl" means a linear or branched alkenyl containing 2-4 carbon atoms. The alkenyl includes, but is not limited to, vinyl, 1-propenyl, 2-propenyl, 1-,2-, or 3-butenyl, etc.

"Alkenyl" can be substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅.

"Alkynyl" refers to an alkyl defined as above consisting of at least two carbon atoms and at least one carbon-carbon triple bond, preferably including a linear or branched alkynyl containing 2-10 or 2-4 carbon atoms. For example, "C₂₋₁₀ alkynyl" means a linear or branched alkynyl containing 2-10 carbon atoms, "C₂₋₄ alkynyl" means a linear or branched alkynyl containing 2-4 carbon atoms. The alkynyl includes, but is not limited to, ethynyl, 1-propynyl, 2-propynyl, 1-,2-, or 3-butynyl, etc.

"Alkynyl" can be substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅.

"Alkoxy" refers to an -O-alkyl group, wherein the alkyl is defined as above. For example, "C₁₋₁₀ alkoxy" means an alkyloxy containing 1-10 carbon atoms, "C₁₋₄ alkoxy" means an alkyloxy containing 1-4 carbon atoms, "C₁₋₂ alkoxy" means an alkyloxy containing 1-2 carbon atoms, including but not limited to, methoxy, ethoxy, propoxy, butoxy, etc.

"Alkoxy" can be optionally substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅.

"Cycloalkoxy" or "cycloalkyloxy" refers to an -O-cycloalkyl group, wherein cycloalkyl is defined as above. For example, "C₃₋₁₂ cycloalkoxy" means a cycloalkyloxy containing 3-12 carbon atoms, "C₃₋₆ cycloalkoxy" means a cycloalkyloxy containing 3-6 carbon atoms, including but not limited to cyclopropoxy, cyclobutoxy, cyclopentoxy, cyclohexoxy, etc.

"Cycloalkoxy" or "cycloalkyloxy" can be optionally substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅.

"Heterocycloxy" or "heterocyclyloxy" refers to an -O-heterocyclyl group, wherein the heterocyclyl is defined as above, including but not limited to, azetidyloxy, oxetanyloxy, azetidyloxy, nitrogen, oxetanyloxy, etc.

"Heterocycloxy" or "heterocyclyloxy" can be optionally substituted or unsubstituted, and when it is substituted, the substituent is preferably one or more (preferably 1, 2, 3 or 4) of the groups independently selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅.

"C₁₋₁₀ alkanoyl" refers to a monovalent atomic group which is obtained after a hydroxy is removed from the C₁₋₁₀ alkyl acid, and is also generally referred to as "C₀₋₉ alkyl-C(O)-". For example, "C₁ alkyl-C(O)-" refers to acetyl; "C₂ alkyl-C(O)-" refers to propanoyl; "C₃ alkyl-C(O)-" refers to butanoyl or isobutanoyl.

"-C₀₋₈ alkyl-S(O)(=N-R₁₀)R₁₁" refers to the sulfur atom in -S(O)(=N-R₁₀)R₁₁ attached to C₀₋₈ alkyl, wherein the C₀₋₈ alkyl is defined as above.

"-C₀₋₈ alkyl-N=S(O)R₁₁R₁₂" refers to the nitrogen atom in -N=S(O)R₁₁R₁₂ attached to C₀₋₈ alkyl, wherein the C₀₋₈ alkyl is defined as above.

"-C₀₋₈ alkyl-N=SR₁₁R₁₂" refers to the nitrogen atom in -N=SR₁₁R₁₂ attached to C₀₋₈ alkyl, wherein the C₀₋₈ alkyl is defined as above.

"-C₀₋₈ alkyl-O-S(O)₂R₁₃" refers to the oxygen atom in -O-S(O)₂R₁₃ attached to C₀₋₈ alkyl, wherein the C₀₋₈ alkyl is defined as above.

"-C₀₋₈ alkyl-S(O)ᵣR₁₃" refers to the sulfur atom in -S(O)ᵣR₁₃ attached to C₀₋₈ alkyl, wherein the C₀₋₈ alkyl is defined as above.

"-C₀₋₈ alkyl-O-R₁₄" refers to the oxygen atom in -O-R₁₄ attached to C₀₋₈ alkyl, wherein the C₀₋₈ alkyl is defined as above.

"-C₀₋₈ alkyl-C(O)OR₁₄" refers to the carbonyl in -C(O)OR₁₄ attached to C₀₋₈ alkyl, wherein the C₀₋₈ alkyl is defined as above.

"-C₀₋₈ alkyl-C(O)SR₁₄" refers to the carbonyl in -C(O)SR₁₄ attached to C₀₋₈ alkyl, wherein the C₀₋₈ alkyl is defined as above.

"-C₀₋₈ alkyl-S-C(O)R₁₅" refers to the sulfur atom in -S-C(O)R₁₅ attached to C₀₋₈ alkyl, wherein the C₀₋₈ alkyl is defined as above.

"-C₀₋₈ alkyl-C(O)R₁₅" refers to the carbonyl in -C(O)R₁₅ attached to C₀₋₈ alkyl, wherein the C₀₋₈ alkyl is defined as above.

"-C₀₋₈ alkyl-O-C(O)R₁₅" refers to the oxygen atom in -O-C(O)R₁₅ attached to C₀₋₈ alkyl, wherein the C₀₋₈ alkyl is defined as above.

"-C₀₋₈ alkyl-P(O)(R₁₅)₂" refers to the phosphorus atom in -P(O)(R₁₅)₂ attached to C₀₋₈ alkyl, wherein the C₀₋₈ alkyl is defined as above.

"-C₀₋₈ alkyl-NR₁₆R₁₇" refers to the nitrogen atom in -NR₁₆R₁₇ attached to C₀₋₈ alkyl, wherein the C₀₋₈ alkyl is defined as above.

"-C₀₋₈ alkyl-C(=NR₁₆)R₁₅" refers to the carbon atom in -C(=NR₁₆)R₁₅ attached to C₀₋₈ alkyl, wherein the C₀₋₈ alkyl is defined as above.

"-C₀₋₈ alkyl-N(R₁₆)-C(=NR₁₇)R₁₅" refers to the nitrogen atom in -N(R₁₆)-C(=NR₁₇)R₁₅ attached to C₀₋₈ alkyl, wherein the C₀₋₈ alkyl is defined as above.

"-C₀₋₈ alkyl-C(O)NR₁₆R₁₇" refers to the carbonyl in -C(O)NR₁₆R₁₇ attached to C₀₋₈ alkyl, wherein the C₀₋₈ alkyl is defined as above.

"-C₀₋₈ alkyl-N(R₁₆)-C(O)R₁₅" refers to the nitrogen atom in -N(R₁₆)-C(O)R₁₅ attached to C₀₋₈ alkyl, wherein the C₀₋₈ alkyl is defined as above.

"C₁₋₁₀ haloalkyl" refers to an alkyl having 1-10 carbon atoms in which hydrogens on the alkyl are optionally substituted with a fluorine, chlorine, bromine and iodine atom, including but not limited to difluoromethyl, dichloromethyl, dibromomethyl, trifluoromethyl, trichloromethyl, tribromomethyl, etc.

"C₁₋₁₀ haloalkoxy" refers to an alkoxy having 1-10 carbon atoms in which hydrogens on the alkyl are optionally substituted with a fluorine, chlorine, bromine, and iodine atom, including but not limited to difluoromethoxy, dichloromethoxy, dibromomethoxy, trifluoromethoxy, trichloromethoxy, tribromomethoxy, etc.

"C₁₋₁₀ deuterioalkyl" refers to an alkyl having 1-10 carbon atoms in which hydrogens on the alkyl are optionally substituted with a deuterium atom, including but not limited to monodeuterium, dideuterium, trideuterium, etc.

"Halogen" refers to fluorine, chlorine, bromine, or iodine.

The term "optional" or "optionally" means that the event or circumstance subsequently described may, but not necessarily, occur, and that the description includes instances where the event or circumstance occurs or dose not occur, i.e., both substituted or unsubstituted instances. For example, "heterocyclyl group optionally substituted with alkyl" means that alkyl may be, but not necessarily, present, and that the description includes instances where the heterocyclyl group is and is not substituted with alkyl.

The term "substituted" means that one or more "hydrogen atoms" in a group are each independently substituted with a corresponding number of substituents. It goes without saying that a substituent is only in its possible chemical position and consistent with the valence bond theory in chemistry, and those skilled in the art will be able to determine (by experiments or theories) possible or impossible substitution without undue efforts. For example, it may be unstable when an amino or hydroxy having a free hydrogen is bound to a carbon atom having an unsaturated bond (such as olefin).

"Stereoisomer" refers to isomers produced by different spatial arrangements of atoms in the molecules. They can be divided into cis-trans isomers and enantiomers and can also be divided into two categories: enantiomers and diastereomers. A stereoisomer produced by the rotation of a single bond is referred to as a conformational stereo-isomer, sometimes also referred to as a rotamer. A stereoisomer produced by bond length, bond angle, double bonds in the molecule, ring and other reasons is referred to as a configuration stereo-isomer, which can be divided into two categories. An isomer produced by the inability of a double bond or a single bond of ring carbon atoms to rotate freely becomes a geometric isomer, also known as a cis-trans isomer, which can be divided into Z and E configurations. For example: cis-2-butene and trans-2-butene are a pair of geometric isomers. Stereoisomers with different optical properties produced by the absence of anti-axial symmetry in the molecules are called optical isomers, which can be divided into R and S configurations. "Stereoisomers" as described in the present invention, if not specifically indicated, are to be understood to include one or more of the enantiomers, configurational isomers, and conformational isomers described above.

"Pharmacologically acceptable salt" in the present invention refers to pharmaceutically acceptable acid addition salts, including inorganic and organic acid salts, which may be prepared by methods known in the art.

"Pharmaceutical composition" refers to a mixture containing one or more of the compounds described herein or a physiologically/pharmaceutically acceptable salt or pro-drug thereof, and other chemical components, for example physiologically/pharmaceutically acceptable carriers and excipients. The purpose of the pharmaceutical composition is to promote the administration to an organism, which facilitates the absorption of the active ingredient, thereby exerting biological activities.

### The present invention is further explained in detail below with reference to examples, which are not intended to limit the present invention, and the present invention is not merely limited to the contents of the examples.

The compound structure of the present invention is determined by nuclear magnetic resonance (NMR) or/and liquid chromatography-mass spectrometry (LC-MS). The NMR chemical shift (δ) is given in parts per million (ppm). The NMR determination is conducted by using a Bruker AVANCE-400/500 nuclear magnetic resonance apparatus, with hexadeuterodimethyl sulfoxide (DMSO-*d₆*), tetradeuteromethanol (CD₃OD) and deuterated chloroform (CDCl₃) as determination solvents, and tetramethylsilane (TMS) as internal standard.

The LC-MS determination is conducted by using an Agilent 6120 mass spectrometer. The HPLC determination is conducted by using an Agilent 1200 DAD high pressure liquid chromatography (Sunfire C18 150 × 4.6 mm chromatographic column) and a Waters 2695-2996 high pressure liquid chromatography (Gimini C18 150 × 4.6 mm chromatographic column).

Yantai Yellow Sea HSGF254 or Qingdao GF254 silica gel plate is adopted as a thin layer chromatography (TLC) silica gel plate. The specification adopted by the TLC is 0.15-0.20 mm, and the specification adopted by the thin layer chromatography for the separation and purification of products is 0.4-0.5 mm. The Yantai Yellow Sea silica gel of 200-300 mesh is generally utilized as a carrier in column chromatography.

Starting materials in the examples of the present invention are known and commercially available, or may be synthesized by using or according to methods known in the art.

Unless otherwise stated, all reactions of the present invention are carried out under a dry nitrogen or argon atmosphere with continuous magnetic stirring, wherein the solvent is a dry solvent and the reaction temperature is in degree centigrade (°C).

### I. Preparation of Intermediates

### Intermediate A1: Preparation of ((2-fluoro-6-(methoxymethoxy)-8-(4,4,5,5-tetramethyl-1,3,2-dioxaborolan-2-yl)nap hthalen-1-yl)ethynyl)triisopropylsilane

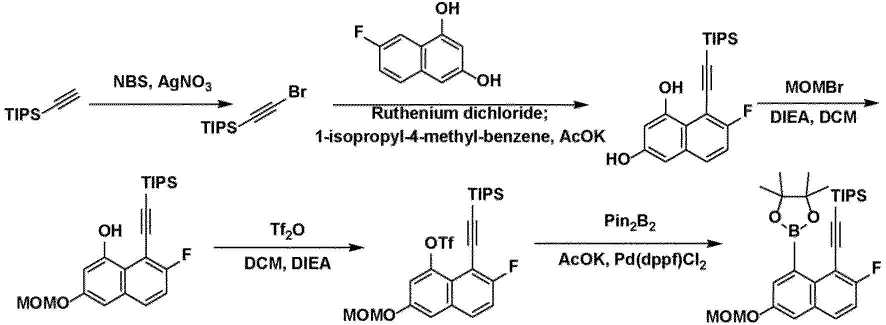

### Step 1: Synthesis of (bromoethynyl)triisopropylsilane

Ethynyltriisopropylsilane (15 g, 82.2 mmol) was dissolved in acetone (100 mL), and silver nitrate (1 g, 6.45 mmol) and N-bromosuccinimide (15 g, 86.3 mmol) was added, respectively. The reaction solution was reacted at room temperature for 1 hour under nitrogen protection. The solution was concentrated under reduced pressure to remove the solvent, and the remaining was added with 300 mL of petroleum ether, slurried and filtered, and the filtrate was concentrated under reduced pressure to remove the solvent to obtain (bromoethynyl)triisopropylsilane (21 g, yield: 97.7%).

¹H NMR (400MHz, CDCl₃) δ 1.10 (m, 21H).

### Step 2: Synthesis of 7-fluoro-8-((triisopropylsilyl)ethynyl)naphthalen-1,3-diol

7-fluoronaphthalen-1,3-diol (5 g, 28.1 mmol) was dissolved in 1,4-dioxane (200 mL), and (bromoethynyl)triisopropylsilane (8.06 g, 30.8 mmol), ruthenium dichloride, 1-isopropyl-4-methyl-benzene (1.2 g, 1.96 mmol) and potassium acetate (5.51 g, 56.2 mmol) were added under nitrogen protection, respectively. The reaction solution was reacted at 110°C for 18 hours under nitrogen protection. The reaction solution was filtered, the filtrate was concentrated under reduced pressure to remove the solvent, and the remaining was separated by using a fast silica gel column to obtain 7-fluoro-8-((triisopropylsilyl)ethynyl)naphthalen-1,3-diol (9.1 g, yield: 90.4%). ESI-MS: 359 [M+1]⁺.

### Step 3: Synthesis of 7-fluoro-3-(methoxymethoxy)-8-((triisopropylsilyl)ethynyl)naphthalen-1-ol

7-fluoro-8-((triisopropylsilyl)ethynyl)naphthalen-1,3-diol (9 g, 25.1 mmol) was dissolved in anhydrous dichloromethane (200 mL), and N,N-diisopropylethylamine (12.4 mL, 75.4 mmol) and bromo(methoxy)methane (4.1 g, 32.6 mmol) were added dropwise, respectively. The reaction solution was reacted at room temperature for 1 hour under nitrogen protection. The reaction solution was washed sequentially with saturated sodium bicarbonate aqueous solution (100 mL) and saturated sodium chloride aqueous solution (100 mL). The organic phase was dried over anhydrous sodium sulfate and concentrated under reduced pressure to remove the solvent, and the remaining was separated by using a fast silica gel column to obtain 7-fluoro-3-(methoxymethoxy)-8-((triisopropylsilyl)ethynyl)naphthalen-1-ol (7.5 g, yield: 74.2%).

¹H NMR (400MHz, CDCl₃) δ 9.12 (s, 1H), 7.65 (dd, *J* = 9.17, 5.62 Hz, 1H), 7.18 (t, *J* = 8.74 Hz, 1H), 6.96 (d, *J* = 2.45 Hz, 1H), 6.80 (d, *J*=1.83 Hz, 1H), 5.24 (s, 2H), 3.50 (s, 3 H), 1.17 - 1.23 (m, 21H).

### Step 4: Synthesis of 7-fluoro-3-(methoxymethoxy)-8-((triisopropylsilyl)ethynyl)naphthalen-1-yl trifluoromethanesulfonate

7-fluoro-3-(methoxymethoxy)-8-((triisopropylsilyl)ethynyl)naphthalen-1-ol (4 g, 9.94 mmol) was dissolved in anhydrous dichloromethane (60 mL), and N,N-diisopropylethylamine was added dropwise (4.93 mL, 29.8 mmol). The reaction solution temperature is reduced to -40°C under nitrogen protection. Trifluoromethanesulfonic anhydride (3.67 g, 14.9 mmol) was slowly added dropwise to the reaction solution. The reaction solution was reacted at -40°C for 0.5 hours. TLC detected that there would be a small amount of raw materials remaining. 200 mL of water was added to the reaction solution. The resulting solution was extracted with 100 mL of dichloromethane. The organic phase was washed with saturated sodium chloride aqueous solution (100 mL), dried over anhydrous sodium sulfate and concentrated under reduced pressure to remove the solvent, and the remaining was separated by using a fast silica gel column to obtain 7-fluoro-3-(methoxymethoxy)-8-((triisopropylsilyl)ethynyl)naphthalen-1-yl trifluoromethanesulfonate (3.05 g, yield: 57.4%).

### Step 5: Synthesis of ((2-fluoro-6-(methoxymethoxy)-8-(4,4,5,5-tetramethyl-1,3,2-dioxaborolan-2-yl)nap hthalen-1-yl)ethynyl)triisopropylsilane

7-fluoro-3-(methoxymethoxy)-8-((triisopropylsilyl)ethynyl)naphthalen-1-yl trifluoromethanesulfonate (5.1 g, 9.54 mmol) was dissolved in anhydrous toluene (100 mL), and bis(pinacolato)diboron (4.84 g, 19.1 mmol), potassium acetate (2.81 g, 28.6 mmol) and Pd(dppf)Cl₂(349 mg, 0.48 mmol) were added, respectively. The reaction solution was reacted under nitrogen protection at 130°C for 3 hours. The reaction solution was diluted with 200 mL ethyl acetate and washed with saturated sodium chloride aqueous solution (100 mL*2). The organic phase was dried over anhydrous sodium sulfate and concentrated under reduced pressure to remove the solvent, and the remaining was separated by using a fast silica gel column to obtain ((2-fluoro-6-(methoxymethoxy)-8-(4,4,5,5-tetramethyl-1,3,2-dioxaborolan-2-yl)naphtha len-1-yl)ethynyl)triisopropylsilane (2.6 g, yield: 53.2%).

¹H NMR (400MHz, CDCl₃) δ 7.66 (dd, *J* = 8.91, 5.65 Hz, 1H), 7.50 (d, *J* = 2.26 Hz, 1H), 7.37 (d, *J* = 2.51 Hz, 1H), 7.22 (t, *J* = 8.78 Hz, 1H), 5.27 (s, 2H), 3.50 (s, 3H), 1.43 (s, 12H), 1.16-1.22 (m, 21H).

### Intermediates A2-A3 can be prepared by selecting the corresponding raw materials with reference to all or part of the synthesis methods of intermediate A1:

| **Intermediate No.** | **Structural Formula** | **Chemical Name** | **ESI-MS: [M+1]⁺** |
|---|---|---|---|
| **A2** | 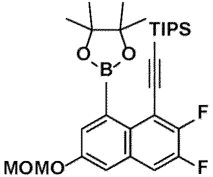 | ((2,3-difluoro-6-(methoxy methoxy)-8-(4,4,5,5-tetram ethyl-1,3,2-dioxaborolan-2-yl)naphthalen-1-yl)ethynyl) triisopropylsilane | 531 |
| **A3** | 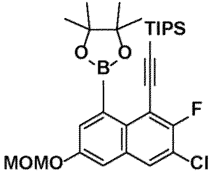 | ((3 -chloro-2-fluoro-6-(meth oxymethoxy)-8-(4,4, 5, 5-tet ramethyl-1,3,2-dioxaborola n-2-yl)naphthalen-1 -yl)ethy nyl)triisopropylsilane | 547 |

### Intermediate B: Preparation of tert-butyl 1-(methyl-d₃)-3,8-diazabicyclo[3.2.1]octan-8-carboxylate

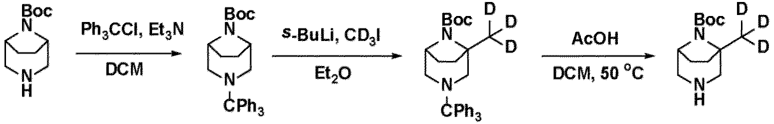

### Step 1: Synthesis of tert-butyl 3-triphenylmethyl-3,8-diazabicyclo[3.2.1]octan-8-carboxylate

At room temperature, tert-butyl 3,8-diazabicyclo[3.2.1]octan-8-carboxylate (7.0 g, 33.0 mmol) was dissolved in 100 mL dichloromethane, and triethylamine (4.6 mL, 33.0 mmol) and (chloromethanetriyl)tribenzene (9.2 g, 33.0 mmol) were added in sequence. After being stirred at room temperature for 24 hours, the solution was added with 2.8 g (chloromethanetriyl)tribenzene. It was again stirred at room temperature for 24 hours, and quenched by adding saturated sodium carbonate aqueous solution dropwise. Then, it was left to stand for layering. The aqueous phase was extracted with dichloromethane (50 mL* 2) and the organic phase was combined. The solution was washed sequentially with water (10 mL) and saturated sodium chloride aqueous solution (10 mL), dried over anhydrous sodium sulfate and concentrated, and the obtained crude product was separated by using a fast silica gel column [eluent: petroleum ether: ethyl acetate = 1: 1] to obtain tert-butyl 3-triphenylmethyl-3,8-diazabicyclo[3.2.1]octan-8-carboxylate (13.0 g, yield: 86%). ESI-MS: 243 [M+1]⁺.

### Step 2: Synthesis of tert-butyl 1-(methyl-d₃)-3-triphenylmethyl-3,8-diazabicyclo[3.2.1]octan-8-carboxylate

Under nitrogen protection, sec-butyllithium solution (3.6 mL, 4.6 mmol, 1.3 M) was dropwise added to anhydrous ether suspension (20 mL) of tert-butyl 3-triphenylmethyl-3,8-diazabicyclo[3.2.1]octan-8-carboxylate (900.0 mg, 2.0 mmol) and N,N,N',N'-tetramethylethylenediamine (526 mg, 4.6 mmol). The reaction temperature was maintained at around -30°C. After the dropwise addition, the reaction solution was stirred at 0°C for half an hour, and then deuterated iodomethane (287mg, 2.0 mmol) was added dropwise. After the dropwise addition, the reaction solution was further stirred at 0°C for 15 minutes. The saturated ammonium chloride aqueous solution (10 mL) was then added dropwise. The solution was left to stand for layering. The aqueous phase was extracted with dichloromethane (10 mL) and the organic phase was combined. The solution was washed sequentially with saturated sodium bicarbonate aqueous solution (10 mL), water (10 mL) and saturated sodium chloride aqueous solution (10 mL), dried over anhydrous sodium sulfate and filtered and concentrated. The obtained crude product was separated by using a fast silica gel column [eluent: petroleum ether: ethyl acetate = 100: 0-10: 1] to obtain tert-butyl 1-(methyl-*d*₃)-3-triphenylmethyl-3,8-diazabicyclo[3.2.1]octan-8-carboxylate (888.0 mg, yield: 77%). ESI-MS: 243 [M+1]⁺.

### Step 3: Synthesis of tert-butyl 1-(methyl-d₃)-3,8-diazabicyclo[3.2.1]octan-8-carboxylate

Tert-butyl 1-(methyl-*d*₃)-3-triphenylmethyl-3,8-diazabicyclo[3.2.1]octan-8-carboxylate (880 mg, 1.87 mmol) was dissolved in dichloromethane (5 mL), 5 mL acetic acid was added, and the solution was heated to 50°C and reacted for 1 hour, and was then spun dry to obtain tert-butyl 1-(methyl-*d*₃)-3,8-diazabicyclo[3.2.1]octan-8-carboxylate, which was used directly for the next reaction. ESI-MS: 230.4 [M+1]⁺.

### Intermediate C1: Preparation of tert-butyl 1-(methoxymethyl)-3,8-diazabicyclo [3.2.1] octan-8-carboxylate

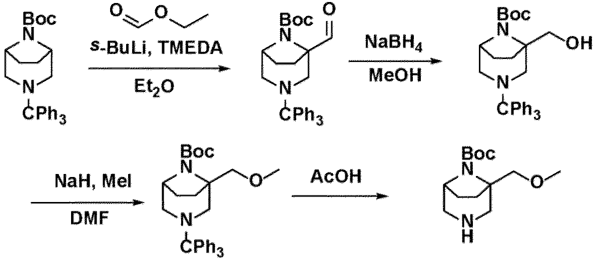

### Step 1: Synthesis of tert-butyl 1-formyl-3-triphenylmethyl-3,8-diazabicyclo[3.2.1]octan-8-carboxylate

Under nitrogen protection, sec-butyllithium solution (14.4 mL, 18.7 mmol, 1.3 M) was dropwise added to anhydrous ether suspension (40 mL) of tert-butyl 3-triphenylmethyl-3,8-diazabicyclo[3.2.1]octan-8-carboxylate (5.0 g, 11.0 mmol) and N,N,N',N'-tetramethylethylenediamine (2.2 g, 18.7 mmol). The reaction temperature was maintained at around 0°C. After the dropwise addition, the reaction solution was stirred at 0°C for 1.5 hours, and then ethyl formate (2.4 g, 33.0 mmol) was added dropwise. After the dropwise addition, the reaction solution was further stirred at 0°C for 15 minutes. The saturated ammonium chloride aqueous solution (20 mL) was then added dropwise. The solution was left to stand for layering. The aqueous phase was extracted with dichloromethane (10 mL) and the organic phase was combined. The solution was washed sequentially with saturated sodium bicarbonate aqueous solution (10 mL), water (10 mL) and saturated sodium chloride aqueous solution (10 mL), dried over anhydrous sodium sulfate and concentrated. The obtained crude product was separated by using a fast silica gel column [eluent: petroleum ether: ethyl acetate = 100: 0-10: 1] to obtain tert-butyl **1-formyl-3-triphenylmethyl-3,8-diazabicyclo[3.2.1]octan-8-carboxylate** (1.9 g, yield: 36%). ESI-MS: 483.2 [M+1]⁺.

### Step 2: Synthesis of tert-butyl 1-(hydroxymethyl)-3-triphenylmethyl-3,8-diazabicyclo [3.2.1]octan-8-carboxylate

Tert-butyl 1-formyl-3-triphenylmethyl-3,8-diazabicyclo[3.2.1]octan-8-carboxylate (560.0 mg, 1.2 mmol) was dissolved in methanol (10 mL), sodium borohydride (78.0 mg, 2.3 mmol) was added under the ice bath, and then the solution was stirred at room temperature for 1 hour until the reaction was completed. The solution was quenched by adding water (15 mL) and ethyl acetate (15 mL), and stirred at room temperature for 5 minutes. It was then left to stand for layering. The aqueous phase was extracted with ethyl acetate (10 mL*2) and the organic phase was combined. The solution was washed sequentially with saturated sodium bicarbonate aqueous solution (10 mL), water (10 mL) and saturated sodium chloride aqueous solution (10 mL), dried over anhydrous sodium sulfate and filtered and concentrated to obtain tert-butyl 1-(hydroxymethyl)-3-triphenylmethyl-3,8-diazabicyclo[3.2.1]octan-8-carboxylate (560.0 mg, yield: 91%). ESI-MS: 485.2 [M+1]⁺.

### Step 3: Synthesis of tert-butyl 1-(methoxymethyl)-3-triphenylmethyl-3,8-diazabicyclo [3.2.1] octan-8-carboxylate

Tert-butyl 1-(hydroxymethyl)-3-triphenylmethyl-3,8-diazabicyclo[3.2.1]octan-8-carboxylate (700 mg, 1.444 mmol) was dissolved in DMF (10 mL) solution, and sodium hydrogen (69.3 mg, 1.73 mmol) was added slowly at 0°C. After the reaction solution was stirred and reacted at 0°C for 2 hours, methyl iodide (270 µL, 4.33 mmol) was added. After the addition, the reaction lasted for 2 hours at 0°C. After the reaction was completed, water (20 mL) was added for quenching and the aqueous phase was extracted with ethyl acetate (20 mL*2). The combined organic phase was washed with saturated brine, dried over anhydrous magnesium sulfate, filtrated and concentrated under reduced pressure to remove the solvent. The remaining was separated with a rapid silica gel column to obtain tert-butyl 1-(methoxymethyl)-3-triphenylmethyl-3,8-diazabicyclo[3.2.1]octan-8-carboxylate (440 mg, yield: 61.1%).

### Step 4: Synthesis of tert-butyl 1-(methoxymethyl)-3,8-diazabicyclo [3.2.1] octan-8-carboxylate

Tert-butyl 1-(methoxymethyl)-3-triphenylmethyl-3,8-diazabicyclo[3.2.1]octan-8-carboxylate (440 mg, 0.88 mmol) was dissolved in acetic acid (5 mL). The mixture was reacted at 25°C for 2 hours. It was concentrated under reduced pressure to remove the solvent, to obtain tert-butyl 1-(methoxymethyl)-3,8-diazabicyclo[3.2.1]octan-8-carboxylate (220 mg, yield: 97.3 %). ESI-MS: 256 [M+1]⁺.

### Intermediates C2-C5 can be prepared by selecting the corresponding raw materials with reference to all or part of the synthesis methods of intermediate C1:

| **Intermediat e No.** | **Structural Formula** | **Chemical Name** | **ESI-MS: [M+1]⁺** |
|---|---|---|---|
| **C2** | 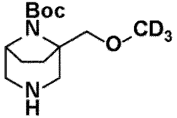 | tert-butyl 1-((methoxy-*d*₃)methyl)-3, 8-diazabicyclo[3.2.1]octan -8-carboxylate | 260 |
| **C3** | 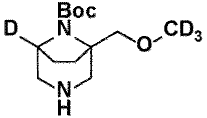 | tert-butyl 1-((methoxy-*d*₃)methyl)-3, 8-diazabicyclo[3.2.1]octan -8-carboxylate-5-*d* | 261 |
| **C4** | 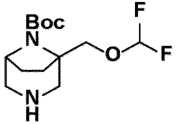 | tert-butyl 1-((difluoromethoxy)meth yl)-3,8-diazabicyclo[3.2. 1] octan-8-carboxylate | 293 |
| **C5** | 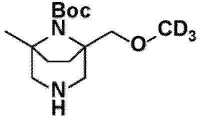 | tert-butyl 1-((methoxy-*d*₃)methyl)-5-methyl-3,8-diazabicyclo[3. 2.1] octan-8-carboxylate | 274 |

### Intermediate D1: Preparation of tert-butyl 1-vinyl-3,8-diazabicyclo[3.2.1]octan-8-carboxylate

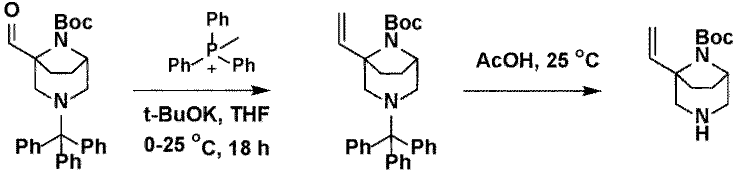

### Step 1: Synthesis of tert-butyl 3-triphenylmethyl-1-vinyl-3,8-diazabicyclo[3.2.1]octan-8-carboxylate

Methyl triphenylphosphane bromide cation (3.66 g, 10.3 mmol) was dissolved in anhydrous tetrahydrofuran (30 mL), and potassium tert-butoxide (1.27 g, 11.3 mmol) was added. The solution was stirred for 10 minutes at 0°C, and tetrahydrofuran solution (10 mL) of tert-butyl 1-formyl-3-triphenylmethyl-3,8-diazabicyclo[3.2.1]octan-8-carboxylate (1.65 g, 3.42 mmol) was slowly added. The mixture was reacted at 25°C for 2 hours. TLC detected that the reaction was complete. The reaction solution was diluted with 30 mL water, and extracted with 30 mL ethyl acetate twice. The organic phase was washed with 20 mL saturated brine once, dried over anhydrous sodium sulfate and concentrated under reduced pressure to remove the solvent. The remaining was separated by using a fast silica gel column to obtain tert-butyl 3-triphenylmethyl-1-vinyl-3,8-diazabicyclo[3.2.1]octan-8-carboxylate (1.19 g, yield: 72.4%).

¹H NMR (400 MHz, CDCl₃) δ 7.50 (br s, 5H), 7.33-7.25 (m, 7H), 7.19-7.09 (m, 3H), 6.16 (dd, J=11.2, 17.9 Hz, 1H), 4.93 (d, *J* = 11.3 Hz, 1H), 4.81 (d, *J* = 18.1 Hz, 1H), 4.16 (d, *J* = 5.5 Hz, 1H), 3.19 (d, *J* = 11.0 Hz, 1H), 2.96 (d, *J* = 11.0 Hz, 1H), 2.58-2.46 (m, 1H), 2.36-2.22 (m, 1H), 2.13-1.89 (m, 2H), 1.92 (d, *J* = 11.0 Hz, 1H), 1.84 (d, *J* = 11.0 Hz, 1H), 1.15 (s, 9H).

### Step 2: Synthesis of tert-butyl 1-vinyl-3,8-diazabicyclo[3.2.1]octan-8-carboxylate

Tert-butyl 3-triphenylmethyl-1-vinyl-3,8-diazabicyclo[3.2.1]octan-8-carboxylate (550 mg, 1.14 mmol) was dissolved in acetic acid (10 mL). The mixture was reacted overnight at 25°C. It was concentrated under reduced pressure to remove the solvent, to obtain tert-butyl 1-vinyl-3,8-diazabicyclo[3.2.1]octan-8-carboxylate (540 mg, crude product). The crude product can be directly used in the next reaction without separation. ESI-MS: 183 [M+1-56]⁺.

### Intermediates D2-D5 can be prepared by selecting the corresponding raw materials with reference to all or part of the synthesis methods of intermediate D1:

| **Intermediate No.** | **Structural Formula** | **Chemical Name** | **ESI-MS: [M+1]⁺** |
|---|---|---|---|
| **D2** | 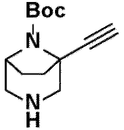 | tert-butyl 1-ethynyl-3, 8-diazabicyclo [3.2.1]octan-8-carboxylate | 237 |
| **D3** | 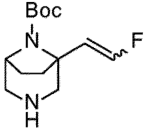 | tert-butyl 1-(2-fluorovinyl)-3,8-diaza bicyclo[3.2.1]octan-8-carb oxylate | 257 |
| **D4** | 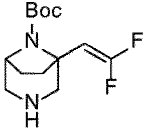 | tert-butyl 1-(2,2-difluorovinyl)-3,8-d iazabicyclo[3.2.1]octan-8-carboxylate | 275 |
| **D5** | 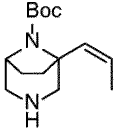 | tert-butyl 1-((Z)-prop-1-en-1-yl)-3,8 -diazabicyclo[3.2.1]octan-8-carboxylate | 253 |

### Intermediate E1: tert-butyl 1-ethyl-3,8-diazabicyclo[3.2.1]octan-8-carboxylate

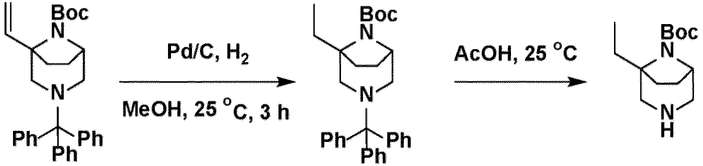

### Step 1: Synthesis of tert-butyl 1-ethyl-3-triphenylmethyl-3,8-diazabicyclo [3.2.1] octan-8-carboxylate

Tert-butyl 3-triphenylmethyl-1-vinyl-3,8-diazabicyclo[3.2.1]octan-8-carboxylate (550 mg, 1.14 mmol) was dissolved in anhydrous methanol solution (20 mL), and wet Pd/C (200 mg, purity: 10%) was added under nitrogen protection. The hydrogen substitution was carried out three times, and the mixture was reacted at 25°C for 3 hours under a hydrogen balloon atmosphere. TLC confirmed that the reaction was complete. The mixture was filtered by diatomite, and the filtrate was concentrated under reduced pressure to remove the solvent, to obtain tert-butyl 1-ethyl-3-triphenylmethyl-3,8-diazabicyclo[3.2.1]octan-8-carboxylate (550 mg, yield: 100%).

### Step 2: Synthesis of tert-butyl 1-ethyl-3,8-diazabicyclo [3.2.1] octan-8-carboxylate

Tert-butyl 1-ethyl-3 -triphenylmethyl-3,8-diazabicyclo[3.2.1]octan-8-carboxylate (550 mg, 1.13 mmol) was dissolved in acetic acid (10 mL). The mixture was reacted overnight at 25°C. It was concentrated under reduced pressure to remove the solvent, to obtain tert-butyl 1-ethyl-3,8-diazabicyclo[3.2.1]octan-8-carboxylate (540 mg, crude product). ESI-MS: 241 [M+1]⁺.

### Intermediates E2-E4 can be prepared by selecting the corresponding raw materials with reference to all or part of the synthesis methods of intermediate E1:

| **Intermediate No.** | **Structural Formula** | **Chemical Name** | **ESI-MS: [M+1]⁺** |
|---|---|---|---|
| **E2** | 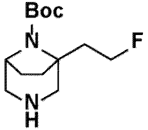 | tert-butyl 1-(2-fluoroethyl)-3,8-diaza bicyclo[3.2.1]octan-8-carb oxylate | 259 |
| **E3** | 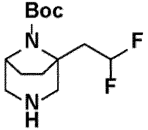 | tert-butyl 1-(2,2-difluoroethyl)-3,8-d iazabicyclo[3.2.1]octan-8-carboxylate | 277 |
| **E4** | 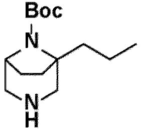 | tert-butyl 1-propyl-3,8-diazabicyclo[ 3.2.1]octan-8-carb oxylate | 255 |

### Intermediate F: Preparation of tert-butyl 1,5-dimethyl-3,8-diazabicyclo[3.2.1]octan-8-carboxylate

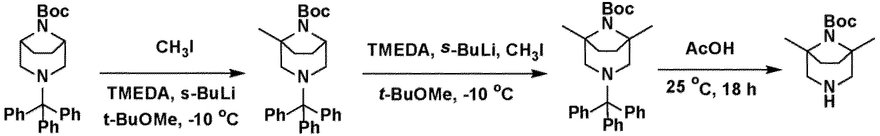

### Step 1: Synthesis of tert-butyl 1-methyl-3-triphenylmethyl-3,8-diazabicyclo [3.2.1] octan-8-carboxylate

Tert-butyl 3-triphenylmethyl-3,8-diazabicyclo[3.2.1]octan-8-carboxylate (5 g, 11.0 mmol) was dissolved in methyl tert-butyl ether solution (50 mL), tetramethylethylenediamine (4.15 mL, 27.5 mmol) was added to the mixture, and sec-butyllithium (21.1 mL, 27.5 mmol) was slowly added dropwise at -10°C. The mixture was reacted at 0°C for 2 hours. Methyl iodide (1.87 g, 13.2 mmol) was added to the mixture at 0°C. The mixture was reacted at 0°C for 2 hours. TLC confirmed that the reaction was complete. The reaction solution was quenched by slowly adding water dropwise, extracted with water and ethyl acetate, and washed with saturated sodium chloride aqueous solution. The organic phase was dried over anhydrous magnesium sulfate and concentrated under reduced pressure to remove the solvent. The remaining was separated by using a fast silica gel column to obtain tert-butyl 1-methyl-3-triphenylmethyl-3,8-diazabicyclo[3.2.1]octan-8-carboxylate (5 g, yield: 97.1%).

¹H NMR (400MHz, CDCl₃) δ 7.49 (s, 6H), 7.30-7.23 (m, 8H), 7.18-7.11 (m, 3H), 4.16 (d, *J* = 5.1 Hz, 1H), 3.00-2.90 (m, 1H), 2.81 (d, *J* = 11.0 Hz, 1H), 2.54-2.46 (m, 1H), 2.41-2.31 (m, 1H), 2.27-2.18 (m, 1H), 1.87-1.81 (m, 1H), 1.38 (s, 3H), 1.18 (s, 9H).

### Step 2: Synthesis of tert-butyl 1,5-dimethyl-3-triphenylmethyl-3,8-diazabicyclo[3.2.1]octan-8-carboxylate

Tert-butyl 1-methyl-3-triphenylmethyl-3,8-diazabicyclo[3.2.1]octan-8-carboxylate (2.5 g, 5.33 mmol) was dissolved in anhydrous methyl tert-butyl ether (40 mL) and tetramethylethylenediamine (1.55 g, 13.3 mmol) was added slowly under nitrogen protection at 0°C. Sec-butyllithium (10.2 mL, 13.3 mmol) was slowly added to this mixture under nitrogen protection at -10°C, and the mixture was reacted at -10°C for 1 hour. Methyl iodide (0.91 g, 6.40 mmol) was slowly added under nitrogen protection at -10-0°C, and the reaction was continued for 2 hours. LC-MS showed that the reaction was completed. The reaction solution was directly concentrated under reduced pressure to remove the solvent, to obtain the crude product tert-butyl 1,5-dimethyl-3-triphenylmethyl-3,8-diazabicyclo[3.2.1]octan-8-carboxylate (2 g), which was directly used for the next reaction. ESI-MS: 483 [M+1]⁺.

### Step 3: Synthesis of tert-butyl 1,5-dimethy-3,8-diazabicyclo [3.2.1] octan-8-carboxylate

Tert-butyl 1,5-dimethyl-3-triphenylmethyl-3,8-diazabicyclo[3.2.1]octan-8-carboxylate (1 g, 2.07 mmol) was dissolved in acetic acid (10 mL) and the mixture was reacted at 25°C for 18 hours. LC-MS showed that the reaction was completed. The reaction solution was directly concentrated under reduced pressure to remove the solvent, to obtain the crude product tert-butyl 1,5-dimethyl-3,8-diazabicyclo[3.2.1]octan-8-carboxylate (content: 500 mg), which was directly used for the next reaction. ESI-MS: 241 [M+1]⁺.

### Intermediate G: Preparation of tert-butyl 1-(1-hydroxyethyl)-3,8-diazabicyclo[3.2.1]octan-8-carboxylate

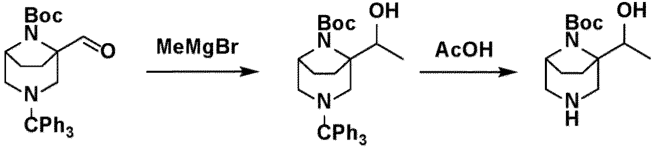

### Step 1: Synthesis of tert-butyl 1-(1-hydroxyethyl)-3-triphenylmethyl-3,8-diazabicyclo [3.2.1] octan-8-carboxylate

Tert-butyl 1-formyl-3-triphenylmethyl-3,8-diazabicyclo[3.2.1]octan-8-carboxylate (4 g, 8.29 mmol) was dissolved in tetrahydrofuran (40 mL), methyl magnesium bromide (8.29 ml, 16.58 mmol) was slowly added at -78°C and the reaction was carried out at room temperature for 2 hours. After the reaction was over, the solution was diluted with ethyl acetate, and the organic phase was separated and washed with saturated brine once. The organic phase was dried and filtered, the filtrate was concentrated, and the remaining was separated by using a fast silica gel column [ethyl acetate: petroleum ether = 0-50%] to obtain tert-butyl 1-(1-hydroxyethyl)-3-triphenylmethyl-3,8-diazabicyclo[3.2.1]octan-8-carboxylate (3.22 g, yield: 78%). ESI-MS: 499.29 [M+1]⁺.

### Step 2: Synthesis of tert-butyl 1-(1-hydroxyethyl)-3,8-diazabicyclo[3.2.1]octan-8-carboxylate

Tert-butyl 1-(1-hydroxyethyl)-3-triphenylmethyl-3,8-diazabicyclo[3.2.1]octan-8-carboxylate (3.22 g, 6.46 mmol) was dissolved in dichloromethane (20 mL) and acetic acid (20 ml), and the reaction was carried out at 50°C for 18 hours. After the reaction was over, the solution was directly concentrated to obtain tert-butyl 1-(1-hydroxyethyl)-3,8-diazabicyclo[3.2.1]octan-8-carboxylate (1.66 g, yield: 100%). ESI-MS: 257.18 [M+1]⁺.

### Intermediate H: Preparation of tert-butyl 1-((methylthio)methyl)-3,8-diazabicyclo[3.2.1]octan-8-carboxylate

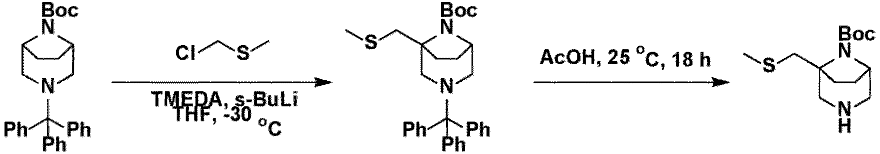

### Step 1: Synthesis of tert-butyl 1-((methylthio)methyl)-3-triphenylmethyl-3,8-diazabicyclo[3.2.1]octan-8-carboxyla te

Tert-butyl 3-triphenylmethyl-3,8-diazabicyclo[3.2.1]octan-8-carboxylate (5 g, 11.0 mmol) was dissolved in diethyl ether (100 mL), N,N,N,N-tetramethylethylenediamine (3.20 g, 27.5 mmol) was added at -10°C under nitrogen protection, and sec-butyllithium (1.3 M, 21.2 mL, 27.5 mmol) was slowly added dropwise. The mixture was stirred and reacted at -10°C for 2 hours, and then chloromethyl methyl sulfide (2.12 g, 22.0 mmol) was added dropwise. After being stirred and reacted at -10°C for 2 hours, the mixture was quenched with saturated ammonium chloride aqueous solution and extracted with ethyl acetate. The organic phase was washed with saturated sodium chloride aqueous solution, dried over anhydrous magnesium sulfate and concentrated under reduced pressure to remove the solvent. The remaining was separated by using a fast silica gel column to obtain tert-butyl 1-((methylthio)methyl)-3-triphenylmethyl-3,8-diazabicyclo[3.2.1]octan-8-carboxylate (1.2 g, yield: 21.2%).

¹H NMR (400 MHz, CDCl₃) δ 7.49 (d, *J* = 7.5 Hz, 5H), 7.34-7.23 (m, 7H), 7.20-7.12 (m, 3H), 4.33 (s, 1H), 4.00 (d, *J* = 2.6 Hz, 1H), 3.24 - 3.05 (m, 1H), 2.86 (t, *J* = 12.4 Hz, 1H), 2.59-2.44 (m, 2H), 2.22-2.09 (m, 4H), 1.79-1.64 (m, 1H), 1.57 (s, 3H), 1.40 (s, 9H).

### Step 2: Synthesis of tert-butyl 1-((methylthio)methyl)-3,8-diazabicyclo[3.2.1]octan-8-carboxylate

Tert-butyl 1-((methanesulfonyl)methyl)-3-triphenylmethyl-3,8-diazabicyclo[3.2.1]octan-8-carboxy late (500 mg, 0.97 mmol) was dissolved in acetic acid (10 mL). After being stirred and reacted at 25°C for 18 hours, the mixture was concentrated under reduced pressure to remove the solvent, with the remaining being the crude product tert-butyl 1-((methylthio)methyl)-3,8-diazabicyclo[3.2.1]octan-8-carboxylate (content: 250 mg, yield: 96.2%). ESI-MS: 273 [M+1]⁺.

### Intermediate I: Preparation of tert-butyl 1-((methanesulfonyl)methyl)-3,8-diazabicyclo[3.2.1]octan-8-carboxylate

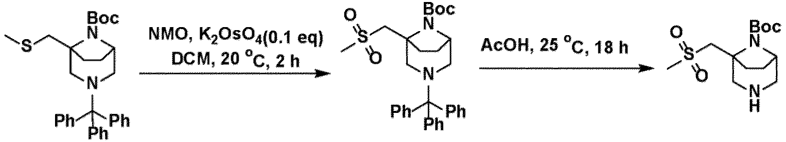

### Step 1: Synthesis of tert-butyl 1-((methanesulfonyl)methyl)-3-triphenylmethyl-3,8-diazabicyclo [3.2.1] octan-8-car boxylate

Tert-butyl 1-((methylthio)methyl)-3-triphenylmethyl-3,8-diazabicyclo[3.2.1]octan-8-carboxylate (660 mg, 1.28 mmol) was dissolved in dichloromethane (20 mL), and N-methylmorpholine oxide (751 mg, 6.41 mmol) and potassium osmate dihydrate (47.2 mg, 0.13 mmol) were added at -40°C under nitrogen protection. The mixture was stirred and reacted at 25°C for 2 hours, quenched with saturated sodium hydroxide aqueous solution, extracted with water and dichloromethane, and washed with saturated sodium chloride aqueous solution. The organic phase was dried over anhydrous magnesium sulfate and concentrated under reduced pressure to remove the solvent. The remaining was tert-butyl 1-((methanesulfonyl)methyl)-3-triphenylmethyl-3,8-diazabicyclo[3.2.1]octan-8-carboxy late (600 mg, yield: 85.6%). The crude product was directly used for the next reaction.

### Step 2: Synthesis of tert-butyl 1-((methanesulfonyl)methyl)-3,8-diazabicyclo[3.2.1]octan-8-carboxylate

Tert-butyl 1-((methanesulfonyl)methyl)-3-triphenylmethyl-3,8-diazabicyclo[3.2.1]octan-8-carboxy late (700 mg, 1.28 mmol) was dissolved in acetic acid (10 mL). After being stirred and reacted at 25°C for 18 hours, the mixture was concentrated under reduced pressure to remove the solvent, with the remaining being the crude product tert-butyl 1-((methanesulfonyl)methyl)-3,8-diazabicyclo[3.2.1]octan-8-carboxylate (700 mg, yield: 89.8%). ESI-MS: 305 [M+1]⁺.

### Intermediate J: Preparation of 8-(tert-butyl) 1-methyl 3,8-diazabicyclo[3.2.1]octan-1,8-dicarboxylate

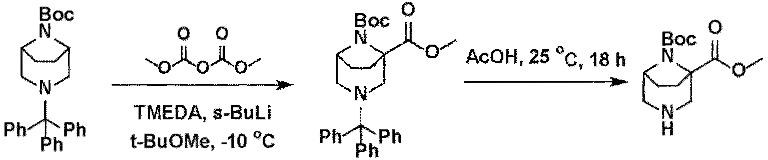

### Step 1: Synthesis of 8-(tert-butyl) 1-methyl 3-triphenylmethyl-3,8-diazabicyclo [3.2.1] octan-1,8-dicarboxylate

Tert-butyl 3-triphenylmethyl-3,8-diazabicyclo[3.2.1]octan-8-carboxylate (5 g, 11.0 mmol) was dissolved in methyl tert-butyl ether solution (60 mL), tetramethylethylenediamine (4.15 mL, 27.5 mmol) was added to the mixture, and sec-butyllithium (21.2 mL, 27.5 mmol) was slowly added dropwise at -10°C. The mixture was reacted at 0°C for 2 hours. Dimethyl dicarbonate (2.95 g, 22.0 mmol) was added to the mixture at 0°C. The mixture was reacted at 0°C for 2 hours. TLC confirmed that the reaction was complete. The reaction solution was quenched by slowly adding water dropwise, diluted with 50 mL water, and extracted with 50 mL ethyl acetate twice. The organic phase was washed with 50 mL saturated brine once, dried over anhydrous magnesium sulfate and concentrated under reduced pressure to remove the solvent. The remaining was separated by using a fast silica gel column to obtain 8-(tert-butyl) 1-methyl 3-triphenylmethyl-3,8-diazabicyclo[3.2.1]octan-1,8-dicarboxylate (3.2 g, yield: 56.7%).

### Step 2: Synthesis of 8-(tert-butyl) 1-methyl 3,8-diazabicyclo[3.2.1]octan-1,8-dicarboxylate

8-(tert-butyl) 1-methyl (1S,5R)-3-triphenylmethyl-3,8-diazabicyclo[3.2.1]octan-1,8-dicarboxylate (1.5 g, 2.93 mmol) was dissolved in acetic acid (15 mL). The mixture was reacted at 25°C for 2 hours. It was concentrated under reduced pressure to remove the solvent, to obtain the crude product 8-(tert-butyl) 1-methyl 3,8-diazabicyclo[3.2.1]octan-1,8-dicarboxylate (content: 0.79 g, yield: 99.9%).

### Intermediate K1: Preparation of 1-(ethoxymethyl)-3,8-diazabicyclo[3.2.1]octan

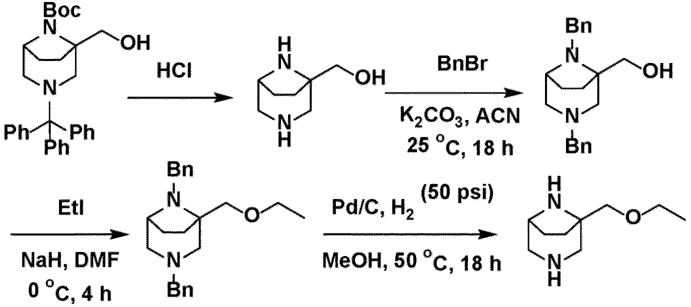

### Step 1: Synthesis of (3,8-diazabicyclo[3.2.1]octan-1-yl)methanol

Tert-butyl 1-(hydroxymethyl)-3-triphenylmethyl-3,8-diazabicyclo[3.2.1]octan-8-carboxylate (4 g, 8.25 mmol) was added to hydrochloric acid ethyl acetate solution (100 mL, 4 M). The mixture was stirred and reacted for 2 hours at room temperature, and solids were precipitated, filtered and collected to obtain (3,8-diazabicyclo[3.2.1]octan-1-yl)methanol (1.78 g, hydrochloride, yield: 100%). ESI-MS: 143 [M+1]⁺.

### Step 2: Synthesis of (3,8-diphenylmethyl-3,8-diazabicyclo[3.2.1]octan-1-yl)methanol

(3,8-diazabicyclo[3.2.1]octan-1-yl)methanol (1.78 g, hydrochloride, 8.25 mmol) and potassium carbonate (3.43 g, 24.8 mmol) were added to acetonitrile (60 mL), and benzyl bromide (3.54 g, 20.7 mmol) was added at room temperature. The reaction solution was stirred at room temperature overnight. The reaction mixture was extracted with water (100 mL) and ethyl acetate (100 mL). The organic phase was adjusted to pH = 1-2 with dilute hydrochloric acid, and extracted with 2 M dilute hydrochloric acid (50 mL). The separated organic phase was discarded. The aqueous phase was neutralized to weak alkalinity with 2 N sodium hydroxide aqueous solution, and extracted with ethyl acetate (100 mL). The extract was dried over magnesium sulfate, and concentrated under reduced pressure to remove the solvent, to obtain (3,8-diphenylmethyl-3,8-diazabicyclo[3.2.1]octan-1-yl)methanol (2.5 g, yield: 93.6%). ESI-MS: 323 [M+1]⁺.

### Step 3: Synthesis of 3,8-diphenylmethyl-1-(ethoxymethyl)-3,8-diazabicyclo[3.2.1]octan

(3,8-diphenylmethyl-3,8-diazabicyclo[3.2.1]octan-1-yl)methanol (0.5 g, 1.551 mmol) was dissolved in N,N-dimethylformamide (15 mL) solution, and sodium hydrogen (0.04 g, purity: 60%, 0.931 mmol) was added slowly at 0°C under nitrogen protection. The mixture was reacted at 0°C for 2 hours. Ethyl iodide (0.12 mL, 1.55 mmol) was added at 0°C. The mixture continued to react for 2 hours at 0°C. TLC confirmed that the reaction was complete. The reaction solution was quenched by slowly adding water dropwise, diluted with 30 mL water, and extracted with 20 mL ethyl acetate twice. The organic phase was washed with 30 mL saturated brine once, dried over anhydrous magnesium sulfate and concentrated under reduced pressure to remove the solvent. The remaining was separated by using a fast silica gel column to obtain 3,8-diphenylmethyl-1-(ethoxymethyl)-3,8-diazabicyclo[3.2.1]octan (510 mg, yield: 93.8%).

### Step 4: Synthesis of 1-(ethoxymethyl)-3,8-diazabicyclo[3.2.1]octan

3,8-diphenylmethyl-1-(ethoxymethyl)-3,8-diazabicyclo[3.2.1]octan (500 mg, 1.45 mmol) was dissolved in methanol (25 mL), and concentrated hydrochloric acid (1 mL) and wet palladium on carbon (200 mg, purity 10%) were added to the mixture. The mixture was reacted at 50°C under hydrogen (50 psi) for 18 hours. Palladium on carbon was filtered out with diatomite, and the filtrate was concentrated under reduced pressure to obtain 1-(ethoxymethyl)-3,8-diazabicyclo[3.2.1]octan (240 mg, yield: 96.9%). ESI-MS: 171 [M+1]⁺.

### Intermediates K2-K3 can be prepared by selecting the corresponding raw materials with reference to all or part of the synthesis methods of intermediate K1:

| **Intermediate No.** | **Structural Formula** | **Chemical Name** | **ESI-MS: [M+1]⁺** |
|---|---|---|---|
| **K2** | 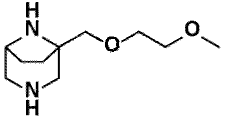 | 1-((2-methoxyethox y)methyl)-3,8-diazab icyclo[3.2.1]octan | 201 |
| **K3** | 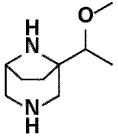 | 1-(1-methoxyethyl)-3,8-diazabicyclo[3.2. 1]octan | 171 |

### Intermediate L: Preparation of tert-butyl 1-(2-methoxyethyl)-3,8-diazabicyclo[3.2.1]octan-8-carboxylate

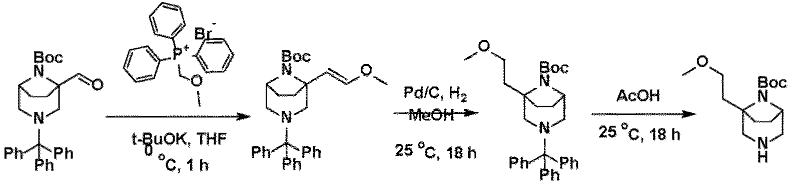

### Step 1: Synthesis of tert-butyl 1-((E)-2-methoxyvinyl)-3-triphenylmethyl-3,8-diazabicyclo [3.2.1] octan-8-carboxyla te

(Methoxymethyl)triphenylphosphonium bromide (2.13 g, 6.21 mmol) was dissolved in anhydrous tetrahydrofuran (20 mL) and potassium tert-butoxide (0.77 g, 6.83 mmol) was added slowly under nitrogen protection at 0°C. The mixture was reacted at 0°C for 10 minutes. Tert-butyl 1-formyl-3-triphenylmethyl-3,8-diazabicyclo[3.2.1]octan-8-carboxylate (1 g, 2.07 mmol) was then dissolved in anhydrous tetrahydrofuran (5 mL), and the mixture was slowly added to the reaction solution. The reaction solution was reacted at 0°C for 1 hour. The reaction solution was diluted with water (30 mL), and extracted with ethyl acetate twice (20 mL*2). The organic phase was dried over anhydrous sodium sulfate, and concentrated under reduced pressure to remove the solvent. The remaining was separated by using a fast silica gel column to obtain tert-butyl 1-((E)-2-methoxyvinyl)-3-triphenylmethyl-3,8-diazabicyclo[3.2.1]octan-8-carboxylate (900 mg, yield: 84.9%).

### Step 2: Synthesis of tert-butyl 1-(2-methoxyethyl)-3-triphenylmethyl-3,8-diazabicyclo[3.2.1]octan-8-carboxylate

Tert-butyl 1-((E)-2-methoxyvinyl)-3-triphenylmethyl-3,8-diazabicyclo[3.2.1]octan-8-carboxylate (0.5 g, 0.98 mmol) was dissolved in anhydrous methanol (10 mL), 10% wet palladium on carbon (1.04 g, 0.98 mmol) was added slowly under argon protection, and the hydrogen substitution was carried out three times. The mixture was reacted overnight at 25°C. The reaction solution was filtered with diatomite, and the filtrate was concentrated under reduced pressure to obtain the product tert-butyl 1-(2-methoxyethyl)-3-triphenylmethyl-3,8-diazabicyclo[3.2.1]octan-8-carboxylate (450 mg, crude product).

### Step 3: Synthesis of tert-butyl 1-(2-methoxyethyl)-3,8-diazabicyclo[3.2.1]octan-8-carboxylate

Tert-butyl 1-(2-methoxyethyl)-3-triphenylmethyl-3,8-diazabicyclo[3.2.1]octan-8-carboxylate (450 mg, 0.88 mmol) was dissolved in acetic acid (5 mL), and the mixture was reacted overnight at 25°C. The reaction solution was concentrated under reduced pressure to remove the solvent, to obtain tert-butyl 1-(2-methoxyethyl)-3,8-diazabicyclo[3.2.1]octan-8-carboxylate (content: 200 mg, crude product).

### Intermediate M1: Preparation of tert-butyl 1-((N-methylacetamido)methyl)-3,8-diazabicyclo[3.2.1]octan-8-carboxylate

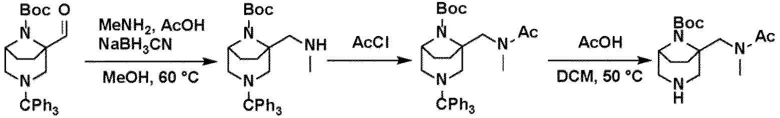

### Step 1: Synthesis of tert-butyl 1-((methylamino)methyl)-3-triphenylmethyl-3,8-diazabicyclo[3.2.1]octan-8-carbox ylate

Tert-butyl 1-formyl--3-triphenylmethyl-3,8-diazabicyclo[3.2.1]octan-8-carboxylate (600 mg, 1.24 mmol) was dissolved in MeOH (30 mL), and methylamine hydrochloride (251.83 mg, 3.73 mmol), diisopropylethylamine (0.62 mL, 3.73 mmol) and acetic acid (0.07 mL, 1.24 mmol) were added. the solution was stirred at room temperature. The reaction solution was stirred and reacted at 60°C for 2 hours. It was cooled to room temperature, then sodium cyanoborohydride (234.37 mg, 3.73 mmol) was added, and it was warmed to 60°C, stirred and reacted for 1 hour. LCMS monitored the reaction, showing that the starting materials had been reacted and the product was formed. The reaction solution was cooled to room temperature, sodium bicarbonate aqueous solution (25 mL) was added to it, and it was concentrated under reduced pressure to remove MeOH. The aqueous phase was extracted with ethyl acetate. The organic phase was extracted and combined, dried over anhydrous sodium sulfate and filtered, and the filtrate was concentrated. The remaining was separated by using a fast silica gel column [silicon dioxide, petroleum ether solution of 0-100% ethyl acetate] to obtain tert-butyl 1-((methylamino)methyl)-3-triphenylmethyl-3,8-diazabicyclo[3.2.1]octan-8-carboxylate (450 mg, 0.90 mmol, yield: 67.14%). ESI-MS: 498.4 [M+1]⁺.

### Step 2: Synthesis of tert-butyl 1-((N-methylacetamido)methyl)-3-triphenylmethyl-3,8-diazabicyclo[3.2.1]octan-8-c arboxylate

Tert-butyl 1-((methylamino)methyl)-3-triphenylmethyl-3,8-diazabicyclo[3.2.1]octan-8-carboxylate (450 mg, 0.90 mmol) was dissolved in DCM (5 mL). While being cooled with ice water and stirred, the reaction solution was added with diisopropylethylamine (0.30 mL, 1.81 mmol) and acetylchloride (0.08 mL, 1.09 mmol) sequentially. The reaction solution was stirred and reacted at 0°C under nitrogen ball protection for 1 hour. TLC showed that the raw materials had been reacted completely. The reaction solution was quenched by adding 10 mL water, and the aqueous phase was extracted with dichloromethane. The organic phase was extracted, dried over anhydrous sodium sulfate and filtered, and the filtrate was concentrated. The remaining was separated by using a fast silica gel column [silicon dioxide, petroleum ether solution of 50% ethyl acetate] to obtain tert-butyl 1-((N-methylacetamido)methyl)-3-triphenylmethyl-3,8-diazabicyclo[3.2.1]octan-8-carb oxylate (450 mg, 0.83 mmol, yield: 92.21%). ESI-MS 562.3 [M+23]⁺.

### Step 3: Synthesis of tert-butyl 1-((N-methylacetamido)methyl)-3,8-diazabicyclo [3.2.1] octan-8-carboxylate

Tert-butyl 1-((N-methylacetamido)methyl)-3-triphenylmethyl-3,8-diazabicyclo[3.2.1]octan-8-carb oxylate (450 mg, 0.83 mmol) was dissolved in 5 mL dichloromethane, and acetic acid (5 mL, 87.26 mmol) was added. The reaction solution was warmed to 50°C, stirred and reacted for 3 hours under nitrogen ball protection. TLC monitored the reaction, showing that the raw materials had been reacted completely. The reaction solution was concentrated to obtain tert-butyl 1-((N-methylacetamido)methyl)-3,8-diazabicyclo[3.2.1]octan-8-carboxylate (700 mg, 0.824 mmol, yield: 98.81%), and the crude product was used directly in the next step. ESI-MS: 298.3 [M+1]⁺.

### Intermediates M2-M3 can be prepared by selecting the corresponding raw materials with reference to all or part of the synthesis methods of intermediate M1:

| **Intermediate No.** | **Structural Formula** | **Chemical Name** | **ESI-MS: [M+1]⁺** |
|---|---|---|---|
| **M2** | 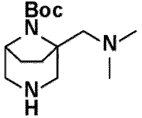 | tert-butyl 1-((dimethylamino)met hyl)-3,8-diazabicyclo[3. 2.1]octan-8-carboxylate | 270 |
| **M3** | 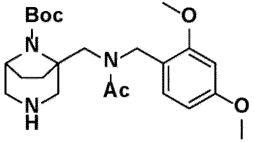 | tert-butyl 1-((N-(2,4-dimethoxybe nzyl)acetamido)methyl) -3,8-diazabicyclo[3.2.1] octan-8-carboxylate | 434 |

### Intermediate N: Preparation of methyl 2-(3,8-diazabicyclo[3.2.1]octan-1-yl)acetate

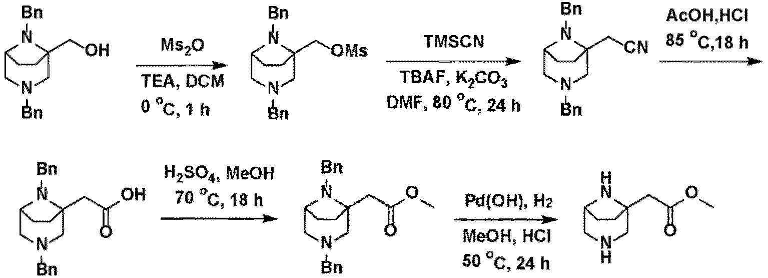

### Step 1: Synthesis of (3,8-diphenylmethyl-3,8-diazabicyclo[3.2.1]octan-1-yl)methyl methanesulfonate

(3,8-diphenylmethyl-3,8-diazabicyclo[3.2.1]octan-1-yl)methanol (1.6 g, 4.96 mmol) and triethylamine (2.76 mL, 20.0 mmol) were dissolved in dichloromethane (50 mL). Methyl sulfonic anhydride (0.95 mL, 7.44 mmol) was slowly added dropwise at 0°C. The reaction mixture was stirred and reacted for 1 hour at 0°C. The reaction mixture was extracted with water (100 mL) and dichloromethane (100 mL*3). The organic phase was separated, dried over magnesium sulfate, filtrated and concentrated under reduced pressure. The remaining was separated by using a fast silica gel column and the organic phase was concentrated under reduced pressure to remove the solvent, to obtain (3,8-diphenylmethyl-3,8-diazabicyclo[3.2.1]octan-1-yl)methyl methanesulfonate (1.8 g, yield: 90.5%). ESI-MS: 401 [M+1]⁺.

### Step 2: Synthesis of 2-(3,8-diphenylmethyl-3,8-diazabicyclo[3.2.1]octan-1-yl)acetonitrile

(3,8-diphenylmethyl-3,8-diazabicyclo[3.2.1]octan-1-yl)methyl methanesulfonate (4.3 g, 10.7 mmol), potassium carbonate (2.97 g, 21.5 mmol), trimethylsilyl cyanide (5.73 mL, 42.9 mmol) and tetrabutylammonium fluoride (1 M tetrahydrofuran solution, 42.9 mL, 42.9 mmol) were dissolved in N,N-dimethylformamide (60 mL). The reaction solution was stirred and reacted for 24 hours at 80°C. The reaction mixture was extracted with water (400 mL) and ethyl acetate (400 mL*3). The organic phase was separated, dried over magnesium sulfate, filtrated and concentrated under reduced pressure. The crude product was separated by using a fast silica gel column and the organic phase was concentrated under reduced pressure to remove the solvent, to obtain 2-(3,8-diphenylmethyl-3,8-diazabicyclo[3.2.1]octan-1-yl)acetonitrile (1.9 g, yield: 53.4%). ESI-MS: 332 [M+1]⁺.

### Step 3: Synthesis of 2-(3,8-diphenylmethyl-3,8-diazabicyclo[3.2.1]octan-1-yl)acetic acid

2-(3,8-diphenylmethyl-3,8-diazabicyclo[3.2.1]octan-1-yl)acetonitrile (1.9 g, 5.73 mmol) was dissolved in the mixture of acetic acid (10 mL) and concentrated hydrochloric acid (3 mL). The reaction solution was stirred and reacted at 85°C for 18 hours, and then concentrated under reduced pressure to remove the solvent, to obtain 2-(3,8-diphenylmethyl-3,8-diazabicyclo[3.2.1]octan-1-yl)acetic acid (2 g, yield: 99.6%). ESI-MS: 351 [M+1]⁺.

### Step 4: Synthesis of methyl 2-(3,8-diphenylmethyl-3,8-diazabicyclo[3.2.1]octan-1-yl)acetate

2-(3,8-diphenylmethyl-3,8-diazabicyclo[3.2.1]octan-1-yl)acetic acid (1.3 g, 3.71 mmol) was dissolved in the mixture of methanol (20 mL) and concentrated sulfuric acid (0.36 g, 3.71 mmol). The reaction solution was stirred and reacted at 70°C for 18 hours. The organic phase was concentrated under reduced pressure to remove the solvent, and the solution was extracted with water (30 mL) and ethyl acetate (30 mL*3). The organic phase was separated, dried over magnesium sulfate, filtrated and concentrated under reduced pressure. The crude product was separated by using a fast silica gel column and the organic phase was concentrated under reduced pressure to remove the solvent, to obtain methyl 2-(3,8-diphenylmethyl-3,8-diazabicyclo[3.2.1]octan-1-yl)acetate (1.3 g, yield: 96.2%). ESI-MS: 365 [M+1]⁺.

### Step 5: Synthesis of methyl 2-(3,8-diazabicyclo[3.2.1]octan-1-yl)acetate

Methyl 2-(3,8-diphenylmethyl-3,8-diazabicyclo[3.2.1]octan-1-yl)acetate (1.4 g, 3.84 mmol) and palladium hydroxide/carbon (0.8 g, purity: 10%) and a catalytic amount of hydrochloric acid were dissolved in methanol (20 mL). The reaction solution was stirred and reacted in hydrogen (50 Psi) atmosphere at 50°C for 24 hours. It was filtered with diatomite, and the filtrate was concentrated under reduced pressure to remove the solvent, to obtain methyl 2-(3,8-diazabicyclo[3.2.1]octan-1-yl)acetate (707 mg, yield: 99.9%), which was directly used in the next step. ESI-MS: 185 [M+1]⁺.

### Intermediate O: Preparation of 2,4,7-trichloro-8-fluoropyrido[4,3-d]pyrimidin

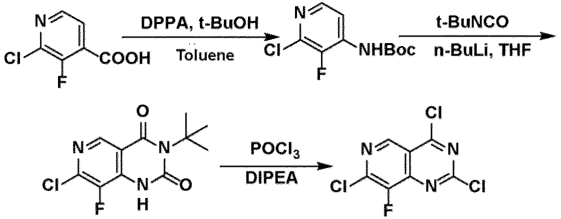

### Step 1: Synthesis of tert-butyl (2-chloro-3-fluoropyridin-4-yl)carbamate

2-chloro-3-fluoroisonicotinic acid (90 g, 513 mmol) was dissolved in toluene (675 mL) and tert-butanol (675 mL). Triethylamine (142 mL, 1.03 mol), diphenylphosphoryl azide (116 mL, 538 mmol) and Boc anhydride (11.0 mL, 51.3 mmol) were added, respectively. Under nitrogen protection, the reaction solution was reacted at room temperature for 0.5 hours, and then reacted at 80°C for 6 hours. After the reaction was completed, the solution was cooled to room temperature, diluted with 800 mL water, and extracted with ethyl acetate (500 mL*2). The organic phase was dried over magnesium sulfate, and concentrated under reduced pressure to remove the solvent. The remaining was separated by using a fast silica gel column to obtain tert-butyl (2-chloro-3-fluoropyridin-4-yl)carbamate (105 g, yield: 83.0%). ESI-MS: 247 [M+1]⁺.

### Step 2: Synthesis of 3-(tert-butyl)-7-chloro-8-fluoropyrido[4,3-d]pyrimidin-2,4(1H,3H)-dione

Tert-butyl (2-chloro-3-fluoropyridin-4-yl)carbamate (30 g, 122 mmol) was dissolved in tetrahydrofuran (300 mL), and n-butyllithium (121 mL, 304 mmol) was added dropwise at -78°C under nitrogen protection. After the dropwise addition, the temperature of the reaction solution was slowly raised to -20°C, and it was reacted at this temperature for 1 hour. Tert-butyl isocyanate (14.3 mL, 243 mmol) was then added dropwise to the reaction solution at -20°C. The temperature of the reaction solution was slowly raised to room temperature and reacted for 1 hour, then heated to 70°C and reacted overnight. The reaction solution was slowly added with saturated sodium bicarbonate aqueous solution (300 mL), and extracted with ethyl acetate (500 mL*2). The organic phase was dried over magnesium sulfate, and concentrated under reduced pressure to remove the solvent. The remaining was separated by using a fast silica gel column to obtain 3-(tert-butyl)-7-chloro-8-fluoropyrido[4,3-d]pyrimidin-2,4(1H,3H)-dione (248 g, yield: 72.6%).

### Step 3: Synthesis of 2,4,7-trichloro-8-fluoropyrido[4,3-d]pyrimidin

3-(tert-butyl)-7-chloro-8-fluoropyrido[4,3-d]pyrimidin-2,4(1H,3H)-dione (24 g, 88.3 mmol) was added slowly in batches to phosphorus oxychloride (300 mL), and N,N-diisopropylethylamine (43.8 mL, 265 mmol) was added slowly dropwise. The mixture was reacted overnight at 110°C. The majority of the phosphorus oxychloride was evaporated under reduced pressure. The remaining was slowly added with ice saturated sodium bicarbonate solution (300 mL), and extracted with ethyl acetate (500 mL*2). The organic phase was washed with saturated sodium chloride aqueous solution (100 mL), dried over anhydrous sodium sulfate and concentrated under reduced pressure to remove the solvent. The remaining was slurried in 200 mL petroleum ether, the mixture was filtered, and the filter cake was dried to obtain 2,4,7-trichloro-8-fluoropyrido[4,3-d]pyrimidin (14.8 g, yield: 66.6%).

¹H NMR (400MHz, DMSO-*d*₆) δ 8.94 (s, 1 H).

### Intermediate P: Preparation of 5,7-dichloro-8-fluoro-2-(methylthio)pyrido [4,3-d] pyrimidin-4(3H)-one

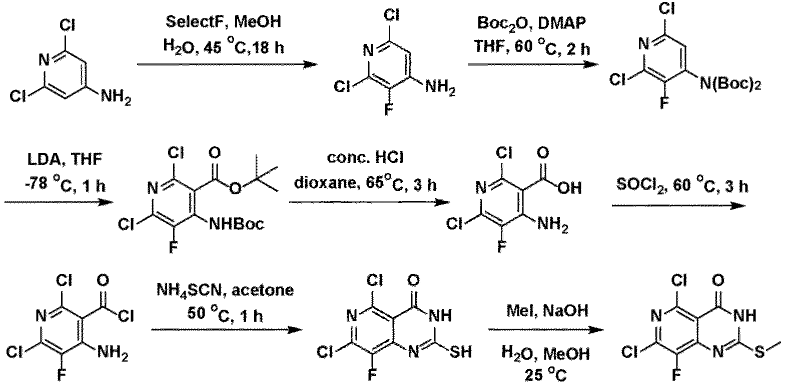

### Step 1: Synthesis of 2,6-dichloro-3-fluoropyridin-4-amine

2,6-dichloropyridin-4-amine (50 g, 306 mmol) was dissolved in methanol (1.25 L) and water (250 mL), and then 1-chloromethyl-4-fluoro-1,4-diazoniabicyclo[2.2.2]octan bis(tetrafluoroborate) was added (245 g, 690 mmoL). The mixture was reacted at 45°C for 18 hours, and LC-MS showed that the reaction was completed. The reaction solution was filtered, the filtrate was spin-dried, and then ethyl acetate (500 mL) was added. The mixture was extracted with water (300 mL). The organic phase was washed with saturated sodium chloride aqueous solution (200 mL), dried over anhydrous sodium sulfate and concentrated under reduced pressure to remove the solvent, to obtain 2,6-dichloro-3-fluoropyridin-4-amine (18.2 g, yield: 32.4%). ESI-MS: 181 [M+1]⁺.

### Step 2: Synthesis of tert-butyl (tert-butoxycarbonyl)(2,6-dichloro-3-fluoropyridin-4-yl)carbamate

2,6-dichloro-3-fluoropyridin-4-amine (18.2 g, 101 mmol) was dissolved in tetrahydrofuran (250 mL), and 4-dimethylaminopyridin (0.62 g, 5.04 mmol) and di-tert-butyl dicarbonate (53.9 mL, 251 mmol) were added. The mixture was reacted at 60°C for 2 hours, and TLC showed that the reaction was completed. The reaction solution was concentrated under reduced pressure to remove the solvent, and then methanol (200 mL) was added. The mixture was slurried for 30 minutes, and then filtered to obtain tert-butyl (tert-butoxycarbonyl)(2,6-dichloro-3-fluoropyridin-4-yl)carbamate (24.3 g, yield: 63.2%).

¹H NMR (400 MHz, CDCl₃) δ 7.18 (d, *J* = 4.3 Hz, 1H), 1.49 (s, 18H).

### Step 3: Synthesis of tert-butyl 4-((tert-butoxycarbonyl)amino)-2,6-dichloro-5-fluoronicotinate

Lithium diisopropylamide (105 mL, 210 mmol) was slowly dropwise added to anhydrous tetrahydrofuran (200 mL) at -78°C. The solution was stirred for 10 minutes. Tert-butyl (tert-butoxycarbonyl)(2,6-dichloro-3-fluoropyridin-4-yl)carbamate (24.3 g, 63.7 mmol) was dissolved in tetrahydrofuran (200 mL), and the mixture was slowly dropwise added to the above solution at -78°C. After the addition, the solution was reacted for 1 hour. TLC showed that the reaction was completed. The reaction solution was quenched by slowly dropwise adding acetic acid (20 mL). The reaction solution was extracted with ethyl acetate (400 mL) and water (300 mL). The organic phase was washed with saturated sodium chloride aqueous solution (200 mL), dried over anhydrous sodium sulfate and concentrated under reduced pressure to remove the solvent, to obtain tert-butyl 4-((tert-butoxycarbonyl)amino)-2,6-dichloro-5-fluoronicotinate (26.6 g, yield: 99.9%).

### Step 4: Synthesis of 4-amino-2,6-dichloro-5-fluoronicotinic acid

Tert-butyl 4-((tert-butoxycarbonyl)amino)-2,6-dichloro-5-fluoronicotinate (26.6 g, 69.6 mmol) was dissolved in dioxane (80 mL), and concentrated hydrochloric acid (30 mL) was added. The mixture was reacted for 3 hours at 65°C, and LCMS showed that the reaction was completed. It was concentrated under reduced pressure to remove the solvent, to obtain 4-amino-2,6-dichloro-5-fluoronicotinic acid (15 g, yield: 95.7%). ESI-MS: 225 [M+1]⁺.

### Step 5: Synthesis of 4-amino-2,6-dichloro-5-fluoronicotinoyl chloride

4-amino-2,6-dichloro-5-fluoronicotinic acid (15 g, 66.7 mmol) was dissolved in thionyl chloride (40 mL). The mixture was reacted at 60°C for 3 hours, and LC-MS showed that the reaction was completed. The reaction solution was concentrated under reduced pressure to remove the solvent, to obtain 4-amino-2,6-dichloro-5-fluoronicotinoyl chloride (16 g, yield: 98.6%).

### Step 6: Synthesis of 5,7-dichloro-8-fluoro-2-mercaptopyrido[4,3-d]pyrimidin-4(3H)-one

4-amino-2,6-dichloro-5-fluoronicotinoyl chloride (16 g, 65.7 mmol) was dissolved in anhydrous acetone (80 mL), and NH₄SCN (15 g, 197 mmol) was dissolved in anhydrous acetone (80 mL), both of which were slowly added to the above solution. The mixture was reacted at 50°C for 1 hour, and TLC showed that the reaction was completed. The reaction solution was poured into 400 mL water. The solution was stirred for 10 minutes, solids were precipitated and filtered, and the filter cake was rinsed with water to obtain 5,7-dichloro-8-fluoro-2-mercaptopyrido[4,3-d]pyrimidin-4(3H)-one (15.9 g, yield: 90.7%).

### Step 7: Synthesis of 5,7-dichloro-8-fluoro-2-(methylthio)pyrido [4,3-d] pyrimidin-4(3H)-one

5,7-dichloro-8-fluoro-2-mercaptopyrido[4,3-d]pyrimidin-4(3H)-one (15.9 g, 59.6 mmol) was dissolved in methanol (130 mL), and then methyl iodide (12.7 g, 894 mmol) and 100 mL 1 M sodium hydroxide aqueous solution were added. The mixture was reacted at 25°C for 3 hours, and LC-MS showed that the reaction was completed. The reaction solution was poured into 400 mL water, the pH was adjusted to about 6 with dilute hydrochloric acid, solids were precipitated and filtered, and the filter cake was rinsed with water to obtain 5,7-dichloro-8-fluoro-2-(methylthio)pyrido[4,3-d]pyrimidin-4(3H)-one (15 g, yield: 80.6%).

¹H NMR (400 MHz, DMSO-*d*₆) δ 13.34 (br s, 1H), 2.60 (s, 3H).

### Intermediate Q: Preparation of ((2-fluoro-6-(methoxymethoxy)-3-methyl-8-(4,4,5,5-tetramethyl-1,3,2-dioxaborolan -2-yl)naphthalen-1-yl)ethynyl)triisopropylsilane

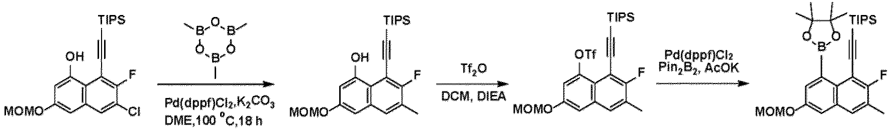

### Step 1: Synthesis of 7-fluoro-3-(methoxymethoxy)-6-methyl-8-((triisopropylsilyl)ethynyl)naphthalen-1-ol

6-chloro-7-fluoro-3-(methoxymethoxy)-8-((triisopropylsilyl)ethynyl)naphthalen-1-ol (2 g, 4.576 mmol) and 2,4,6-trimethyl-1,3,5,2,4,6-trioxatriborinane (1.15 g, 9.153 mmol) were dissolved in ethylene glycol dimethyl ether (15 mL), and potassium carbonate (1.89 g, 13.73 mmol) and Pd(dppf)Cl₂(0.33 g, 0.458 mmol) were added. The mixture was reacted at 100°C for 18 hours. After the reaction was over, the solution was diluted with ethyl acetate, and the organic phase was separated and washed with saturated brine once. The organic phase was dried and filtered, the filtrate was concentrated, and the remaining was separated by using a fast silica gel column [0-50% ethyl acetate: petroleum ether] to obtain 7-fluoro-3-(methoxymethoxy)-6-methyl-8-((triisopropylsilyl)ethynyl)naphthalen-1-ol (715 mg, yield: 37.4%). ESI-MS: 417.2 [M+1]⁺.

### Step 2: Synthesis of 7-fluoro-3-(methoxymethoxy)-6-methyl-8-((triisopropylsilyl)ethynyl)naphthalen-1-yl trifluoromethanesulfonate

7-fluoro-3-(methoxymethoxy)-6-methyl-8-((triisopropylsilyl)ethynyl)naphthalen-1 -ol (1.43 g, 3.432 mmol) and N,N-diisopropylethylamine (2.21 g, 17.16 mmol) were dissolved in dichloromethane (20 mL), and trifluoromethanesulfonic anhydride (1.45 g, 5.149 mmol) was added at 0°C. The mixture was reacted at 0°C for 1 hour. After the reaction was over, the solution was diluted with ethyl acetate, and the organic phase was separated and washed with saturated brine once. The organic phase was dried and filtered, the filtrate was concentrated, and the remaining was separated by using a fast silica gel column [0-50% ethyl acetate: petroleum ether] to obtain 7-fluoro-3-(methoxymethoxy)-6-methyl-8-((triisopropylsilyl)ethynyl)naphthalen-1-yl trifluoromethanesulfonate (1.04 g, yield: 55.3%). ESI-MS: 549.2 [M+1]⁺.

### Step 3: Synthesis of ((2-fluoro-6-(methoxymethoxy)-3-methyl-8-(4,4,5,5-tetramethyl-1,3,2-dioxaborolan -2-yl)naphthalen-1-yl)ethynyl)triisopropylsilane

7-fluoro-3-(methoxymethoxy)-6-methyl-8-((triisopropylsilyl)ethynyl)naphthalen-1 -yl trifluoromethanesulfonate (1.04 g, 1.896 mmol), 4,4,5,5-tetramethyl-2-(4,4,5,5-tetramethyl-1,3,2-dioxaborolan-2-yl)-1,3,2-dioxaborolan (1.44 g, 5.687 mmol) and potassium acetate (560 mg, 5.687 mmol) were dissolved in toluene (20 mL), and Pd(dppf)Cl₂ (140 mg, 0.19 mmol) was added. The nitrogen substitution was carried out three times, and the mixture was reacted at 135°C for 18 hours. After the reaction was over, the solution was diluted with ethyl acetate, and the organic phase was separated and washed with saturated brine once. The organic phase was dried and filtered, the filtrate was concentrated, and the remaining was separated by using a fast silica gel column [0-50% ethyl acetate: petroleum ether] to obtain ((2-fluoro-6-(methoxymethoxy)-3-methyl-8-(4,4,5,5-tetramethyl-1,3,2-dioxaborolan-2-yl)naphthalen-1-yl)ethynyl)triisopropylsilane (564 mg, yield: 56.5%). ESI-MS: 527.3 [M+1]⁺.

### Intermediate R: Preparation of ((2,3-difluoro-6-(methoxymethoxy)-8-(4,4,5,5-tetramethyl-1,3,2-dioxaborolan-2-yl) naphthalen-1-yl)ethynyl)triisopropylsilane

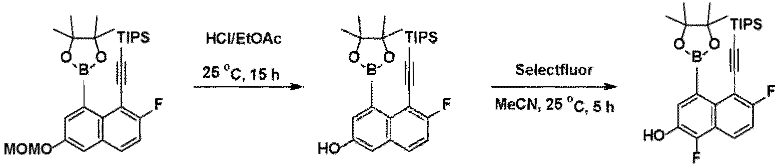

### Step 1: Synthesis of 6-fluoro-4-(4,4,5,5-tetramethyl-1,3,2-dioxaborolan-2-yl)-5-((triisopropylsilyl)ethyny l)naphthalen-2-ol

((2-fluoro-6-(methoxymethoxy)-8-(4,4,5,5-tetramethyl-1,3,2-dioxaborolan-2-yl)na phthalen-1-yl)ethynyl)triisopropylsilane (5000 mg, 9.76 mmol) was added to the hydrochloric acid ethyl acetate solution (24.4 mL). The mixture was reacted at 25°C for 5 hours. The mixture was concentrated. The crude product was separated by using a fast silica gel column to obtain 6-fluoro-4-(4,4,5,5-tetramethyl-1,3,2-dioxaborolan-2-yl)-5-((triisopropylsilyl)ethynyl)n aphthalen-2-ol (1500 mg, yield: 32.8%).

¹H NMR (400MHz, DMSO-*d*₆) δ 9.88 (s, 1H), 7.83 (dd, *J* = 5.9, 9.1 Hz, 1H), 7.85-7.76(m, 1H), 7.40 (t, *J* = 9.0 Hz, 1H),7.32 (d, *J* = 2.3 Hz, 1H), 7.23 (d, *J* = 2.4 Hz, 1H), 1.34 (s, 12H), 1.14-1.08 (m, 21H).

### Step 2: Synthesis of 1,6-difluoro-4-(4,4,5,5-tetramethyl-1,3,2-dioxaborolan-2-yl)-5-((triisopropylsilyl)et hynyl)naphthalen-2-ol

6-fluoro-4-(4,4,5,5-tetramethyl-1,3,2-dioxaborolan-2-yl)-5-((triisopropylsilyl)ethyn yl)naphthalen-2-ol (500 mg, 1.07 mmol) was dissolved in acetonitrile (10 mL) under nitrogen atmosphere, and 1-chloromethyl-4-fluoro-1,4-diazoniabicyclo[2.2.2]octan bis(tetrafluoroborate) (416 mg, 1.17 mmol) was added to the solution. The mixture was reacted at 25°C for 5 hours. The reaction mixture was extracted with water (20 mL) and ethyl acetate (20 mL*3). The organic phase was separated, dried over magnesium sulfate, filtrated and concentrated under reduced pressure. The crude product was separated by using a fast silica gel column to obtain 1, 6-difluoro-4-(4,4, 5, 5-tetramethyl-1,3,2-dioxaborolan-2-yl)-5-((triisopropylsilyl)ethyn yl)naphthalen-2-ol (250 mg, yield: 48.2%).

¹H NMR (400MHz, CDCl₃) δ 7.85 (dd, *J* = 5.6, 9.2 Hz, 1H), 7.46 (d, *J* = 9.1 Hz, 1H), 7.25-7.20 (m, 1H), 5.33 (br d, *J* = 3.0 Hz, 1H), 1.35 (s, 12H), 1.10-1.05 (m, 21H).

### Intermediate S: Preparation of tert-butyl 1-((methanesulfonyl)methyl)-3,8-diazabicyclo[3.2.1]octan-8-carboxylate

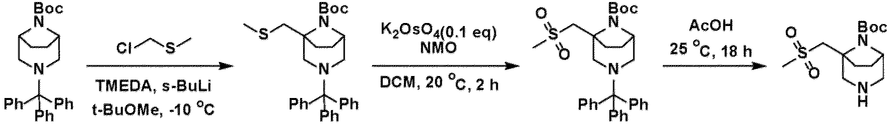

### Step 1: Synthesis of tert-butyl 1-((methylthio)methyl)-3-triphenylmethyl-3,8-diazabicyclo[3.2.1]octan-8-carboxyla te

Tert-butyl 3-triphenylmethyl-3,8-diazabicyclo[3.2.1]octan-8-carboxylate (5 g, 11.0 mmol) was dissolved in diethyl ether (100 mL), N,N,N,N-tetramethylethylenediamine (3.20 g, 27.5 mmol) was added at -10°C under nitrogen protection, and sec-butyllithium (1.3 M, 21.2 mL, 27.5 mmol) was slowly added dropwise. The mixture was reacted at -10°C for 2 hours, and then chloromethyl methyl sulfide (2.12 g, 22.0 mmol) was added dropwise. After being reacted at -10°C for 2 hours, the mixture was quenched with saturated ammonium chloride aqueous solution and extracted with ethyl acetate. The organic phase was washed with saturated sodium chloride aqueous solution, dried over anhydrous magnesium sulfate and concentrated under reduced pressure to remove the solvent. The remaining was separated by using a fast silica gel column to obtain tert-butyl (1R,5S)-1-((methylthio)methyl)-3-triphenylmethyl-3,8-diazabicyclo[3.2.1]octan-8-carb oxylate (1.2 g, yield: 21.2%).

¹H NMR (400 MHz, CDCl₃) δ 7.49 (d, *J* = 7.5 Hz, 5H), 7.34 - 7.23 (m, 7H), 7.20-7.12 (m, 3H), 4.33 (s, 1H), 4.00 (d, *J* = 2.6 Hz, 1H), 3.24-3.05 (m, 1H), 2.86 (t, *J* = 12.4 Hz, 1H), 2.59-2.44 (m, 2H), 2.22-2.09 (m, 4H), 1.79-1.64 (m, 1H), 1.57 (s, 3H), 1.40 (s, 9H).

### Step 2: Synthesis of tert-butyl 1-((methanesulfonyl)methyl)-3-triphenylmethyl-3,8-diazabicyclo [3.2.1] octan-8-car boxylate

Tert-butyl 1-((methylthio)methyl)-3-triphenylmethyl-3,8-diazabicyclo[3.2.1]octan-8-carboxylate (660 mg, 1.28 mmol) was dissolved in dichloromethane (20 mL), and N-methylmorpholine oxide (751 mg, 6.41 mmol) and potassium osmate dihydrate (47.2 mg, 0.13 mmol) were added at -40°C under nitrogen protection. The mixture was reacted at 25°C for 2 hours, quenched with saturated sodium hydroxide aqueous solution, extracted with water and dichloromethane, and washed with saturated sodium chloride aqueous solution. The organic phase was dried over anhydrous magnesium sulfate and concentrated under reduced pressure to remove the solvent. The remaining was tert-butyl 1-((methanesulfonyl)methyl)-3-triphenylmethyl-3,8-diazabicyclo[3.2.1]octan-8-carboxy late (600 mg, yield: 85.6%). The crude product was directly used for the next reaction.

### Step 3: Synthesis of tert-butyl 1-((methanesulfonyl)methyl)-3,8-diazabicyclo[3.2.1]octan-8-carboxylate

Tert-butyl 1-((methanesulfonyl)methyl)-3-triphenylmethyl-3,8-diazabicyclo[3.2.1]octan-8-carboxy late (700 mg, 1.28 mmol) was dissolved in acetic acid (10 mL), reacted at 25°C for 18 hours and then concentrated under reduced pressure to remove the solvent. The remaining was crude product tert-butyl 1-((methanesulfonyl)methyl)-3,8-diazabicyclo[3.2.1]octan-8-carboxylate (700 mg, yield: 89.8%). ESI-MS: 305 [M+1]⁺.

### II. Preparation of Specific Examples

### Example 1: Preparation of 5-ethynyl-6-fluoro-4-(8-fluoro-2-(((2R,7aS)-2-fluorotetrahydro-1H-pyrrolizin-7a-yl )methoxy)-4-(1-(methoxymethyl)-3,8-diazabicyclo[3.2.1]octan-3-yl)pyrido[4,3-d]py rimidin-7-yl)naphthalen-2-ol

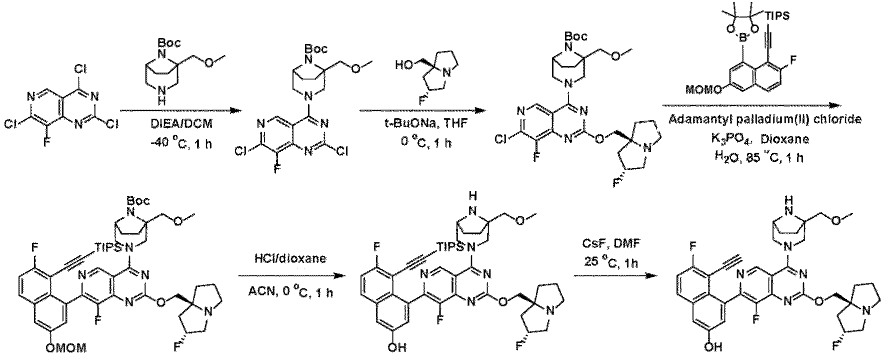

### Step 1: Synthesis of tert-butyl 3-(2,7-dichloro-8-fluoropyrido [4,3-d] pyrimidin-4-yl)-1-(methoxymethyl)-3,8-diaza bicyclo [3.2.1] octan-8-carboxylate

Tert-butyl 1-(methoxymethyl)-3,8-diazabicyclo[3.2.1]octan-8-carboxylate (220 mg, 0.87 mmol) and 2,4,7-trichloro-8-fluoropyrido[4,3-d]pyrimidin (223 mg, 0.87 mmol) were dissolved in anhydrous dichloromethane (10 mL). DIEA (0.72 mL, 4.35 mmol) was added slowly under nitrogen protection at -40°C, and the reaction was continued for 1 hour at -40°C under nitrogen protection. The reaction solution was diluted with water (20 mL), extracted with dichloromethane (20 mL*2), and washed with saturated sodium chloride aqueous solution (20 mL). The organic phase was dried over anhydrous sodium sulfate and concentrated under reduced pressure to remove the solvent. The remaining was separated by using a rapid silica gel column to obtain tert-butyl 3-(2,7-dichloro-8-fluoropyrido[4,3-d]pyrimidin-4-yl)-1-(methoxymethyl)-3,8-diazabicy clo[3.2.1]octan-8-carboxylate (370 mg, yield: 89.9%). ESI-MS: 472 [M+1]⁺.

### Step 2: Synthesis of tert-butyl 3-(7-chloro-8-fluoro-2-(((2R,7aS)-2-fluorotetrahydro-1H-pyrrolizin-7a-yl)methoxy) pyrido[4,3-d]pyrimidin-4-yl)-1-(methoxymethyl)-3,8-diazabicyclo[3.2.1]octan-8-car boxylate

Tert-butyl 3-(2,7-dichloro-8-fluoropyrido[4,3-d]pyrimidin-4-yl)-1-(methoxymethyl)-3,8-diazabicy clo[3.2.1]octan-8-carboxylate (370 mg, 0.78 mmol) and ((2R,7aS)-2-fluorotetrahydro-1H-pyrrolizin-7a-yl) methanol (124 mg, 0.78 mmol) were dissolved in anhydrous tetrahydrofuran (10 mL), and sodium tert-butoxide (150 mg, 1.56 mmol) was slowly added under nitrogen protection at 0°C. After the addition is completed, the reaction was continued for 1 hour at 0°C. The reaction solution was diluted with water (20 mL), extracted with ethyl acetate (20 mL*2), and washed with saturated sodium chloride aqueous solution (20 mL). The organic phase was dried over anhydrous sodium sulfate and filtered and concentrated under reduced pressure to remove the solvent. The remaining was separated by using a rapid silica gel column to obtain tert-butyl 3-(7-chloro-8-fluoro-2-(((2R,7aS)-2-fluorotetrahydro-1H-pyrrolizin-7a-yl)methoxy)pyri do[4,3-d]pyrimidin-4-yl)-1-(methoxymethyl)-3,8-diazabicyclo[3.2.1]octan-8-carboxylat e (200 mg, yield: 42.9%). ESI-MS: 595 [M+1]⁺.

### Step 3: Synthesis of tert-butyl 3-(8-fluoro-7-(7-fluoro-3-(methoxymethoxy)-8-((triisopropylsilyl)ethynyl)naphthale n-1-yl)-2-(((2R,7aS)-2-fluorotetrahydro-1H-pyrrolizin-7a-yl)methoxy)pyrido[4,3-d]pyrimi din-4-yl)-1-(methoxymethyl)-3,8-diazabicyclo[3.2.1]octan-8-carboxylate

Tert-butyl 3-(7-chloro-8-fluoro-2-(((2R,7aS)-2-fluorotetrahydro-1H-pyrrolizin-7a-yl)methoxy)pyri do[4,3-d]pyrimidin-4-yl)-1-(methoxymethyl)-3,8-diazabicyclo[3.2.1]octan-8-carboxylat e (200 mg, 0.34 mmol), ((2-fluoro-6-(methoxymethoxy)-8-(4,4,5,5-tetramethyl-1,3,2-dioxaborolan-2-yl)naphtha len-1-yl)ethynyl)triisopropylsilane (189 mg, 0.37 mmol) and potassium phosphate (142 mg, 0.67 mmol) were dissolved in anhydrous dioxane (5 mL) and water (1 mL). Adamantyl palladium(II) chloride (11.2 mg, 0.02 mmol) was slowly added under nitrogen protection at 25°C, and reacted at 85°C for 1 hour under nitrogen protection. The reaction solution was diluted with water (20 mL), extracted with ethyl acetate (20 mL*2), and washed with saturated sodium chloride aqueous solution (20 mL). The organic phase was dried over anhydrous sodium sulfate and filtered and concentrated under reduced pressure to remove the solvent. The remaining was separated by using a rapid silica gel column to obtain tert-butyl 3-(8-fluoro-7-(7-fluoro-3-(methoxymethoxy)-8-((triisopropylsilyl)ethynyl)naphthalen-1 -yl)-2-(((2R,7aS)-2-fluorotetrahydro-1H-pyrrolizin-7a-yl)methoxy)pyrido[4,3-d]pyrimi din-4-yl)-1-(methoxymethyl)-3,8-diazabicyclo[3.2.1]octan-8-carboxylate (260 mg, yield: 81.8%). ESI-MS: 945 [M+1]⁺.

### Step 4: Synthesis of 6-fluoro-4-(8-fluoro-2-(((2R,7aS)-2-fluorotetrahydro-1H-pyrrolizin-7a-yl)methoxy) -4-(1-(methoxymethyl)-3,8-diazabicyclo[3.2.1]octan-3-yl)pyrido[4,3-d]pyrimidin-7-yl)-5-((triisopropylsilyl)ethynyl)naphthalen-2-ol

Tert-butyl 3-(8-fluoro-7-(7-fluoro-3-(methoxymethoxy)-8-((triisopropylsilyl)ethynyl)naphthalen-1 -yl)-2-(((2R,7aS)-2-fluorotetrahydro-1H-pyrrolizin-7a-yl)methoxy)pyrido[4,3-d]pyrimidin-4 -yl)-1-(methoxymethyl)-3,8-diazabicyclo[3.2.1]octan-8-carboxylate (250 mg, 0.26 mmol) was dissolved in acetonitrile (2 mL), and hydrochloric acid/dioxane (2 mL) was slowly added dropwise at 25°C. Reaction lasted for 1 hour at 0°C. After concentration under reduced pressure to remove the solvent, 6-fluoro-4-(8-fluoro-2-(((2R,7aS)-2-fluorotetrahydro-1H-pyrrolizin-7a-yl)methoxy)-4-( 1-(methoxymethyl)-3,8-diazabicyclo[3.2.1]octan-3-yl)pyrido[4,3-d]pyrimidin-7-yl)-5-(( triisopropylsilyl)ethynyl)naphthalen-2-ol (180 mg, 0.23 mmol, yield: 85.0%) was obtained. ESI-MS: 801 [M+1]⁺.

### Step 5: Synthesis of 5-ethynyl-6-fluoro-4-(8-fluoro-2-(((2R,7aS)-2-fluorotetrahydro-1H-pyrrolizin-7a-yl )methoxy)-4-(1-(methoxymethyl)-3,8-diazabicyclo[3.2.1]octan-3-yl)pyrido[4,3-d]py rimidin-7-yl)naphthalen-2-ol

6-fluoro-4-(8-fluoro-2-(((2R,7aS)-2-fluorotetrahydro-1H-pyrrolizin-7a-yl)methoxy )-4-(1-(methoxymethyl)-3,8-diazabicyclo[3.2.1]octan-3-yl)pyrido[4,3-d]pyrimidin-7-yl) -5-((triisopropylsilyl)ethynyl)naphthalen-2-ol (170 mg, 0.21 mmol) was dissolved in DMF (10 mL), and cesium fluoride (1.61 g, 10.6 mmol) was added at 25°C. The reaction lasted for 1 hour at 25°C. The reaction solution was filtered, the filtrate was concentrated under reduced pressure to remove the solvent, and the remaining was separated by using a reversed-phase column to obtain 5-ethynyl-6-fluoro-4-(8-fluoro-2-(((2R,7aS)-2-fluorotetrahydro-1H-pyrrolizin-7a-yl)met hoxy)-4-(1-(methoxymethyl)-3,8-diazabicyclo[3.2.1]octan-3-yl)pyrido[4,3-d]pyrimidin-7-yl)naphthalen-2-ol (22.4 mg, yield: 11.94%, purity: 98.471%). ESI-MS: 645 [M+1]⁺.

¹H NMR (400 MHz, DMSO-*d*₆) δ 10.47-9.95 (m, 1H), 9.04 (s, 1H), 7.98 (dd, *J* = 5.9, 9.1 Hz, 1H), 7.58-7.34 (m, 2H), 7.27-7.15 (m, 1H), 5.43-5.16 (m, 1H), 4.62-4.25 (m, 2H), 4.20-3.84 (m, 3H), 3.67-3.51 (m, 2H), 3.49-3.38 (m, 4H), 3.34 (s, 3H), 3.09 (d, *J* = 7.3 Hz, 2H), 3.02 (s, 1H), 2.88-2.76 (m, 1H), 2.17-1.97 (m, 3H), 1.89-1.73 (m, 4H), 1.70-1.63 (m, 2H), 1.39-1.39 (m, 1H).

### Examples 2-31 can be prepared by selecting the corresponding raw materials with reference to all or part of the synthesis methods of Example 1:

| **Example No.** | **Structural Formula** | **Chemical Name** | **ESI-MS: [M+1]⁺** |
|---|---|---|---|
| **Example 2** | 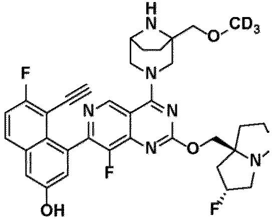 | 5-ethynyl-6-fluoro-4-(8-fluoro-2-(((2R,7aS)-2-fluorotetrahydr o-1H-pyrrolizin-7a(5H)-yl)met hoxy)-4-(1-((methoxy-*d*₃)meth yl)-3,8-diazabicyclo[3.2.1]octa n-3-yl)pyrido[4,3-d]pyrimidin-7-yl)naphthalen-2-ol | 648 |
| **Example 3** | 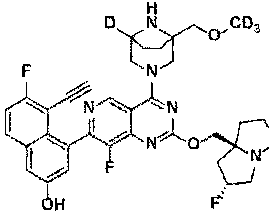 | 5-ethynyl-6-fluoro-4-(8-fluoro-2-(((2R,7aS)-2-fluorotetrahydr o-1H-pyrrolizin-7a(5H)-yl)met hoxy)-4-(1-((methoxy-*d*₃)meth yl)-3,8-diazabicyclo[3.2.1]octa n-3-yl-5-*d*)pyrido[4,3-d]pyrimi din-7-yl)naphthalen-2-ol | 649 |
| **Example 4** | 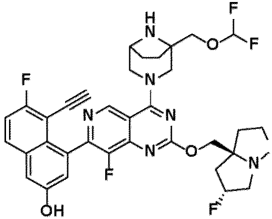 | 4-(4-(1-((difluoromethoxy)met hyl)-3,8-diazabicyclo[3.2.1]oct an-3 -yl)-8-fluoro-2-(((2R,7aS)-2-fluorotetrahydro-1H-pyrroliz in-7a(5H)-yl)methoxy)pyrido[4 ,3-d]pyrimidin-7-yl)-5-ethynyl-6-fluoronaphthalen-2-ol | 681 |
| **Example 5** | 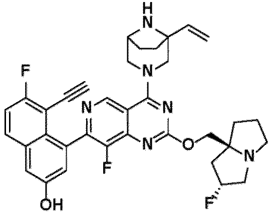 | 5-ethynyl-6-fluoro-4-(8-fluoro-2-(((2R,7aS)-2-fluorotetrahydr o-1H-pyrrolizin-7a(5H)-yl)met hoxy)-4-(1-vinyl-3,8-diazabicy clo[3.2.1]octan-3-yl)pyrido[4,3 -d]pyrimidin-7-yl)naphthalen-2 -ol | 627 |
| **Example 6** | 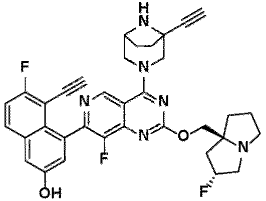 | 5-ethynyl-4-(4-(1-ethynyl-3,8-diazabicyclo[3.2.1]octan-3-yl)-8-fluoro-2-(((2R,7aS)-2-fluorot etrahydro-1H-pyrrolizin-7a(5H )-yl)methoxy)pyrido[4,3 -d]pyri midin-7-yl)-6-fluoronaphthalen -2-ol | 625 |
| **Example 7** | 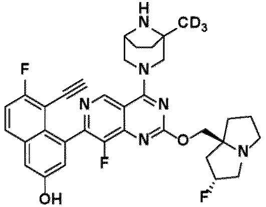 | 5-ethynyl-6-fluoro-4-(8-fluoro-2-(((2R,7aS)-2-fluorotetrahydr o-1H-pyrrolizin-7a(5H)-yl)met hoxy)-4-(1-(methyl-*d*₃)-3,8-dia zabicyclo[3.2.1]octan-3-yl)pyri do[4,3-d]pyrimidin-7-yl)napht halen-2-ol | 618 |
| **Example 8** | 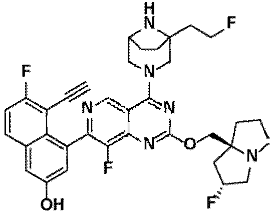 | 5-ethynyl-6-fluoro-4-(8-fluoro-4-(1-(2-fluoroethyl)-3,8-diazab icyclo[3.2.1]octan-3-yl)-2-(((2 R,7aS)-2-fluorotetrahydro-1H-pyrrolizin-7a(5H)-yl)methoxy) pyrido[4,3-d]pyrimidin-7-yl)na phthalen-2-ol | 647 |
| **Example 9** | 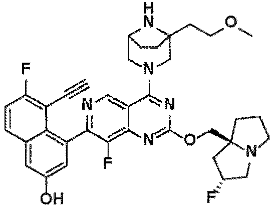 | 5-ethynyl-6-fluoro-4-(8-fluoro-2-(((2R,7aS)-2-fluorotetrahydr o-1H-pyrrolizin-7a(5H)-yl)met hoxy)-4-(1-(2-methoxyethyl)-3 ,8-diazabicyclo[3.2.1]octan-3-y l)pyrido[4,3-d]pyrimidin-7-yl) naphthalen-2-ol | 659 |
| **Example 10** | 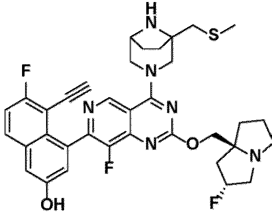 | 5-ethynyl-6-fluoro-4-(8-fluoro-2-(((2R,7aS)-2-fluorotetrahydr o-1H-pyrrolizin-7a(5H)-yl)met hoxy)-4-(1-((methylthio)methy l)-3,8-diazabicyclo[3.2.1]octan -3-yl)pyrido[4,3-d]pyrimidin-7 -yl)naphthalen-2-ol | 661 |
| **Example 11** | 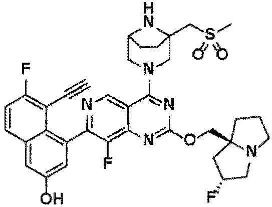 | 5-ethynyl-6-fluoro-4-(8-fluoro-2-(((2R,7aS)-2-fluorotetrahydr o-1H-pyrrolizin-7a(5H)-yl)met hoxy)-4-(1-((methanesulfonyl) methyl)-3,8-diazabicyclo[3.2.1 ]octan-3 -yl)pyrido[4,3 -d]pyrim idin-7-yl)naphthalen-2-ol | 693 |
| **Example 12** | 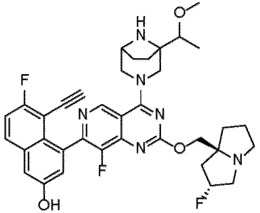 | 5-ethynyl-6-fluoro-4-(8-fluoro-2-(((2R,7aS)-2-fluorotetrahydr o-1H-pyrrolizin-7a(5H)-yl)met hoxy)-4-(1-(1-methoxyethyl)-3 ,8-diazabicyclo[3.2.1]octan-3-y l)pyrido[4,3-d]pyrimidin-7-yl) naphthalen-2-ol | 659 |
| **Example 13** | 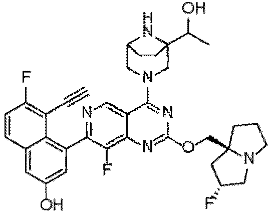 | 5-ethynyl-6-fluoro-4-(8-fluoro-2-(((2R,7aS)-2-fluorotetrahydr o-1H-pyrrolizin-7a(5H)-yl)met hoxy)-4-(1-(1-hydroxyethyl)-3, 8-diazabicyclo[3.2.1]octan-3-yl )pyrido[4,3-d]pyrimidin-7-yl)n aphthalen-2-ol | 645 |
| **Example 14** | 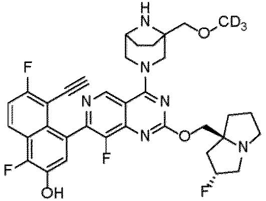 | 5-ethynyl-1,6-difluoro-4-(8-flu oro-2-(((2R,7aS)-2-fluorotetrah ydro-1H-pyrrolizin-7a(5H)-yl) methoxy)-4-(1-((methoxy-*d*₃)m ethyl)-3,8-diazabicyclo[3.2.1]o ctan-3-yl)pyrido[4,3-d]pyrimid in-7-yl)naphthalen-2-ol | 666 |
| **Example 15** | 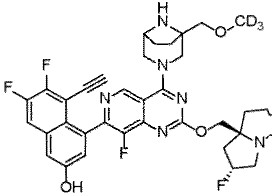 | 5-ethynyl-6,7-difluoro-4-(8-flu oro-2-(((2R,7aS)-2-fluorotetrah ydro-1H-pyrrolizin-7a(5H)-yl) methoxy)-4-(1-((methoxy-*d*₃)m ethyl)-3,8-diazabicyclo[3.2.1]o ctan-3-yl)pyrido[4,3-d]pyrimid in-7-yl)naphthalen-2-ol | 666 |
| **Example 16** | 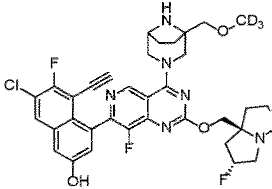 | 7-chloro-5-ethynyl-6-fluoro-4-( 8-fluoro-2-(((2R,7aS)-2-fluorot etrahydro-1H-pyrrolizin-7a(5H )-yl)methoxy)-4-(1-((methoxy-*d*₃)methyl)-3,8-diazabicyclo[3. 2.1]octan-3-yl)pyrido[4,3-d]py rimidin-7-yl)naphthalen-2-ol | 682 |
| **Example 17** | 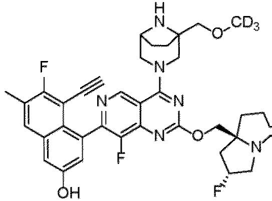 | 5-ethynyl-6-fluoro-4-(8-fluoro-2-(((2R,7aS)-2-fluorotetrahydr o-1H-pyrrolizin-7a(5H)-yl)met hoxy)-4-(1-((methoxy-*d*₃)meth yl)-3,8-diazabicyclo[3.2.1]octa n-3-yl)pyrido[4,3-d]pyrimidin-7-yl)-7-methylnaphthalen-2-ol | 662 |
| **Example 18** | 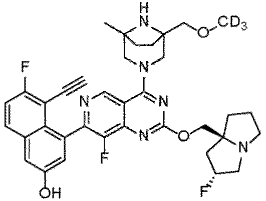 | 5-ethynyl-6-fluoro-4-(8-fluoro-2-(((2R,7aS)-2-fluorotetrahydr o-1H-pyrrolizin-7a(5H)-yl)met hoxy)-4-(1-((methoxy-*d*₃)meth yl)-5-methyl-3,8-diazabicyclo[ 3.2.1]octan-3-yl)pyrido[4,3-d]p yrimidin-7-yl)naphthalen-2-ol | 662 |
| **Example 19** | 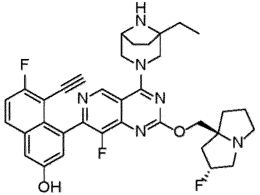 | 4-(4-(1-ethyl-3,8-diazabicyclo[ 3.2.1]octan-3-yl)-8-fluoro-2-((( 2R,7aS)-2-fluorotetrahydro-1H -pyrrolizin-7a(5H)-yl)methoxy )pyrido[4,3-d]pyrimidin-7-yl)-5-ethynyl-6-fluoronaphthalen-2 -ol | 629 |
| **Example 20** | 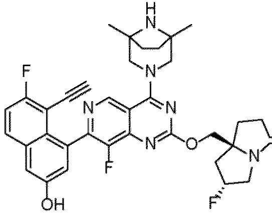 | 4-(4-(1,5-dimethyl-3,8-diazabi cyclo[3.2.1]octan-3-yl)-8-fluor o-2-(((2R,7aS)-2-fluorotetrahy dro-1H-pyrrolizin-7a(5H)-yl)m ethoxy)pyrido[4,3-d]pyrimidin -7-yl)-5-ethynyl-6-fluoronapht halen-2-ol | 629 |
| **Example 21** | 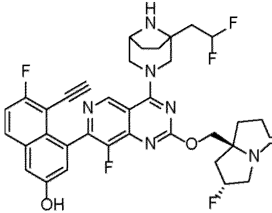 | 4-(4-(1-(2,2-difluoroethyl)-3,8-diazabicyclo[3.2.1]octan-3-yl)-8-fluoro-2-(((2R,7aS)-2-fluorot etrahydro-1H-pyrrolizin-7a(5H )-yl)methoxy)pyrido[4,3 -d]pyri midin-7-yl)-5-ethynyl-6-fluoro naphthalen-2-ol | 665 |
| **Example 22** | 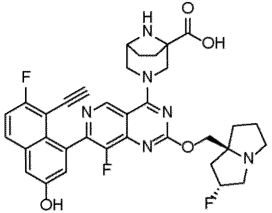 | 3-(7-(8-ethynyl-7-fluoro-3-hyd roxynaphthalen-1-yl)-8-fluoro-2-(((2R,7aS)-2-fluorotetrahydr o-1H-pyrrolizin-7a(5H)-yl)met hoxy)pyrido[4,3-d]pyrimidin-4 -yl)-3,8-diazabicyclo[3.2.1]oct an-1-carboxylic acid | 645 |
| **Example 23** | 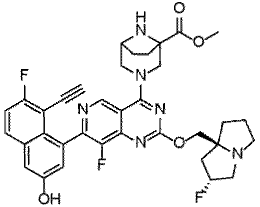 | methyl 3-(7-(8-ethynyl-7-fluoro-3-hyd roxynaphthalen-1-yl)-8-fluoro-2-(((2R,7aS)-2-fluorotetrahydr o-1H-pyrrolizin-7a(5H)-yl)met hoxy)pyrido[4,3-d]pyrimidin-4 -yl)-3,8-diazabicyclo[3.2.1]oct an-1-carboxylate | 659 |
| **Example 24** | 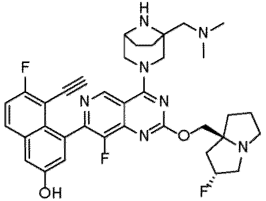 | 4-(4-(1-((dimethylamino)meth yl)-3,8-diazabicyclo[3.2.1]octa n-3-yl)-8-fluoro-2-(((2R,7aS)-2 -fluorotetrahydro-1H-pyrrolizi n-7a(5H)-yl)methoxy)pyrido[4, 3-d]pyrimidin-7-yl)-5-ethynyl-6-fluoronaphthalen-2-ol | 658 |
| **Example 25** | 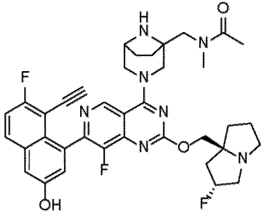 | N-((3-(7-(8-ethynyl-7-fluoro-3-hydroxynaphthalen-1 -yl)-8-flu oro-2-(((2R,7aS)-2-fluorotetrah ydro-1H-pyrrolizin-7a(5H)-yl) methoxy)pyrido[4,3-d]pyrimidi n-4-yl)-3,8-diazabicyclo[3.2.1] octan-1-yl)methyl)-N-methylac etamide | 686 |
| **Example 26** | 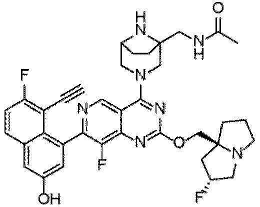 | N-((3-(7-(8-ethynyl-7-fluoro-3-hydroxynaphthalen-1 -yl)-8-flu oro-2-(((2R,7aS)-2-fluorotetrah ydro-1H-pyrrolizin-7a(5H)-yl) methoxy)pyrido[4,3-d]pyrimidi n-4-yl)-3,8-diazabicyclo[3.2.1] octan-1 -yl)methyl)acetamide | 672 |
| **Example 27** | 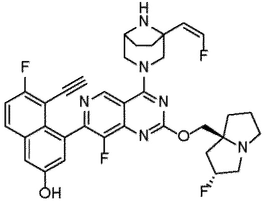 | 5-ethynyl-6-fluoro-4-(8-fluoro-2-(((2R,7aS)-2-fluorotetrahydr o-1H-pyrrolizin-7a(5H)-yl)met hoxy)-4-(1-((Z)-2-fluorovinyl)-3,8-diazabicyclo[3.2.1]octan-3-yl)pyrido[4,3-d]pyrimidin-7-yl )naphthalen-2-ol | 645 |
| **Example 28** | 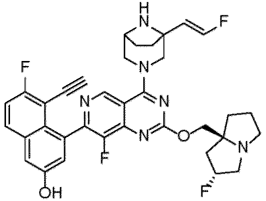 | 5-ethynyl-6-fluoro-4-(8-fluoro-2-(((2R,7aS)-2-fluorotetrahydr o-1H-pyrrolizin-7a(5H)-yl)met hoxy)-4-(1-((E)-2-fluorovinyl)-3,8-diazabicyclo[3.2.1]octan-3-yl)pyrido[4,3-d]pyrimidin-7-yl )naphthalen-2-ol | 645 |
| **Example 29** | 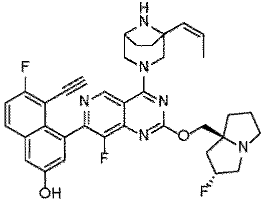 | 5-ethynyl-6-fluoro-4-(8-fluoro-2-(((2R,7aS)-2-fluorotetrahydr o-1H-pyrrolizin-7a(5H)-yl)met hoxy)-4-(1-((Z)-prop-1-en-1-yl )-3,8-diazabicyclo[3.2.1]octan-3-yl)pyrido[4,3-d]pyrimidin-7-yl)naphthalen-2-ol | 641 |
| **Example 30** | 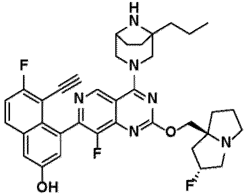 | 5-ethynyl-6-fluoro-4-(8-fluoro-2-(((2R,7aS)-2-fluorotetrahydr o-1H-pyrrolizin-7a(5H)-yl)met hoxy)-4-(1-propyl-3,8-diazabic yclo[3.2.1]octan-3-yl)pyrido[4, 3-d]pyrimidin-7-yl)naphthalen-2-ol | 643 |
| **Example 31** | 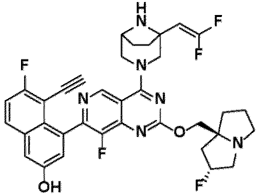 | 4-(4-(1 -(2,2-difluorovinyl)-3,8-diazabicyclo[3.2.1]octan-3-yl)-8-fluoro-2-(((2R,7aS)-2-fluorot etrahydro-1H-pyrrolizin-7a(5H )-yl)methoxy)pyrido[4,3 -d]pyri midin-7-yl)-5-ethynyl-6-fluoro naphthalen-2-ol | 663 |

### HNMR data for the compounds prepared in the above examples are as follows:

| **Example No.** | **¹H NMR (400 MHz)** |
|---|---|
| **Example 2** | ¹H NMR (400 MHz, DMSO-*d*₆) δ 10.16 (s, 1H), 9.03 (s, 1H), 7.98 (dd, *J* = 5.8, 9.2 Hz, 1H), 7.47 (t, *J* = 9.0 Hz, 1H), 7.39 (d, *J* = 2.6 Hz, 1H), 7.18 (s, 1H), 5.28 (d, *J* = 54.2 Hz, 1H), 4.56-4.38 (m, 2H), 4.18-3.98 (m, 2H), 3.91 (d, *J* = 27.5 Hz, 1H), 3.65-3.37 (m, 5H), 3.09 (d, *J* = 8.1 Hz, 2H), 3.01 (s, 1H), 2.86-2.80 (m, 1H), 2.18-1.96 (m, 4H), 1.90-1.60 (m, 7H). |
| **Example 3** | ¹H NMR (400 MHz, DMSO-*d*₆) δ 10.17 (s, 1H), 9.04 (s, 1H), 7.98 (dd, *J* = 5.9, 9.2 Hz, 1H), 7.47 (t, *J* = 9.0 Hz, 1H), 7.40 (d, *J* = 2.5 Hz, 1H), 7.19 (s, 1H), 5.39-5.17 (m, 1H), 4.58-4.30 (m, 2H), 4.17-3.99 (m, 2H), 3.96-3.87 (m, 1H), 3.67-3.50 (m, 1H), 3.49-3.45 (m, 1H), 3.41 (td, *J* = 4.2, 9.0 Hz, 2H), 3.13-3.00 (m, 3H), 2.88-2.78 (m, 1H), 2.15-1.98 (m, 3H), 1.89-1.61 (m, 7H), 1.46 (s, 1H). |
| **Example 4** | ¹H NMR (400 MHz, DMSO-*d*₆) δ 10.19 (s, 1H), 9.05 (d, *J* = 2.8 Hz, 1H), 7.98 (dd, *J* = 6.0, 9.3 Hz, 1H), 7.47 (t, *J* = 9.0 Hz, 1H), 7.39 (d, *J* = 2.5 Hz, 1H), 7.18 (s, 1H), 6.76 (dt, *J* = 1.8, 75.8 Hz, 1H), 5.28 (d, *J* = 54.0 Hz, 1H), 4.62-4.28 (m, 2H), 4.18-4.08 (m, 1H), 4.08-3.99 (m, 1H), 3.95 (s, 2H), 3.89 (s, 1H), 3.67-3.55 (m, 2H), 3.43 (d, *J* = 12.3 Hz, 2H), 3.12-3.04 (m, 2H), 3.01 (s, 1H), 2.87-2.78 (m, 1H), 2.15-1.94 (m, 3H), 1.90-1.61 (m, 6H), 1.54-1.43 (m, 1H). |
| **Example 5** | ¹H NMR (400 MHz, DMSO-*d*₆) δ 10.22 (br s, 1H), 9.05 (d, *J* = 1.3 Hz, 1H), 7.97 (dd, *J* = 5.9, 9.1 Hz, 1H), 7.46 (t, *J* = 9.0 Hz, 1H), 7.38 (d, *J* = 2.4 Hz, 1H), 7.18 (s, 1H), 6.08 (dd, *J* = 10.9, 17.6 Hz, 1H), 5.38-5.12 (m, 3H), 4.61-4.28 (m, 2H), 4.18-4.09 (m, 1H), 4.07-3.99 (m, 1H), 3.93 (d, *J* = 8.4 Hz, 1H), 3.67-3.54 (m, 2H), 3.43-3.34 (m, 2H), 3.08 (d, *J* = 7.3 Hz, 2H), 3.01 (s, 1H), 2.88-2.78 (m, 1H), 2.16-1.98 (m, 3H), 1.89-1.74 (m, 5H), 1.68 (d, *J* = 8.9 Hz, 1H), 1.53-1.41 (m, 1H). |
| **Example 6** | ¹H NMR (400 MHz, DMSO-*d*₆) δ 9.01 (d, *J* = 4.4 Hz, 1H), 7.84 (t, *J* = 7.6 Hz, 1H), 7.36 (t, *J* = 9.0 Hz, 1H), 7.22 (s, 1H), 7.15-7.09 (m, 1H), 5.28 (d, *J* = 53.9 Hz, 1H), 4.57 (dd, *J* = 12.2, 50.2 Hz, 1H), 4.26 (dd, *J* = 12.0, 62.8 Hz, 1H), 4.15-3.98 (m, 2H), 3.87 (d, *J* = 3.0 Hz, 1H), 3.71-3.42 (m, 4H), 3.19-2.97 (m, 6H), 2.89-2.79 (m, 1H), 2.18-1.94 (m, 4H), 1.93-1.58 (m, 5H). |
| **Example 7** | ¹H NMR (400 MHz, DMSO-*d*₆) δ 10.16 (s, 1H), 9.05 (s, 1H), 7.98 (dd, *J* = 5.9, 9.2 Hz, 1H), 7.47 (t, *J* = 9.0 Hz, 1H), 7.39 (d, *J* = 2.6 Hz, 1H), 7.17 (d, *J* = 2.5 Hz, 1H), 5.28 (d, *J* = 54.2 Hz, 1H), 4.49-4.21 (m, 2H), 4.17-3.98 (m, 2H), 3.94 (d, *J* = 1.5 Hz, 1H), 3.59-3.39 (m, 3H), 3.14-3.05 (m, 3H), 3.01 (s, 1H), 2.87-2.78 (m, 1H), 2.15-1.99 (m, 4H), 1.88-1.60 (m, 6H). |
| **Example 8** | ¹H NMR (400 MHz, DMSO-*d*₆) δ 9.05 (s, 1H), 8.20 (s, 1H), 7.98 (dd, *J* = 6.0, 9.2 Hz, 1H), 7.47 (t, *J* = 9.0 Hz, 1H), 7.40 (d, *J* = 2.4 Hz, 1H), 7.19 (d, *J* = 2.3 Hz, 1H), 5.37-5.21 (m, 1H), 4.78-4.71 (m, 1H), 4.63-4.55 (m, 1H), 4.45 (d, *J* = 12.2 Hz, 1H), 4.33 (d, *J* = 11.6 Hz, 1H), 4.14 (dt, *J* = 4.6, 10.4 Hz, 2H), 4.05 (dt, *J* = 4.6, 10.5 Hz, 2H), 3.91 (d, *J* = 15.9 Hz, 2H), 3.63 (d, *J* = 9.3 Hz, 2H), 3.42 (t, *J* = 11.0 Hz, 1H), 3.13-3.02 (m, 3H), 2.88-2.81 (m, 1H), 2.15-1.97 (m, 5H), 1.88-1.63 (m, 7H), 1.54-1.42 (m, 1H), 1.00-0.94 (m, 1H). |
| **Example 9** | ¹H NMR (400 MHz, DMSO-*d*₆) δ 10.40-9.93 (m, 1H), 9.04 (s, 1H), 7.97 (d, *J* = 6.2 Hz, 1H), 7.59-7.32 (m, 2H), 7.19 (s, 1H), 5.47-5.10 (m, 1H), 4.68-4.55 (m, 1H), 4.54-4.38 (m, 1H), 4.29 (d, *J* = 11.2 Hz, 1H), 4.12 (s, 1H), 4.03 (d, *J* = 4.5 Hz, 1H), 3.92 (d, *J* = 12.7 Hz, 1H), 3.64-3.43 (m, 3H), 3.26 (d, *J* = 6.1 Hz, 3H), 3.17-2.98 (m, 3H), 2.84 (d, *J* = 5.6 Hz, 2H), 2.23-1.94 (m, 3H), 1.92-1.57 (m, 9H), 1.42 (d, *J* = 10.6 Hz, 1H). |
| **Example 10** | ¹H NMR (400 MHz, DMSO-*d*₆) δ 8.88-8.69 (m, 1H), 7.98 (dd, *J* = 9.0, 6.0 Hz, 1H), 7.47 (t, *J* = 8.9 Hz, 1H), 7.39 (d, *J* = 2.5 Hz, 1H), 7.22-7.15 (m, 1H), 6.23 (d, *J* = 11.0 Hz, 1H), 5.39-5.19 (m, 1H), 4.95-4.80 (m, 1H), 4.32-4.18 (m, 1H), 4.13-3.97 (m, 3H), 3.16-2.98 (m, 5H), 2.88-2.77 (m, 1H), 2.32 (d, *J* = 1.5 Hz, 3H), 2.20-2.12 (m, 2H), 2.04 (m, 4H), 1.91-1.73 (m, 4H), 1.17 (m, 4H). |
| **Example 11** | ¹H NMR (400 MHz, DMSO-*d*₆) δ 9.31 (d, *J* = 1.9 Hz, 1H), 7.97 (dd, *J* = 5.9, 9.2 Hz, 1H), 7.46 (t, *J* = 9.0 Hz, 1H), 7.39 (d, *J* = 2.4 Hz, 1H), 7.17 (dd, *J* = 2.3, 7.9 Hz, 1H), 6.97 (s, 1H), 5.41-5.20 (m, 1H), 4.98-4.80 (m, 1H), 4.49-4.34 (m, 1H), 4.14-4.03 (m, 5H), 3.13-3.08 (m, 2H), 3.03 (d, *J* = 5.5 Hz, 5H), 2.89-2.79 (m, 2H), 2.17-2.13 (m, 1H), 2.09-2.00 (m, 4H), 1.92-1.72 (m, 5H), 1.70-1.48 (m, 2H). |
| **Example 12** | ¹H NMR (400 MHz, DMSO-*d*₆) δ 10.18 (s, 1H), 9.06-8.98 (m, 1H), 7.97 (dd, *J* = 5.9, 9.2 Hz, 1H), 7.46 (t, *J* = 9.0 Hz, 1H), 7.38 (d, *J* = 2.5 Hz, 1H), 7.19 (s, 1H), 5.28 (m, 1H), 4.74-4.22 (m, 2H), 4.19-4.00 (m, 2H), 3.88 (d, *J* = 44.1 Hz, 1H), 3.69-3.44 (m, 2H), 3.37 (t, *J* = 6.2 Hz, 4H), 3.09 (d, *J* = 8.6 Hz, 2H), 3.01 (s, 1H), 2.88-2.81 (m, 1H), 2.42 (s, 1H), 2.05 (m, 3H), 1.91-1.65 (m, 5H), 1.63-1.43 (m, 3H), 1.14 (m, 3H). |
| **Example 13** | ¹H NMR (400 MHz, CD₃OD) δ 9.02 (d, *J* = 4.8 Hz, 1H), 7.86 (dd, *J* = 5.8, 9.0 Hz, 1H), 7.37-7.29 (m, 2H), 7.21 (t, *J* = 2.6 Hz, 1H), 5.42-5.21 (m, 1H), 4.68-4.62 (m, 1H), 4.57-4.51 (m, 1H), 4.36-4.23 (m, 3H), 3.96-3.83 (m, 2H), 3.79-3.58 (m, 4H), 3.07-3.01 (m, 1H), 2.40-2.10 (m, 4H), 2.06-1.91 (m, 4H), 1.82-1.63 (m, 3H), 1.28 (m, 3H). |
| **Example 14** | ¹H NMR (400 MHz, DMSO-*d*₆) δ 9.06-9.01 (m, 1H), 8.18-8.10 (m, 1H), 7.61 (t, *J* = 9.0 Hz, 1H), 7.38 (d, *J* = 8.7 Hz, 1H), 5.43-5.14 (m, 1H), 4.62-4.27 (m, 2H), 4.16-4.07 (m, 1H), 4.07-3.95 (m, 2H), 3.66-3.57 (m, 2H), 3.49 (s, 1H), 3.11-2.99 (m, 4H), 2.83 (d, *J* = 6.7 Hz, 1H), 2.15-2.11 (m, 1H), 2.07-1.99 (m, 2H), 1.87-1.66 (m, 6H), 1.48 (d, *J* = 8.2 Hz, 1H). |
| **Example 15** | ¹H NMR (400 MHz, DMSO-*d*₆) δ 10.34 (s, 1H), 9.03 (s, 1H), 8.02 (dd, *J* = 8.5, 11.3 Hz, 1H), 7.37 (d, *J* = 2.5 Hz, 1H), 7.16 (s, 1H), 5.38-5.16 (m, 1H), 4.57-4.28 (m, 2H), 4.18-3.96 (m, 3H), 3.63-3.51 (m, 2H), 3.49-3.38 (m, 3H), 3.16-2.98 (m, 3H), 2.87-2.78 (m, 1H), 2.13 (s, 1H), 2.08-1.95 (m, 2H), 1.89-1.72 (m, 4H), 1.70-1.61 (m, 2H), 1.46 (d, *J* = 11.2 Hz, 1H). |
| **Example 16** | ¹H NMR (400 MHz, CD₃OD) δ 9.01 (d, *J* = 4.5 Hz, 1H), 7.99 (d, *J* = 7.6 Hz, 1H), 7.29 (d, *J* = 2.4 Hz, 1H), 7.22 (d, *J* = 2.4 Hz, 1H), 5.30 (d, *J* = 54.5 Hz, 1H), 4.64 (m, 2H), 4.37-4.20 (m, 2H), 3.76-3.64 (m, 2H), 3.60 (dd, *J* = 4.8, 12.4 Hz, 1H), 3.55-3.45 (m, 3H), 3.29-3.18 (m, 3H), 3.07-2.96 (m, 1H), 2.37-2.10 (m, 3H), 2.04-1.88 (m, 4H), 1.76 (t, *J* = 9.4 Hz, 2H), 1.68-1.56 (m, 1H). |
| **Example 17** | ¹H NMR (400 MHz, DMSO-*d*₆) δ 10.06 (s, 1H), 9.02 (s, 1H), 7.85-7.77 (m, 1H), 7.28 (d, *J* = 2.5 Hz, 1H), 7.10 (d, *J* = 1.8 Hz, 1H), 5.28 (d, *J* = 56.1 Hz, 1H), 4.51 (dd, *J* = 12.5, 26.8 Hz, 1H), 4.36 (dd, *J* = 12.5, 26.8 Hz, 1H), 4.17-3.97 (m, 2H), 3.85 (d, *J* = 29.3 Hz, 1H), 3.64-3.50 (m, 2H), 3.48-3.38 (m, 2H), 3.09 (d, *J* = 9.4 Hz, 2H), 3.01 (s, 1H), 2.85-2.82 (m, 1H), 2.40-2.31 (m, 4H), 2.14-2.13 (m, 1H), 2.05-2.00 (m, 2H), 1.90-1.61 (m, 7H), 1.48-1.43 (m, 1H). |
| **Example 18** | ¹H NMR (400 MHz, DMSO-*d*₆) δ 9.05 (s, 1H), 8.18 (s, 1H), 7.98 (dd, *J* = 5.9, 9.2 Hz, 1H), 7.47 (t, *J* = 9.0 Hz, 1H), 7.40 (d, *J* = 2.4 Hz, 1H), 7.20 (s, 1H), 5.38-5.20 (m, 1H), 4.56-4.26 (m, 4H), 4.15 (td, *J* = 3.2, 10.4 Hz, 2H), 4.09-4.02 (m, 2H), 3.96-3.86 (m, 2H), 3.40 (d, *J* = 4.0 Hz, 1H), 3.10 (d, *J* = 7.8 Hz, 2H), 3.03 (s, 1H), 2.87-2.81 (m, 1H), 2.15 (d, *J* = 4.1 Hz, 1H), 2.07 (s, 1H), 1.82-1.54 (m, 7H), 1.46 (dt, *J* = 3.6, 11.8 Hz, 1H), 1.26 (s, 3H). |
| **Example 19** | ¹H NMR (400 MHz, DMSO-*d*₆) δ 9.06 (s, 1H), 8.19 (s, 2H), 7.97 (dd, *J* = 6.0, 9.3 Hz, 1H), 7.46 (t, *J* = 9.0 Hz, 1H), 7.39 (d, *J* = 2.5 Hz, 1H), 7.18 (s, 1H), 5.41-5.18 (m, 1H), 4.56-4.46 (m, 1H), 4.36 (dd, *J* = 12.7, 20.0 Hz, 1H), 4.16-4.10 (m, 1H), 4.04 (dt, *J* = 5.1, 10.2 Hz, 1H), 3.93 (d, *J* = 14.8 Hz, 1H), 3.72-3.56 (m, 3H), 3.44-3.35 (m, 1H), 315-3.00 (m, 3H), 2.88-2.77 (m, 1H), 2.13 (s, 1H), 2.07-1.98 (m, 2H), 1.83-1.62 (m, 8H), 1.41 (d, *J* = 7.5 Hz, 1 H), 1.00-0.92 (m, 3H). |
| **Example 20** | ¹H NMR (400 MHz, DMSO-*d*₆) δ 10.25-10.11 (m, 1H), 9.07 (s, 1H), 7.98 (dd, *J* = 5.8, 9.3 Hz, 1H), 7.47 (t, *J* = 9.0 Hz, 1H), 7.40 (d, *J* = 2.5 Hz, 1H), 7.19 (d, *J* = 1.8 Hz, 1H), 5.37-5.20 (m, 1H), 4.40-4.34 (m, 1H), 4.28 (dd, *J* = 2.9, 12.2 Hz, 1H), 4.14 (dd, *J* = 4.0, 10.5 Hz, 1H), 4.03 (dd, *J* = 4.1, 10.4 Hz, 1H), 3.94 (s, 1H), 3.29 (s, 1H), 3.12-3.07 (m, 2H), 3.02 (s, 1H), 2.88-2.80 (m, 1H), 2.15-2.12 (m, 1H), 2.07-2.00 (m, 2H), 1.92-1.68 (m, 7H), 1.46 (d, *J* = 8.3 Hz, 2H), 1.23 (s, 6H). |
| **Example 21** | ¹H NMR (400 MHz, DMSO-*d*₆) δ 10.19 (s, 1H), 9.05 (s, 1H), 7.98 (dd, *J* = 6.1, 9.1 Hz, 1H), 7.47 (t, *J* = 9.0 Hz, 1H), 7.40 (d, *J* = 2.4 Hz, 1H), 7.20-7.17 (m, 1H), 6.47-6.14 (m, 1H), 5.29 (d, *J* = 54.1 Hz, 1H), 4.62-4.26 (m, 2H), 4.09 (dtd, *J* = 4.8, 10.4, 36.7 Hz, 2H), 3.92 (d, *J* = 17.6 Hz, 1H), 3.64-3.52 (m, 3H), 3.09 (d, *J* = 8.4 Hz, 2H), 3.02 (s, 1H), 2.86-2.80 (m, 1H), 2.26-2.00 (m, 5H), 1.89-1.61 (m, 7H), 1.58-1.46 (m, 1H). |
| **Example 22** | ¹H NMR (400 MHz, DMSO-*d*₆) δ 9.07 (d, *J* = 2.8 Hz, 1H), 8.20 (s, 1H), 7.98 (dd, *J* = 8.9, 6.2 Hz, 1H), 7.52-7.37 (m, 2H), 7.22 (dd, *J* = 1.8, 12.1 Hz, 1H), 5.40-5.15 (m, 1H), 4.97-4.33 (m, 2H), 4.19-4.10 (m, 1H), 4.09-3.93 (m, 4H), 3.05-2.99 (m, 2H), 2.90-2.77 (m, 3H), 2.15 (s, 1 H), 2.11-1.95 (m, 4H), 1.92-1.74 (m, 6H), 1.24 (s, 1H). |
| **Example 23** | ¹H NMR (400 MHz, DMSO-d₆ ) δ 9.02 (s, 1H), 8.30 (t, *J* = 4.2 Hz, 1H), 7.96 (dd, *J* = 6.4, 9.0 Hz, 1H), 7.45 (t, *J* = 9.1 Hz, 1H), 7.37 (s, 1H), 7.18 (d, *J* = 5.9 Hz, 1H), 5.37-5.18 (m, 1H), 4.56-4.40 (m, 1H), 4.31 (s, 1H), 4.19 (d, *J* = 13.0 Hz, 1H), 4.15-4.09 (m, 1H), 4.04 (d, *J* = 10.4 Hz, 1H), 3.92 (d, *J* = 6.8 Hz, 1H), 3.85-3.71 (m, 2H), 3.69-3.57 (m, 2H), 3.12-3.00 (m, 5H), 2.92 (s, 1H), 2.82 (dd, *J* = 7.3, 14.0 Hz, 2H), 2.16-1.93 (m, 7H), 1.90-1.71 (m, 7H), 1.60 (s, 2H), 1.37 (s, 1H), 1.28-1.12 (m, 1H). |
| **Example 24** | ¹H NMR (400 MHz, DMSO-*d*₆) δ 9.03 (d, *J* = 2.0 Hz, 1H), 7.97 (dd, *J* = 5.9, 9.2 Hz, 1H), 7.46 (t, *J* = 9.0 Hz, 1H), 7.39 (d, *J* = 2.6 Hz, 1H), 7.18 (s, 1H), 5.28 (d, J = 54.4 Hz, 1H), 4.58-4.28 (m, 2H), 4.17-4.10 (m, 1H), 4.07-4.00 (m, 1H), 3.91 (d, *J* = 13.6 Hz, 1H), 3.64-3.49 (m, 4H), 3.14-2.78 (m, 6H), 2.47-2.35 (m, 2H), 2.27 (d, *J* = 9.0 Hz, 6H), 2.14-1.95 (m, 3H), 1.87-1.58 (m, 6H), 1.46-1.35 (m, 1H). |
| **Example 25** | ¹H NMR (400 MHz, DMSO-*d*₆) δ 10.17 (s, 1H), 8.98 (d, *J* = 3.5 Hz, 1H), 7.97 (dd, *J* = 5.9, 9.2 Hz, 1H), 7.46 (t, *J* = 9.0 Hz, 1H), 7.39 (d, *J* = 2.6 Hz, 1H), 7.20-7.15 (m, 1H), 5.28 (d, *J* = 54.4 Hz, 1H), 4.52-4.25 (m, 2H), 4.16-4.07 (m, 1H), 4.06-3.96 (m, 1H), 3.94-3.88 (m, 1H), 3.75-3.54 (m, 4H), 3.14-3.06 (m, 5H), 3.02 (s, 1H), 2.94 (s, 1H), 2.86-2.80 (m, 1H), 2.15-2.00 (m, 6H), 1.87-1.69 (m, 5H), 1.61-1.40 (m, 2H). |
| **Example 26** | ¹H NMR (400 MHz, DMSO-*d*₆) δ 10.16 (s, 1H), 9.02 (d, *J* = 7.9 Hz, 1H), 8.03-7.93 (m, 2H), 7.46 (t, *J* = 9.0 Hz, 1H), 7.39 (d, *J* = 2.5 Hz, 1H), 7.18 (s, 1H), 5.28 (d, J = 54.4 Hz, 1H), 4.56-4.25 (m, 2H), 4.15-4.07 (m, 1H), 4.05-3.97 (m, 1H), 3.94-3.85 (m, 1H), 3.65-3.51 (m, 2H), 3.46-3.36 (m, 2H), 3.24-2.99 (m, 5H), 2.86-2.80 (m, 1H), 2.15-1.99 (m, 3H), 1.86 (d, *J* = 6.7 Hz, 3H), 1.84-1.70 (m, 4H), 1.67-1.56 (m, 2H), 1.48-1.37 (m, 1H). |
| **Example 27** | ¹H NMR (400 MHz, DMSO-*d*₆) δ 10.18 (s, 1H), 9.03 (s, 1H), 7.98 (dd, *J* = 5.9, 9.2 Hz, 1H), 7.54-7.33 (m, 2H), 7.19 (dd, *J* = 2.3, 6.1 Hz, 1H), 6.96-6.53 (m, 1H), 5.40-5.18 (m, 1H), 5.18-5.02 (m, 1H), 4.81-4.57 (m, 1H), 4.44-4.18 (m, 1H), 4.17-3.99 (m, 2H), 3.96-3.81 (m, 1H), 3.71-3.46 (m, 3H), 3.11-3.00 (m, 3H), 2.88-2.81 (m, 1H), 2.43-2.37 (m, 1H), 2.17-1.95 (m, 4H), 1.88-1.73 (m, 4H), 1.67-1.55 (m, 2H). |
| **Example 28** | ¹H NMR (400 MHz, DMSO-*d*₆) δ 10.17 (s, 1H), 9.07 (d, *J* = 2.2 Hz, 1H), 7.98 (dd, *J* = 5.9, 9.2 Hz, 1H), 7.47 (t, *J* = 8.9 Hz, 1H), 7.40 (d, *J* = 2.4 Hz, 1H), 7.18 (s, 1H), 7.15-6.89 (m, 1H), 5.86-5.71 (m, 1H), 5.38-5.14 (m, 1H), 4.56-4.27 (m, 2H), 4.18-3.99 (m, 2H), 3.94 (d, *J* = 3.7 Hz, 1H), 3.67-3.52 (m, 2H), 3.41-3.36 (m, 1H), 3.09 (d, *J* = 9.0 Hz, 2H), 3.02 (s, 1H), 2.83 (d, *J* = 7.3 Hz, 1H), 2.45 (s, 1H), 2.13 (s, 1H), 2.08-1.98 (m, 2H), 1.89-1.73 (m, 5H), 1.72-1.64 (m, 1H), 1.51 (d, *J* = 4.0 Hz, 1H). |
| **Example 29** | ¹H NMR (400 MHz, CD₃OD) 6 9.00 (s, 1H), 7.88-7.84 (m, 1H), 7.39-7.27 (m, 2H), 7.21 (dd, *J* = 2.5, 7.2 Hz, 1H), 5.78-5.57 (m, 2H), 5.31 (d, *J* = 53.8 Hz, 1H), 4.53-4.24 (m, 3H), 3.82 (dd, *J* = 12.4, 26.2 Hz, 1H), 3.63 (t, *J* = 7.7 Hz, 1H), 3.47-3.35 (m, 2H), 3.25-2.96 (m, 4H), 2.43-1.56 (m, 15H). |
| **Example 30** | ¹H NMR (400 MHz, CD₃OD) 6 9.01 (s, 1H), 7.86 (dd, *J* = 5.7, 9.1 Hz, 1H), 7.38-7.26 (m, 2H), 7.21 (dd, *J* = 2.6, 4.5 Hz, 1H), 5.30 (d, *J* = 53.9 Hz, 1H), 4.70 (dt, *J* = 5.9, 12.0 Hz, 1H), 4.60-4.49 (m, 1H), 4.30-4.24 (m, 2H), 3.77-3.62 (m, 2H), 3.45-3.32 (m, 2H), 3.28-3.11 (m, 3H), 3.03-3.01 (m, 1H), 2.41-1.35 (m, 15H), 1.02 (t, *J* = 7.0 Hz, 3H). |
| **Example 31** | ¹H NMR (400 MHz, DMSO-*d*₆) δ 10.18 (s, 1H), 9.05 (d, *J* = 2.4 Hz, 1H), 7.98 (dd, *J* = 6.1, 8.9 Hz, 1H), 7.56-7.32 (m, 2H), 7.19 (d, *J* = 3.3 Hz, 1H), 5.41-5.17 (m, 1H), 4.96-4.81 (m, 1H), 4.68-4.44 (m, 1H), 4.44-4.20 (m, 1H), 4.18-4.09 (m, 1H), 4.08-3.99 (m, 1H), 3.97-3.88 (m, 1H), 3.72-3.54 (m, 2H), 3.44 (dd, *J* = 12.5, 16.7 Hz, 1H), 3.13-3.06 (m, 2H), 3.02 (s, 1H), 2.88-2.79 (m, 1H), 2.16-1.95 (m, 4H), 1.90-1.73 (m, 4H), 1.69-1.55 (m, 2H). |

### Example 32: Preparation of 4-(4-(1-(ethoxymethyl)-3,8-diazabicyclo [3.2.1] octan-3-yl)-8-fluoro-2-(((2R,7aS)-2-fl uorotetrahydro-1H-pyrrolizin-7a(SH)-yl)methoxy)pyrido [4,3-d] pyrimidin-7-yl)-5-e thynyl-6-fluoronaphthalen-2-ol

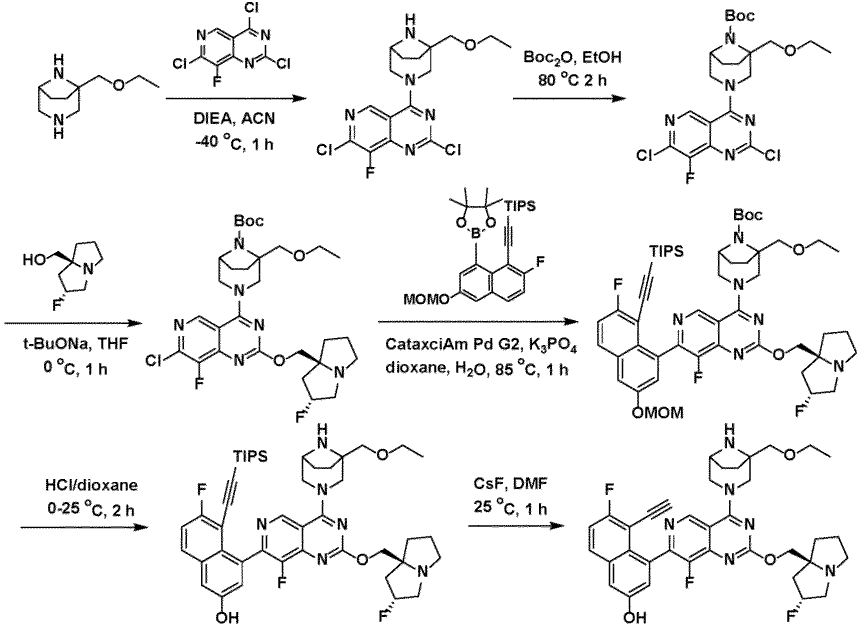

### Step 1: Synthesis of 2,7-dichloro-4-(1-(ethoxymethyl)-3,8-diazabicyclo[3.2.1]octan-3-yl)-8-fluoropyrido[ 4,3-d]pyrimidin

1-(ethoxymethyl)-3,8-diazabicyclo[3.2.1]octan (240 mg, 1.41 mmol) and 2,4,7-trichloro-8-fluoropyrido[4,3-d]pyrimidin (355 mg, 1.41 mmol) were dissolved in anhydrous dichloromethane (5 mL). N,N-diisopropylethylamine (0.93 mL, 5.63 mmol) was added slowly under nitrogen protection at -40°C, and the reaction lasted for 1 hour under nitrogen protection at -40°C. The reaction solution was diluted with water (10 mL), extracted with dichloromethane (10 mL*2), and washed with saturated sodium chloride aqueous solution (10 mL). The organic phase was dried over anhydrous sodium sulfate and concentrated under reduced pressure to remove the solvent. The remaining was separated by using a rapid silica gel column to obtain 2,7-dichloro-4-(1-(ethoxymethyl)-3,8-diazabicyclo[3.2.1]octan-3-yl)-8-fluoropyrido[4,3 -d]pyrimidin (510 mg, yield: 93.6%). ESI-MS: 386 [M+1]⁺.

### Step 2: Synthesis of tert-butyl 3-(2,7-dichloro-8-fluoropyrido[4,3-d]pyrimidin-4-yl)-1-(ethoxymethyl)-3,8-diazabic yclo [3.2.1] octan-8-carboxylate

2,7-dichloro-4-(1-(ethoxymethyl)-3,8-diazabicyclo[3.2.1]octan-3-yl)-8-fluoropyrid o[4,3-d]pyrimidin (510 mg, 1.32 mmol) was dissolved in ethanol (10 mL), and BOC anhydride (0.28 mL, 1.32 mmol) was added to the mixture. The mixture was reacted at 80°C for 2 hours. The solvent was removed by concentration under reduced pressure, and the remaining was separated by using a rapid silica gel column to obtain tert-butyl 3-(2,7-dichloro-8-fluoropyrido[4,3-d]pyrimidin-4-yl)-1-(ethoxymethyl)-3,8-diazabicycl o[3.2.1]octan-8-carboxylate (240 mg, yield: 37.3%). ESI-MS: 486 [M+1]⁺.

### Step 3: Synthesis of tert-butyl 3-(7-chloro-8-fluoro-2-(((2R,7aS)-2-fluorotetrahydro-1H-pyrrolizin-7a(5H)-yl)meth oxy)pyrido [4,3-d] pyrimidin-4-yl)-1-(ethoxymethyl)-3,8-diazabicyclo [3.2.1] octan-8-carboxylate

Tert-butyl 3-(2,7-dichloro-8-fluoropyrido[4,3-d]pyrimidin-4-yl)-1-(ethoxymethyl)-3,8-diazabicycl o[3.2.1]octan-8-carboxylate (460 mg, 0.95 mmol) and ((2R,7aS)-2-fluorotetrahydro-1H-pyrrolizin-7a(5H)-yl) methanol (150 mg, 0.95 mmol) were dissolved in anhydrous tetrahydrofuran (15 mL). Sodium tert-butoxide (181 mg, 1.89 mmol) was slowly added under nitrogen protection at 0°C, and the reaction lasted for 1 hour at 0°C under nitrogen protection. The reaction solution was diluted with water (10 mL), extracted with ethyl acetate (10 mL*2), and washed with saturated sodium chloride aqueous solution (10 mL). The organic phase was dried over anhydrous sodium sulfate and concentrated under reduced pressure to remove the solvent. The remaining was separated by using a rapid silica gel column to obtain tert-butyl 3-(7-chloro-8-fluoro-2-(((2R,7aS)-2-fluorotetrahydro-1H-pyrrolizin-7a(5H)-yl)methoxy )pyrido[4,3-d]pyrimidin-4-yl)-1-(ethoxymethyl)-3,8-diazabicyclo[3.2.1]octan-8-carboxy late (270 mg, yield: 46.87%). ESI-MS: 609 [M+1]⁺.

### Step 4: Preparation of tert-butyl 1-(ethoxymethyl)-3-(8-fluoro-7-(7-fluoro-3-(methoxymethoxy)-8-((triisopropylsilyl) )ethynyl)naphthalen-1-yl)-2-(((2R,7aS)-2-fluorotetrahydro-1H-pyrrolizin-7a(5H)-yl )methoxy)pyrido[4,3-d]pyrimidin-4-yl)-3,8-diazabicyclo[3.2.1]octan-8-carboxylate

Tert-butyl 3-(7-chloro-8-fluoro-2-(((2R,7aS)-2-fluorotetrahydro-1H-pyrrolizin-7a(5H)-yl)methoxy )pyrido[4,3-d]pyrimidin-4-yl)-1-(ethoxymethyl)-3,8-diazabicyclo[3.2.1]octan-8-carboxy late (270 mg, 0.44 mmol), ((2-fluoro-6-(methoxymethoxy)-8-(4,4,5,5-tetramethyl-1,3,2-dioxaborolan-2-yl)naphtha len-1-yl)ethynyl)triisopropylsilane (272 mg, 0.53 mmol) and potassium phosphate (282 mg, 1.33 mmol) were dissolved in anhydrous dioxane (5 mL) and water (0.5 mL). Adamantyl palladium(II) catalyst (14.8 mg, 0.02 mmol) was slowly added under nitrogen protection at 25°C, and reacted at 85°C for 1 hour under nitrogen protection. The reaction solution was diluted with water (10 mL), extracted with ethyl acetate (10 mL*2), and washed with saturated sodium chloride aqueous solution (10 mL). The organic phase was dried over anhydrous sodium sulfate and concentrated under reduced pressure to remove the solvent. The remaining was separated by using a rapid silica gel column to obtain tert-butyl 1-(ethoxymethyl)-3-(8-fluoro-7-(7-fluoro-3-(methoxymethoxy)-8-((triisopropylsilyl))et hynyl)naphthalen-1-yl)-2-(((2R,7aS)-2-fluorotetrahydro-1H-pyrrolizin-7a(5H)-yl)metho xy)pyrido[4,3-d]pyrimidin-4-yl)-3,8-diazabicyclo[3.2.1]octan-8-carboxylate (340 mg, yield: 79.9%). ESI-MS: 959 [M+1]⁺.

### Step 5: Synthesis of 4-(4-(1-(ethoxymethyl)-3,8-diazabicyclo[3.2.1]octan-3-yl)-8-fluoro-2-(((2R,7aS)-2-fl uorotetrahydro-1H-pyrrolizin-7a(5H)-yl)methoxy)pyrido[4,3-d]pyrimidin-7-yl)-6-f luoro-5-((triisopropylsilyl)ethynyl)naphthalen-2-ol

Tert-butyl 1-(ethoxymethyl)-3-(8-fluoro-7-(7-fluoro-3-(methoxymethoxy)-8-((triisopropylsilyl))et hynyl)naphthalen-1-yl)-2-(((2R,7aS)-2-fluorotetrahydro-1H-pyrrolizin-7a(5H)-yl)metho xy)pyrido[4,3-d]pyrimidin-4-yl)-3,8-diazabicyclo[3.2.1]octan-8-carboxylate (340 mg, 0.35 mmol) was dissolved in acetonitrile (6 mL), and hydrochloric acid/dioxane (2 mL) was slowly added dropwise at 25°C. Reaction lasted for 1 hour at 0°C. The solvent was removed by concentration under reduced pressure to obtain 4-(4-(1-(ethoxymethyl)-3,8-diazabicyclo[3.2.1]octan-3-yl)-8-fluoro-2-(((2R,7aS)-2-fluo rotetrahydro-1H-pyrrolizin-7a(5H)-yl)methoxy)pyrido[4,3-d]pyrimidin-7-yl)-6-fluoro-5 -((triisopropylsilyl)ethynyl)naphthalen-2-ol (280 mg, yield: 96.9%). ESI-MS: 815 [M+1]⁺.

### Step 6: Synthesis of 4-(4-(1-(ethoxymethyl)-3,8-diazabicyclo [3.2.1] octan-3-yl)-8-fluoro-2-(((2R,7aS)-2-fl uorotetrahydro-1H-pyrrolizin-7a(5H)-yl)methoxy)pyrido[4,3-d]pyrimidin-7-yl)-5-e thynyl-6-fluoronaphthalen-2-ol

4-(4-(1-(ethoxymethyl)-3,8-diazabicyclo[3.2.1]octan-3-yl)-8-fluoro-2-(((2R,7aS)-2 -fluorotetrahydro-1H-pyrrolizin-7a(5H)-yl)methoxy)pyrido[4,3-d]pyrimidin-7-yl)-6-flu oro-5-((triisopropylsilyl)ethynyl)naphthalen-2-ol (280 mg, 0.344 mmol) was dissolved in DMF (10 mL), and cesium fluoride (2.61 g, 17.2 mmol) was added at 25°C. The reaction lasted for 1 hour at 25°C. The reaction solution was filtered, the filtrate was concentrated under reduced pressure to remove the solvent, and the remaining was separated by using a reversed-phase column to obtain 4-(4-(1-(ethoxymethyl)-3,8-diazabicyclo[3.2.1]octan-3-yl)-8-fluoro-2-(((2R,7aS )-2-flu orotetrahydro-1H-pyrrolizin-7a(5H)-yl)methoxy)pyrido[4,3-d]pyrimidin-7-yl)-5-ethyny l-6-fluoronaphthalen-2-ol (87 mg, yield: 37.49%, purity: 97.51%). ESI-MS: 659 [M+1]⁺.

¹H NMR (400 MHz, DMSO-*d*₆) δ 10.25-10.09 (m, 1H), 9.03 (d, *J* = 3.3 Hz, 1H), 7.98 (dd, *J* = 5.9, 9.2 Hz, 1H), 7.53-7.34 (m, 2H), 7.19 (s, 1H), 5.41-5.14 (m, 1H), 4.63-4.26 (m, 2 H), 4.19-3.98 (m, 2H), 3.97-3.82 (m, 1H), 3.68-3.56 (m, 2H), 3.55-3.49 (m, 3H), 3.48 (s, 3H), 3.13-3.07 (m, 2H), 3.04-3.00 (m, 1H), 2.89-2.78 (m, 1H), 2.00 (s, 3H), 1.89-1.73 (m, 4H), 1.72-1.63 (m, 2H), 1.53-1.40 (m, 1H), 1.15 (dt, *J* = 3.7, 6.9 Hz, 3H).

### Examples 33-35 can be prepared by selecting the corresponding raw materials with reference to all or part of the synthesis methods of Example 32:

| **Example No.** | **Structural Formula** | **Chemical Name** | **ESI-MS: [M+1]⁺** |
|---|---|---|---|
| **Example 33** | 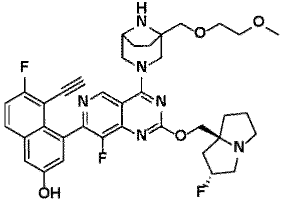 | 5-ethynyl-6-fluoro-4-(8-fluo ro-2-(((2R,7aS)-2-fluorotetr ahydro-1H-pyrrolizin-7a(5H )-yl)methoxy)-4-(1-(2-meth oxyethyl)-3,8-diazabicyclo[ 3.2.1]octan-3-yl)pyrido[4,3-d]pyrimidin-7-yl)naphthalen -2-ol | 689 |
| **Example 34** | 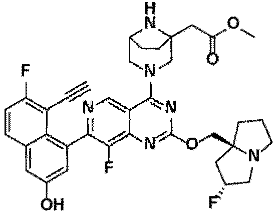 | Methyl 2-(3-(7-(8-ethynyl-7-fluoro-3-hydroxynaphthalen-1-yl)-8-fluoro-2-(((2R,7aS)-2-fluo rohexahydro- 1H-pyrrolizin-7a-yl)methoxy)pyrido[4,3-d ]pyrimidin-4-yl)-3,8-diazabi cyclo[3.2.1]octan-1-yl)aceta te | 673 |
| **Example 35** | 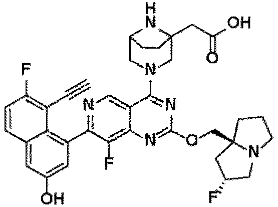 | 2-(3-(7-(8-ethynyl-7-fluoro-3-hydroxynaphthalen-I-yl)-8-fluoro-2-(((2R,7aS)-2-fluo rohexahydro- 1H-pyrrolizin-7a-yl)methoxy)pyrido[4,3-d ]pyrimidin-4-yl)-3, 8-diazabi cyclo[3.2.1]octan-1 -yl)aceti c acid | 659 |

### HNMR data for the compounds prepared in the above examples are as follows:

| **Example No.** | **¹H NMR (400 MHz)** |
|---|---|
| **Example 33** | ¹H NMR (400 MHz, DMSO-*d*₆) δ 10.17 (s, 1H), 9.04 (d, *J* = 2.8 Hz, 1H), 7.98 (dd, *J* = 6.0, 9.1 Hz, 1H), 7.47 (t, *J* = 9.1 Hz, 1H), 7.40 (d, *J* = 2.5 Hz, 1H), 7.19 (s, 1H), 5.39-5.19 (m, 1H), 4.61-4.28 (m, 2H), 4.18-3.98 (m, 2H), 3.96-3.83 (m, 1H), 3.64-3.45 (m, 9H), 3.27 (d, *J* = 3.1 Hz, 3H), 3.09 (d, *J* = 7.8 Hz, 2H), 3.02 (s, 1H), 2.88-2.79 (m, 1H), 2.17-2.11 (m, 1H), 2.08-1.96 (m, 2H), 1.93-1.60 (m, 7H), 1.52-1.40 (m, 1H). |
| **Example 34** | ¹H NMR (400 MHz, DMSO-*d*₆) δ 10.18 (s, 1H), 9.05 (d, *J* = 5.3 Hz, 1H), 7.98 (dd, *J* = 5.9, 9.2 Hz, 1H), 7.47 (t, *J* = 9.0 Hz, 1H), 7.40 (d, *J* = 2.4 Hz, 1H), 7.22-7.12 (m, 1H), 5.39-5.19 (m, 1H), 4.85-4.61 (m, 1H), 4.45-4.21 (m, 1H), 4.17-3.99 (m, 2H), 3.96-3.87 (m, 1H), 3.63 (d, *J* = 6.4 Hz, 3H), 3.56 (d, *J* = 7.2 Hz, 1H), 3.42 (d, *J* = 12.8 Hz, 1H), 3.13-3.04 (m, 2H), 3.02 (s, 1H), 2.89-2.79 (m, 1H), 2.76-2.71 (m, 1H), 2.61 (dd, *J* = 7.0, 15.2 Hz, 1H), 2.14 (d, *J* = 7.6 Hz, 1H), 2.07-1.97 (m, 2H), 1.92-1.73 (m, 5H), 1.64 (s, 1H), 1.55-1.42 (m, 1H). |
| **Example 35** | ¹H NMR (400MHz, DMSO-d₆ ) δ 9.07 (d, *J* = 8.8 Hz, 1H), 7.98 (dd, *J* = 6.2, 9.2 Hz, 1H), 7.47 (t, *J* = 9.1 Hz, 1H), 7.40 (d, *J* = 2.2 Hz, 1H), 7.18 (s, 1H), 5.38-5.19 (m, 1H), 4.67 (d, *J* = 11.0 Hz, 1H), 4.52 (d, *J* = 10.3 Hz, 1H), 4.38 (d, *J* = 12.5 Hz, 1H), 4.17-4.04 (m, 3H), 3.93 (d, *J* = 14.3 Hz, 2H), 3.71 (s, 1H), 3.10 (d, *J* = 9.2 Hz, 3H), 3.02 (s, 1H), 2.83 (d, *J* = 6.2 Hz, 2H), 2.59 (s, 1H), 2.13 (s, 1H), 2.08-1.98 (m, 2H), 1.93-1.75 (m, 6H), 1.56 (s, 1H). |

### Example 36: Preparation of 5-ethynyl-6-fluoro-4-(8-fluoro-2-(((2R,7aS)-2-fluorotetrahydro-1H-pyrrolizin-7a(5 H)-yl)methoxy)-5-methoxy-4-(1-((methoxy-d₃)methyl)-3,8-diazabicyclo[3.2.1]octan-3-yl)pyrido[4,3-d]pyrimidin-7-yl)naphthalen-2-ol

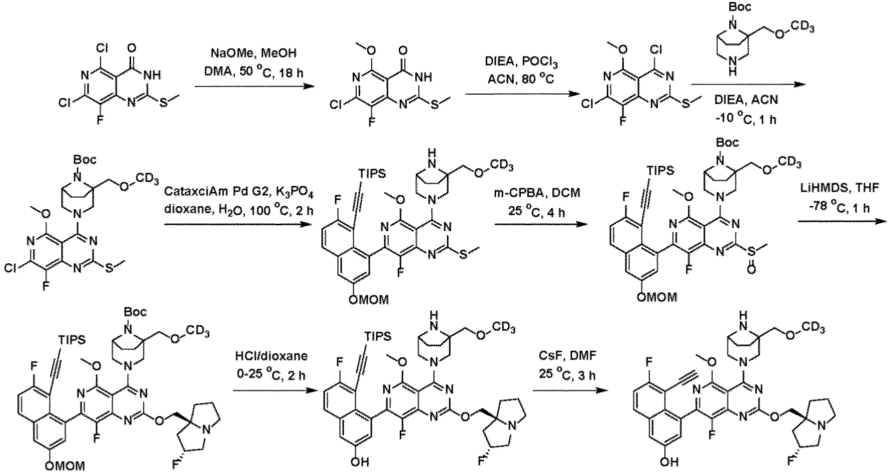

### Step 1: Synthesis of 7-chloro-8-fluoro-5-methoxy-2-(methylthio)pyrido[4,3-d]pyrimidin-4(3H)-one

5,7-dichloro-8-fluoro-2-(methylthio)pyrido[4,3-d]pyrimidin-4(3H)-one (3 g, 10.7 mmol) was dissolved in N,N-dimethylacetamide (30 mL), and added in methanol solution of sodium methoxide (3.86 g, 21.4 mmol). It was stirred and reacted at 50°C for 2 hours and the pH value was adjusted to 3. Water was added for filtration, and the filter cake was collected and dried to obtain 7-chloro-8-fluoro-5-methoxy-2-(methylthio)pyrido[4,3-d]pyrimidin-4(3H)-one (2.69 g, yield: 91.1%). ESI-MS: 276 [M+1]⁺.

### Step 2: Synthesis of 4,7-dichloro-8-fluoro-5-methoxy-2-(methylthio)pyrido [4,3-d] pyrimidin

7-chloro-8-fluoro-5-methoxy-2-(methylthio)pyrido[4,3-d]pyrimidin-4(3H)-one (900 mg, 3.26 mmol) was dissolved in acetonitrile (20 mL), and then N,N-diisopropylethylamine (3128 mg, 24.2 mmol) and phosphorus oxychloride (6580 mg, 42.9 mmol) were added. It was stirred and reacted at 80°C for 1 hour. The reaction solution was concentrated under reduced pressure to remove most of the phosphorus oxychloride. The remaining reaction solution was slowly poured into water, extracted with ethyl acetate and saturated sodium bicarbonate solution, washed with saturated sodium chloride aqueous solution. The organic phase was dried over anhydrous magnesium sulfate and concentrated under reduced pressure to remove the solvent. The remaining was separated by using a rapid silica gel column to obtain 4,7-dichloro-8-fluoro-5-methoxy-2-(methylthio)pyrido[4,3-d]pyrido (900 mg, yield: 93.7%). ESI-MS: 295 [M+1]⁺.

### Step 3: Synthesis of tert-butyl 3-(7-chloro-8-fluoro-5-methoxy-2-(methylthio)pyrido[4,3-d]pyrimidin-4-yl)-1-((met hoxyl-d₃)methyl)-3,8-diazabicyclo[3.2.1]octan-8-carboxylate

4,7-dichloro-8-fluoro-5-methoxy-2-(methylthio)pyrido[4,3-d]pyrimidin (900 mg, 3.06 mmol) was dissolved in dichloromethane (20 mL). Under nitrogen protection, tert-butyl 1-((methoxy-*d*₃)methyl)-3,8-diazabicyclo[3.2.1]octan-8-carboxylate (600 mg, 2.31 mmol), and N,N-diisopropylethylamine (664 mg, 5.14 mmol) were added at -40°C. It was stirred and reacted at -40°C for 1 hour, extracted with water and dichloromethane, and washed with saturated sodium chloride aqueous solution. The organic phase was dried over anhydrous magnesium sulfate and concentrated under reduced pressure to remove the solvent. The remaining was separated by using a rapid silica gel column to obtain tert-butyl 3-(7-chloro-8-fluoro-5-methoxy-2-(methylthio)pyrido[4,3-d]pyrimidin-4-yl)-1-((methox y-*d*₃)methyl)-3,8-diazabicyclo[3.2.1]octan-8-carboxylate (520 mg, yield: 39.1%). ESI-MS 517 [M]⁺

### Step 4: Synthesis of 8-fluoro-7-(7-fluoro-3-(methoxymethoxy)-8-((triisopropylsilyl)ethynyl)naphthalen-1-yl)-5-methoxy-4-(1-((methoxy-d₃)methyl)-3,8-diazabicyclo[3.2.1]octan-3-yl)-2-(m ethylthio)pyrido[4,3-d]pyrimidin

Tert-butyl 3-(7-chloro-8-fluoro-5-methoxy-2-(methylthio)pyrido[4,3-d]pyrimidin-4-yl)-1-((methox y-*d*₃)methyl)-3,8-diazabicyclo[3.2.1]octan-8-carboxylate (420 mg, 0.812 mmol) was dissolved in dioxane (9 mL), and ((2-fluoro-6-(methoxymethoxy)-8-(4,4,5,5-tetramethyl-1,3,2-dioxaborolan-2-yl)naphtha len-1-yl)ethynyl)triisopropylsilane (624 mg, 1.22 mmol), potassium phosphate (689 mg, 3.24 mmol) and 3 mL water were added. [(Bis(1-adamantyl)-N-butylphosphine)-2-(2-aminobiphenyl)palladium(II) chloride was added under nitrogen protection, and was stirred and reacted at 100°C for 1 hour. The reaction solution was slowly poured into 20 mL water, extracted with ethyl acetate (50 mL), and washed with saturated sodium chloride aqueous solution (30 mL). The organic phase was dried over anhydrous magnesium sulfate and concentrated under reduced pressure to remove the solvent. The remaining was separated by using a rapid silica gel column to obtain 8-fluoro-7-(7-fluoro-3-(methoxymethoxy)-8-((triisopropylsilyl)ethynyl)naphthalen-1-yl )-5-methoxy-4-(1-((methoxy-*d*₃)methyl)-3,8-diazabicyclo[3.2.1]octan-3-yl)-2-(methylth io)pyrido[4,3-d]pyrimidin (750 mg). ESI-MS: 867 [M+1]⁺.

¹H NMR (400 MHz, CDCl₃) δ 7.76 (dd, *J* = 5.6, 8.9 Hz, 1H), 7.49 (d, *J* = 1.3 Hz, 1H), 7.37 (dd, *J* = 2.5, 18.6 Hz, 1H), 7.31-7.26 (m, 1H), 5.29 (d, *J* = 2.3 Hz, 2H), 4.96-4.64 (m, 1H), 4.43-4.29 (m, 1H), 4.03-3.87 (m, 4H), 3.77-3.69 (m, 1H), 3.61-3.49 (m, 5H), 3.25-3.06 (m, 1H), 2.60 (d, *J* = 3.8 Hz, 3H), 1.99-1.88 (m, 2H), 1.81-1.58 (m, 5H), 1.51 (s, 9H), 0.90-0.84 (m, 18H).

### Step 5: Synthesis of tert-butyl 3-(8-fluoro-7-(7-fluoro-3-(methoxymethoxy)-8-((triisopropylsilyl)ethynyl)naphthale n-1-yl)-5-methoxy-2-(methylsulfinyl(sulfinyl))pyrido [4,3-d] pyrimidin-4-yl)-1-((met hoxy-d₃)methyl)-3,8-diazabicyclo[3.2.1]octan-8-carboxylate

8-fluoro-7-(7-fluoro-3-(methoxymethoxy)-8-((triisopropylsilyl)ethynyl)naphthalen -1-yl)-5-methoxy-4-(1-((methoxy-*d*₃)methyl)-3,8-diazabicyclo[3.2.1]octan-3-yl)-2-(met hylthio)pyrido[4,3-d]pyrimidin (650 mg, 0.750 mmol) was dissolved in dichloromethane (5 mL), and m-chloroperoxybenzoic acid (0.40 mL, 1.12 mmol) was added. Then it was stirred and reacted at 25°C for 2 hours. The reaction solution was extracted with ethyl acetate and saturated sodium bicarbonate solution, and washed with saturated sodium chloride aqueous solution. The organic phase was dried over anhydrous magnesium sulfate and concentrated under reduced pressure to remove the solvent. The remaining was separated by using a rapid silica gel column to obtain tert-butyl 3-(8-fluoro-7-(7-fluoro-3-(methoxymethoxy)-8-((triisopropylsilyl)ethynyl)naphthalen-1 -yl)-5-methoxy-2-(methylsulfinyl(sulfinyl))pyrido[4,3-d]pyrimidin-4-yl)-1-((methoxy-d ₃)methyl)-3,8-diazabicyclo[3.2.1]octan-8-carboxylate (800 mg, crude product). ESI-MS: 883 [M+1]⁺.

### Step 6: Synthesis of tert-butyl 3-(8-fluoro-7-(7-fluoro-3-(methoxymethoxy)-8-((triisopropylsilyl)ethynyl)naphthale n-1-yl)-2-(((2R,7aS)-2-fluorotetrahydro-1H-pyrrolizin-7a(SH)-yl)methoxy)-5-meth oxypyrido[4,3-d]pyrimidin-4-yl)-1-((methoxy-d₃)methyl)-3,8-diazabicyclo[3.2.1]oct an-8-carboxylate

Tert-butyl 3-(8-fluoro-7-(7-fluoro-3-(methoxymethoxy)-8-((triisopropylsilyl)ethynyl)naphthalen-1 -yl)-5-methoxy-2-(methylsulfinyl(sulfinyl))pyrido[4,3-d]pyrimidin-4-yl)-1-((methoxy-d ₃)methyl)-3,8-diazabicyclo[3.2.1]octan-8-carboxylate (800 mg, 0.82 mmol) was dissolved in tetrahydrofuran (10 mL), and ((2R,7aS)-2-fluorotetrahydro-1H-pyrrolizin-7a(SH)-yl)methanol (194 mg, 1.22 mmol) was added. Lithium bis(trimethylsilyl)amide (0.98 mL, 0.98 mmol) was added under nitrogen protection, and was stirred and reacted at 0°C for 1 hour. The reaction solution was slowly poured into 30 mL water, extracted with ethyl acetate (80 mL), and washed with saturated sodium chloride aqueous solution (30 mL). The organic phase was dried over anhydrous magnesium sulfate and concentrated under reduced pressure to remove the solvent. The remaining was separated by using a rapid silica gel column to obtain tert-butyl 3-(8-fluoro-7-(7-fluoro-3-(methoxymethoxy)-8-((triisopropylsilyl)ethynyl)naphthalen-1 -yl)-2-(((2R,7aS)-2-fluorotetrahydro-1H-pyrrolizin-7a(SH)-yl)methoxy)-5-methoxypyri do[4,3-d]pyrimidin-4-yl)-1-((methoxy-*d*₃)methyl)-3,8-diazabicyclo[3.2.1]octan-8-carbo xylate (580 mg, yield: 52.2%). ESI-MS: 979 [M+1]⁺.

### Step 7: Synthesis of 6-fluoro-4-(8-fluoro-2-(((2R,7aS)-2-fluorotetrahydro-1H-pyrrolizin-7a(5H)-yl)meth oxy)-5-methoxy-4-(1-((methoxy-d₃)methyl)-3,8-diazabicyclo[3.2.1]octan-3-yl)pyrido [4,3-d]pyrimidin-7-yl)-5-((triisopropylsilyl)ethynyl)naphthalen-2-ol

Tert-butyl 3-(8-fluoro-7-(7-fluoro-3-(methoxymethoxy)-8-((triisopropylsilyl)ethynyl)naphthalen-1 -yl)-2-(((2R,7aS)-2-fluorotetrahydro-1H-pyrrolizin-7a(5H)-yl)methoxy)-5-methoxypyri do[4,3-d]pyrimidin-4-yl)-1-((methoxy-*d*₃)methyl)-3,8-diazabicyclo[3.2.1]octan-8-carbo xylate (500 mg, 0.51 mmol) was dissolved in acetonitrile (3 mL), and hydrochloric acid/dioxane (3 mL, 4 M) was added. It was stired and reacted at 25°C for 1 hour. The solvent was removed by concentration under reduced pressure to obtain 6-fluoro-4-(8-fluoro-2-(((2R,7aS)-2-fluorotetrahydro-1H-pyrrolizin-7a(5H)-yl)methoxy )-5-methoxy-4-(1-((methoxy-*d*₃)methyl)-3,8-diazabicyclo[3.2.1]octan-3-yl)pyrido[4,3-d ]pyrimidin-7-yl)-5-((triisopropylsilyl)ethynyl)naphthalen-2-ol (500 mg, yield: 93.0%). The crude product was directly used for the next reaction. ESI-MS: 834 [M+1]⁺.

### Step 8: Synthesis of 5-ethynyl-6-fluoro-4-(8-fluoro-2-(((2R,7aS)-2-fluorotetrahydro-1H-pyrrolizin-7a(5 H)-yl)methoxy)-5-methoxy-4-(1-((methoxy-d₃)methyl)-3,8-diazabicyclo[3.2.1]octan-3-yl)pyrido[4,3-d]pyrimidin-7-yl)naphthalen-2-ol

6-fluoro-4-(8-fluoro-2-(((2R,7aS)-2-fluorotetrahydro-1H-pyrrolizin-7a(5H)-yl)met hoxy)-5-methoxy-4-(1-((methoxy-*d*₃)methyl)-3,8-diazabicyclo[3.2.1]octan-3-yl)pyrido[ 4,3-d]pyrimidin-7-yl)-5-((triisopropylsilyl)ethynyl)naphthalen-2-ol (0.430 g, 0.52 mmol) was dissolved in N,N-dimethylformamide (15 mL), and cesium fluoride (15.7 g, 103 mmol) was added. It was stired and reacted at 25°C for 1 hour. After the reaction solution was filtered, the organic phase was concentrated under reduced pressure to remove the solvent, and the remaining was separated by using a reversed-phase column to obtain 5-ethynyl-6-fluoro-4-(8-fluoro-2-(((2R,7aS)-2-fluorotetrahydro-1H-pyrrolizin-7a(5H)-y l)methoxy)-5-methoxy-4-(1-((methoxy-*d*₃)methyl)-3,8-diazabicyclo[3.2.1]octan-3-yl)py rido[4,3-d]pyrimidin-7-yl)naphthalen-2-ol (100 mg, yield: 26.2%, purity: 95.44%). ESI-MS: 678 [M+1]⁺.

¹H NMR (400 MHz, DMSO-*d*₆) δ 10.56-9.78 (m, 1H), 8.18-8.16 (m, 1H), 7.98 (dd, *J* = 5.9, 9.1 Hz, 1H), 7.47 (t, *J* = 9.0 Hz, 1H), 7.39 (d, *J =* 2.5 Hz, 1H), 7.23 (d, *J* = 2.1 Hz, 1H), 5.39-5.19 (m, 1H), 4.28-4.17 (m, 1H), 4.16-4.09 (m, 1H), 4.06-3.99 (m, 1H), 3.89 (d, *J* = 1.3 Hz, 3H), 3.54 (s, 2H), 3.42 (d, *J* = 4.9 Hz, 2H), 3.21 (d, *J* = 12.1 Hz, 2H), 3.09 (d, *J* = 6.5 Hz, 2H), 3.02 (s, 1H), 2.88-2.78 (m, 1H), 2.53 (s, 1H), 2.13 (s, 1H), 2.07-1.97 (m, 2H), 1.91-1.62 (m, 6H), 1.57-1.37 (m, 2H).

### Examples 37-38 can be prepared by selecting the corresponding raw materials with reference to all or part of the synthesis methods of Example 36:

| **Examp le No.** | **Structural Formula** | **Chemical Name** | **ESI-MS: [M+1]⁺** |
|---|---|---|---|
| **Examp le 37** | 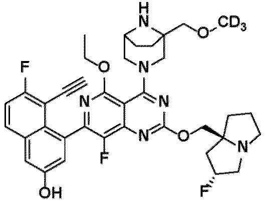 | 4-(5-ethoxy-8-fluoro-2-(((2R,7 aS)-2-fluorotetrahydro- 1H-pyrr olizin-7a(5H)-yl)methoxy)-4-(( 1S,5R)-1-((methoxy-*d*₃)methyl )-3,8-diazabicyclo[3.2.1]octan-3 -yl)pyrido[4,3-d]pyrimidin-7-yl)-5-ethynyl-6-fluoronaphthal en-2-ol | 692 |
| **Examp le 38** | 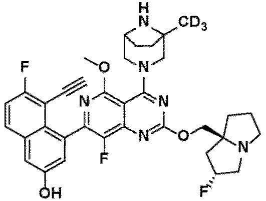 | 5-ethynyl-6-fluoro-4-(8-fluoro-2-(((2R,7aS)-2-fluorotetrahydr o-1H-pyrrolizin-7a(5H)-yl)met hoxy)-5-methoxy-4-(1-(methyl *d*₃)-3,8-diazabicyclo[3.2.1]octa n-3-yl)pyrido[4,3-d]pyrimidin-7-yl)naphthalen-2-ol | 648 |

### HNMR data for the compounds prepared in the above examples are as follows:

| **Example No.** | **¹H NMR (400 MHz)** |
|---|---|
| **Example 37** | ¹H NMR (400 MHz, DMSO-*d*₆) δ 8.20 (s, 1H), 8.01-7.93 (m, 1H), 7.47 (t, *J* = 8.9 Hz, 1H), 7.38 (s, 1H), 7.22 (s, 1H), 5.40-5.16 (m, 1H), 4.48-4.25 (m, 3H), 4.18-3.96 (m, 2H), 3.89 (s, 1H), 3.79 (s, 1H), 3.33 (d, *J* = 8.5 Hz, 4H), 3.09 (d, *J* = 4.5 Hz, 3H), 3.02 (s, 1H), 2.83 (d, *J* = 6.5 Hz, 1H), 2.57 (s, 2H), 2.12 (s, 1H), 2.07-1.97 (m, 2H), 1.89-1.76 (m, 3H), 1.72-1.59 (m, 2H), 1.43-1.28 (m, 5H). |
| **Example 38** | ¹H NMR (400 MHz, DMSO-*d*₆) δ 10.14 (s, 1H), 7.97 (dd, *J* = 4.0, 8.0 Hz, 1H), 7.46 (t, *J* = 8.0 Hz, 1H), 7.38 (d, *J* = 4.0 Hz, 1H), 7.23-7.21 (m, 1H), 5.35-5.20 (m, 1H), 4.14-4.08 (m, 1H), 4.03-3.97 (m, 2H), 3.88 (d, *J* = 4.0 Hz, 5H), 3.50-3.46 (m, 1H), 3.13-3.08 (m, 3H), 3.01 (s, 1H), 2.85-2.79 (m, 1H), 2.12 (s, 1H), 2.08-1.98 (m, 2H), 1.85-1.53 (m, 7H), 1.31-1.23 (m, 1H). |

### Example 39: Preparation of 5-ethynyl-6-fluoro-4-(8-fluoro-2-(((2R,7aS)-2-fluorotetrahydro-1H-pyrrolizin-7a(5 H)-yl)methoxy)-4-(1-((methoxy-d₃)methyl)-3,8-diazabicyclo[3.2.1]octan-3-yl)-5-met hylpyrido[4,3-d]pyrimidin-7-yl)naphthalen-2-ol

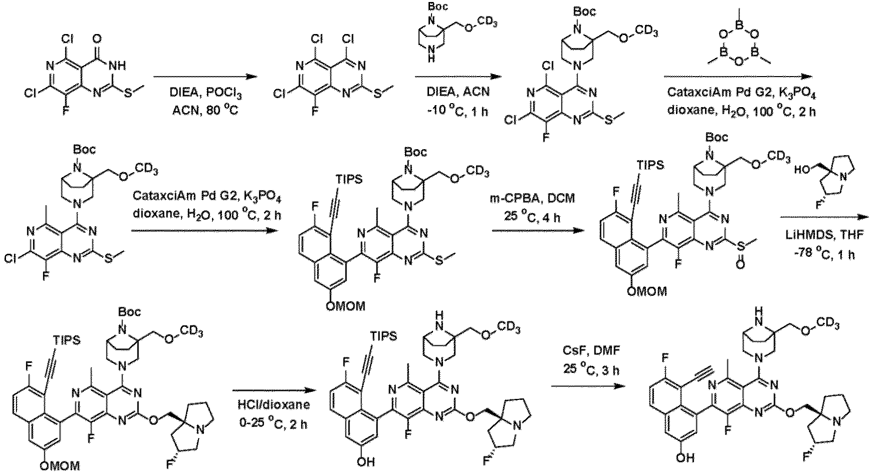

### Step 1: Synthesis of 4,5,7-trichloro-8-fluoro-2-(methylthio)pyrido [4,3-d] pyrimidin

5,7-dichloro-8-fluoro-2-(methylthio)pyrido[4,3-d]pyrimidin-4(3H)-one (1 g, 3.57 mmol) was dissolved in anhydrous acetonitrile (10 mL), and N,N-diisopropylethylamine (2.36 mL, 14.3 mmol) and phosphorus oxychloride (0.67 mL, 7.14 mmol) were added. The mixture was reacted at 80°C for 1 hour. LC-MS showed that the reaction was completed. The reaction solution was diluted with ethyl acetate (100 mL), and washed with saturated sodium chloride aqueous solution (100 mL). The organic phase was dried over anhydrous sodium sulfate and concentrated under reduced pressure to remove solvent to obtain 4,5,7-trichloro-8-fluoro-2-(methylthio)pyrido[4,3-d]pyrimidin (0.9 g, yield: 84.1%). ESI-MS: 298 [M+1]⁺.

### Step 2: Synthesis of tert-butyl 3-(5,7-dichloro-8-fluoro-2-(methylthio)pyrido[4,3-d]pyrimidin-4-yl)-1-((methoxy-d₃ )methyl)-3,8-diazabicyclo[3.2.1]octan-8-carboxylate

4,5,7-trichloro-8-fluoro-2-(methylthio)pyrido[4,3-d]pyrimidin (0.9 g, 3.0 mmol) and N,N-diisopropylethylamine (2.0 mL, 12.1 mmol) were dissolved in anhydrous acetonitrile (15 mL), and tert-butyl 1-((methoxy-*d*₃)methyl)-3,8-diazabicyclo[3.2.1]octan-8-carboxylate (700 mg, 2.67mmol) was added at -10°C. The mixture was reacted at -10°C for 1 hour. LC-MS showed that the reaction was completed. The reaction solution was poured into 100 mL water, extracted with ethyl acetate (100 mL), and washed with saturated sodium chloride aqueous solution (100 mL). The organic phase was dried over anhydrous sodium sulfate and concentrated under reduced pressure to remove the solvent. The remaining was separated by using a rapid silica gel column to obtain tert-butyl 3-(5,7-dichloro-8-fluoro-2-(methylthio)pyrido[4,3-d]pyrimidin-4-yl)-1-((methoxy-*d*₃)m ethyl)-3,8-diazabicyclo[3.2.1]octan-8-carboxylate (0.78 g, yield: 50%). ESI-MS: 521 [M+1]⁺.

### Step 3: Synthesis of tert-butyl 3-(7-chloro-8-fluoro-5-methyl-2-(methylthio)pyrido [4,3-d] pyrimidin-4-yl)-1-((meth oxyl-d₃)methyl)-3,8-diazabicyclo[3.2.1] octan-8-carboxylate

Tert-butyl 3-(5,7-dichloro-8-fluoro-2-(methylthio)pyrido[4,3-d]pyrimidin-4-yl)-1-((methoxy-*d*₃)m ethyl)-3,8-diazabicyclo[3.2.1]octan-8-carboxylate (0.53 g, 1.02 mmol) and 2,4,6-trimethyl-1,3,5,2,4,6-trioxatriborinane (64 mg, 0.51 mmol) were dissolved in anhydrous 1,4-dioxane (10 mL). Potassium phosphate (647 mg, 3.05 mmol) and adamantane palladium(II) catalyst (68 mg, 0.102 mmol) were added respectively, and the mixture was reacted at 100°C for 3 hours. LC-MS showed that the reaction was completed. The reaction solution was poured into 50 mL water, extracted with ethyl acetate (50 mL), and washed with saturated sodium chloride aqueous solution (50 mL). The organic phase was dried over anhydrous sodium sulfate and concentrated under reduced pressure to remove the solvent. The remaining was separated by using a rapid silica gel column to obtain tert-butyl 3-(7-chloro-8-fluoro-5-methyl-2-(methylthio)pyrido[4,3-d]pyrimidin-4-yl)-1-((methoxy -*d*₃)methyl)-3,8-diazabicyclo[3.2.1]octan-8-carboxylate (261 mg, yield: 51.8%). ESI-MS: 501 [M+1]⁺.

### Step 4: Synthesis of tert-butyl 3-(8-fluoro-7-(7-fluoro-3-(methoxymethoxy)-8-((triisopropylsilyl)ethynyl)naphthale n-1-yl)-5-methyl-2-(methylthio)pyrido[4,3-d]pyrimidin-4-yl)-1-((methoxy-d₃)methy l)-3,8-diazabicyclo[3.2.1]octan-8-carboxylate

Tert-butyl 3-(7-chloro-8-fluoro-5-methoxy-2-(methylthio)pyrido[4,3-d]pyrimidin-4-yl)-1-((methox y-*d*₃)methyl)-3,8-diazabicyclo[3.2.1]octan-8-carboxylate (0.21 g, 0.42 mmol) and ((2-fluoro-6-(methoxymethoxy)-8-(4,4,5,5-tetramethyl-1,3,2-dioxaborolan-2-yl)naphtha len-1-yl)ethynyl)triisopropylsilane (322 mg, 0.63 mmol) were dissolved in anhydrous 1,4-dioxane (10 mL) and water (3 mL). Potassium phosphate (356 mg, 1.68 mmol) and adamantane palladium(II) catalyst (56 mg, 0.09 mmol) were added respectively, and the mixture was reacted at 100°C for 1 hour. LC-MS showed that the reaction was completed. The reaction solution was poured into 50 mL water, extracted with ethyl acetate (50 mL), and washed with saturated sodium chloride aqueous solution (50 mL). The organic phase was dried over anhydrous sodium sulfate and concentrated under reduced pressure to remove the solvent. The remaining was separated by using column chromatography to obtain tert-butyl 3-(8-fluoro-7-(7-fluoro-3-(methoxymethoxy)-8-((triisopropylsilyl)ethynyl)naphthalen-1 -yl)-5-methyl-2-(methylthio)pyrido[4,3-d]pyrimidin-4-yl)-1-((methoxy-*d*₃)methyl)-3,8-diazabicyclo[3.2.1]octan-8-carboxylate (105 mg, yield: 29.2%). ESI-MS: 851 [M+1]⁺.

### Step 5: Synthesis of tert-butyl 3-(8-fluoro-7-(7-fluoro-3-(methoxymethoxy)-8-((triisopropylsilyl)ethynyl)naphthale n-1-yl)-5-methyl-2-(methylsulfinyl(sulfinyl))pyrido[4,3-d]pyrimidin-4-yl)-1-((metho xy-d₃)methyl)-3,8-diazabicyclo[3.2.1]octan-8-carboxylate

Tert-butyl 3-(8-fluoro-7-(7-fluoro-3-(methoxymethoxy)-8-((triisopropylsilyl)ethynyl)naphthalen-1 -yl)-5-methyl-2-(methylthio)pyrido[4,3-d]pyrimidin-4-yl)-1-((methoxy-*d*₃)methyl)-3,8-diazabicyclo[3.2.1]octan-8-carboxylate (105 mg, 0.123 mmol) was dissolved in anhydrous dichloromethane (10 mL), m-CPBA (32 mg, 0.186 mmol) was added, and the mixture was reacted at 25°C for 3 hours. LC-MS showed that the reaction was completed. The reaction solution was poured into 50 mL water, extracted with ethyl acetate (50 mL), and washed with saturated sodium chloride aqueous solution (50 mL). The organic phase was dried over anhydrous sodium sulfate and concentrated under reduced pressure to remove the solvent to obtain tert-butyl 3-(8-fluoro-7-(7-fluoro-3-(methoxymethoxy)-8-((triisopropylsilyl)ethynyl)naphthalen-1 -yl)-5-methyl-2-(methylsulfinyl(sulfinyl))pyrido[4,3-d]pyrimidin-4-yl)-1-((methoxy-*d*₃) methyl)-3,8-diazabicyclo[3.2.1]octan-8-carboxylate (0.1 g, yield: 90.9%). ESI-MS: 867 [M+1]⁺.

### Step 6: Synthesis of tert-butyl 3-(8-fluoro-7-(7-fluoro-3-(methoxymethoxy)-8-((triisopropylsilyl)ethynyl)naphthale n-1-yl)-2-(((2R,7aS)-2-fluorotetrahydro-1H-pyrrolizin-7a(SH)-yl)methoxy)-5-meth ylpyrido[4,3-d]pyrimidin-4-yl)-1-((methoxy-d₃)methyl)-3,8-diazabicyclo[3.2.1]octa n-8-carboxylate

Tert-butyl 3-(8-fluoro-7-(7-fluoro-3-(methoxymethoxy)-8-((triisopropylsilyl)ethynyl)naphthalen-1 -yl)-5-methyl-2-(methylsulfinyl(sulfinyl))pyrido[4,3-d]pyrimidin-4-yl)-1-((methoxy-*d*₃) methyl)-3,8-diazabicyclo[3.2.1]octan-8-carboxylate (0.1 g, 0.115 mmol) and ((2R,7aS)-2-fluorotetrahydro-1H-pyrrolizin-7a(SH)-yl)methanol (24 mg, 0.15 mmol) were dissolved in anhydrous tetrahydrofuran (10 mL), and LHMDS (0.14 mL, 0.14 mmol) was added at 0°C, and the mixture was reacted at 0°C for 1 hour. LC-MS showed that the reaction was completed. The reaction solution was poured into 50 mL water, extracted with ethyl acetate (50 mL), and washed with saturated sodium chloride aqueous solution (50 mL). The organic phase was dried over anhydrous sodium sulfate and concentrated under reduced pressure to remove the solvent to obtain tert-butyl 3-(8-fluoro-7-(7-fluoro-3-(methoxymethoxy)-8-((triisopropylsilyl)ethynyl)naphthalen-1 -yl)-2-(((2R,7aS)-2-fluorotetrahydro-1H-pyrrolizin-7a(5H)-yl)methoxy)-5-methylpyrido [4,3-d]pyrimidin-4-yl)-1-((methoxy-*d*₃)methyl)-3,8-diazabicyclo[3.2.1]octan-8-carboxyl ate (106 mg, yield: 95.5%). ESI-MS: 963 [M+1]⁺.

### Step 7: Synthesis of 6-fluoro-4-(8-fluoro-2-(((2R,7aS)-2-fluorotetrahydro-1H-pyrrolizin-7a(SH)-yl)meth oxy)-4-(1-((methoxy-d₃)methyl)-3,8-diazabicyclo[3.2.1]octan-3-yl)-5-methylpyrido[ 4,3-d]pyrimidin-7-yl)-5-((triisopropylsilyl)ethynyl)naphthalen-2-ol

Tert-butyl 3-(8-fluoro-7-(7-fluoro-3-(methoxymethoxy)-8-((triisopropylsilyl)ethynyl)naphthalen-1 -yl)-2-(((2R,7aS)-2-fluorotetrahydro-1H-pyrrolizin-7a(5H)-yl)methoxy)-5-methylpyrido [4,3-d]pyrimidin-4-yl)-1-((methoxy-*d*₃)methyl)-3,8-diazabicyclo[3.2.1]octan-8-carboxyl ate (82 mg, 0.09 mmol) was dissolved in acetonitrile (3 mL), and hydrochloric acid/1,4-dioxane solution (3 mL, 4 M) was added. The mixture was reacted at 25°C for 2 hours. LC-MS showed that the reaction was completed. The reaction solution was concentrated under reduced pressure to remove the solvent to obtain 6-fluoro-4-(8-fluoro-2-(((2R,7aS)-2-fluorotetrahydro-1H-pyrrolizin-7a(5H)-yl)methoxy )-4-(1-((methoxy-*d*₃)methyl)-3,8-diazabicyclo[3.2.1]octan-3-yl)-5-methylpyrido[4,3-d]p yrimidin-7-yl)-5-((triisopropylsilyl)ethynyl)naphthalen-2-ol (90 mg, crude product). ESI-MS: 818 [M+1]⁺.

### Step 8: Synthesis of 5-ethynyl-6-fluoro-4-(8-fluoro-2-(((2R,7aS)-2-fluorotetrahydro-1H-pyrrolizin-7a(5 H)-yl)methoxy)-4-(1-((methoxy-d₃)methyl)-3,8-diazabicyclo[3.2.1]octan-3-yl)-5-met hylpyrido[4,3-d]pyrimidin-7-yl)naphthalen-2-ol

6-fluoro-4-(8-fluoro-2-(((2R,7aS)-2-fluorotetrahydro-1H-pyrrolizin-7a(5H)-yl)met hoxy)-4-(1-((methoxy-*d*₃)methyl)-3,8-diazabicyclo[3.2.1]octan-3-yl)-5-methylpyrido[4, 3-d]pyrimidin-7-yl)-5-((triisopropylsilyl)ethynyl)naphthalen-2-ol (90 mg, 0.11 mmol) was dissolved in N,N-dimethylformamide (6 mL), and cesium fluoride (3.34 g, 22 mmol) was added. The reaction lasted for 1 hour at room temperature. LC-MS showed that the reaction was completed. The reaction solution was filtered, the filtrate was concentrated under reduced pressure to remove the solvent, and the remaining was separated by using a reversed-phase column to obtain 5-ethynyl-6-fluoro-4-(8-fluoro-2-(((2R,7aS)-2-fluorotetrahydro-1H-pyrrolizin-7a(5H)-y l)methoxy)-4-(1-((methoxy-*d*₃)methyl)-3,8-diazabicyclo[3.2.1]octan-3-yl)-5-methylpyri do[4,3-d]pyrimidin-7-yl)naphthalen-2-ol (5 mg, yield: 6.68%, purity: 97.2%). ESI-MS: 662 [M+1]⁺.

¹H NMR (400MHz, DMSO-*d*₆ ) δ 10.14 (s, 1H), 8.05-7.92 (m, 1H), 7.51-7.35 (m, 2H), 7.23 (s, 1H), 5.41-5.17 (m, 1H), 4.23-3.95 (m, 2H), 3.75-3.47 (m, 3H), 3.47-3.40 (m, 2H), 3.36 (m, 3H), 3.15-2.97 (m, 3H), 2.84 (s, 1H), 2.68 (s, 1H), 2.34 (s, 2H), 2.19-1.93 (m, 4H), 1.78 (s, 4H), 1.72-1.48 (m, 1H), 1.43-1.19 (m, 1H), 0.86 (s, 1H).

### Examples 40 can be prepared by selecting the corresponding raw materials with reference to all or part of the synthesis methods of Example 39:

| **Examp le No.** | **Structural Formula** | **Chemical Name** | **MS m/z [M+1]⁺** |
|---|---|---|---|
| **Examp le 40** | 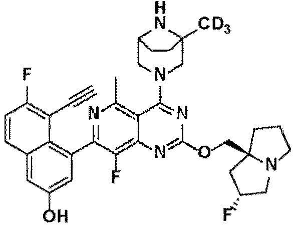 | 5-ethynyl-6-fluoro-4-(8-fluoro-2-(((2R,7aS)-2-fluorotetrahydr o-1H-pyrrolizin-7a(5H)-yl)met hoxy)-5-methyl-4-(1-(methyl-*d* ₃)-3,8-diazabicyclo[3.2.1]octan -3-yl)pyrido[4,3-d]pyrimidin-7 -yl)naphthalen-2-ol | 632 |

### HNMR data for the compounds prepared in the above examples are as follows:

| **Example No.** | **¹H NMR (400 MHz)** |
|---|---|
| **Example 40** | ¹H NMR (400 MHz, DMSO-*d*₆) δ 10.13 (s, 1H), 7.97 (dd, *J* = 8.0, 4.0 Hz, 1H), 7.46 (t, *J* = 8.0 Hz, 1H), 7.38 (d, *J* = 4.0 Hz, 1H), 7.22-7.20 (m, 1H), 5.35-5.20 (m, 1H), 4.17-4.10 (m, 1H), 4.06-3.99(m, 1H), 3.72-3.58 (m, 2H), 3.46 (s, 2H), 3.14-3.01 (m, 4H), 2.86-2.80 (m, 1H), 2.56 (s, 3H), 2.14-1.93 (m, 4H), 1.89-1.60 (m, 5H), 1.26-1.23 (m, 2H). |

### Examples 41 and 42: Preparation of 5-ethynyl-6-fluoro-4-(8-fluoro-2-(((2R,7aS)-2-fluorotetrahydro-1H-pyrrolizin-7a-yl )methoxy)-4-((1R,5S)-1-(methoxymethyl)-3,8-diazabicyclo[3.2.1]octan-3-yl)pyrido[ 4,3-d]pyrimidin-7-yl)naphthalen-2-ol and 5-ethynyl-6-fluoro-4-(8-fluoro-2-(((2R,7aS)-2-fluorotetrahydro-1H-pyrrolizin-7a-yl )methoxy)-4-((1S,SR)-1-(methoxymethyl)-3,8-diazabicyclo[3.2.1]octan-3-yl)pyrido[ 4,3-d]pyrimidin-7-yl)naphthalen-2-ol

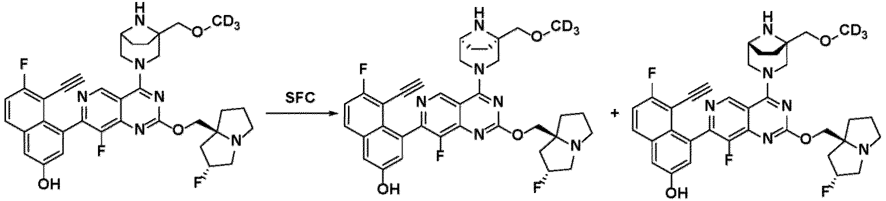

5-ethynyl-6-fluoro-4-(8-fluoro-2-(((2R,7aS)-2-fluorotetrahydro-1H-pyrrolizin-7a-y l)methoxy)-4-(1-(methoxymethyl)-3,8-diazabicyclo[3.2.1]octan-3-yl)pyrido[4,3-d]pyri midin-7-yl)naphthalen-2-ol (1.9 g, 2.93 mmol) was separated through chiral resolution (Column: DAICEL CHIRALPAK AD (250 mm*50 mm, 10 um), Condition: 0.1% ammonia in IPA), and examples 41 and 42 were obtained respectively.

### Example 41, (663 mg, yield: 34.3%), ESI-MS: 648[M+1]⁺.

¹H NMR (400 MHz, DMSO-*d*₆) δ 9.00 (s, 1H), 7.78 (dd, *J* = 6.0, 9.1, Hz, 1H), 7.32 (t, *J=* 9.0 Hz, 1H), 7.19-7.06 (m, 2H), 5.37-5.17 (m, 1H), 4.57-4.29 (m, 2H), 4.12 (d, *J=* 10.4 Hz, 1 H), 3.99 (d, *J* = 10.4 Hz, 1 H), 3.88-3.74 (m, 1H), 3.63-3.51 (m, 5H), 3.14-2.97 (m, 3H), 2.87-2.75 (m, 1H), 2.13 (d, *J* = 3.6 Hz, 1H), 2.07-1.93 (m, 2H), 1.90-1.56 (m, 7H), 1.45 (d, *J* = 8.8 Hz, 1H).

### Example 42, (767 mg, yield: 39.5%), ESI-MS: 648[M+1]⁺.

¹H NMR (400 MHz, DMSO-*d*₆) δ 9.00 (s, 1H), 7.79 (dd, *J* = 6.1, 9.1 Hz, 1H), 7.32 (t, *J* = 9.0 Hz, 1H), 7.17 (d, *J* = 2.3 Hz, 1 H), 7.11 (d, *J* = 2.3 Hz, 1H), 5.37-5.17 (m, 1H), 4.56-4.30 (m, 2H), 4.13-3.99 (m, 2H), 3.86-3.76 (m, 1H), 3.61-3.53 (m, 5H) 3.14-2.98 (m, 3H), 2.86-2.77 (m, 1H), 2.12 (s, 1H), 2.06-1.96 (m, 2H), 1.90-1.55 (m, 7H), 1.45 (d, *J* = 10.0 Hz, 1H).

### Examples 43 and 44: Preparation of 5-ethynyl-6-fluoro-4-(8-fluoro-2-(((2R,7aS)-2-fluorotetrahydro-1H-pyrrolizin-7a(5 H)-yl)methoxy)-4-((1R,5S)-1-(methyl-d₃)-3,8-diazabicyclo[3.2.1]octan-3-yl)pyrido[4 ,3-d]pyrimidin-7-yl)naphthalen-2-ol and 5-ethynyl-6-fluoro-4-(8-fluoro-2-(((2R,7aS)-2-fluorotetrahydro-1H-pyrrolizin-7a(5 H)-yl))methoxy)-4-((1S,5R)-1-(methyl-d₃)-3,8-diazabicyclo[3.2.1]octan-3-yl)pyrido[ 4,3-d]pyrimidin-7-yl)naphthalen-2-ol

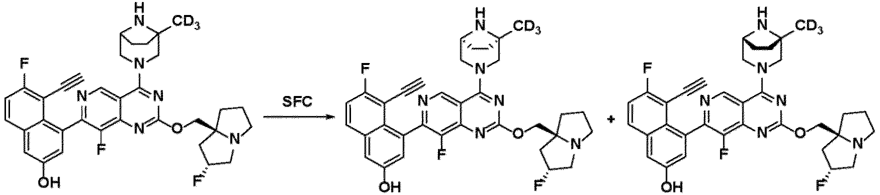

5-ethynyl-6-fluoro-4-(8-fluoro-2-(((2R,7aS)-2-fluorotetrahydro-1H-pyrrolizin-7a(5 H)-yl)methoxy)-4-(1-(methyl-*d*₃)-3,8-diazabicyclo[3.2.1]octan-3-yl)pyrido[4,3-d]pyrimi din-7-yl)naphthalen-2-ol was separated through chiral resolution (Column: DAICEL CHIRALPAK OX (250 mm*50 mm, 10 um), Condition: CO₂/MeOH (0.2% NH₃) = 45/55), and examples 43 and 44 were obtained respectively.

### Example 43, ESI-MS: 618[M+1]⁺.

¹H NMR (400 MHz, DMSO-*d*₆) δ 9.03 (s, 1H), 7.95 (dd, *J* = 6.0, 9.2 Hz, 1H), 7.45 (t, *J* = 9.0 Hz, 1H), 7.36 (d, *J* = 2.5 Hz, 1H), 7.17 (d, *J* = 2.5 Hz, 1H), 5.28 (d, *J* = 52 Hz, 1H), 4.55-4.31 (m, 2H), 4.14-4.00 (m, 2H), 3.89 (d, *J* = 27.6 Hz, 1H), 3.64-3.36 (m, 6H), 3.13-3.06 (m, 2H), 3.01 (s, 1H), 2.86-2.77 (m, 1H), 2.14-1.95 (m, 4H), 1.88-1.40 (m, 7H).

### Example 44, ESI-MS: 618[M+1]⁺.

¹H NMR (400 MHz, DMSO-*d*₆) δ 9.02 (d, *J* = 2.7 Hz, 1H), 7.94 (t, *J* = 7.8 Hz, 1H), 7.44 (t, *J* = 9.0 Hz, 1H), 7.35 (s, 1H), 7.17 (d, *J* = 2.9 Hz, 1H), 5.27 (d, *J* = 54.2 Hz, 1H), 4.57-4.28 (m, 2H), 4.19-3.97 (m, 2H), 3.89 (d, *J* = 28.9 Hz, 1H), 3.66-3.37 (m, 5H), 3.15-2.97 (m, 4H), 2.86-2.76 (m, 1H), 2.19-1.93 (m, 4H), 1.88-1.39 (m, 7H).

### Examples 45 and 46: Preparation of 5-ethynyl-6-fluoro-4-(8-fluoro-2-(((2R,7aS)-2-fluorotetrahydro-1H-pyrrolizin-7a(5 H)-yl)methoxy)-4-((1S,SR)-1-(methoxymethyl)-3,8-diazabicyclo[3.2.1]octan-3-yl)py rido[4,3-d]pyrimidin-7-yl)naphthalen-2-ol and 5-ethynyl-6-fluoro-4-(8-fluoro-2-(((2R,7aS)-2-fluorotetrahydro-1H-pyrrolizin-7a(5 H)-yl)methoxy)-4-((1R,5S)-1-(methoxymethyl)-3,8-diazabicyclo[3.2.1]octan-3-yl)py rido[4,3-d]pyrimidin-7-yl)naphthalen-2-ol

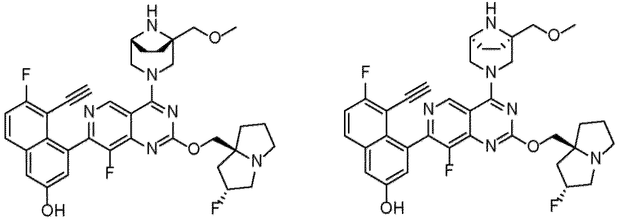

Examples 45 and 46 may be prepared by selecting appropriate conditions with reference to the methods of examples 41 and 42.

### Example 45: ESI-MS: 645 [M+1]⁺.

¹H NMR (400 MHz, DMSO-*d*₆) δ 9.03 (s, 1H), 7.96 (dd, *J* = 5.9, 9.2 Hz, 1H), 7.45 (t, *J* = 9.0 Hz, 1H), 7.38 (d, *J* = 2.3 Hz, 1H), 7.18 (d, *J* = 2.3 Hz, 1H), 5.38-5.17 (m, 1H), 4.57-4.28 (m, 2H), 4.15-4.00 (m, 2H), 3.95-3.84 (m, 1H), 3.67-3.49 (m, 3H), 3.49-3.38 (m, 5H), 3.13-2.99 (m, 3H), 2.87-2.78 (m, 1H), 2.15-1.95 (m, 3H), 1.91-1.58 (m, 7H), 1.46 (d, *J* = 8.8 Hz, 1H).

### Example 46: ESI-MS: 645 [M+1]⁺.

¹H NMR (400MHz, DMSO-*d*₆) δ 10.18 (s, 1H), 9.03 (s, 1H), 7.98 (dd, *J* = 5.8, 9.0 Hz, 1H), 7.47 (t, *J* = 9.0 Hz, 1H), 7.39 (d, *J* = 2.5 Hz, 1H), 7.21-7.16 (m, 1H), 5.37-5.17 (m, 1H), 4.58-4.29 (m, 2H), 4.14 (dd, *J* = 3.9, 10.4 Hz, 1H), 4.01 (dd, *J* = 3.3, 10.3 Hz, 1H), 3.96-3.86 (m, 1H), 3.65-3.51 (m, 2H), 3.49-3.37 (m, 5H), 3.14-2.99 (m, 3H), 2.87-2.78 (m, 1H), 2.13 (br s, 1H), 2.08-1.95 (m, 2H), 1.89-1.60 (m, 7H), 1.46 (d, *J* = 9.0 Hz, 1H).

### Examples 47 and 48: Preparation of 5-ethynyl-6-fluoro-4-(8-fluoro-2-(((2R,7aS)-2-fluorotetrahydro-1H-pyrrolizin-7a(5 H)-yl)methoxy)-4-((1S,5R)-1-((methoxy-d₃)methyl)-3,8-diazabicyclo[3.2.1]octan-3-y l-5-d)pyrido[4,3-d]pyrimidin-7-yl)naphthalen-2-ol and 5-ethynyl-6-fluoro-4-(8-fluoro-2-(((2R,7aS)-2-fluorotetrahydro-1H-pyrrolizin-7a(5 H)-yl)methoxy)-4-((1R,5S)-1-((methoxy-d₃)methyl)-3,8-diazabicyclo[3.2.1]octan-3-y l-5-d)pyrido[4,3-d]pyrimidin-7-yl)naphthalen-2-ol

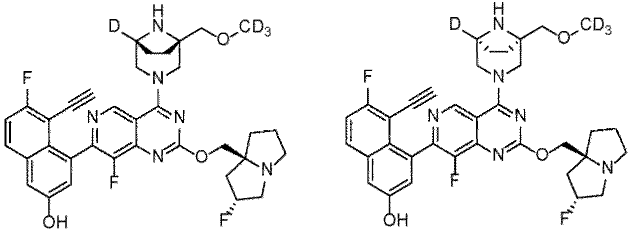

Examples 47 and 48 may be prepared by selecting appropriate conditions with reference to the methods of examples 41 and 42.

**Example 47:** ¹H NMR (400 MHz, DMSO-*d*₆) δ 10.22-10.07 (m, 1H), 9.01 (s, 1H), 7.95 (dd, *J* = 6.0, 9.1 Hz, 1H), 7.44 (t, *J* = 9.0 Hz, 1H), 7.37 (d, *J* = 2.4 Hz, 1H), 7.19-7.13 (m, 1H), 5.43-5.14 (m, 1H), 4.56-4.26 (m, 2H), 4.12 (dd, *J* = 3.8, 10.4 Hz, 1H), 4.17-3.95 (m, 1H), 3.99 (dd, *J* = 3.2, 10.3 Hz, 1H), 3.93-3.81 (m, 1H), 3.64-3.48 (m, 1H), 3.46-3.42 (m, 1H), 3.41-3.35 (m, 2H), 3.13-2.96 (m, 3H), 2.86-2.74 (m, 1H), 2.13-1.94 (m, 3H), 1.89-1.57 (m, 7H), 1.51-1.35 (m, 1H).

**Example 48:** ¹H NMR (400 MHz, DMSO-*d*₆) δ 10.17 (s, 1H), 9.04 (s, 1H), 7.98 (dd, *J* = 6.0, 9.1 Hz, 1H), 7.47 (t, *J* = 9.1 Hz, 1H), 7.40 (d, *J* = 2.4 Hz, 1H), 7.19 (d, *J* = 2.3 Hz, 1H), 5.39-5.17 (m, 1H), 4.58-4.29 (m, 2H), 4.15-4.00 (m, 2H), 3.97-3.85 (m, 1H), 3.65-3.50 (m, 1H), 3.49-3.45 (m, 1H), 3.45-3.37 (m, 2H), 3.18-2.99 (m, 3H), 2.88-2.79 (m, 1H), 2.16-1.98 (m, 3H), 1.93-1.55 (m, 7H), 1.51-1.42 (m, 1H).

### Examples 49 and 50: Preparation of 5-ethynyl-6-fluoro-4-(8-fluoro-2-(((2R,7aS)-2-fluorotetrahydro-1H-pyrrolizin-7a(5 H)-yl)methoxy)-5-methoxy-4-((1S,5R)-1-((methoxy-d₃)methyl)-3,8-diazabicyclo[3.2. 1]octan-3-yl)pyrido[4,3-d]pyrimidin-7-yl)naphthalen-2-ol and 5-ethynyl-6-fluoro-4-(8-fluoro-2-(((2R,7aS)-2-fluorotetrahydro-1H-pyrrolizin-7a(5 H)-yl)methoxy)-5-methoxy-4-((1R,5S)-1-((methoxy-d₃)methyl)-3,8-diazabicyclo[3.2. 1]octan-3-yl)pyrido[4,3-d]pyrimidin-7-yl)naphthalen-2-ol

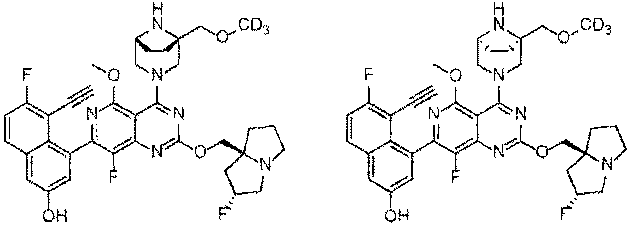

Examples 49 and 50 may be prepared by selecting appropriate conditions with reference to the methods of examples 41 and 42.

**Example 49:** ¹H NMR (400 MHz, DMSO-*d*₆) δ 10.47-9.89 (m, 1H), 7.98 (dd, *J* = 5.9, 9.1 Hz, 1H), 7.47 (t, *J* = 8.9 Hz, 1H), 7.39 (d, *J* = 2.4 Hz, 1H), 7.23 (t, *J* = 2.9 Hz, 1H), 5.36-5.20 (m, 1H), 4.26-4.18 (m, 1H), 4.12-4.08 (m, 1H), 4.05-4.02 (m, 1H), 3.89 (s, 3H), 3.09 (d, *J =* 5.0 Hz, 3H), 3.01 (s, 1H), 2.88-2.78 (m, 2H), 2.51 (s, 3H), 2.12 (s, 1H), 2.06-1.97 (m, 3H), 1.85-1.65 (m, 7H), 1.42 (d, *J* = 12.1 Hz, 2H).

**Example 50:** ¹H NMR (400 MHz, DMSO-*d*₆) δ 11.45-9.14 (m, 1H), 7.97 (dd, *J* = 6.1, 8.9 Hz, 1H), 7.47 (t, *J* = 9.0 Hz, 1H), 7.39 (d, *J* = 2.1 Hz, 1H), 7.28-7.19 (m, 1H), 5.36-5.20 (m, 1H), 4.20 (d, *J* = 16.4 Hz, 1H), 4.14 (d, *J* = 10.4 Hz, 1H), 3.99 (dd, *J* = 2.6, 10.3 Hz, 1H), 3.89 (s, 3H), 3.52 (s, 2H), 3.08 (d, *J* = 6.9 Hz, 3H), 3.01 (s, 1H), 2.86-2.79 (m, 1H), 2.51 (s, 2H), 2.13-1.96 (m, 4H), 1.87-1.63 (m, 7H), 1.47-1.35 (m, 2H).

### Examples 51 and 52: Preparation of 4-(5-ethoxy-8-fluoro-2-(((2R,7aS)-2-fluorotetrahydro-1H-pyrrolizin-7a(5H)-yl)met hoxy)-4-((1S,5R)-1-((methoxy-d₃)methyl)-3,8-diazabicyclo[3.2.1]octan-3-yl)pyrido[ 4,3-d]pyrimidin-7-yl)-5-ethynyl-6-fluoronaphthalen-2-ol and 4-(5-ethoxy-8-fluoro-2-(((2R,7aS)-2-fluorotetrahydro-1H-pyrrolizin-7a(5H)-yl)met hoxy)-4-((1R,5S)-1-((methoxy-d₃)methyl)-3,8-diazabicyclo[3.2.1]octan-3-yl)pyrido[ 4,3-d] pyrimidin-7-yl)-5-ethynyl-6-fluoronaphthalen-2-ol

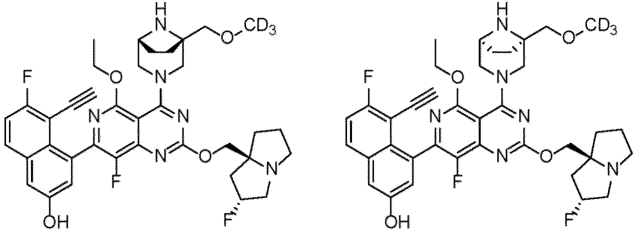

Examples 51 and 52 may be prepared by selecting appropriate conditions with reference to the methods of examples 41 and 42.

**Example 51:** ¹H NMR (400 MHz, DMSO-*d*₆) δ 10.17 (s, 1H), 8.01-7.94 (m, 1H), 7.47 (t, *J* = 9.0 Hz, 1H), 7.38 (d, *J* = 2.1 Hz, 1H), 7.22 (s, 1H), 5.39-5.16 (m, 1H), 4.48-4.26 (m, 2H), 4.13-4.07 (m, 1H), 4.06-4.01 (m, 1H), 3.90-3.76 (m, 1H), 3.52 (s, 1H), 3.45-3.39 (m, 4H), 3.32-3.31 (m, 1H), 3.09 (d, *J* = 4.4 Hz, 2H), 3.04-3.00 (m, 1H), 3.02 (s, 1H), 2.87-2.79 (m, 1H), 2.12 (s, 1H), 2.07-1.97 (m, 2H), 1.89-1.74 (m, 3H), 1.72-1.46 (m, 3H), 1.42-1.29 (m, 5H).

**Example 52:** ¹H NMR (400 MHz, DMSO-*d*₆) δ 10.17 (s, 1H), 7.98 (dd, *J* = 5.9, 9.2 Hz, 1H), 7.47 (t, *J* = 9.0 Hz, 1H), 7.38 (d, *J* = 2.3 Hz, 1H), 7.22 (dd, *J* = 2.4, 4.9 Hz, 1H), 5.38-5.18 (m, 1H), 4.50-4.26 (m, 2H), 4.14 (d, *J* = 10.4 Hz, 1H), 3.99 (dd, *J* = 3.3, 10.3 Hz, 1H), 3.90-3.75 (m, 1H), 3.52 (s, 1H), 3.45-3.39 (m, 3H), 3.30-3.25 (m, 2H), 3.09 (d, *J* = 6.5 Hz, 2H), 3.01 (s, 1H), 2.88-2.79 (m, 1H), 2.12 (s, 1H), 2.09-2.07 (m, 1H), 2.09-2.03 (m, 1H), 1.99 (br s, 1H), 1.90-1.75 (m, 3H), 1.72-1.57 (m, 2H), 1.51 (s, 1H), 1.43-1.28 (m, 5H).

### Examples 53 and 54: Preparation of 4-(4-((1S,5R)-1-ethyl-3,8-diazabicyclo[3.2.1]octan-3-yl)-8-fluoro-2-(((2R,7aS)-2-fluo rotetrahydro-1H-pyrrolizin-7a(5H)-yl)methoxy)pyrido[4,3-d]pyrimidin-7-yl)-5-eth ynyl-6-fluoronaphthalen-2-ol and 4-(4-((1R,5S)-1-ethyl-3,8-diazabicyclo[3.2.1]octan-3-yl)-8-fluoro-2-(((2R,7aS)-2-fluo rotetrahydro-1H-pyrrolizin-7a(5H)-yl)methoxy)pyrido[4,3-d]pyrimidin-7-yl)-5-eth ynyl-6-fluoronaphthalen-2-ol

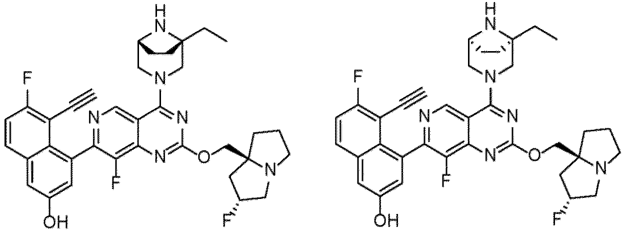

Examples 53 and 54 may be prepared by selecting appropriate conditions with reference to the methods of examples 41 and 42.

**Example 53:** ¹H NMR (400 MHz, DMSO-*d*₆) δ 9.02 (s, 1 H), 7.76 (dd, *J* = 5.9, 9.2 Hz, 1H), 7.30 (t, *J* = 9.0 Hz, 1H), 7.11 (d, *J* = 9.9 Hz, 2H), 5.44-5.14 (m, 1H), 4.54-4.24 (m, 2H), 4.17-4.00 (m, 2H), 3.80 (d, *J* = 15.9 Hz, 2H), 3.58-3.53 (m, 2H), 3.34-3.29 (m, 1H), 3.09 (d, *J* = 8.4 Hz, 2H), 3.01 (s, 1H), 2.90-2.75 (m, 1H), 2.16-2.11 (m, 1H), 2.07-1.98 (m, 2H), 1.88-1.74 (m, 4H), 1.68-1.53 (m, 4H), 1.33 (d, *J* = 7.3 Hz, 1H), 0.94 (dt, *J* = 3.5, 7.5 Hz, 3H).

**Example 54:** ¹H NMR (400 MHz, DMSO-*d*₆) δ 9.02 (s, 1H), 7.80 (dd, *J* = 6.1, 9.1 Hz, 1H), 7.33 (t, *J* = 9.0 Hz, 1H), 7.19 (s, 1H), 7.12 (t, *J* = 2.6 Hz, 1H), 5.38-5.16 (m, 1H), 4.53-4.27 (m, 2H), 4.13 (dd, *J* = 2.1, 10.2 Hz, 1H), 4.01 (d, *J* = 10.3 Hz, 1H), 3.82 (d, *J* = 19.1 Hz, 1H), 3.63-3.51 (m, 2H), 3.31 (t, *J* = 12.9 Hz, 2H), 3.11-2.99 (m, 3H), 2.88-2.75 (m, 1H), 2.19-1.96 (m, 3H), 1.90-1.50 (m, 8H), 1.34 (dd, *J* = 6.2, 11.2 Hz, 1H), 0.94 (dt, *J* = 3.7, 7.5 Hz, 3H).

### Examples 55 and 56: Preparation of 5-ethynyl-6-fluoro-4-(8-fluoro-4-((1R,5R)-1-(2-fluoroethyl)-3,8-diazabicyclo[3.2.1]o ctan-3-yl)-2-(((2R,7aS)-2-fluorotetrahydro-1H-pyrrolizin-7a(5H)-yl)methoxy)pyrid o[4,3-d]pyrimidin-7-yl)naphthalen-2-ol and 5-ethynyl-6-fluoro-4-(8-fluoro-4-((1S,5S)-1-(2-fluoroethyl)-3,8-diazabicyclo[3.2.1]oc tan-3-yl)-2-(((2R,7aS)-2-fluorotetrahydro-1H-pyrrolizin-7a(5H)-yl)methoxy)pyrido [4,3-d]pyrimidin-7-yl)naphthalen-2-ol

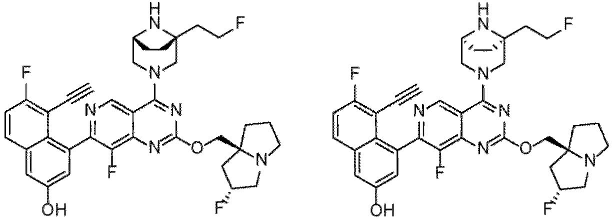

Examples 55 and 56 may be prepared by selecting appropriate conditions with reference to the methods of examples 41 and 42.

**Example 55:** ¹H NMR (400 MHz, DMSO-*d*₆) δ 9.03 (s, 1H), 7.99-7.95 (m, 1H), 7.46 (t, *J* = 9.0 Hz, 1H), 7.39 (s, 1H), 7.19 (s, 1H), 5.35-5.21 (m, 1H), 4.73 (d, *J* = 4.6 Hz, 1H), 4.62-4.57 (m, 1H), 4.44 (d, *J* = 12.3 Hz, 1H), 4.31 (d, *J* = 11.9 Hz, 1H), 4.16-4.09 (m, 2H), 4.08-4.01 (m, 2H), 3.90 (d, *J* = 16.3 Hz, 2H), 3.57 (s, 3H), 3.10-3.03 (m, 3H), 2.83 (d, *J* = 6.6 Hz, 1H), 2.05 (s, 5H), 1.77 (s, 7H), 1.46 (s, 1H).

**Example 56:** ¹H NMR (400 MHz, DMSO-*d*₆) δ 9.04 (s, 1H), 7.98 (dd, *J* = 6.0, 9.0 Hz, 1H), 7.47 (t, *J* = 9.0 Hz, 1H), 7.39 (d, *J* = 2.3 Hz, 1H), 7.18 (s, 1H), 5.36-5.20 (m, 1H), 4.73 (dd, *J* = 3.1, 6.4 Hz, 1H), 4.65-4.59 (m, 1H), 4.43 (d, *J* = 12.0 Hz, 1H), 4.31 (d, *J* = 11.4 Hz, 1H), 4.14 (dd, *J* = 5.0, 10.2 Hz, 2H), 4.02 (dd, *J* = 4.4, 10.3 Hz, 2H), 3.91 (d, *J* = 17.9 Hz, 2H), 3.61-3.53 (m, 3H), 3.09 (d, *J* = 9.3 Hz, 2H), 3.02 (s, 1H), 2.83 (d, *J* = 6.5 Hz, 1H), 2.12-1.96 (m, 5H), 1.83-1.63 (m, 7H), 1.45 (d, *J* = 4.9 Hz, 1H).

### Examples 57 and 58: Preparation of 5-ethynyl-6-fluoro-4-(8-fluoro-2-(((2R,7aS)-2-fluorotetrahydro-1H-pyrrolizin-7a(5 H)-yl)methoxy)-4-((1R,5R)-1-vinyl-3,8-diazabicyclo[3.2.1]octan-3-yl)pyrido[4,3-d]p yrimidin-7-yl)naphthalen-2-ol and 5-ethynyl-6-fluoro-4-(8-fluoro-2-(((2R,7aS)-2-fluorotetrahydro-1H-pyrrolizin-7a(5 H)-yl)methoxy)-4-((1S,5S)-1-vinyl-3,8-diazabicyclo[3.2.1octan-3-yl)pyrido[4,3-d]p yrimidin-7-yl)naphthalen-2-ol

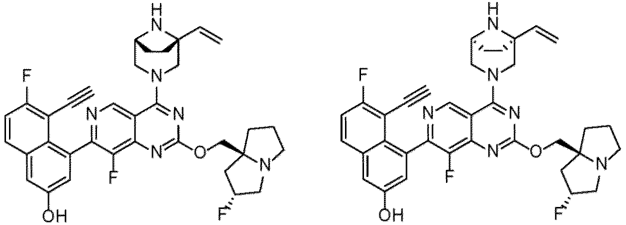

Examples 57 and 58 may be prepared by selecting appropriate conditions with reference to the methods of examples 41 and 42.

**Example 57:** ¹H NMR (400 MHz, DMSO-*d*₆) δ 9.05 (s, 1H), 7.93 (dd, *J* = 6.1, 9.1 Hz, 1H), 7.43 (t, *J* = 9.0 Hz, 1H), 7.35 (d, *J* = 2.2 Hz, 1H), 7.18 (d, *J* = 2.7 Hz, 1H), 6.09 (dd, *J* = 10.9, 17.5 Hz, 1H), 5.41-5.31 (m, 1H), 5.30-5.12 (m, 2H), 4.56 (d, *J* = 12.0 Hz, 1H), 4.44 (br dd, *J* = 6.1, 11.4 Hz, 1H), 4.33 (d, *J* = 11.9 Hz, 1H), 4.15 (dd, *J* = 3.1, 10.4 Hz, 1H), 4.03 (d, *J* = 10.4 Hz, 1H), 3.90 (d, *J* = 10.0 Hz, 1H), 3.69-3.54 (m, 2H), 3.09 (d, *J* = 10.1 Hz, 2H), 3.02 (s, 1H), 2.91-2.77 (m, 1H), 2.68 (s, 1H), 2.20-2.11 (m, 1H), 2.09-1.98 (m, 2H), 1.94-1.60 (m, 7H), 1.48 (t, *J* = 10.6 Hz, 1H), 1.04 (d, *J* = 6.1 Hz, 1H).

**Example 58:** ¹H NMR (400 MHz, DMSO-*d*₆) δ 9.05 (s, 1H), 7.93 (dd, *J* = 6.1, 9.1 Hz, 1H), 7.43 (t, *J* = 9.0 Hz, 1H), 7.34 (d, *J* = 2.2 Hz, 1H), 7.17 (s, 1H), 6.09 (dd, *J* = 10.9, 17.6 Hz, 1H), 5.38-5.31 (m, 1H), 5.30-5.12 (m, 2H), 4.55 (d, *J* = 12.6 Hz, 1H), 4.49-4.39 (m, 1H), 4.31 (s, 1H), 4.17-4.09 (m, 1H), 4.08-4.00 (m, 1H), 3.90 (d, *J* = 7.5 Hz, 1H), 3.68-3.54 (m, 2H), 3.09 (d, *J* = 9.5 Hz, 2H), 3.02 (s, 1H), 2.83 (d, *J* = 6.4 Hz, 1H), 2.68 (s, 1H), 2.14 (d, *J* = 4.8 Hz, 1H), 2.07-1.99 (m, 2H), 1.91-1.61 (m, 7H), 1.53-1.44 (m, 1H).

### Examples 59 and 60: Preparation of 5-ethynyl-6-fluoro-4-(8-fluoro-2-(((2R,7aS)-2-fluorotetrahydro-1H-pyrrolizin-7a(5 H)-yl)methoxy)-4-((1R,5R)-1-((Z)-2-fluorovinyl)-3,8-diazabicyclo[3.2.1]octan-3-yl) pyrido[4,3-d]pyrimidin-7-yl)naphthalen-2-ol and 5-ethynyl-6-fluoro-4-(8-fluoro-2-(((2R,7aS)-2-fluorotetrahydro-1H-pyrrolizin-7a(5 H)-yl)methoxy)-4-((1S,5S)-1-((Z)-2-fluorovinyl)-3,8-diazabicyclo[3.2.1]octan-3-yl)p yrido[4,3-d]pyrimidin-7-yl)naphthalen-2-ol

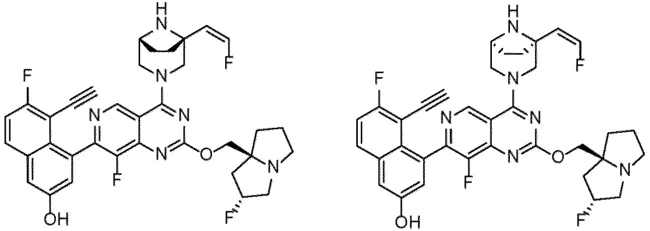

Examples 59 and 60 may be prepared by selecting appropriate conditions with reference to the methods of examples 41 and 42.

**Example 59:** ¹H NMR (400 MHz, DMSO-*d*₆) δ 10.17 (s, 1H), 9.03 (d, *J* = 0.7 Hz, 1H), 7.98 (dd, *J* = 6.0, 9.2 Hz, 1H), 7.51-7.36 (m, 2H), 7.19 (dd, *J* = 2.5, 7.2 Hz, 1H), 6.89-6.59 (m, 1H), 5.40-5.00 (m, 2H), 4.83-4.56 (m, 1H), 4.46-4.19 (m, 1H), 4.15 (dd, *J* = 5.5, 10.4 Hz, 1H), 4.03 (dd, *J* = 6.5, 10.3 Hz, 1H), 3.97-3.81 (m, 1H), 3.71-3.39 (m, 4H), 3.14 (d, *J* = 2.7 Hz, 2H), 3.02 (s, 1H), 2.88-2.79 (m, 1H), 2.19-2.04 (m, 3H), 1.99 (s, 1H), 1.89-1.74 (m, 4H), 1.70-1.56 (m, 2H).

**Example 60:** ¹H NMR (400 MHz, DMSO-*d*₆) δ 10.19 (s, 1H), 9.04(s, 1H), 7.98 (dd, *J* = 5.9, 9.2 Hz, 1H), 7.53-7.36 (m, 2H), 7.25-7.15 (m, 1H), 6.91-6.62 (m, 1H), 5.33-5.09 (m, 1H), 4.81-4.58 (m, 1H), 4.54 (t, *J* = 6.9 Hz, 1H), 4.33-4.19 (m, 1H), 4.17-4.04 (m, 2H), 3.98-3.80 (m, 1 H), 3.73-3.55 (m, 3H), 3.12 (d, *J* = 5.5 Hz, 2H), 3.06-3.01 (m, 1H), 2.92-2.77 (m, 1H), 2.18-2.05 (m, 4H), 2.03-1.97 (m, 1H), 1.89-1.74 (m, 4H), 1.70-1.57 (m, 2H).

### Examples 61 and 62: Preparation of 5-ethynyl-6-fluoro-4-(8-fluoro-2-(((2R,7aS)-2-fluorotetrahydro-1H-pyrrolizin-7a(5 H)-yl)methoxy)-4-((1R,5R)-1-((E)-2-fluorovinyl)-3,8-diazabicyclo[3.2.1]octan-3-yl) pyrido[4,3-d]pyrimidin-7-yl)naphthalen-2-ol and 5-ethynyl-6-fluoro-4-(8-fluoro-2-(((2R,7aS)-2-fluorotetrahydro-1H-pyrrolizin-7a(5 H)-yl)methoxy)-4-((1S,5S)-1-((E)-2-fluorovinyl)-3,8-diazabicyclo[3.2.1]octan-3-yl)p yrido[4,3-d]pyrimidin-7-yl)naphthalen-2-ol

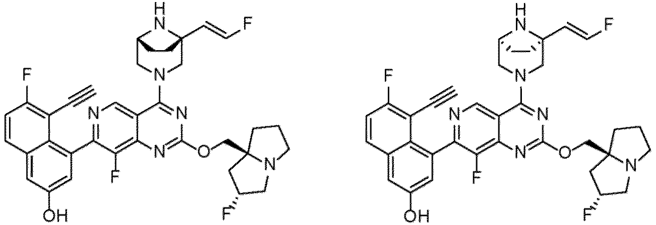

Examples 61 and 62 may be prepared by selecting appropriate conditions with reference to the methods of examples 41 and 42.

**Example 61:** ¹H NMR (400 MHz, DMSO-*d*₆) δ10.18 (s, 1H), 9.06 (d, *J* = 2.0 Hz, 1H), 7.98 (dd, *J* = 6.0, 9.2 Hz, 1H), 7.55-7.35 (m, 2H), 7.19 (t, *J* = 2.8 Hz, 1H), 7.13 (dd, *J* = 2.6, 11.3 Hz, 1H), 5.86-5.68 (m, 1H), 5.40-5.17 (m, 1H), 4.57-4.28 (m, 2H), 4.19-3.88 (m, 3H), 3.65-3.53 (m, 2H), 3.13-3.00 (m, 3H), 2.89-2.78 (m, 2H), 2.16-2.05 (m, 2H), 2.01 (s, 1H), 1.92-1.81 (m, 3H), 1.81-1.71 (m, 3H), 1.51 (d, *J* = 4.3 Hz, 2H).

**Example 62:** ¹H NMR (400 MHz, DMSO-*d*₆) δ 10.19 (s, 1 H), 9.06 (d, *J* = 2.2 Hz, 1 H), 7.98 (dd, *J* = 5.9, 9.2 Hz, 1H), 7.56-7.35 (m, 2H), 7.26-7.05 (m, 2H), 6.92 (dd, *J* = 2.6, 11.3 Hz,1H), 5.87-5.69 (m, 1H), 5.40-5.14 (m, 1H), 4.57-4.27 (m, 2H), 4.15-4.01 (m, 2H), 3.94 (dd, *J* = 0.7, 2.7 Hz, 1H), 3.66-3.51 (m, 2H), 3.09 (d, *J* = 8.1 Hz, 2H), 3.02 (s, 1H), 2.83 (d, *J* = 6.2 Hz, 1H), 2.16-2.11 (m, 1H), 2.08-1.98 (m, 2H), 1.91-1.75 (m, 6H), 1.72-1.61 (m, 1 H), 1.57-1.44 (m, 1H).

### Biological Test Evaluation

### I. KRAS^{G12D}/cRAF Binding Test

### 1. Compound Preparation

The compound was dissolved in 100% DMSO at a starting concentration of 5 mM and diluted in DMSO with a 3-fold serial dilution method for a total of 8 concentration points. 1 µL of the compound or DMSO at each concentration point was added into 99 µL of the experimental buffer to obtain 10X stock solution.

### 2. Test Steps

1) 4 µL of the working mixture solution of His-tagged KRAS^{G12D} and GMPPNP was added to the 384-well plate.
2) The 10X compound stock solution or 1% DMSO was added to the 384-well plate.
3) The plate was centrifuged at 1000 rpm for 1 minute.
4) 4 µL of GST-tagged cRAF solution was added to the 384-well plate.
5) The plate was centrifuged at 1000 rpm for 1 minute.
6) The plate was incubated at room temperature for 15 minutes.
7) 10 µL of detection working solution was added to the 384-well plate.
8) The plate was centrifuged at 1000 rpm for 1 minute.
9) The plate was incubated at room temperature for 60 minutes.
10) The 384-well plate was placed into Envision and assay data was obtained.

### 3. Data Processing

The ratio for each compound concentration was calculated from the signal of the DMSO control group contained in each assay plate and the Alphalisa signal in each compound well. The compound concentration required for inhibitory concentration at 50% (IC₅₀) was determined using a four-parameter logarithmic dose-response equation by testing the concentration of the compound and the value of the ratio. The endpoint value (IC₅₀) of the reference compound was assessed in each experiment as a quality control measure. If the endpoint value is within three times the expected value, the experiment may be considered acceptable.

**Table 1: Biology Test Results**

| **Example No.** | **KRAS G12D/ cRAF Alphalisa IC₅₀ (nM)** | **Example No.** | **KRAS G12D/ cRAF Alphalisa IC₅₀ (nM)** |
|---|---|---|---|
| **Example 1** | 100 | **Example 2** | ** |
| **Example 3** | NT | **Example 4** | ** |
| **Example 5** | 72 | **Example 6** | ** |
| **Example 7** | 4.3 | **Example 8** | *** |
| **Example 9** | ** | **Example 10** | * |
| **Example 11** | * | **Example 12** | ** |
| **Example 13** | NT | **Example 14** | NT |
| **Example 15** | ** | **Example 16** | * |
| **Example 17** | NT | **Example 18** | ** |
| **Example 19** | ** | **Example 20** | ** |
| **Example 21** | ** | **Example 22** | ** |
| **Example 23** | ** | **Example 24** | ** |
| **Example 25** | ** | **Example 26** | NT |
| **Example 27** | 54 | **Example 28** | ** |
| **Example 29** | ** | **Example 30** | NT |
| **Example 31** | NT | **Example 32** | ** |
| **Example 33** | ** | **Example 34** | *** |
| **Example 35** | NT | **Example 36** | ** |
| **Example 37** | ** | **Example 38** | *** |
| **Example 39** | *** | **Example 40** | *** |
| **Examples 41 and 42** | ***or* | **Examples 43 and 44** | 4.6 or 19 |
| **Examples 45 and 46** | *or** | **Examples 47 and 48** | ** or** |
| **Examples 49 and 50** | 262 or 10 | **Examples 51 and 52** | ** |
| **Examples 53 and 54** | 221 or 55 | **Examples 55 and 56** | NT |
| **Examples 57 and 58** | 15 or 47 | **Examples 59 and 60** | 15 or 25 |
| **Examples 61 and 62** | 876 or 18 | **Positive Compounds** | *** |
| **Notes** | 1. "NT" is an abbreviation of "Not Tested", which means that it has not been tested for the time being. | | |
| | 2. "*" means IC₅₀ ≥ 500 nM; "**" means 500 nM > IC₅₀ ≥ 50 nM; and "***" means 50 nM > IC₅₀. | | |

The "positive compound" used in the biological test evaluation of this application was Example 252 of WO2021041671A1 (the same positive compound used in the following anti-proliferative 2D CTG assay and mouse pharmacokinetic experiment) with the chemical structure as follows:

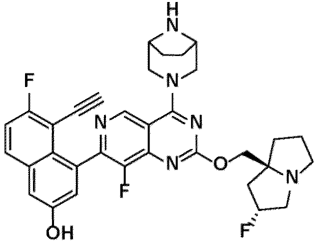

### II. Anti-Proliferative 2D CTG Assay

### 1. Experiment Steps:

### 1.1 Day 0 Plating

When the cell confluence was about 80%, the cells were isolated with 0.25% trypsin. Isolated cells were resuspended in 5 mL fresh cell culture medium and centrifuged for cell collection. Meanwhile, the number of cells was also calculated. The cells were then suspended in culture medium of medium concentration. The cells were placed into a 96-well plate with 1000 cells/well for AGS and 1000 cells/well for GP2D. The 96-well plate was placed in an incubator at 37°C and incubated overnight.

### 1.2 Day 1 Compound Treatment

From the 10 mM stock solution, with a ratio of 1:5, 10-point serial dilutions were performed. The 10X medium containing the compound was transferred into corresponding wells of the 96 wells. The final maximum compound concentration was 10 µM and the final DMSO concentration was 0.1%. The 96-well plate was placed in a 37°C incubator, the AGS cells were incubated for 3 days, and the GP2D cells were incubated for 4 days.

### 1.3 Day 4 Baseline Readings

### 1.4 Signal Reading

50 µL/well of detection reagent (CTG) was added to the AGS cells, and the signal was read in an Envision machine.

### 1.5 Day 5 Signal Reading

50 µL/well of detection reagent (CTG) was added to the GP2D cells, and the signal was read in an Envision machine.

### 2. Data Processing

The percent inhibition (%) at each compound concentration was calculated from the signal in the HPE and ZPE control wells and the fluorescence signal in the individual compound wells included in each assay plate. The ZPE control wells containing enzyme and substrate had an inhibition rate of 0%, and the HPE control wells containing only substrate had an inhibition rate of 100%. The compound concentration required for inhibitory concentration at 50% (IC₅₀) was determined using a four-parameter logarithmic dose-response equation by testing the concentration of the compound and the inhibition percentage value. The endpoint value (IC₅₀) of the reference compound was assessed in each experiment as a quality control measure. If the endpoint value is within three times the expected value, the experiment may be considered acceptable.

**Table 2: Biology Test Results**

| **Example No.** | **AGS cell IC₅₀ (nM)** | **Example No.** | **AGS cell IC₅₀ (nM)** |
|---|---|---|---|
| **Example 1** | 20 | **Example 2** | *** |
| **Example 3** | *** | **Example 4** | ** |
| **Example 5** | 15 | **Example 6** | *** |
| **Example 7** | 28 | **Example 8** | *** |
| **Example 9** | ** | **Example 10** | ** |
| **Example 11** | * | **Example 12** | *** |
| **Example 13** | ** | **Example 14** | * |
| **Example 15** | ** | **Example 16** | ** |
| **Example 17** | * | **Example 18** | ** |
| **Example 19** | *** | **Example 20** | *** |
| **Example 21** | *** | **Example 22** | * |
| **Example 23** | ** | **Example 24** | *** |
| **Example 25** | * | **Example 26** | NT |
| **Example 27** | 11 | **Example 28** | *** |
| **Example 29** | *** | **Example 30** | NT |
| **Example 31** | NT | **Example 32** | *** |
| **Example 33** | ** | **Example 34** | ** |
| **Example 35** | NT | **Example 36** | ** |
| **Example 37** | *** | **Example 38** | *** |
| **Example 39** | *** | **Example 40** | *** |
| **Examples 41 and 42** | ***or** | **Examples 43 and 44** | 1.5 or 6.1 |
| **Examples 45 and 46** | * or *** | **Examples 47 and 48** | ** or *** |
| **Examples 49 and 50** | 288 or 6.5 | **Examples 51 and 52** | *** |
| **Examples 53 and 54** | 708 or 7.3 | **Examples 55 and 56** | ** or *** |
| **Examples 57 and 58** | 8.1 or 127 | **Examples 59 and 60** | 13 or 79 |
| **Examples 61 and 62** | 816 or 27 | **Positive Compounds** | *** |
| **Notes** | 1. "NT" is an abbreviation of "Not Tested", which means that it has not been tested for the time being. | | |
| | 2. "*" means IC₅₀ ≥ 500 nM; "**" means 500 nM > IC₅₀ ≥ 50 nM; and "***" means 50 nM > IC₅₀. | | |

From the activity data of the compounds in specific examples, it can be seen that the series of compounds of the present invention had a strong inhibitory effect on KRAS cell activity. Under the same test conditions, the cell inhibitory activity of compounds in some examples was equivalent to that of the positive compounds, or improved to a certain extent.

### III. Pharmacokinetic Experiment in Mice

### 1. Test Drugs

The compounds used in this trial are from the compounds in the specific examples of the present invention.

### 2. Test Animals

ICR mice male N=3 original source: Shanghai Sippr-Bk Lab Animal Co., Ltd.

### 3. Drug Formulation and Administration

Single oral (PO) administration in ICR mice: The compounds were weighed and added to the solvent of 0.5% CMC + 1% Tween 80 respectively, well shaken, and ultrasonicated to get a light yellow suspension. 3 mice were administered orally after fasting overnight. The dose was 10 mg/kg.

ICR mice were administered a single intravenous (IV) dose: The compounds were weighed and added to the solvent of 50mM pH 4.7 Acetate buffer of 20% HP-β-CD respectively, well shaken, and ultrasonicated to get a light yellow solution. 3 mice were administered via tail vein injection at a dose of 2 mg/kg after fasting overnight.

### 4. Sample Collection:

About 90 µL/timepoint blood was collected through the orbital cavity, anticoagulated with sodium heparin, placed on ice after collection, and centrifuged to separate the plasma within 1 hour (centrifugation conditions: 8000 rpm, 6 min, 2-8°C). The blood collection timepoints were 0, 0.25, 0.5, 1, 2, 4, 6, 8 and 24 h. The samples were stored in a -20°C refrigerator.

The plasma sample was 40 µL, 160 µL of ice-cold acetonitrile containing internal standard was added, vortexed for 1 minute, and centrifuged at 18,000 rpm for 10 minutes. The supernatant was transferred to a 96-well plate, and 5 µL was injected into LC-MS/MS for analysis.

**Table 3: Pharmacokinetic Data**

| **Example No.** | **Administration Method (Dose)** | **AUCₗₐₛₜ (hr*ng/mL)** | **T_{1/2} (h)** | **Bioavailabili ty F (%)** |
|---|---|---|---|---|
| Example 2 | PO (10 mg/kg) | 602 | 8.3 | NT |
| Example 5 | PO (10 mg/kg) | 489 | 6.3 | NT |
| Example 12 | PO (10 mg/kg) | 1738 | 7.2 | NT |
| Example 14 | PO (10 mg/kg) | 607 | 5.8 | NT |
| Example 32 | PO (10 mg/kg) | 2201 | 4.6 | NT |
| Example 38 | PO (10 mg/kg) | 115 | 9.2 | NT |
| Example 41 | IV (2 mg/kg) | 2502 | 9.9 | 10 |
| | PO (10 mg/kg) | 1230 | 4.8 | |
| Example 43 | IV (2 mg/kg) | 214 | 5.4 | 5.8 |
| | PO (10 mg/kg) | 733 | 15.7 | |
| Example 50 | IV (2 mg/kg) | 1101 | 6.7 | 48 |
| | PO (10 mg/kg) | 2659 | 6.4 | |
| Example 51 | IV (2 mg/kg) | 1567 | 5.4 | 28 |
| | PO (10 mg/kg) | 1121 | 6.2 | |
| Example 54 | IV (2 mg/kg) | 460 | 7.7 | 51 |
| | PO (10 mg/kg) | 1165 | 9.8 | |
| Example 55 | PO (10 mg/kg) | 296 | 7.4 | NT |
| Example 57 | IV (2 mg/kg) | 375 | 5.1 | 95 |
| | PO (10 mg/kg) | 1788 | 4.5 | |
| Positive Compounds | IV (2 mg/kg) | 337 | 3.1 | 2.4 |
| | PO (10 mg/kg) | 40 | 1.6 | |
| Notes | "NT" is an abbreviation of "Not Tested", which means that it has not been tested for the time being. | | | |

From the pharmacokinetic data of the compounds in the above specific examples, this series of compounds of the present invention were absorbed into the mice body via IV or oral administration, and showed very good PK data. Compared with the positive compounds, the IV and oral AUC of this series of compounds of the present invention were significantly improved. In particular, the oral AUC of the compounds in some examples was improved by more than ten times, or even dozens of times, showing significantly improved bioavailability. The test demonstrated that this series of compounds of the present invention have very good development prospects, and are likely to solve the problem that positive compounds cannot be taken orally and can only be administered intravenously.

All documents mentioned in the present invention are incorporated by reference in this application, just as each document is cited separately as a reference. In addition, it should be understood that various modifications or changes may be made by those skilled in the art after reading the above disclosed content of the present invention, and these equivalent forms also fall within the scope defined by the claims appended hereto.

## Claims

1. A compound of formula (I), a stereoisomer or pharmaceutically acceptable salt thereof: 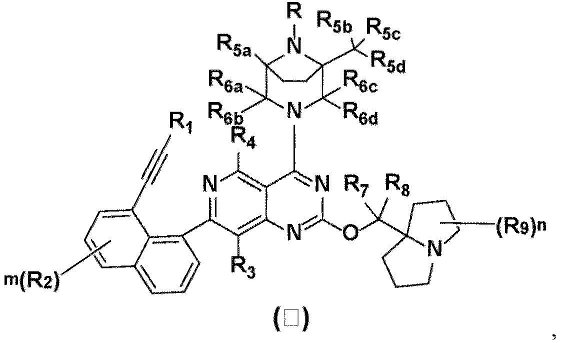
wherein, R is selected from the group consisting of hydrogen, C₁₋₁₀ alkyl, C₂₋₁₀ alkenyl, C₃₋₁₂ cycloalkyl, 3-12 membered heterocyclyl, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-C(O)R₁₅, -C₀₋₈ alkyl-O-C(O)R₁₅, -C₀₋₈ alkyl-NR₁₆R₁₇, -C₀₋₈ alkyl-C(O)NR₁₆R₁₇ and -C₀₋₈ alkyl-N(R₁₆)-C(O)R₁₅, above groups are independently optionally further substituted with one or more substituents selected from the group consisting of deuterium, halogen, cyano, nitro, azido, C₁₋₁₀ alkyl, C₁₋₁₀ haloalkyl, C₁₋₁₀ deuterioalkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
R₁ is selected from the group consisting of hydrogen, deuterium, halogen, cyano, C₁₋₄ alkyl, C₃₋₆ cycloalkyl, 3-6 membered heterocyclyl and -C₀₋₈ alkyl-NR₁₆R₁₇, above groups are independently optionally further substituted with one or more substituents selected from the group consisting of deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl and 5-10 membered heteroaryl, above groups are independently optionally further substituted with one or more substituents selected from the group consisting of deuterium and halogen;
each R₂ is independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, above groups are independently optionally further substituted with one or more substituents selected from the group consisting of deuterium and halogen;
R₃ is selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, above groups are independently optionally further substituted with one or more substituents selected from the group consisting of deuterium and halogen;
R₄ is selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, above groups are independently optionally further substituted with one or more substituents selected from the group consisting of deuterium and halogen;
R₅ₐ is selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, above groups are independently optionally further substituted with one or more substituents selected from the group consisting of deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, above groups are independently optionally further substituted with one or more substituents selected from the group consisting of deuterium and halogen;
R_{5b}, R_{5c} and R_{5d} are each independently selected from the group consisting of hydrogen, deuterium, halogen, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-NH-S(O)₂R₁₃, -C₀₋₈ alkyl-NH-S(O)₂NR₁₆R₁₇, -C₀₋₈ alkyl-S(O)ᵣR₁₃, -C₀₋₈ alkyl-S(O)ᵣNR₁₆R₁₇, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, above groups are independently optionally further substituted with one or more substituents selected from the group consisting of deuterium, halogen, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, above groups are independently optionally further substituted with one or more substituents selected from the group consisting of deuterium and halogen;
or, R_{5b} and R_{5c}, together with the carbon atom directly attached thereto, form a C(O), C₃₋₁₀ cycloalkyl or 3-10 membered heterocyclyl, above groups are independently optionally further substituted with one or more substituents selected from the group consisting of deuterium, halogen, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, above groups are independently optionally further substituted with one or more substituents selected from the group consisting of deuterium and halogen, wherein R_{5d} is defined as above;
or, R_{5b} and R_{5c}, together with the carbon atom directly attached thereto, form 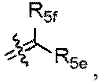 wherein R_{5d} is defined as above;
or, R_{5b}, R_{5c} and R_{5d}, together with the carbon atom directly attached thereto, form 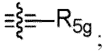
R₅ₑ, R_{5f} and R_{5g} are each independently selected from the group consisting of hydrogen, deuterium, halogen, cyano, C₁₋₄ alkyl, C₃₋₆ cycloalkyl, 3-6 membered heterocyclyl and -C₀₋₈ alkyl-NR₁₆R₁₇, above groups are independently optionally further substituted with one or more substituents selected from the group consisting of deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl and 5-10 membered heteroaryl, above groups are independently optionally further substituted with one or more substituents selected from the group consisting of deuterium and halogen;
R₆ₐ, R_{6b}, R_{6c} and R_{6d} are each independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, or, R₆ₐ and R_{6b}, R_{6c} and R_{6d}, together with the carbon atom directly attached thereto, form a C₃₋₁₀ cycloalkyl or 3-10 membered heterocyclyl, above groups are independently optionally further substituted with one or more substituents selected from the group consisting of deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, above groups are independently optionally further substituted with one or more substituents selected from the group consisting of deuterium and halogen;
R₇ and R₈ are each independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, or, R₇ and R₈, together with the carbon atom directly attached thereto, form a C₃₋₁₀ cycloalkyl or 3-10 membered heterocyclyl, above groups are independently optionally further substituted with one or more substituents selected from the group consisting of deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, above groups are independently optionally further substituted with one or more substituents selected from the group consisting of deuterium and halogen;
each R₉ is independently selected from the group consisting of hydrogen, deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, or, two adjacent R₉, together with the moiety to which they are directly attached thereto, form a C₃₋₁₀ cycloalkyl or 3-10 membered heterocyclyl, above groups are independently optionally further substituted with one or more substituents selected from the group consisting of deuterium, halogen, cyano, nitro, azido, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, =O, =S, -C₀₋₈ alkyl-SF₅, -C₀₋₈ alkyl-S(O)(=N-R₁₀)R₁₁, -C₀₋₈ alkyl-N=S(O)R₁₁R₁₂, -C₀₋₈ alkyl-N=SR₁₁R₁₂, -C₀₋₈ alkyl-O-S(O)₂R₁₃, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)SR₁₄, -C₀₋₈ alkyl-S-C(O)R₁₅, -C₀₋₈ alkyl-C(O)R₁₅, -C₀₋₈ alkyl-O-C(O)R₁₅, -C₀₋₈ alkyl-P(O)(R₁₅)₂, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅, above groups are independently optionally further substituted with one or more substituents selected from the group consisting of deuterium and halogen;
each R₁₀ is independently selected from the group consisting of hydrogen, deuterium, C₁₋₁₀ alkyl, C₂₋₁₀ alkenyl, C₂₋₁₀ alkynyl, C₃₋₁₀ cycloalkyl, 3-10 membered heterocyclyl, C₆₋₁₀ aryl, 5-10 membered heteroaryl, -C₀₋₈ alkyl-S(O)ᵣR₁₃, -C₀₋₈ alkyl-C(O)OR₁₄, -C₀₋₈ alkyl-C(O)R₁₅ and -C₀₋₈ alkyl-C(O)NR₁₆R₁₇, above groups are optionally further substituted with one or more substituents selected from the group consisting of deuterium, halogen, cyano, nitro, azido, C₁₋₁₀ alkyl, C₂₋₁₀ alkenyl, C₂₋₁₀ alkynyl, C₁₋₁₀ haloalkyl, C₁₋₁₀ deuterioalkyl, C₃₋₁₀ cycloalkyl, 3-10 membered heterocyclyl, C₆₋₁₀ aryl, 5-10 membered heteroaryl, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)R₁₅, -C₀₋₈ alkyl-O-C(O)R₁₅, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
R₁₁ and R₁₂ are each independently selected from the group consisting of hydrogen, deuterium, hydroxy, C₁₋₁₀ alkyl, C₂₋₁₀ alkenyl, C₂₋₁₀ alkynyl, C₃₋₁₀ cycloalkyl, 3-10 membered heterocyclyl, C₆₋₁₀ aryl and 5-10 membered heteroaryl, or, R₁₁ and R₁₂, together with the sulfur atom directly attached thereto, form a 3-10 membered heterocyclyl, above groups are optionally further substituted with one or more substituents selected from the group consisting of deuterium, halogen, cyano, nitro, azido, C₁₋₁₀ alkyl, C₂₋₁₀ alkenyl, C₂₋₁₀ alkynyl, C₁₋₁₀ haloalkyl, C₁₋₁₀ deuterioalkyl, C₃₋₁₀ cycloalkyl, 3-10 membered heterocyclyl, C₆₋₁₀ aryl, 5-10 membered heteroaryl, -C₀₋₈ alkyl-S(O)ᵣR₁₃, -C₀₋₈ alkyl-O-R₁₄, -C₀₋₈ alkyl-C(O)OR₁₄, -C₀₋₈ alkyl-C(O)R₁₅, -C₀₋₈ alkyl-O-C(O)R₁₅, -C₀₋₈ alkyl-NR₁₆R₁₇, -C₀₋₈ alkyl-C(=NR₁₆)R₁₅, -C₀₋₈ alkyl-N(R₁₆)-C(=NR₁₇)R₁₅, -C₀₋₈ alkyl-C(O)NR₁₆R₁₇ and -C₀₋₈ alkyl-N(R₁₆)-C(O)R₁₅;
each R₁₃ is independently selected from the group consisting of hydrogen, deuterium, hydroxy, C₁₋₁₀ alkyl, C₂₋₁₀ alkenyl, C₃₋₁₂ cycloalkyl, 3-12 membered heterocyclyl, C₆₋₁₀ aryl, 5-10 membered heteroaryl and -NR₁₆R₁₇, above groups are independently optionally further substituted with one or more substituents selected from the group consisting of deuterium, halogen, hydroxy, =O, C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₁₆R₁₇;
each R₁₄ is independently selected from the group consisting of hydrogen, deuterium, C₁₋₁₀ alkyl, C₂₋₁₀ alkenyl, C₃₋₁₂ cycloalkyl, 3-12 membered heterocyclyl, C₆₋₁₀ aryl and 5-10 membered heteroaryl, above groups are independently optionally further substituted with one or more substituents selected from the group consisting of deuterium, halogen, hydroxy, =O, cyano, C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₁₆R₁₇;
each R₁₅ is independently selected from the group consisting of hydrogen, deuterium, hydroxy, C₁₋₁₀ alkyl, C₁₋₁₀ alkoxy, C₂₋₁₀ alkenyl, C₂₋₁₀ alkynyl, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₁₆R₁₇, above groups are independently optionally further substituted with one or more substituents selected from the group consisting of deuterium, halogen, hydroxy, =O, cyano, C₁₋₁₀ alkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy and -NR₁₆R₁₇;
R₁₆ and R₁₇ are each independently selected from the group consisting of hydrogen, deuterium, hydroxy, C₁₋₁₀ alkyl, C₂₋₁₀ alkenyl, C₂₋₁₀ alkynyl, C₃₋₁₂ cycloalkyl, 3-12 membered heterocyclyl, C₆₋₁₀ aryl, 5-10 membered heteroaryl, sulfinyl, sulfonyl, methanesulfonyl, isopropylsulfonyl, cyclopropylsulfonyl, p-toluenesulfonyl, aminosulfonyl, dimethylaminosulfonyl and C₁₋₁₀ alkanoyl, above groups are independently optionally further substituted with one or more substituents selected from the group consisting of deuterium, halogen, hydroxy, =O, C₁₋₁₀ alkyl, C₂₋₁₀ alkenyl, C₂₋₁₀ alkynyl, C₁₋₁₀ haloalkyl, C₁₋₁₀ deuterioalkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy, amino, monoC₁₋₁₀ alkylamino, diC₁₋₁₀ alkylamino and C₁₋₁₀ alkanoyl;
or, R₁₆ and R₁₇, together with the nitrogen atom directly attached thereto, form a 4-10 membered heterocyclyl or 5-10 membered heteroaryl, the 4-10 membered heterocyclyl or 5-10 membered heteroaryl is optionally further substituted with one or more substituents selected from the group consisting of deuterium, halogen, hydroxy, =O, C₁₋₁₀ alkyl, C₂₋₁₀ alkenyl, C₂₋₁₀ alkynyl, C₁₋₁₀ haloalkyl, C₁₋₁₀ deuterioalkyl, C₁₋₁₀ alkoxy, C₃₋₁₂ cycloalkyl, C₃₋₁₂ cycloalkoxy, 3-12 membered heterocyclyl, 3-12 membered heterocyclyloxy, C₆₋₁₀ aryl, C₆₋₁₀ aryloxy, 5-10 membered heteroaryl, 5-10 membered heteroaryloxy, amino, monoC₁₋₁₀ alkylamino, diC₁₋₁₀ alkylamino and C₁₋₁₀ alkanoyl;
m is 0, 1, 2, 3, 4, 5 or 6;
n is 0, 1, 2, 3, 4, 5 or 6; and
each r is independently 0, 1 or 2.

2. The compound of formula (I), the stereoisomer or pharmaceutically acceptable salt thereof according to claim 1, **characterized in that** R is selected from the group consisting of hydrogen, C₁₋₄ alkyl, C₂₋₄ alkenyl, C₃₋₆ cycloalkyl, 3-6 membered heterocyclyl, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-C(O)R₁₅, -C₀₋₄ alkyl-O-C(O)R₁₅, -C₀₋₄ alkyl-NR₁₆R₁₇, -C₀₋₄ alkyl-C(O)NR₁₆R₁₇ and -C₀₋₄ alkyl-N(R₁₆)-C(O)R₁₅, above groups are independently optionally further substituted with one or more substituents selected from the group consisting of deuterium, halogen, cyano, nitro, azido, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
R₁ is selected from the group consisting of hydrogen, deuterium, halogen, cyano, C₁₋₄ alkyl, C₃₋₆ cycloalkyl, 3-6 membered heterocyclyl and -C₀₋₄ alkyl-NR₁₆R₁₇, above groups are independently optionally further substituted with one or more substituents selected from the group consisting of deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₆₋₈ aryl and 5-8 membered heteroaryl, above groups are independently optionally further substituted with one or more substituents selected from the group consisting of deuterium and halogen;
each R₂ is independently selected from the group consisting of hydrogen, deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, above groups are independently optionally further substituted with one or more substituents selected from the group consisting of deuterium and halogen;
R₃ is selected from the group consisting of hydrogen, deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, above groups are independently optionally further substituted with one or more substituents selected from the group consisting of deuterium and halogen;
R₄ is selected from the group consisting of hydrogen, deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, above groups are independently optionally further substituted with one or more substituents selected from the group consisting of deuterium and halogen;
R₅ₐ is selected from the group consisting of hydrogen, deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, above groups are independently optionally further substituted with one or more substituents selected from the group consisting of deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, above groups are more independently optionally further substituted with one or more substituents selected from the group consisting of deuterium and halogen;
R_{5b}, R_{5c} and R_{5d} are each independently selected from the group consisting of hydrogen, deuteriu, halogen, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-NH-S(O)₂R₁₃, -C₀₋₄ alkyl-NH-S(O)₂NR₁₆R₁₇, -C₀₋₄ alkyl-S(O)ᵣR₁₃, -C₀₋₄ alkyl-S(O)ᵣNR₁₆R₁₇, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, above groups are independently optionally further substituted with one or more substituents selected from the group consisting of deuterium, halogen, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, above groups are independently optionally further substituted with one or more substituents selected from the group consisting of deuterium and halogen;
or, R_{5b} and R_{5c}, together with the carbon atom directly attached thereto, form a C(O), C₃₋₆ cycloalkyl or 3-6 membered heterocyclyl, above groups are independently optionally further substituted with one or more substituents selected from the group consisting of deuterium, halogen, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, above groups are independently optionally further substituted with one or more substituents selected from the group consisting of deuterium and halogen, wherein R_{5d} is defined as above;
or, R_{5b} and R_{5c}, together with the carbon atom directly attached thereto, form 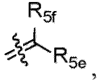 wherein R_{5d} is defined as above;
or, R_{5b}, R_{5c} and R_{5d}, together with the carbon atom directly attached thereto, form 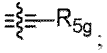
R₅ₑ, R_{5f} and R_{5g} are each independently selected from the group consisting of hydrogen, deuterium, halogen, cyano, C₁₋₄ alkyl, C₃₋₆ cycloalkyl, 3-6 membered heterocyclyl and -NR₁₆R₁₇, above groups are independently optionally further substituted with one or more substituents selected from the group consisting of deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl and 5-8 membered heteroaryl, above groups are more independently optionally further substituted with one or more substituents selected from the group consisting of deuterium and halogen;
R₆ₐ, R_{6b}, R_{6c} and R_{6d} are each independently selected from the group consisting of hydrogen, deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, or, R₆ₐ and R_{6b}, R₆, and R_{6d}, together with the carbon atom directly attached thereto, form a C₃₋₁₀ cycloalkyl or 3-10 membered heterocyclyl, above groups are independently optionally further substituted with one or more substituents selected from the group consisting of deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, above groups are more independently optionally further substituted with one or more substituents selected from the group consisting of deuterium and halogen;
R₇ and R₈ are each independently selected from the group consisting of hydrogen, deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, or, R₇ and R₈, together with the carbon atom directly attached thereto, form a C₃₋₁₀ cycloalkyl or 3-10 membered heterocyclyl, above groups are independently optionally further substituted with one or more substituents selected from the group consisting of deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, above groups are more independently optionally further substituted with one or more substituents selected from the group consisting of deuterium and halogen;
each R₉ is independently selected from the group consisting of hydrogen, deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, or, two adjacent R₉, together with the moiety to which they are directly attached thereto, form a C₃₋₁₀ cycloalkyl or 3-10 membered heterocyclyl, above groups are independently optionally further substituted with one or more substituents selected from the group consisting of deuterium, halogen, cyano, nitro, azido, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, above groups are more independently optionally further substituted with one or more substituents selected from the group consisting of deuterium and halogen;
wherein, R₁₀, R₁₁, R₁₂, R₁₃, R₁₄, R₁₅, R₁₆, R₁₇ and r are defined as in claim 1.

3. The compound of formula (I), the stereoisomer or pharmaceutically acceptable salt thereof according to claim 1, **characterized in that** each R₁₀ is independently selected from the group consisting of hydrogen, deuterium, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-S(O)ᵣR₁₃, -C₀₋₄ alkyl-C(O)OR₁₄, -C₀₋₄ alkyl-C(O)R₁₅ and -C₀₋₄ alkyl-C(O)NR₁₆R₁₇, above groups are optionally further substituted with one or more substituents selected from the group consisting of deuterium, halogen, cyano, nitro, azido, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, C₆₋₈ aryl, 5-8 membered heteroaryl, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)R₁₅, -C₀₋₄ alkyl-O-C(O)R₁₅, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
R₁₁ and R₁₂ are each independently selected from the group consisting of hydrogen, deuterium, hydroxy, C₁₋₄ alkyl, C₂₋₄ alkenyl, C₂₋₄ alkynyl, C₃₋₆ cycloalkyl, 3-6 membered heterocyclyl, C₆₋₈ aryl and 5-8 membered heteroaryl, or, R₁₁ and R₁₂, together with the sulfur atom directly attached thereto, form a 3-6 membered heterocyclyl, above groups are optionally further substituted with one or more substituents selected from the group consisting of deuterium, halogen, cyano, nitro, azido, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl, C₆₋₈ aryl, 5-8 membered heteroaryl, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)R₁₅, -C₀₋₄ alkyl-O-C(O)R₁₅, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅;
each R₁₃ is independently selected from the group consisting of hydrogen, deuterium, hydroxy, C₁₋₄ alkyl, C₂₋₄ alkenyl, C₃₋₆ cycloalkyl, 3-6 membered heterocyclyl, C₆₋₈ aryl, 5-8 membered heteroaryl and -NR₁₆R₁₇, above groups are independently optionally further substituted with one or more substituents selected from the group consisting of deuterium, halogen, hydroxy, =O, C₁₋₄ alkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₁₆R₁₇;
each R₁₄ is independently selected from the group consisting of hydrogen, deuterium, C₁₋₄ alkyl, C₂₋₄ alkenyl, C₃₋₆ cycloalkyl, 3-6 membered heterocyclyl, C₆₋₈ aryl and 5-8 membered heteroaryl, above groups are independently optionally further substituted with one or more substituents selected from the group consisting of deuterium, halogen, hydroxy, =O, cyano, C₁₋₄ alkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₁₆R₁₇;
each R₁₅ is independently selected from the group consisting of hydrogen, deuterium, hydroxy, C₁₋₄ alkyl, C₁₋₄ alkoxy, C₂₋₄ alkenyl, C₂₋₄ alkynyl, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₁₆R₁₇, above groups are independently optionally further substituted with one or more substituents selected from the group consisting of deuterium, halogen, hydroxy, =O, cyano, C₁₋₄ alkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy and -NR₁₆R₁₇;
R₁₆ and R₁₇ are each independently selected from the group consisting of hydrogen, deuterium, hydroxy, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, sulfinyl, sulfonyl, methanesulfonyl, isopropylsulfonyl, cyclopropylsulfonyl, p-toluenesulfonyl, aminosulfonyl, dimethylaminosulfonyl and C₁₋₄ alkanoyl, above groups are independently optionally further substituted with one or more substituents selected from the group consisting of deuterium, halogen, hydroxy, =O, C₁₋₄ alkyl, C₂₋₄ alkenyl, C₂₋₄ alkynyl, C₁₋₄ haloalkyl, C₁₋₄ deuterioalkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy, amino, monoC₁₋₄ alkylamino, diC₁₋₄ alkylamino and C₁₋₄ alkanoyl;
or, R₁₆ and R₁₇, together with the nitrogen atom directly attached thereto, form a 4-8 membered heterocyclyl or 5-8 membered heteroaryl, the 4-8 membered heterocyclyl or 5-8 membered heteroaryl is optionally further substituted with one or more substituents selected from the group consisting of deuterium, halogen, hydroxy, =O, C₁₋₄ alkyl, C₂₋₄ alkenyl, C₂₋₄ alkynyl, C₁₋₄ haloalkyl, C₁₋₄ deuterioalkyl, C₁₋₄ alkoxy, C₃₋₆ cycloalkyl, C₃₋₆ cycloalkoxy, 3-6 membered heterocyclyl, 3-6 membered heterocyclyloxy, C₆₋₈ aryl, C₆₋₈ aryloxy, 5-8 membered heteroaryl, 5-8 membered heteroaryloxy, amino, monoC₁₋₄ alkylamino, diC₁₋₄ alkylamino and C₁₋₄ alkanoyl.

4. The compound of formula (I), the stereoisomer or pharmaceutically acceptable salt thereof according to claim 1, **characterized in that** the compound of formula (I) is a compound of formula (II) as shown below: 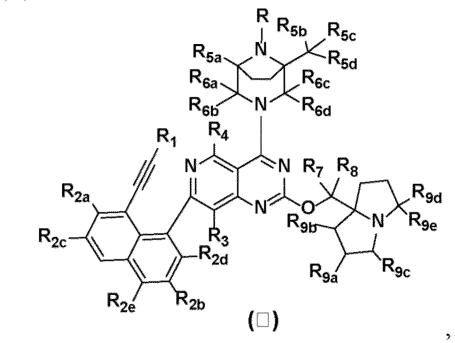
wherein, R is selected from the group consisting of hydrogen, C₁₋₄ alkyl, C₂₋₄ alkenyl, C₃₋₆ cycloalkyl, 3-6 membered heterocyclyl, -S(O)ᵣR₁₃, -O-R₁₄, -C(O)OR₁₄, -C(O)SR₁₄, -C(O)R₁₅, -O-C(O)R₁₅, -NR₁₆R₁₇, -C(O)NR₁₆R₁₇ and -N(R₁₆)-C(O)R₁₅, above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
R₁ is selected from the group consisting of hydrogen, deuterium, halogen, cyano, C₁₋₄ alkyl, C₃₋₆ cycloalkyl, 3-6 membered heterocyclyl and -C₀₋₄ alkyl-NR₁₆R₁₇, above groups are independently optionally further substituted with one or more substituents selected from the group consisting of deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl and 5-8 membered heteroaryl, above groups are independently optionally further substituted with one or more substituents selected from the group consisting of deuterium and halogen;
R₂ₐ is selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, above groups are independently optionally further substituted with one or more substituents selected from the group consisting of deuterium and halogen;
R_{2b} is selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, above groups are independently optionally further substituted with one or more substituents selected from the group consisting of deuterium and halogen;
R_{2c} is selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, above groups are independently optionally further substituted with one or more substituents selected from the group consisting of deuterium and halogen;
R_{2d} is selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, above groups are independently optionally further substituted with one or more substituents selected from the group consisting of deuterium and halogen;
R₂ₑ is selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, above groups are independently optionally further substituted with one or more substituents selected from the group consisting of deuterium and halogen;
R₃ is selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, above groups are independently optionally further substituted with one or more substituents selected from the group consisting of deuterium and halogen;
R₄ is selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, above groups are independently optionally further substituted with one or more substituents selected from the group consisting of deuterium and halogen;
R₅ₐ is selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, above groups are independently optionally further substituted with one or more substituents selected from the group consisting of deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, above groups are more independently optionally further substituted with one or more substituents selected from the group consisting of deuterium and halogen;
R_{5b}, R_{5c} and R_{5d} are each independently selected from the group consisting of hydrogen, deuterium, halogen, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-NH-S(O)₂R₁₃, -C₀₋₄ alkyl-NH-S(O)₂NR₁₆R₁₇, -C₀₋₄ alkyl-S(O)ᵣR₁₃, -C₀₋₄ alkyl-S(O)ᵣNR₁₆R₁₇, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, above groups are independently optionally further substituted with one or more substituents selected from the group consisting of deuterium, halogen, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, above groups are independently optionally further substituted with one or more substituents selected from the group consisting of deuterium and halogen;
or, R_{5b} and R_{5c}, together with the carbon atom directly attached thereto, form a C(O), C₃₋₆ cycloalkyl or 3-6 membered heterocyclyl, above groups are independently optionally further substituted with one or more substituents selected from the group consisting of deuterium, halogen, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -C₀₋₄ alkyl-SF₅, -C₀₋₄ alkyl-S(O)(=N-R₁₀)R₁₁, -C₀₋₄ alkyl-N=S(O)R₁₁R₁₂, -C₀₋₄ alkyl-N=SR₁₁R₁₂, -C₀₋₄ alkyl-O-S(O)₂R₁₃, -C₀₋₄ alkyl-S(O)ᵣR₁₃, -C₀₋₄ alkyl-O-R₁₄, -C₀₋₄ alkyl-C(O)OR₁₄, -C₀₋₄ alkyl-C(O)SR₁₄, -C₀₋₄ alkyl-S-C(O)R₁₅, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(=NR₁₆)R₁₅, -C₀₋₄ alkyl-N(R₁₆)-C(=NR₁₇)R₁₅, -C₀₋₄ alkyl-C(O)NR₁₆R₁₇ and -C₀₋₄ alkyl-N(R₁₆)-C(O)R₁₅, above groups are independently optionally further substituted with one or more substituents selected from the group consisting of deuterium and halogen, wherein R_{5d} is defined as above;
or, R_{5b} and R_{5c}, together with the carbon atom directly attached thereto, form 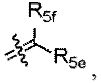 wherein R_{5d} is defined as above; or, R_{5b}, R_{5c} and R_{5d}, together with the carbon atom directly attached thereto, form 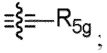
R₅ₑ, R_{5f} and R_{5g} are each independently selected from the group consisting of hydrogen, deuterium, halogen, C₁₋₄ alkyl, C₃₋₆ cycloalkyl, 3-6 membered heterocyclyl and -NR₁₆R₁₇, above groups are independently optionally further substituted with one or more substituents selected from the group consisting of deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl and 5-8 membered heteroaryl, above groups are independently optionally further substituted with one or more substituents selected from the group consisting of deuterium and halogen;
R₆ₐ, R_{6b}, R_{6c} and R_{6d} are each independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, or, R₆ₐ and R_{6b}, R_{6c} and R_{6d}, together with the carbon atom directly attached thereto, form a C₃₋₆ cycloalkyl and 3-6 membered heterocyclyl, above groups are independently optionally further substituted with one or more substituents selected from the group consisting of deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, above groups are more independently optionally further substituted with one or more substituents selected from the group consisting of deuterium and halogen;
R₇ and R₈ are each independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, or, R₇ and R₈, together with the carbon atom directly attached thereto, form a C₃₋₆ cycloalkyl and 3-6 membered heterocyclyl, above groups are independently optionally further substituted with one or more substituents selected from the group consisting of deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, above groups are more independently optionally further substituted with one or more substituents selected from the group consisting of deuterium and halogen;
R₉ₐ, R_{9b}, R_{9c}, R_{9d} and R₉ₑ are each independently selected from the group consisting of hydrogen, deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, or, R_{9d} and R₉ₑ, together with the carbon atom directly attached thereto, form a C₃₋₆ cycloalkyl and 3-6 membered heterocyclyl, and the other three are defined as above, the C₃₋₆ cycloalkyl and 3-6 membered heterocyclyl are independently optionally further substituted with one or more substituents selected from the group consisting of deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅, above groups are independently optionally further substituted with one or more substituents selected from the group consisting of deuterium and halogen;
wherein, R₁₀, R₁₁, R₁₂, R₁₃, R₁₄, R₁₅, R₁₆, R₁₇ and r are defined as in claim 1.

5. The compound of formula (I), the stereoisomer or pharmaceutically acceptable salt thereof according to claim 1, **characterized in that** the compound of formula (I) is a compound of formula (III) as shown below: 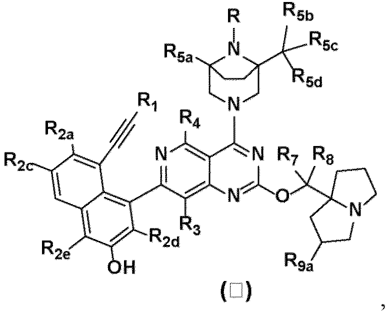
wherein, R is selected from the group consisting of hydrogen, C₁₋₄ alkyl, C₂₋₄ alkenyl, C₃₋₆ cycloalkyl, 3-6 membered heterocyclyl, -S(O)ᵣR₁₃, -O-R₁₄, -C(O)OR₁₄, -C(O)SR₁₄, -C(O)R₁₅, -O-C(O)R₁₅, -NR₁₆R₁₇, -C(O)NR₁₆R₁₇ and -N(R₁₆)-C(O)R₁₅, above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
R₁ is selected from the group consisting of hydrogen, deuterium, halogen, cyano, C₁₋₄ alkyl, C₁₋₄ haloalkyl, C₁₋₄ deuterioalkyl, C₃₋₆ cycloalkyl and 6 membered heterocyclyl;
R₂ₐ is selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, -O-R₁₄ and -S-R₁₃;
R_{2c} is selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, -O-R₁₄ and -S-R₁₃;
R_{2d} is selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, -O-R₁₄ and -S-R₁₃;
R₂ₑ is selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, -O-R₁₄ and -S-R₁₃;
R₃ is selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, -O-R₁₄ and -S-R₁₃;
R₄ is selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, -O-R₁₄, -S-R₁₃ and -NR₁₆R₁₇;
R₅ₐ is selected from the group consisting of hydrogen, deuterium, halogen, cyano, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl and -C₀₋₄ alkyl-O-R₁₄;
R_{5b} and R_{5c} are each independently selected from the group consisting of hydrogen, deuterium, halogen, C₁₋₄ alkyl, C₂₋₄ alkenyl, C₂₋₄ alkynyl, C₃₋₆ cycloalkyl, 3-6 membered heterocyclyl and -O-R₁₄, above groups are independently optionally further substituted with one or more substituents selected from the group consisting of deuterium, halogen, C₁₋₄ alkyl, C₂₋₄ alkenyl, C₂₋₄ alkynyl, C₃₋₆ cycloalkyl, 3-6 membered heterocyclyl, =O, =S, -SF₅, -S(O)ᵣR₁₃ and -O-R₁₄;
R_{5d} is selected from the group consisting of hydrogen, deuterium, halogen, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, -C₀₋₄ alkyl-SF₅, -C₀₋₄ alkyl-NH-S(O)₂R₁₃, -C₀₋₄ alkyl-NH-S(O)₂NR₁₆R₁₇, -C₀₋₄ alkyl-S(O)ᵣR₁₃, -C₀₋₄ alkyl-S(O)ᵣNR₁₆R₁₇, -C₀₋₄ alkyl-O-R₁₄, -C₀₋₄ alkyl-C(O)OR₁₄, -C₀₋₄ alkyl-C(O)R₁₅, -C₀₋₄ alkyl-O-C(O)R₁₅, -C₀₋₄ alkyl-P(O)(R₁₅)₂, -C₀₋₄ alkyl-NR₁₆R₁₇, -C₀₋₄ alkyl-C(O)NR₁₆R₁₇ and -C₀₋₄ alkyl-N(R₁₆)-C(O)R₁₅, above groups are independently optionally further substituted with one or more substituents selected from the group consisting of deuterium, halogen, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)ᵣR₁₃, -O-R₁₄, -C(O)OR₁₄, -C(O)R₁₅, -O-C(O)R₁₅, -P(O)(R₁₅)₂, -NR₁₆R₁₇, -C(O)NR₁₆R₁₇ and -N(R₁₆)-C(O)R₁₅, above groups are independently optionally further substituted with one or more substituents selected from the group consisting of deuterium and halogen;
or, R_{5b} and R_{5c}, together with the carbon atom directly attached thereto, form a C(O), C₃₋₆ cycloalkyl or 3-6 membered heterocyclyl, above groups are independently optionally further substituted with one or more substituents selected from the group consisting of deuterium, halogen, C₁₋₄ alkyl, C₂₋₄ alkenyl, C₂₋₄ alkynyl, C₃₋₆ cycloalkyl, 3-6 membered heterocyclyl, =O, =S, -SF₅, -S(O)ᵣR₁₃ and -O-R₁₄, above groups are independently optionally further substituted with one or more substituents selected from the group consisting of deuterium and halogen, and R_{5d} is selected from the group consisting of hydrogen, deuterium, halogen, C₁₋₄ alkyl, C₂₋₄ alkenyl, C₂₋₄ alkynyl, C₃₋₆ cycloalkyl, 3-6 membered heterocyclyl and -O-R₁₄, above groups are independently optionally further substituted with one or more substituents selected from the group consisting of deuterium and halogen;
or, R_{5b} and R_{5c}, together with the carbon atom directly attached thereto, form 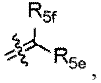 and R_{5d} is selected from the group consisting of hydrogen, deuterium, halogen, C₁₋₄ alkyl, C₂₋₄ alkenyl, C₂₋₄ alkynyl, C₃₋₆ cycloalkyl, 3-6 membered heterocyclyl and -O-R₁₄, above groups are independently optionally further substituted with one or more substituents selected from the group consisting of deuterium and halogen;
or, R_{5b}, R_{5c} and R_{5d}, together with the carbon atom directly attached thereto, form 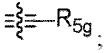
R₅ₑ, R_{5f} and R_{5g} are each independently selected from the group consisting of hydrogen, deuterium, halogen and C₁₋₄ alkyl, above groups are independently optionally further substituted with one or more substituents selected from the group consisting of deuterium, halogen, cyano, C₁₋₄ alkyl, C₃₋₆ cycloalkyl, 3-6 membered heterocyclyl, C₆₋₈ aryl and 5-8 membered heteroaryl, above groups are independently optionally further substituted with one or more substituents selected from the group consisting of deuterium and halogen;
R₇ and R₈ are each independently selected from the group consisting of hydrogen, deuterium, halogen, cyano, C₁₋₄ alkyl, C₂₋₄ alkenyl, C₂₋₄ alkynyl, C₃₋₆ cycloalkyl and 3-6 membered heterocyclyl, or, R₇ and R₈, together with the carbon atom directly attached thereto, form a C₃₋₆ cycloalkyl and 3-6 membered heterocyclyl, above groups are independently optionally further substituted with one or more substituents selected from the group consisting of deuterium, halogen, cyano, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
R₉ₐ is selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl and 3-6 membered heterocyclyl;
wherein, R₁₀, R₁₁, R₁₂, R₁₃, R₁₄, R₁₅, R₁₆, R₁₇ and r are defined as in claim 1.

6. The compound of formula (I), the stereoisomer or pharmaceutically acceptable salt thereof according to claim 5, **characterized in that** R_{5b} and R_{5c} are each independently selected from the group consisting of hydrogen, deuterium, fluorine, chlorine, bromine, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl, methoxy, ethoxy, propoxy, isopropoxy, trifluoromethoxy, difluoromethoxy, monofluoromethoxy, trideuteriomethoxy, dideuteriomethoxy, trifluoroethoxy, trideuterioethoxy, cyclopropoxy and cyclobutoxy;
R_{5d} is selected from the group consisting of hydrogen, deuterium, fluorine, chlorine, bromine, C₁₋₄ alkyl, C₂₋₄ alkenyl, C₂₋₄ alkynyl, C₃₋₆ cycloalkyl, 3-6 membered heterocyclyl, 5-8 membered heteroaryl, -NH-S(O)₂R₁₃, -NH-S(O)₂NR₁₆R₁₇, -S(O)ᵣR₁₃, -S(O)ᵣNR₁₆R₁₇, -O-R₁₄, -C(O)OR₁₄, -C(O)R₁₅, -P(O)(R₁₅)₂, -NR₁₆R₁₇, -C(O)NR₁₆R₁₇ and -N(R₁₆)-C(O)R₁₅, above groups are independently optionally further substituted with one or more substituents selected from the group consisting of deuterium, fluorine, chlorine, bromine, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -S(O)ᵣR₁₃, -O-R₁₄, -C(O)OR₁₄, -C(O)R₁₅ and -C(O)NR₁₆R₁₇, above groups are independently optionally further substituted with one or more substituents selected from the group consisting of deuterium and halogen;
or, R_{5b} and R_{5c}, together with the carbon atom directly attached thereto, form a C(O), C₃₋₆ cycloalkyl or 3-6 membered heterocyclyl, above groups are independently optionally further substituted with one or more substituents selected from the group consisting of deuterium, fluorine, chlorine, bromine, C₁₋₄ alkyl, C₂₋₄ alkenyl, C₂₋₄ alkynyl, C₃₋₆ cycloalkyl, =O, =S, methylthio, ethylthio, methoxy, ethoxy and isopropoxy, above groups are independently optionally further substituted with one or more substituents selected from the group consisting of deuterium and halogen, and R_{5d} is selected from the group consisting of hydrogen, deuterium, fluorine, chlorine, bromine, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl, methoxy, ethoxy and isopropoxyl;
or, R_{5b} and R_{5c}, together with the carbon atom directly attached thereto, form 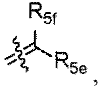 and R_{5d} is selected from the group consisting of hydrogen, deuterium, fluorine, chlorine, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl and azacyclobutyl;
or, R_{5b}, R_{5c} and R_{5d}, together with the carbon atom directly attached thereto, form 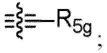
R₅ₑ, R_{5f} and R_{5g} are each independently selected from the group consisting of hydrogen, deuterium, fluorine, chlorine, methyl, ethyl, propyl, isopropyl, trifluoromethyl, difluoromethyl, monofluoromethyl, trideuteriomethyl, dideuteriomethyl and monodeuteriomethyl;
wherein, R₁₃, R₁₄, R₁₅, R₁₆, R₁₇ and r are defined as in claim 5.

7. The compound of formula (I), the stereoisomer or pharmaceutically acceptable salt thereof according to claim 5, **characterized in that** R₅ₐ is selected from the group consisting of hydrogen, deuterium, fluorine, chlorine, bromine, cyano, hydroxy, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl, methoxy, ethoxy, propoxy, isopropoxy, trifluoromethoxy, difluoromethoxy, trideuteriomethoxy, dideuteriomethoxy, trifluoroethoxy, trideuterioethoxy, cyclopropoxy and cyclobutoxy.

8. The compound of formula (I), the stereoisomer or pharmaceutically acceptable salt thereof according to claim 5, **characterized in that** R is selected from the group consisting of hydrogen, methyl, ethyl, propyl, isopropyl, cyclopropyl, cyclobutyl, oxacyclobutyl, azacyclobutyl and -C(O)OR₁₄, above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
wherein, R₁₀, R₁₁, R₁₂, R₁₃, R₁₄, R₁₅, R₁₆, R₁₇ and r are defined as in claim 5.

9. The compound of formula (I), the stereoisomer or pharmaceutically acceptable salt thereof according to claim 5, **characterized in that** R₁ is selected from the group consisting of hydrogen, deuterium, fluorine and methyl;
R₂ₐ is selected from the group consisting of hydrogen, deuterium, fluorine, chlorine, bromine, cyano, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl, methoxy, ethoxy, propoxy, isopropoxy, trifluoromethoxy, difluoromethoxy, trideuteriomethoxy, dideuteriomethoxy, trifluoroethoxy, trideuterioethoxy, cyclopropoxy, cyclobutoxy, methylthio and ethylthio;
R_{2c} is selected from the group consisting of hydrogen, deuterium, fluorine, chlorine, bromine, cyano, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl, methoxy, ethoxy, propoxy, isopropoxy, trifluoromethoxy, difluoromethoxy, trideuteriomethoxy, dideuteriomethoxy, trifluoroethoxy, trideuterioethoxy, cyclopropoxy, cyclobutoxy, methylthio and ethylthio;
R_{2d} is selected from the group consisting of hydrogen, deuterium, fluorine, chlorine, bromine, cyano, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl, methoxy, ethoxy, propoxy, isopropoxy, trifluoromethoxy, difluoromethoxy, trideuteriomethoxy, dideuteriomethoxy, trifluoroethoxy, trideuterioethoxy, cyclopropoxy, cyclobutoxy, methylthio and ethylthio;
R₂ₑ is selected from the group consisting of hydrogen, deuterium, fluorine, chlorine, bromine, cyano, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl, methoxy, ethoxy, propoxy, isopropoxy, trifluoromethoxy, difluoromethoxy, trideuteriomethoxy, dideuteriomethoxy, trifluoroethoxy, trideuterioethoxy, cyclopropoxy, cyclobutoxy, methylthio and ethylthio;
R₃ is selected from the group consisting of hydrogen, deuterium, fluorine, chlorine, bromine, cyano, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl, methoxy, ethoxy, propoxy, isopropoxy, trifluoromethoxy, difluoromethoxy, trideuteriomethoxy, dideuteriomethoxy, trifluoroethoxy, trideuterioethoxy, cyclopropoxy, cyclobutoxy, methylthio and ethylthio.

10. The compound of formula (I), the stereoisomer or pharmaceutically acceptable salt thereof according to claim 5, **characterized in that** R₄ is selected from the group consisting of hydrogen, deuterium, fluorine, chlorine, bromine, cyano, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl, hydroxy, methoxy, ethoxy, propoxy, isopropoxy, trifluoromethoxy, difluoromethoxy, monofluoromethoxy, trideuteriomethoxy, dideuteriomethoxy, trifluoroethoxy, trideuterioethoxy, cyclopropoxy, cyclobutoxy, methylthio, ethylthio, amino, monomethylamino, monoethylamino and dimethylamino.

11. The compound of formula (I), the stereoisomer or pharmaceutically acceptable salt thereof according to claim 5, **characterized in that** R₇ and R₈ are each independently selected from the group consisting of hydrogen, deuterium, fluorine, chlorine, bromine, cyano, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl and azacyclobutyl;
R₉ₐ is selected from the group consisting of hydrogen, deuterium, fluorine, chlorine, bromine, cyano, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl and azacyclobutyl.

12. The compound of formula (I), the stereoisomer or pharmaceutically acceptable salt thereof according to claim 1, **characterized in that** the compound of formula (I) is a compoundof formula (IV-a) as shown below: 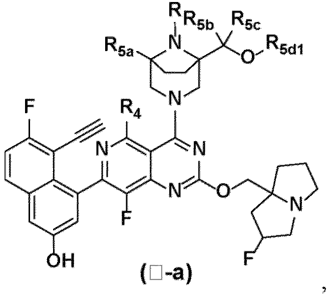
wherein, R is selected from the group consisting of hydrogen, C₁₋₄ alkyl, C₂₋₄ alkenyl, C₃₋₆ cycloalkyl, 3-6 membered heterocyclyl, -S(O)ᵣR₁₃, -O-R₁₄, -C(O)OR₁₄, -C(O)SR₁₄, -C(O)R₁₅, -O-C(O)R₁₅, -NR₁₆R₁₇, -C(O)NR₁₆R₁₇ and -N(R₁₆)-C(O)R₁₅, above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(O)NR₁₆R₁₇ and -N(R₁₆)-C(O)R₁₅;
R₄ is selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, -O-R₁₄, -S-R₁₃ and -NR₁₆R₁₇;
R₅ₐ is selected from the group consisting of hydrogen, deuterium, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl and -C₀₋₄ alkyl-O-R₁₄;
R_{5b} and R_{5c} are each independently selected from the group consisting of hydrogen, deuterium, halogen and C₁₋₄ alkyl, above groups are independently optionally further substituted with one or more substituents selected from the group consisting of deuterium, halogen, C₁₋₄ alkyl, C₃₋₆ cycloalkyl, 3-6 membered heterocyclyl and -O-R₁₄;
R_{5d1} is selected from the group consisting of hydrogen, deuterium, C₁₋₄ alkyl, C₃₋₆ cycloalkyl or 3-6 membered heterocyclyl, above groups are independently optionally further substituted with one or more substituents selected from the group consisting of deuterium, halogen, C₁₋₄ alkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)ᵣR₁₃, -O-R₁₄, -C(O)OR₁₄, -C(O)R₁₅, -O-C(O)R₁₅, -P(O)(R₁₅)₂, -NR₁₆R₁₇, -C(O)NR₁₆R₁₇ and -N(R₁₆)-C(O)R₁₅, above groups are independently optionally further substituted with one or more substituents selected from the group consisting of deuterium and halogen;
or, R_{5b} and R_{5c}, together with the carbon atom directly attached thereto, form a C₃₋₆ cycloalkyl or 3-6 membered heterocyclyl, above groups are independently optionally further substituted with one or more substituents selected from the group consisting of deuterium, halogen, C₁₋₄ alkyl, C₂₋₄ alkenyl, C₂₋₄ alkynyl, C₃₋₆ cycloalkyl, 3-6 membered heterocyclyl and -O-R₁₄, above groups are independently optionally further substituted with one or more substituents selected from the group consisting of deuterium and halogen, and Rsai is selected from the group consisting of hydrogen, deuterium, C₁₋₄ alkyl, C₃₋₆ cycloalkyl and 3-6 membered heterocyclyl, above groups are independently optionally further substituted with one or more substituents selected from the group consisting of deuterium and halogen;
wherein, R₁₃, R₁₄, R₁₅, R₁₆, R₁₇ and r are defined as in claim 1.

13. The compound of formula (I), the stereoisomer or pharmaceutically acceptable salt thereof according to claim 12, **characterized in that** R is selected from the group consisting of hydrogen, methyl, ethyl, propyl, isopropyl, cyclopropyl, cyclobutyl, oxacyclobutyl, azacyclobutyl and -C(O)OR₁₄, above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -SF₅, -S(O)(=N-R₁₀)R₁₁, -N=S(O)R₁₁R₁₂, -N=SR₁₁R₁₂, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(=NR₁₆)R₁₅, -N(R₁₆)-C(=NR₁₇)R₁₅, -C(O)NR₁₆R₁₇ and -N(R₁₆)-C(O)R₁₅;
wherein, R₁₀, R₁₁, R₁₂, R₁₃, R₁₄, R₁₅, R₁₆, R₁₇ and r are defined as in claim 12;
preferably, R is selected from the group consisting of hydrogen, methyl, ethyl, propyl, isopropyl, cyclopropyl, cyclobutyl, oxacyclobutyl, azacyclobutyl and -C(O)OR₁₄, above groups are independently optionally further substituted with one or more substituents selected from the group consisting of deuterium, fluorine, chlorine, cyano, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl and -O-C(O)R₁₅;
wherein, R₁₄ and R₁₅ are defined as in claim 12.

14. The compound of formula (I), the stereoisomer or pharmaceutically acceptable salt thereof according to claim 12, **characterized in that** R₄ is selected from the group consisting of hydrogen, deuterium, fluorine, chlorine, bromine, cyano, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl, hydroxy, methoxy, ethoxy, propoxy, isopropoxy, trifluoromethoxy, difluoromethoxy, monofluoromethoxy, trideuteriomethoxy, dideuteriomethoxy, trifluoroethoxy, trideuterioethoxy, cyclopropoxy, cyclobutoxy, methylthio, ethylthio, amino, monomethylamino, monoethylamino and dimethylamino;
preferably, R₄ is selected from the group consisting of hydrogen, fluorine, chlorine, cyano, methyl, ethyl, propyl, isopropyl, trifluoromethyl, difluoromethyl, monofluoromethyl, trideuteriomethyl, dideuteriomethyl, monodeuteriomethyl, hydroxy, methoxy, ethoxy, propoxy, isopropoxy, trifluoromethoxy, difluoromethoxy, monofluoromethoxy, trideuteriomethoxy, dideuteriomethoxy, trifluoroethoxy, trideuterioethoxy, methylthio, ethylthio, amino, monomethylamino, monoethylamino and dimethylamino.

15. The compound of formula (I), the stereoisomer or pharmaceutically acceptable salt thereof according to claim 12, **characterized in that** R₅ₐ is selected from the group consisting of hydrogen, deuterium, fluorine, chlorine, bromine, cyano, hydroxy, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl, methoxy, ethoxy, propoxy, isopropoxy, trifluoromethoxy, difluoromethoxy, trideuteriomethoxy, dideuteriomethoxy, trifluoroethoxy, trideuterioethoxy, cyclopropoxy and cyclobutoxy;
preferably, R₅ₐ is selected from the group consisting of hydrogen, deuterium, cyano, hydroxy, methyl, ethyl, propyl, isopropyl, trifluoromethyl, difluoromethyl, monofluoromethyl, trideuteriomethyl, dideuteriomethyl, monodeuteriomethyl, methoxy, ethoxy, propoxy, isopropoxy, trifluoromethoxy, difluoromethoxy, trideuteriomethoxy, dideuteriomethoxy, trifluoroethoxy and trideuterioethoxy.

16. The compound of formula (I), the stereoisomer or pharmaceutically acceptable salt thereof according to claim 12, **characterized in that** R_{5b} and R_{5c} are each independently selected from the group consisting of hydrogen, deuterium, fluorine, chlorine, bromine, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl, methoxy, ethoxy, propoxy, isopropoxy, trifluoromethoxy, difluoromethoxy, monofluoromethoxy, trideuteriomethoxy, dideuteriomethoxy, trifluoroethoxy, trideuterioethoxy, cyclopropoxy and cyclobutoxy;
Rsai is selected from the group consisting of hydrogen, deuterium, methyl, ethyl, propyl, isopropyl, cyclopropyl, cyclobutyl, oxacyclobutyl and azacyclobutyl, above groups are independently optionally further substituted with one or more substituents selected from the group consisting of deuterium, fluorine, chlorine, bromine, methyl, ethyl, propyl, isopropyl, cyclopropyl, cyclobutyl, oxacyclobutyl, azacyclobutyl, =O, =S, -S(O)ᵣR₁₃ and -O-R₁₄, above groups are independently optionally further substituted with one or more substituents selected from the group consisting of deuterium and halogen;
or, R_{5b} and R_{5c}, together with the carbon atom directly attached thereto, form a C₃₋₆ cycloalkyl or 3-6 membered heterocyclyl, above groups are independently optionally further substituted with one or more substituents selected from the group consisting of deuterium, fluorine, chlorine, bromine, C₁₋₄ alkyl, C₂₋₄ alkenyl, C₂₋₄ alkynyl, C₃₋₆ cycloalkyl, =O, =S, methylthio, ethylthio, methoxy, ethoxy and isopropoxy, above groups are independently optionally further substituted with one or more substituents selected from the group consisting of deuterium and halogen, and R_{5d1} is selected from the group consisting of hydrogen, deuterium, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl and azacyclobutyl;
wherein, R₁₃, R₁₄ and r are defined as in claim 12;
preferably, R_{5b} and R_{5c} are each independently selected from the group consisting of hydrogen, deuterium, fluorine, chlorine, bromine, methyl, ethyl, propyl, isopropyl, trifluoromethyl, difluoromethyl, monofluoromethyl, trideuteriomethyl, dideuteriomethyl, monodeuteriomethyl, methoxy, ethoxy, propoxy, isopropoxy, trifluoromethoxy, difluoromethoxy, monofluoromethoxy, trideuteriomethoxy, dideuteriomethoxy, trifluoroethoxy and trideuterioethoxy;
R_{5d1} is methyl, ethyl, isopropyl or trideuteriomethyl.

17. The compound of formula (I), the stereoisomer or pharmaceutically acceptable salt thereof according to claim 1, **characterized in that** the compound of formula (I) is a compound of formula (IV-b) as shown below: 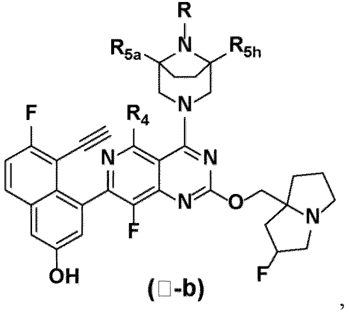
wherein, R is selected from the group consisting of hydrogen, C₁₋₄ alkyl, C₂₋₄ alkenyl, C₃₋₆ cycloalkyl, 3-6 membered heterocyclyl, -S(O)ᵣR₁₃, -O-R₁₄, -C(O)OR₁₄, -C(O)SR₁₄, -C(O)R₁₅, -O-C(O)R₁₅, -NR₁₆R₁₇, -C(O)NR₁₆R₁₇ and -N(R₁₆)-C(O)R₁₅, above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, C₆₋₈ aryl, 5-8 membered heteroaryl, =O, =S, -O-S(O)₂R₁₃, -S(O)ᵣR₁₃, -O-R₁₄, -C(O)OR₁₄, -C(O)SR₁₄, -S-C(O)R₁₅, -C(O)R₁₅, -O-C(O)R₁₅, -P(O)(R₁₅)₂, -NR₁₆R₁₇, -C(O)NR₁₆R₁₇ and -N(R₁₆)-C(O)R₁₅;
R₄ is selected from the group consisting of hydrogen, deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl, 3-6 membered heterocyclyl, -O-R₁₄, -S-R₁₃ and -NR₁₆R₁₇;
R₅ₐ is selected from the group consisting of hydrogen, deuterium, C₁₋₄ alkyl, C₂₋₄ alkenyl, C₂₋₄ alkynyl, C₁₋₄ haloalkyl, C₁₋₄ deuterioalkyl, C₃₋₆ cycloalkyl, 3-6 membered heterocyclyl and -C₀₋₄ alkyl-O-R₁₄;
R₅ₕ is selected from the following groups:
1) 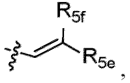 wherein R₅ₑ and R_{5f} are each independently selected from the group consisting of hydrogen, deuterium, halogen and C₁₋₄ alkyl, above groups are independently optionally further substituted with one or more substituents selected from the group consisting of deuterium, halogen, cyano, C₁₋₄ alkyl, C₃₋₆ cycloalkyl, 3-6 membered heterocyclyl, C₆₋₈ aryl and 5-8 membered heteroaryl, above groups are independently optionally further substituted with one or more substituents selected from the group consisting of deuterium and halogen;
2) 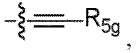 wherein R_{5g} is selected from the group consisting of hydrogen, deuterium, halogen and C₁₋₄ alkyl, above groups are independently optionally further substituted with one or more substituents selected from the group consisting of deuterium, halogen, cyano, C₁₋₄ alkyl, C₃₋₆ cycloalkyl, 3-6 membered heterocyclyl, C₆₋₈ aryl and 5-8 membered heteroaryl, above groups are independently optionally further substituted with one or more substituents selected from the group consisting of deuterium and halogen;
3) C₁₋₄ alkyl, C₃₋₆ cycloalkyl or 3-6 membered heterocyclyl, above C₁₋₄ alkyl is independently optionally further substituted with one or more substituents selected from the group consisting of deuterium, halogen, C₁₋₄ alkyl, C₂₋₄ alkenyl, C₂₋₄ alkynyl, C₃₋₆ cycloalkyl, 3-6 membered heterocyclyl, =O, =S, -SF₅, -S(O)ᵣR₁₃ and -O-R₁₄, above groups are independently optionally further substituted with one or more substituents selected from the group consisting of deuterium and halogen, provided that R₅ₕ is not methyl substituted with -O-R₁₄, C₃₋₆ cycloalkyl or 3-6 membered heterocyclyl;
wherein, R₁₃, R₁₄, R₁₅, R₁₆, R₁₇ and r are defined as in claim 1.

18. The compound of formula (I), the stereoisomer or pharmaceutically acceptable salt thereof according to claim 17, **characterized in that** the compound of formula (I) is a compound of formula (IV-b1) as shown below: 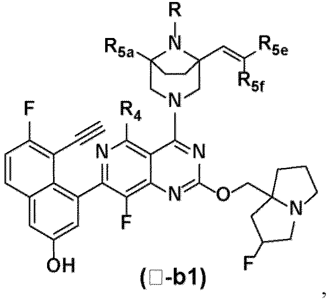
wherein, R is selected from the group consisting of hydrogen, methyl, ethyl, propyl, isopropyl, cyclopropyl, cyclobutyl, oxacyclobutyl, azacyclobutyl and -C(O)OR₁₄, above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl and 3-6 membered heterocyclyl;
R₄ is selected from the group consisting of hydrogen, deuterium, fluorine, chlorine, bromine, cyano, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl, hydroxy, methoxy, ethoxy, propoxy, isopropoxy, trifluoromethoxy, difluoromethoxy, monofluoromethoxy, trideuteriomethoxy, dideuteriomethoxy, trifluoroethoxy, tri deuteri oethoxy, cyclopropoxy, cyclobutoxy, methylthio, ethylthio, amino, monomethylamino, monoethylamino and dimethylamino;
R₅ₐ is selected from the group consisting of hydrogen, deuterium, fluorine, chlorine, bromine, cyano, hydroxy, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl, methoxy, ethoxy, propoxy, isopropoxy, trifluoromethoxy, difluoromethoxy, trideuteriomethoxy, dideuteriomethoxy, trifluoroethoxy, trideuterioethoxy, cyclopropoxy and cyclobutoxy;
R₅, and R_{5f} are each independently selected from the group consisting of hydrogen, fluorine, chlorine, methyl, ethyl and isopropyl, above groups are independently optionally further substituted with one or more substituents selected from the group consisting of deuterium, fluorine, chlorine, cyano, methyl, ethyl, propyl, isopropyl, cyclopropyl, cyclobutyl, oxacyclobutyl and azacyclobutyl, above groups are more independently optionally further substituted with one or more substituents selected from the group consisting of deuterium, fluorine and chlorine;
wherein, R₁₄ is defined as in claim 17;
preferably, R is hydrogen; R₄ is hydrogen; R₅ₐ is hydrogen; R₅ₑ and R_{5f} are each independently selected from the group consisting of hydrogen, fluorine and methyl.

19. The compound of formula (I), the stereoisomer or pharmaceutically acceptable salt thereof according to claim 17, **characterized in that** the compound of formula (I) is a compound of formula (IV-b2) as shown below: 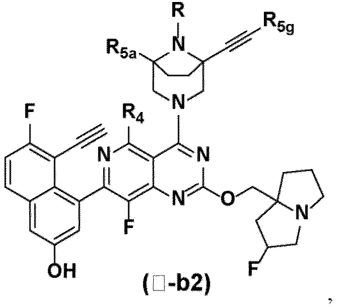
wherein, R is selected from the group consisting of hydrogen, methyl, ethyl, propyl, isopropyl, cyclopropyl, cyclobutyl, oxacyclobutyl, azacyclobutyl and -C(O)OR₁₄, above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl and 3-6 membered heterocyclyl;
R₄ is selected from the group consisting of hydrogen, deuterium, fluorine, chlorine, bromine, cyano, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl, hydroxy, methoxy, ethoxy, propoxy, isopropoxy, trifluoromethoxy, difluoromethoxy, monofluoromethoxy, trideuteriomethoxy, dideuteriomethoxy, trifluoroethoxy, tri deuteri oethoxy, cyclopropoxy, cyclobutoxy, methylthio, ethylthio, amino, monomethylamino, monoethylamino and dimethylamino;
R₅ₐ is selected from the group consisting of hydrogen, deuterium, fluorine, chlorine, bromine, cyano, hydroxy, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl, methoxy, ethoxy, propoxy, isopropoxy, trifluoromethoxy, difluoromethoxy, trideuteriomethoxy, dideuteriomethoxy, trifluoroethoxy, trideuterioethoxy, cyclopropoxy and cyclobutoxy;
R_{5g} is selected from the group consisting of hydrogen, fluorine, chlorine, methyl, ethyl and isopropyl, above groups are independently optionally further substituted with one or more substituents selected from the group consisting of deuterium, fluorine, chlorine, cyano, methyl, ethyl, propyl, isopropyl, cyclopropyl, cyclobutyl, oxacyclobutyl and azacyclobutyl, above groups are independently optionally further substituted with one or more substituents selected from the group consisting of deuterium, fluorine and chlorine;
wherein, R₁₄ is defined as in claim 17;
preferably, R is hydrogen; R₄ is hydrogen; R₅ₐ is hydrogen; R_{5g} is selected from the group consisting of hydrogen, fluorine and methyl.

20. The compound of formula (I), the stereoisomer or pharmaceutically acceptable salt thereof according to claim 17, **characterized in that** the compound of formula (I) is a compound of formula (IV-b3) as shown below: 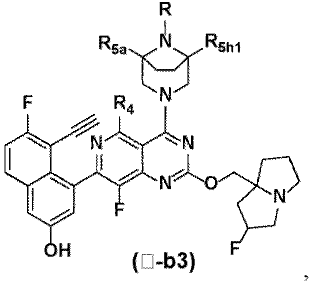
wherein, R is selected from the group consisting of hydrogen, methyl, ethyl, propyl, isopropyl, cyclopropyl, cyclobutyl, oxacyclobutyl, azacyclobutyl and -C(O)OR₁₄, above groups are independently optionally further substituted with one or more substituents selected from the group consisting of deuterium, halogen, cyano, C₁₋₄ alkyl, C₁₋₄ haloalkyl, C₁₋₄ deuterioalkyl, C₂₋₄ alkenyl, C₂₋₄ alkynyl, C₃₋₆ cycloalkyl and 3-6 membered heterocyclyl;
R₄ is selected from the group consisting of hydrogen, deuterium, fluorine, chlorine, bromine, cyano, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl, hydroxy, methoxy, ethoxy, propoxy, isopropoxy, trifluoromethoxy, difluoromethoxy, monofluoromethoxy, trideuteriomethoxy, dideuteriomethoxy, trifluoroethoxy, tri deuteri oethoxy, cyclopropoxy, cyclobutoxy, methylthio, ethylthio, amino, monomethylamino, monoethylamino and dimethylamino;
R₅ₐ is selected from the group consisting of hydrogen, deuterium, fluorine, chlorine, bromine, cyano, hydroxy, methyl, ethyl, propyl, isopropyl, trifluoromethyl, difluoromethyl, monofluoromethyl, trideuteriomethyl, dideuteriomethyl, monodeuteriomethyl, cyclopropyl, cyclobutyl, oxacyclobutyl, azacyclobutyl, methoxy, ethoxy, propoxy, isopropoxy, trifluoromethoxy, difluoromethoxy, trideuteriomethoxy, dideuteriomethoxy, trifluoroethoxy, trideuterioethoxy, cyclopropoxy and cyclobutoxy;
R₅ₕ₁ is methyl, ethyl, propyl, isopropyl, cyclopropyl, cyclobutyl, oxacyclobutyl and azacyclobutyl, above groups are independently optionally further substituted with one or more substituents selected from the group consisting of deuterium, halogen, C₁₋₄ alkyl, C₂₋₄ alkenyl, C₂₋₄ alkynyl, C₃₋₆ cycloalkyl, 3-6 membered heterocyclyl, =O, =S, -SF₅, -S(O)ᵣR₁₃ and -O-R₁₄, above groups are independently optionally further substituted with one or more substituents selected from the group consisting of deuterium and halogen, provided that R₅ₕ₁ is not methyl substituted with -O-R₁₄, cyclopropyl, cyclobutyl, oxacyclobutyl and azacyclobutyl;
wherein, R₁₃, R₁₄ and r are defined as in claim 17;
preferably, R is hydrogen; R₄ is hydrogen, methyl, ethyl, methoxy or methylthio; R₅ₐ is hydrogen or deuterium;
R₅ₕ₁ is methyl, ethyl, propyl or isopropyl, above groups are independently optionally further substituted with one or more substituents selected from the group consisting of deuterium, fluorine, chlorine, C₃₋₆ cycloalkyl, 3-6 membered heterocyclyl, =O, =S, -SF₅, -S(O)ᵣR₁₃ and -O-R₁₄, above groups are independently optionally further substituted with one or more substituents selected from the group consisting of deuterium and halogen; provided that R₅ₕ₁ is not methylsubstituted with -O-R₁₄;
wherein, R₁₃, R₁₄ and r are defined as in claim 17.

21. The compound of formula (I), the stereoisomer or pharmaceutically acceptable salt thereof according to any one of claims 1-20, **characterized in that** it is selected from the group consisting of:

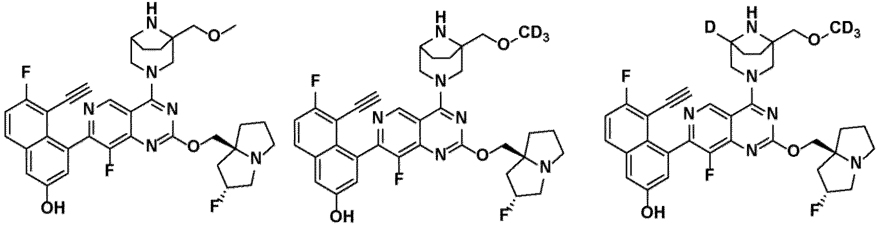

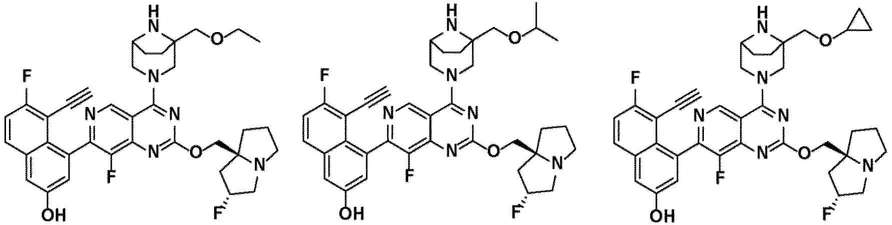

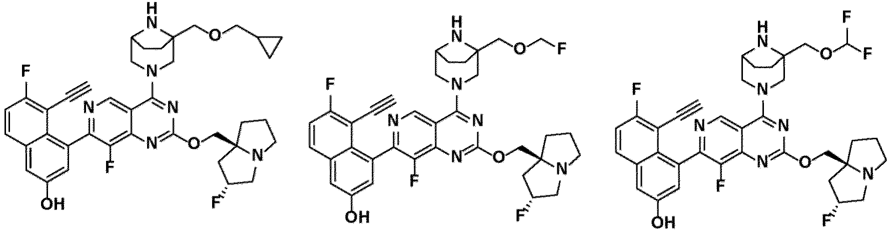

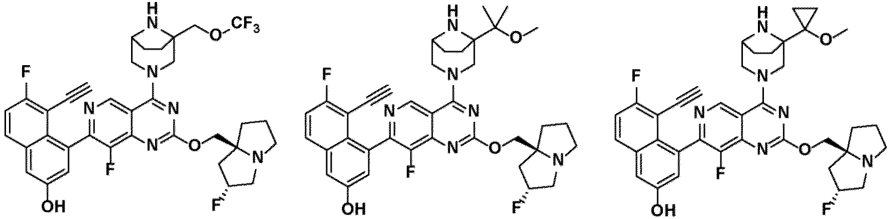

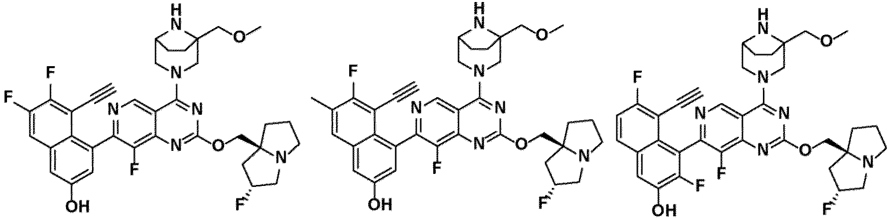

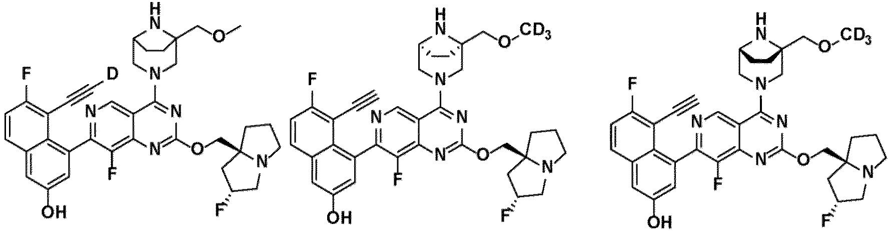

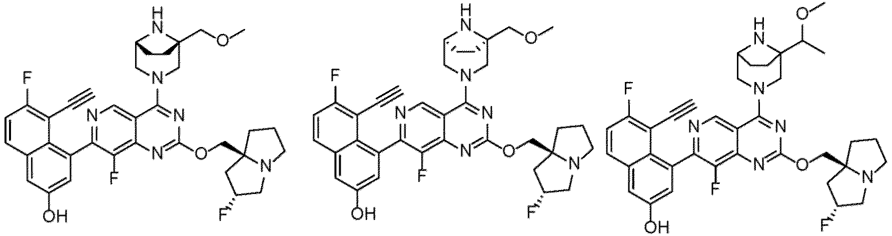

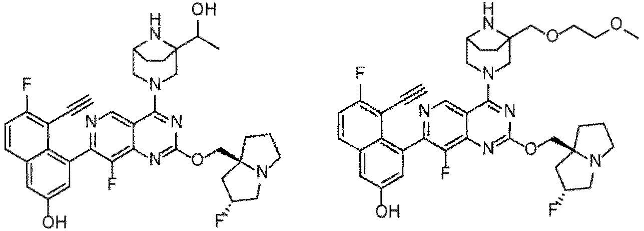

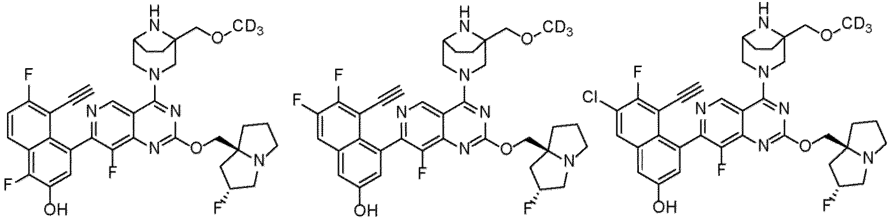

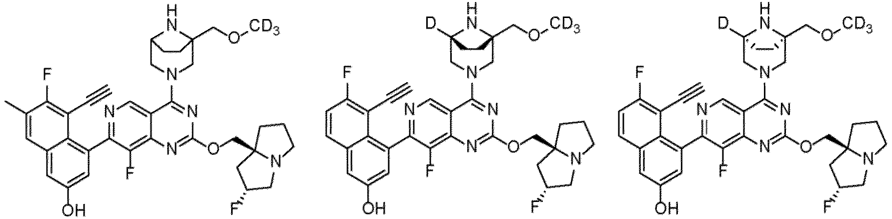

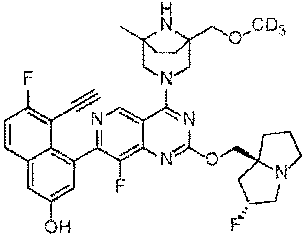

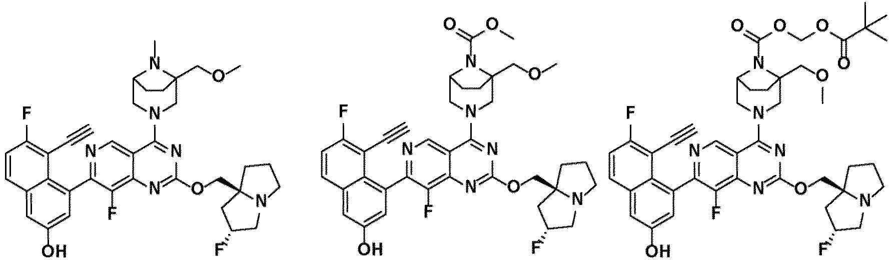

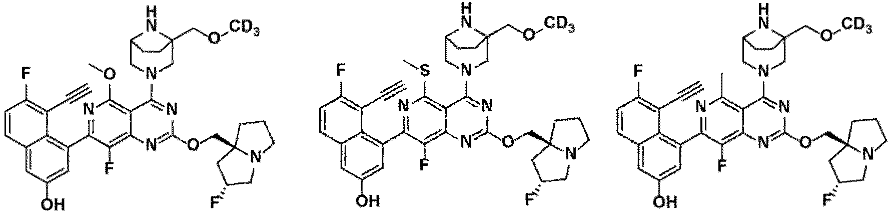

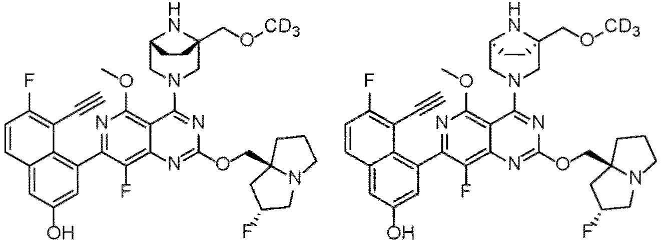

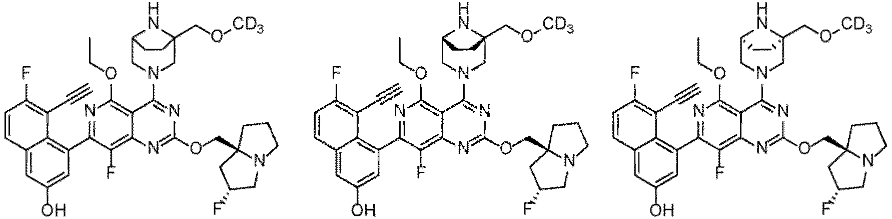

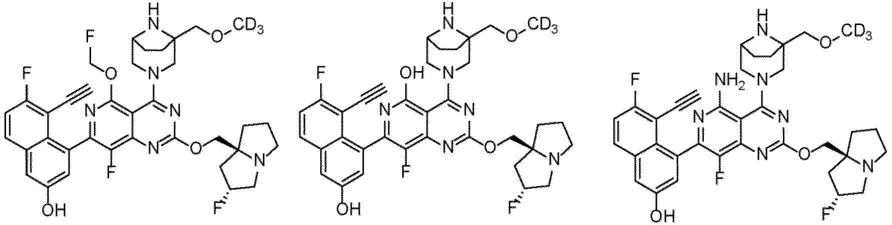

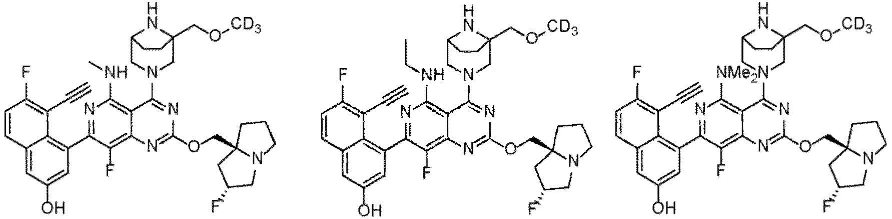

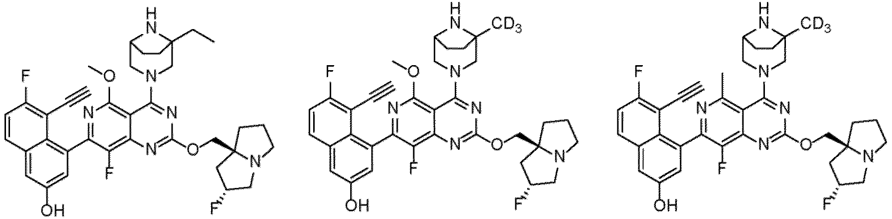

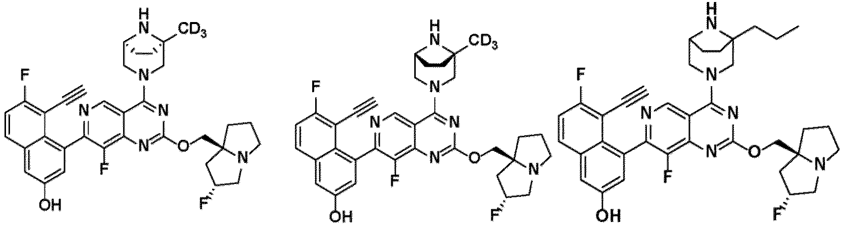

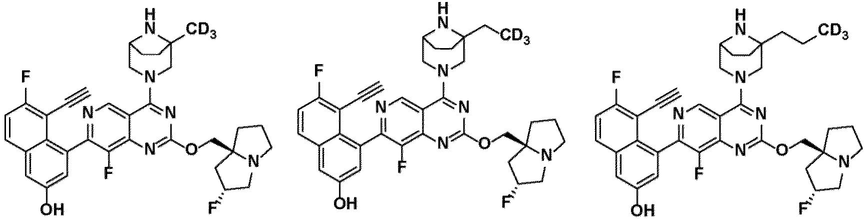

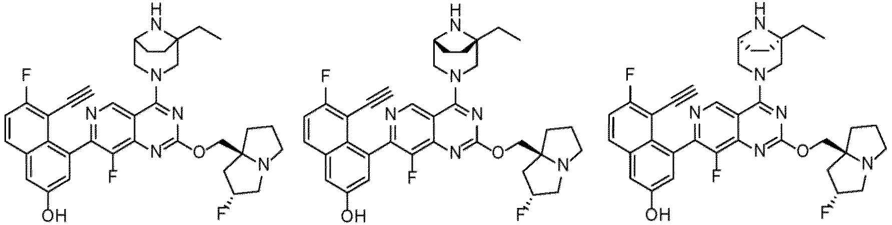

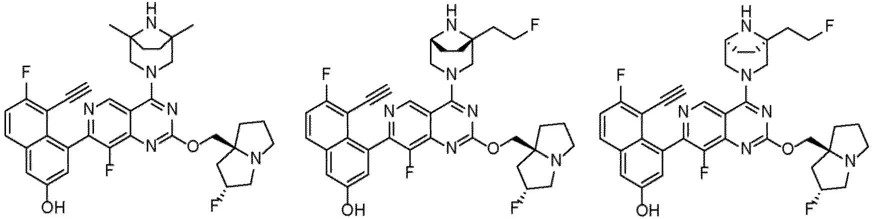

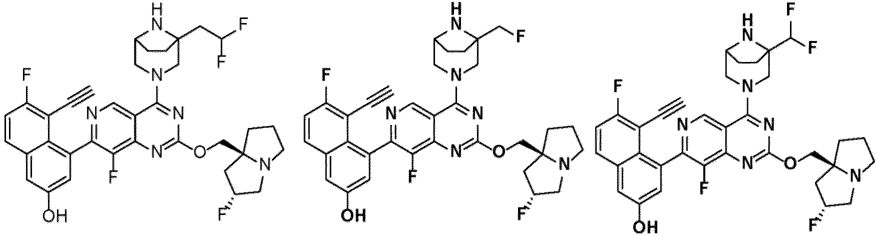

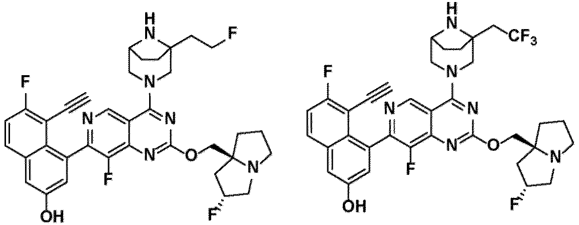

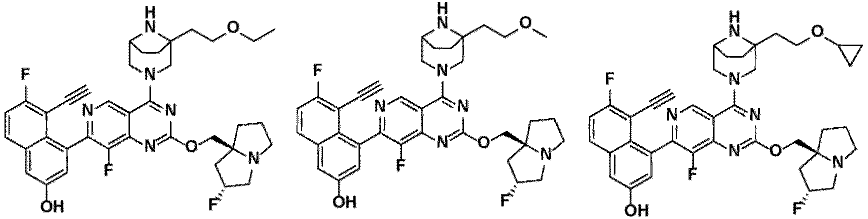

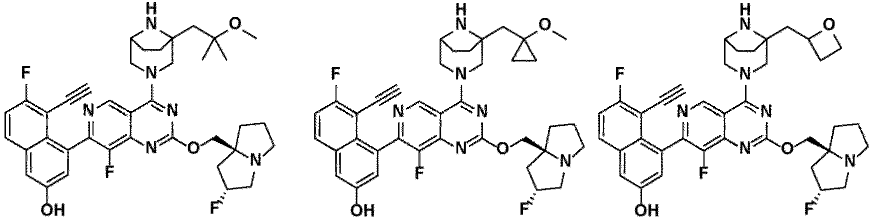

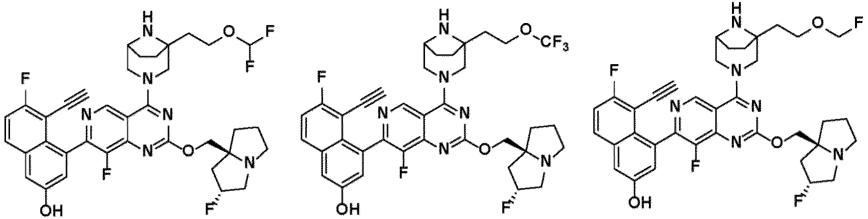

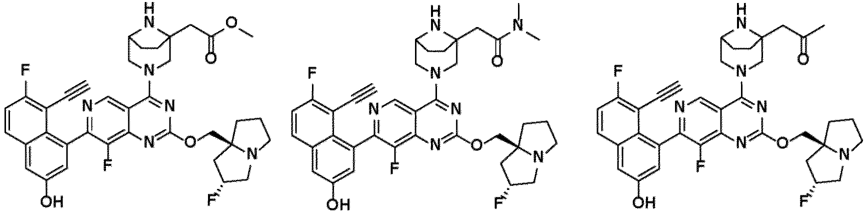

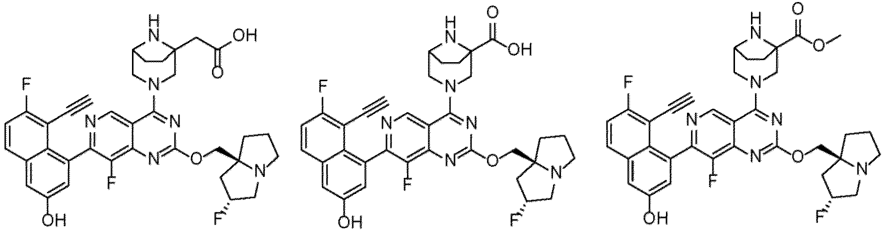

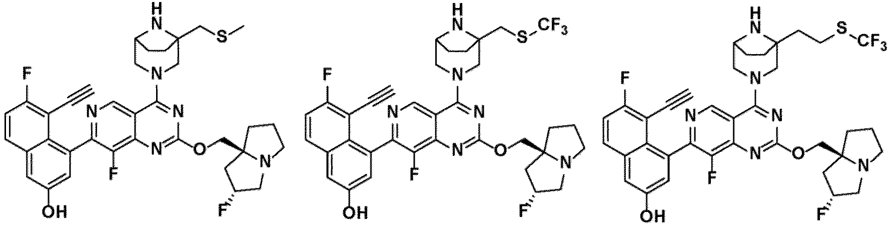

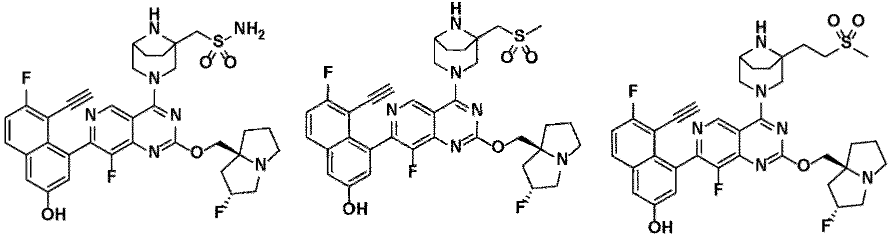

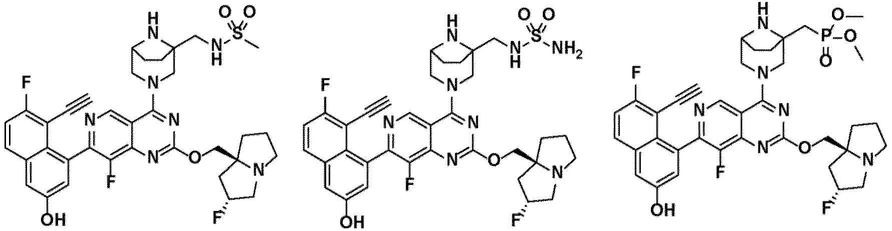

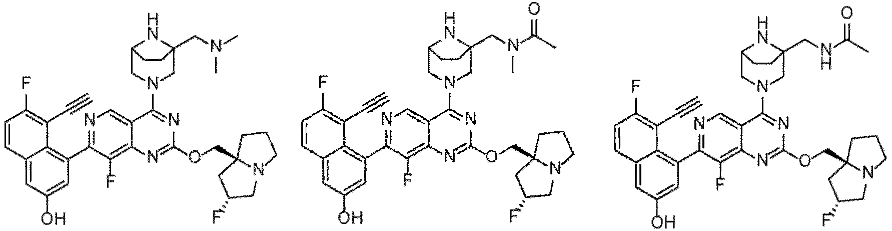

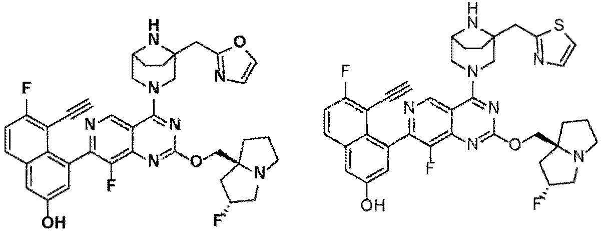

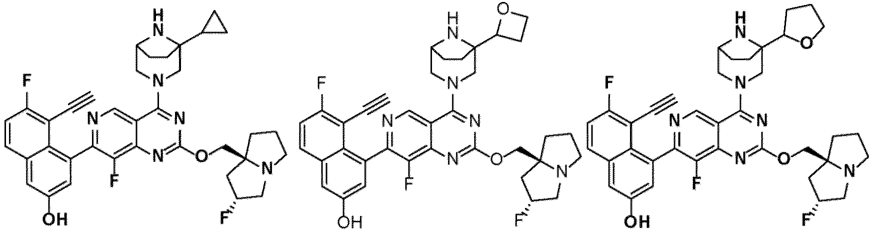

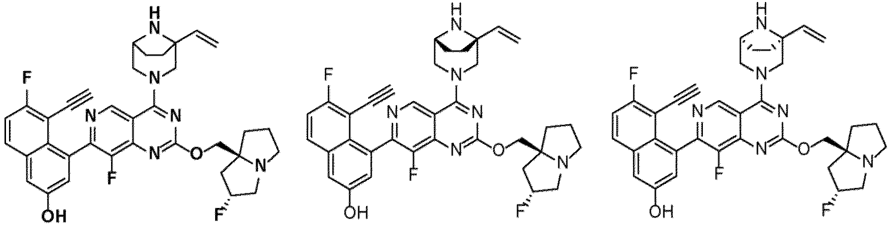

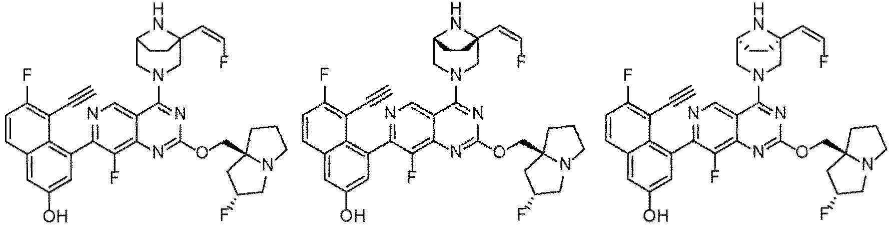

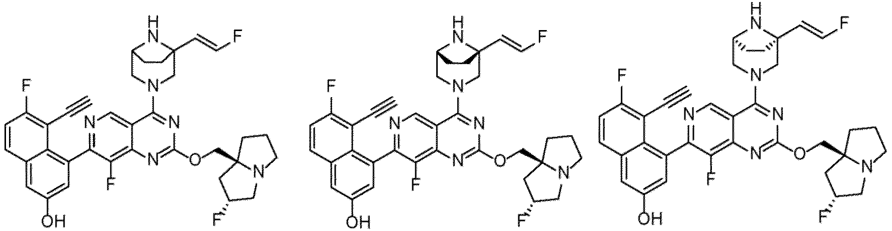

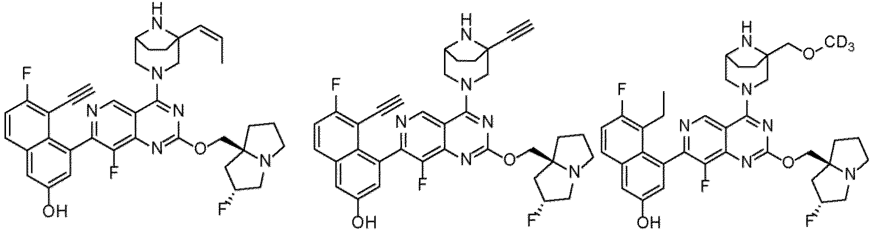

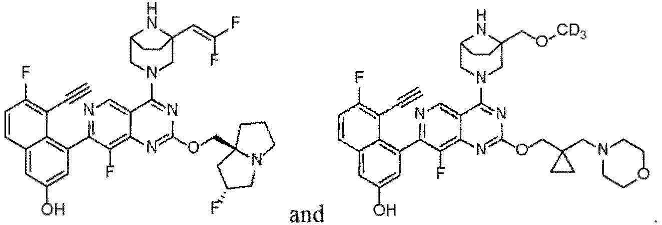

22. A pharmaceutical composition comprising the compound of formula (I), the stereoisomer or pharmaceutically acceptable salt thereof of any one of claims 1-21, and a pharmaceutically acceptable carrier.

23. Use of the compound of formula (I), the stereoisomer or pharmaceutically acceptable salt thereof of any one of claims 1-21 in the preparation of medicaments for the treatment and/or prevention of cancers or tumors related to KRas G12D.

24. Use of the compound of formula (I), the stereoisomer or pharmaceutically acceptable salt thereof of any one of claims 1-21 in the preparation of medicaments for the treatment and/or prevention of KRas G12D-related sarcoma (angiosarcoma, fibrosarcoma, rhabdomyosarcoma, liposarcoma), myxoma, rhabdomyoma, fibroma, lipoma, teratoma; bronchial carcinoma (squamous cell carcinoma, undifferentiated small cell carcinoma, undifferentiated large cell carcinoma, adenocarcinoma), alveolar (bronchiolar carcinoma) carcinoma, bronchial adenoma, lymphoma, chondromatoid hamartoma, mesothelioma; esophageal carcinoma (squamous cell carcinoma, adenocarcinoma, leiomyosarcoma, lymphoma), gastric carcinoma (lymphoma, leiomyosarcoma), pancreatic carcinoma (ductal adenocarcinoma, insulinoma, glucagonoma, gastrinoma, carcinoid tumor, vasoactive intestinal peptide tumor), small intestine cancer (adenocarcinoma, lymphoma, carcinoid tumor, Kaposi's sarcoma, leiomyoma, hemangioma, lipoma, neurofibroma, fibroma), colorectal carcinoma (adenocarcinoma, tubular adenoma, villous adenoma, hamartoma, leiomyoma); kidney cancer (adenocarcinoma, Wilms' tumor (nephroblastoma), lymphoma, leukemia), bladder cancer and urethral cancer (squamous cell carcinoma, transitional cell carcinoma, adenocarcinoma), prostate cancer (adenocarcinoma, sarcoma), testicular cancer (seminoma, teratoma, embryonal carcinoma, terato-epithelial cancer, choriocarcinoma, sarcoma, mesenchymal cell carcinoma, fibroma, fibroadenoma, adenomatoid tumor, lipoma); liver cancer (hepatocellular carcinoma), cholangiocarcinoma, hepatoblastoma, angiosarcoma, hepatocellular adenoma, hemangioma; gallbladder cancer, ampullary carcinoma, cholangiocarcinoma; osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochondroma (osteochondral exostosis), benign chondroma, chondroblastoma, chondromyxoid fibroma, osteoid osteoma and giant cell tumor; skull cancer (osteoma, hemangioma, granuloma, xanthoma, osteitis deformans), meningeal carcinomatosis (meningioma, meningeal sarcoma, gliomatosis), brain cancer (astrocytoma, medulloblastoma, glioma, ependymoma, blastoma (pineal tumor), glioblastoma multiforme, oligodendroglioma, neurilemmoma, retinoblastoma, congenital tumor, spinal neurofibroma, meningioma, glioma, sarcoma); uterine cancer (endometrial cancer (serous cystadenocarcinoma, mucinous cystadenocarcinoma, unclassified cancer), granulosa-theca cell tumor, Sertoli-Leydig cell tumor, dysgerminoma, malignant teratoma), vulvar cancer (squamous cell carcinoma, intraepithelial carcinoma, adenocarcinoma, fibrosarcoma, melanoma), vaginal cancer (clear cell carcinoma, squamous cell carcinoma, botryoid sarcoma (embryonal rhabdomyosarcoma)), fallopian tube (carcinoma); hematologic cancer (myelogenous leukemia (acute and chronic), acute lymphoblastic leukemia, chronic lymphocytic leukemia, myeloproliferative disease, multiple myeloma, myelodysplastic syndrome), Hodgkin's disease, non-Hodgkin's lymphoma (malignant lymphoma); malignant melanoma, basal cell carcinoma, squamous cell carcinoma, Kaposi's sarcoma, nevus, dysplastic nevus, lipoma, hemangioma, dermatofibroma, keloid, psoriasis, adrenal tumor and neuroblastoma.

25. The compound of formula (I), the stereoisomer or pharmaceutically acceptable salt thereof of any one of claims 1-21, for use in the treatment and/or prevention of cancers or tumors related to KRas G12D.
